# EUROPÄISCHE PATENTSCHRIFT

(11) **EP 1 742 925 B1**
(45) Veröffentlichungstag und Bekanntmachung des Hinweises auf die Patenterteilung: **23.04.2014**
(21) Anmeldenummer: 05735696.6
(22) Anmeldetag: 27.04.2005
(51) Int. Cl.: C07D 239/84, A61K 31/517, A61P 43/00, C07D 471/04

(54) **SUBSTITUIERTE 5, 6, 7, 8-TETRAHYDRO-PYRIDO[4, 3-d]PYRIMIDIN-2-YL- UND 5, 6, 7, 8-TETRAHYDRO-CHINAZOLIN-2-YL-VERBINDUNGEN**
SUBSTITUTED 5, 6, 7, 8-TETRAHYDRO-PYRIDO[4, 3-D]PYRIMIDINE-2-YL COMPOUNDS AND 5, 6, 7, 8-TETRAHYDRO-QUINAZOLINE-2-YL COMPOUNDS
COMPOSES DE 5, 6, 7, 8-TETRAHYDRO-PYRIDO[4, 3-D]PYRIMIDINE-2-YL ET DE 5, 6, 7, 8-TETRAHYDRO-QUINAZOLINE-2-YL SUBSTITUEES

(30) Priorität: 28.04.2004 DE 102004020908
(43) Veröffentlichungstag der Anmeldung: 17.01.2007
(73) Patentinhaber: Grünenthal GmbH, 52078 Aachen (DE)
(72) Erfinder: OBERBÖRSCH, Stefan, 52078 Aachen (DE); SUNDERMANN, Bernd, 61381 Friedrichsdorf (DE); SUNDERMANN, Corinna, 61381 Friedrichsdorf (DE); HAURAND, Michael, 52078 Aachen (DE); HENNIES, Hagen-Heinrich, 52152 Simmerath (DE); BIJSTERVELD, Edward, NL-6546 BB Nijmegen (NL)
(74) Vertreter: Brosch, Oliver
(86) Internationale Anmeldenummer: PCT/EP2005/004489
(87) Internationale Veröffentlichungsnummer: WO 2005/105759

(56) Entgegenhaltungen:
- WO-A-01/44246
- WO-A-99/21857
- WO-A-02/096422
- WO-A-2004/016596
- WO-A-2004/022542

## Beschreibung

Die vorliegende Erfindung betrifft substituierte 5,6,7,8-Tetrahydro-pyrido[4,3-d]pyrimidin-2-yl- und 5,6,7,8-Tetrahydro-chinazolin-2-yl-Verbindungen, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des weiteren sind sie bei neuropathischen Schmerzen, unter denen insbesondere Tumorpatienten leiden, häufig nicht ausreichend wirksam.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Prophylaxe und/oder Behandlung von Schmerzen, insbesondere akuten Schmerzen, chronischen Schmerzen, neuropathischen oder viszeralen Schmerzen.

Es wurde nun überraschenderweise gefunden, daß sich die substituierten 5,6,7,8-Tetrahydro-pyrido[4,3-d]pyrimidin-2-yl- und 5,6,7,8-Tetrahydro-chinazolin-2-yl Verbindungen der nachstehend angegebenen allgemeinen Formel I zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation eignen und daher insbesondere als pharmazeutische Wirkstoffe . In Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituferte 5,6,7,8-Tetrahydro-pyrido[4,3-d]pyrimidin-2-yl- und 5,6,7,8-Tetrahydro-chinazolin-2-yl Verbindungen der allgemeinen Formel L 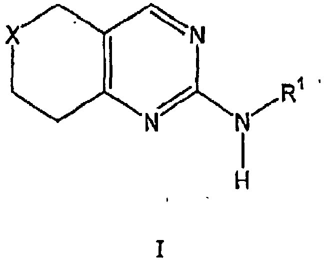 worin
- X: für eine C(H)(NHR²)-Gruppe oder für eine NR^{2a}-Gruppe steht,
- R¹: für eine -C(=O)-R³-Gruppe oder für eine -C(=O)-O-R⁴-Gruppe steht,
- R², R^{2a},: unabhängig voneinander, jeweils für eine -C(=O)R⁵-Gruppe oder für eine -SC=O)₂-R⁶-Gruppe stehen,
- R³: für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₈-Rest, für einen unsubstituierten oder wenigstens einfach substituierten, gesättigten oder ungesättigten, ggf. wenigstens ein Heteroatom als Ringglied aufweisenden, 3-bis 8-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋3-Alkylen-Gruppe gebunden sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₃-Alkylen-Gruppe gebunden sein kann, steht,
- R⁴: für einen unsubstituierten oder wenigstens einfach substituierten 5- bis 14-gliedrigen Aryl- oder Heteroaryl-Rest steht, der über eine lineare oder verzweigte, unsubstituierte oder wenigstens einfach substituierte C₁₋₃-Alkylen-Gruppe gebunden sein kann,
- R⁵: für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₁₀ Rest,
für einen unsubstituierten oder wenigstens einfach substituierten, gesättigten oder ungesättigten, ggf. wenigstens ein Heteroatom als Ringglied aufweisenden 3- bis 8-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₆Alkylen-Gruppe gebunden und/oder mit einem unsubstituierten oder wenigstens einfach substituierten mono- oder polyzyklischen Ringssystem kondensiert sein kann,
für einen unsubstituierten oder wenigstens einfach substituierten, 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₆Alkylen-, C₂₋₆-Alkenylen- oder C₂₋₆-Alkinylen-Gruppe gebunden und/oder mit einem unsubstituierten oder wenigstens einfach substituierten, mono- oder polyzylklischen Ringsystem kondensiert sein kann,
für einen -C(=O)-R⁷-Rest, der über eine lineare oder verzweigte, unsubstituierte oder wenigstens einfach substituierte C₁₋₃-Alkylen-Gruppe gebunden sein kann,
für einen -C(=O)-O-R⁸-Rest, der über eine lineare oder verzweigte, unsubstituierte oder wenigstens einfach substituierte C₁₋₃Alkylen-Gruppe gebunden sein kann, oder
für einen -N(H)-C(=O)-O-R⁹-Rest, der über eine lineare oder verzweigte, unsubstituierte oder wenigstens einfach substituierte, ggf. wenigstens eine -N(H)-C(=O)- oder wenigstens eine -C(=O)-N(H)-Gruppierung als Kettenglied aufweisende C₁₋₈-Alkylen-Gruppe gebunden sein kann, steht,
- R⁶: für eine Gruppe -NR¹⁰R¹¹,
für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₁₀ Rest,
für einen unsubstituierten oder wenigstens einfach substituierten, gesättigten oder ungesättigten, ggf. wenigstens ein Heteroatom als Ringglied aufweisenden, ggf. mit einem unsubstituierten oder wenigstens einfach substituierten, mono- oder polyzyklischen Ringsystem kondensierten drei- bis acht-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₆-Alkylen-Gruppe gebunden und/oder mit einer linearen oder verzweigten C₁₋₆ Alkylen-Gruppe überbrückt sein kann, oder
für einen unsubstituierten oder wenigstens einfach substituierten 5- bis 14-gliedrigen Aryl- oder Heteroaryl-Rest steht, der über eine lineare oder verzweigte, unsubstituierte oder wenigstens einfach substituierte C₁₋₈Alkylen-Gruppe gebunden und/oder mit einem unsubstituierten oder wenigstens einfach substituierten mono- oder polyzyklischen Ringsystem kondensiert sein kann, steht,
- R⁷: für einen linearen oder verzweigten, unsubstituierten oder wenigstens einfach substituierten C₁₋₃-Alkyl-Rest für einen unsubstituierten oder wenigtens einfach substituierten 5- bis 14- gliedrigen Aryl- oder Heteroaryl-Rest, für einen unsubstituierten oder wenigstens einfach substituierten, gesättigten oder ungesättigten, ggf. wenigstens ein Heteroatom als Ringglied aufweisenden 5-, 6- oder 7-gliedrigen cycloaliphatischen Rest, der mit einem unsubstituierten oder wenigstens einfach substituierten monozyklischen Ringsystem kondensiert sein kann, oder für einen -NR^{7a}R^{7b}-Rest steht, worin die Reste R^{7a} und R^{7b}, gleich oder verschieden, jeweils für einen linearen oder verzweigten C₁₋₅Alkyl-Rest stehen.
- R⁸: für einen linearen oder verzweigten, unsubstituierten oder wenigstens einfach substituierten C₁₋₃-Alkyl-Rest oder für einen unsubstituierten oder wenigstens einfach substituierten, ggf. über eine lineare oder verzweigte C₁₋₃-Alkylen-Gruppe gebundenen 5- oder 6-gliedrigen Aryl- oder Heteroaryl-Rest steht,
- R⁹: für einen linearen oder verzweigten, unsubstituierten oder wenigstens einfach substituierten C₁₋₃-Alkyl-Rest, für einen unsubstituierten oder wenigtens einfach substituierten, ggf. über eine lineare oder verzweigte C₁₋₃-Alkylen-Gruppe gebundenen, 5- oder 6-gliedrigen Aryl- oder Hetetoaryl-Rest, oder für einen unsubstituierten oder wenigstens einfach substituierten, gesättigten oder ungesättigten, 5-oder 6-gliedrigen cycloaliphatischen Rest, der mit wenigstens einem unsubstituierten oder wenigstens einfach substituierten monozyklischen Ringsystem kondensiert sein kann, steht,
- R¹⁰ und R¹¹,: gleich oder verschieden, jeweils für einen linearen oder verzweigten C₁₋₅Alkyl-Rest stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastemomeren, in einem beliebigen Mischungsverhältnis, oder jeweils in Form entsprechender Salze, oder jeweils in Form entsprechender Solvate.

Sofern einer oder mehrere der Substituenten R³ und R⁵ -R⁹ für einen gesättigten oder ungesättigten aliphatischen Rest, d.h. für einen Alkyl-, Alkenyl- oder Alkinyl-Rest, stehen, der 1-, 2-, 3-, 4-, 5-, 6-, 7-, 8- oder 9-fach substituiert ist, sind dessen Substituenten, jeweils unabhängig voneinander, ausgewählt aus der Gruppe bestehend aus F, Cl, Br, -OH, -SH und -NH₂. Alkenyl-Reste weisen wenigstens eine C-C-Doppelbindung und Alkinyl-Reste wenigstens eine C-C-Dreifachbindung auf.

Als geeignete Alkyl-, Alkenyl- und Alkinyl-Reste, die einfach oder mehrfach substituiert sein können, seien beispielsweise Methyl, Ethyl, n-Propyl, iso-Propyl, n-Butyl, iso-Butyl, sec-Butyl, tert-Butyl, n-Pentyl, iso-Pentyl, neo-Pentyl, n-Hexyl, 2-Hexyl, n-Heptyl, n-Octyl, n-Nonyl, n-Decyl, -C(H)(C₂H₅)₂, -C(H)(n-C₃H₇)₂, -CH₂-CH₂-C(H)(CH₃)-(CH₂)₃-CH₃, Vinyl, Ethinyl, Propenyl, Allyl, Propinyl, Butenyl, Butenyl, Pentenyl, Pentinyl, Hexenyl, Hexinyl, -CH=CH-CH=CH-CH₃ und -CH₂-CH₂-CH=CH₂ genannt.

Sofern einer oder mehrere der Substituenten R³, R⁵ und R⁶ für einen gesättigten oder ungesättigten aliphatischen Rest, d.h. für einen Alkyl-, Alkenyl- oder Alkinyl-Rest, stehen, der 1, 2, 3, 4 oder 5, Heteroatome als Kettenglied(er) aufweist, sind diese Heteroatome, jeweils unabhängig voneinander, ausgewählt aus der Gruppe bestehend aus Sauerstoff, Schwefei und Stickstoff (NH). Vorzugsweise befinden sich diese Heteroatome in einer nicht-endständigen Postion des jeweiligen Restes. Beispielhaft seien Reste wie -CH₂-CH₂-S-CH₃, -CH₂-O-CH₂-CH₂-O-CH₃, -CH₂-O-CH₃ oder-CH₂-CH₂-O-CH₃ genannt.

Sofern einer oder mehrere der Substituenten R³, R⁵-R⁷ und R⁹ für einen cycloaliphatischen Rest steht oder einen cycloaliphatischen Rest aufweist, der 1-, 2-, 3-, 4- oder 5-fach, substituiert ist, sind dessen Substituenten, jeweils unabhängig voneinander, ausgewählt aus der Gruppe bestehend aus -C₁₋₅-Alkyl, -C(=O)-O-C₁₋₅-Alkyl, - C(-O)-CF₃, -S(=O)₂-C₁₋₅Alkyl, -S(=O)₂-Phenyl, oxo (=O) und Phenyl, wobei die C₁₋₅-Alkyl-Reste jeweils linear oder verzweigt und die Phenyl-Reste jeweils 1-, 2-, 3-, 4- oder 5-fach, gleich oder verschieden, mit einem Substituenten ausgewählt aus der Gruppe bestehend aus F, Cl, Br und I substituiert sein können. Besonders bevorzugt können die Substituenten, jeweils unabhängig voneinander, ausgewählt werden aus der Gruppe bestehend aus-C(=O)-O-tert.-Butyl, -C(=O)-CF₃, -S(=O)₂-Methyl, -S(=O)₂-Phenyl, oxo, und Phenyl, wobei der phenyl-Rest jeweils 1-, 2-, 3-, 4-oder 6-fach, gleich oder verschieden, mit einem Substituenten ausgewählt aus der Gruppe bestehend aus F, Cl und Br substituiert sein kann.

Sofern die cycloaliphatischen Reste 1, 2 oder 3, Heteroatome als Ringglieder aufweisen, sind diese, jeweils unabhängig voneinander, ausgewählt aus der Gruppe bestehend aus Stickstoff, Sauerstoff und Schwefei.

Als geeignete cycloaliphatische Reste, die einfach oder mehrfach substituiert sein können, seien beispielsweise Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl und Dithiolanyl genannt.

Sofern einer oder mehrere der Substituenten R³-R⁹ für einen Aryl- oder Heteroaryl-Rest stehen oder einen Aryl- oder Heteroaryl-Rest aufweisen, der 1-, 2-, 3-, 4- oder 5-fach, substituiert ist, sind dessen Substituenten, jeweils unabhängig voneinander, ausgewählt aus der Gruppe bestehend aus Halogen, -CN, -NO₂, -OH, -SH, C₁₋₅Alkyl, C₁₋₅-Alkoxy, -S-C₁₋₅-Alkyl, -CF₃, -CHF₂, -CH₂F, -O-CF_{3,} -O-CHF₂, -O-CH₂F, -C(=O)-CF₃, -S-CF₃, -S-CHF₂, -S-CH₂F, -S(=O)₂-Phenl, -S(=O)₂-C₁₋₅-Alkyl, N(C₁₋₅Alkyl)(C₁₋₅-Alkyl), -C(=O)-O-C₁₋₅Alkyl, -CH₂-O-C(=O)-Phenyl, -O-C(=O)-Phenyl, -NH-C(=O)-C₁₋₅-Alkyl, -C≡C-Phenyl, -C≡C-Naphthyl, -C=C-Pyrrolyl, -C≡C-Indolyl, -C≡C-Furyl (-C≡C-Furanyl), - C≡C-Benzo[b]furanyl, -C≡C-Thienyl (-C≡C-Thiophenyl), -C≡C-Benzo[b]thienyl, -C≡C-Pyrazolyl, -C≡C-Imidazolyl, -C≡C-Thiazolyl, -C≡C-Thiadiazolyl, -C≡C-Triazolyl, -C≡C-Oxazolyl, -C≡C-Isoxazoly, -C≡C-Pyridinyl, -C≡C-Pyridazinyl, -C≡C-Pyrimidinyl, -C≡C-Pyrazinyl, -C≡C-Pyranyl, -C≡C-Indazolyl, -C≡C-Purinyl, -C≡C-Indolizinyl, -C≡C-Chinolinyl, -C≡C-Isochinolinyl, -C≡C-Chinazolinyl, Pyrazolyl, Phenyl, Furyl (Furanyl), Thiadiazolyl, Thiophenyl (Thienyl), Phenoxy und Benzyl, wobei die zyklischen Substituenten selbst jeweils 1-, 2-, 3-, 4- oder 5-fach, gleich oder verschieden, mit einem Substituenten ausgewählt aus der Gruppe bestehend aus F. Cl, Br, substituiert sein können.

Besonders Bevorzugt können die Substituenten, jeweils unabhängig voneinander, ausgewählt werden aus der Gruppe bestehend aus F, Cl, Br, I, -CN, -NO₂, -OH, -SH, Methyl, Ethyl, n-Propyl, iso-Propyl, n-Butyl, iso-Butyl, sec-Butyl, tert.-Butyl, n-Pentyl, neo-Pentyl, Methoxy, Ethoxy, n-Propoxy, iso-Propoxy, n-Butyloxy, iso-Butyloxy, sec-Butyloxy, tert.-Butyloxy, -S-Methyl, -S-Ethyl, -CF₃, -CHF₂, -CH₂F, -O-CF₃, -O-CHF₂, - O-CH₂F, -C(=O)-CF₃, -S-CF₃, -S-CHF₂, -S-CH₂F, -S(=O)₂-Phenyl, Pyrazolyl, Phenyl, -N(CH₃)₂, -N(C₂H₅)₂, -CH₂-O-C(=O)-Phenyl, -C(=O)-O-CH₃, -C(=O)-O-C₂H₅, -NH-C(=O)-CH₃, -O-C(=O)-Phenyl, -C≡C-Phenyl, -C≡C-Furyl (-C≡C-Furanyl), -C≡C-Thiadiazolyl, -C≡C-Thiophenyl (-C≡C-Thienyl), Phenyl, Furyl (Furanyl), Thiadiazolyl, Thiophenyl (Thienyl), Phenoxy und Benzyl, wobei die zyklischen Substituenten selbst jeweils 1-, 2-, 3-, 4- oder 5-fach, gleich oder verschieden, mit einem Substituenten ausgewählt aus der Gruppe bestehend aus F, Cl, Br, I substituiert sein können.

Als geeignete Aryl-Reste seien beispielsweise Phenyl-, 1-Naphthyl und 2-Naphthyl genannt.

Sofern einer oder mehrere der Substituenten R³-R⁹ für einen Heteroaryl-Rest stehen oder einen Heteroaryl-Rest aufweisen, sind dessen Heteroatom(e), jeweils unabhängig voneinander, ausgewählt aus der Gruppe bestehend aus Sauerstoff, Schwefel und Stickstoff. Erfindungsgemäß weisen die Heteroaryl-Reste 1, 2, 3, 4 oder 5 Heteroatome auf.

Als geeignete Heteroaryl-Reste seien beispielsweise Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl genannt.

Unter einem mono- bzw. polyzyklischen Ringsystem werden im Sinne der vorliegenden Erfindung mono- bzw. polyzyklische Kohlenwasserstoffreste verstanden, die gesättigt, ungesättigt oder aromatisch sein und ggf. 1, 2, 3, 4 oder 5, Heteroatome als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert (anneliert) sein.

Sofern ein polyzyklisches Ringsystem vorlegt, können die verschiedenen Ringe, jeweils unabhängig voneinander, einen unterschiedlichen Sättigungsgrad aufweisen, d.h. gesättigt, ungesättigt oder aromatisch sein. Die Heteroatome jedes Ringes können, jeweils gleich oder verschieden, ausgewählt werden aus der Gruppe bestehend aus Sauerstoff, Stickstoff und Schwefel. Vorzugsweise sind die jeweiligen Ringe des mono- oder polyzyklischen Ringsystems 5- oder 6-gliedrig. Vorzugsweise sind unter polyzyklischen Ringsystemen bizyklische Ringsysteme zu verstehen.

Sofern einer oder mehrere der Substituenten R⁵, R⁶, R⁷ und R⁹ ein monozyklisches oder polyzyklisches Ringsystem aufweisen, das 1-, 2-, 3-, 4- oder 5-flach, substituiert ist, sind dessen Substituenten, jeweils unabhängig voneinander, ausgewählt aus der Gruppe bestehend aus Halogen, -CN, -NO₂, -OH, -SH, C₁₋₅-Alkyl, C₁₋₅-Alkoxy, -S-C₁₋₅-Alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, N(C₁₋₅Alkyl₁₋₅-Alkyl), -C(=O)-O-C₁₋₅-Alkyl, CH₂-O-C(=O)-Phenyl, -O-C(=O)-Phenyl, -NH-C(=O)-C₁₋₅-Alkyl, -C≡C-Phenyl, -C≡C-Naphthyl, -C≡C-Pyrrolyl, -C≡C-Indolyl, -C≡C-Furyl (-C≡C-Furanyl), - C≡C-Benzo[b]furanyl, -C≡C-Thienyl (-C≡C-Thiophenyl), -C≡C-Benzo[b]thienyl, -C≡C-Pyrazolyl, -C≡C-Imidazolyl, -C≡C-Thiazolyl, -C≡C-Thiadiazolyl, -C≡C-Triazolyl, -C≡C-Oxazolyl, -C≡C-Isoxazolyl, -C≡C-Pyridinyl, -C≡C-Pyridazinyl, -C≡C-Pyrimidinyl, -C≡C-Pyrazinyl, -C≡C-Pyranyl, -C≡C-Indazolyl, -C≡C-Purinyl, -C≡C-Indolizinyl, -C≡C-Chinolinyl, -C≡C-Isochinolinyl, -C≡C-Chinazolinyl Pyrazolyl, Phenyl, Furyl (Furanyl), Thiadiazolyl, Thiophenyl (Thienyl), Phenoxy und Benzyl, wobei die zyktischen Substituenten selbst jeweils 1-, 2-, 3-, 4- oder 5-fach, gleich oder verschieden, mit einem Substituenten ausgewählt aus der Gruppe bestehend aus F, Cl, Br, I substrtuiert sein können. Besonders bevorzugt können die Substituenten, jeweils unabhängig voneinander, ausgewählt werden aus der Gruppe bestehend aus F, Cl, Br, I, -CN, -NO₂, -OH, -SH, Methyl, Ethyl, n-Propyl, iso-Propyl, n-Butyl, iso-Butyl, sec-Butyl, tert.-Butyl, n-Pentyl, neo-Pentyl, Methoxy, Ethoxy, n-Propoxy, iso-Propoxy, n-Butyloxy, iso-Butyloxy, sec-Butyloxy, tert.-Butyloxy, -S-Methyl, -S-Ethyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, Phenyl, -N(CH₃)₂, -N(C₂H₅)₂,-CH₂-O-C(=O)-Phenyl, -C(=O)-O-CH₃, -C(=O)-O-C₂H₅, -NH-C(=O)-CH₃, -O-C(=O)-Phenyl, Furyl (Furanyl), Thiadiazolyl, Thiophenyl (Thienyl), Phenoxy und Benzyl, wobei die zyklischen Substituenten selbst jeweils 1-, 2-, 3-, 4- oder 5-fach, gleich oder verschieden, mit einem Substituenten ausgewählt aus der Gruppe bestehend aus F, Cl, Br, I substituiert sein können.

Sofern einer der vorstehend genannten Substituenten R³-R⁶ eine lineare oder verzweigte Alkylen-, Alkenylen- oder Alkinylen-Gruppe aufweist, die 1-, 2-, 3-, 4- oder 5-fach substituiert ist, sind deren Substituenten, jeweils unabhängig voneinander, ausgewählt aus der Gruppe bestehend aus F, Cl, Br, Hydroxy und unsubstituiertem Phenyl.

Sofern die Alkylen-, Alkenylen- oder Alkinylen-Gruppe, 1, 2, 3, 4 oder 5, Heteroatome als Kettenglied(er) aufweist, sind diese ausgewählt aus der Gruppe bestehend aus Sauerstoff, Schwefel und Stickstoff (NH).

Beispielhaft seien Alkylen-, Alkenylen oder Alkinylen-Gruppen wie -CH₂)-, -(CH₂)₂-,-(CH₂)₃-, -(CH₂)₄-, -(CH₂)₅-, -C(H)(CH₂-Phenyl)-, -C(H)(Phenyl), -C(H)(C(H)(CH₃)₂)-C(C₂H₅)(H)-, -(CH₂)-O-, -(CH₂)₂-O-, -(CH₂)₃-O-, -(CH₂)₄-O-, -O-(CH₂)-, -O-(CH₂)₂-O-(CH₂)₃-, -O-(CH₂)₄-, -C(C₂H₅)(H)-O-, -O-C(C₂H₅)(H)-, -CH₂-O-H₂-, -CH₂-S-CH₂-, - C(CH₃)₂-, -C(H)(CH₃)-, -CH=CH- und -C≡C- genannt.

Bevorzugt sind ferner substituierte 5,6,7,8-Tetrahydro-pyrido[4,3-d]pyrimidin-2-yl-und 5,6,7,8-Tetrahydro-chinazolin-2-yl- Verbindungen der vorstehend angegebenen allgemeinen Formel I, in denen R³ für einen linearen oder verzweigten, unsubstituierten oder wenigstens einfach substituierten, ggf. eines oder mehrere Sauerstoffatome und/oder eine oder mehrere NH-Gruppen als Kettenglied(er) aufweisenden C₁₋₈-Alkyl-Rest, für einen unsubstituierten oder wenigstens einfach substituierten, gesättigten oder ungesättigten, ggf. wenigstens ein Heteroatom als Ringglied aufweisenden, 5-, 6- oder 7-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₃-Alkylen-Gruppe gebunden sein kann, oder für einen unsubstituierten oder wenigstens einfach substituierten Phenyl-Rest, Thiophenyl-Rest (Thienyl-Rest), Furanyl-Rest (Furyl-Rest), Pyridinyl-Rest oder Naphthyl-Rest, der über eine lineare oder verzweigte, unsubstituierte oder wenigstens einfach substituierte C₁₋₃-Alkylen-Gruppe gebunden sein kann, steht,
vorzugsweise für einen Methyl-, Ethyl-, n-Propyl-, iso-Propyl-, n-Butyl-, iso-Butyl-, sec-Butyl- oder tert.-Butyl-Rest, für einen unsubstituierten, gesättigten oder ungesättigten, ggf. eines oder mehrere Sauerstoffatome und/oder eines oder mehrere Stickstoffatome als Ringglied(er) aufweisenden, 5-, 6- oder 7-gliedrigen cycloaliphatischen Rest, der über eine -(CH₂)-, -(CH₂)₂- oder-(CH₂)₃-Gruppe gebunden sein kann, oder für einen Phenyl-Rest, Thiophenyl-Rest (Thienyl-Rest), Furanyl-Rest (Furyl-Rest), Pyridinyl-Rest oder Naphthyl-Rest, der über eine -(CH₂)-, -(CH₂)₂- oder -(CH₂)₃-Gruppe gebunden und/oder einfach oder mehrfach, gleich oder verschieden, mit einem Substituenten ausgewählt aus der Gruppe bestehend aus Methyl, Ethyl, n-Propyl, iso-Propyl, n-Butyl, iso-Butyl, sec-Butyl, tert.-Butyl, Methoxy, Ethoxy, n-Propoxy, iso-Propoxy, n-Butoxy, iso-Butoxy, sec-Butoxy, tert.-Butoxy, F, Cl, Br, I, -CN und -CF₃ substituiert sein kann, steht,
und jeweils die übrigen Reste X, R¹, R², R^{2a} und R⁴ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I, worin der Rest R⁴ für einen gegebenenfalls über eine -(CH₂)-, -(CH₂)₂- oder -(CH₂)₃-Brücke gebundenen Phenyl-Rest steht, wobei der Phenyl-Ring unsubstituiert oder wenigstens einfach, gleich oder verschieden, mit einem Substituenten ausgewählt aus der Gruppe bestehend Methyl, Ethyl, n-Propyl, iso-Propyl, n-Butyl, iso-Butyl, sec-Butyl, tert.-Butyl, Methoxy, Ethoxy, n-Propoxy, iso-Propoxy, n-Butoxy, iso-Butoxy, sec-Butoxy, tert.-Butoxy, F, Cl, Br, I, -CN und -CF₃ substituiert sein kann,
vorzugsweise für einen unsubstituierten Benzyl-Rest steht,
und jeweils die übrigen Reste X, R¹, R², R^{2a}, R³ und R⁵ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I, in denen R⁵ für einen linearen oder verzweigten, ggf. eines oder mehrere Sauerstoffatome und/oder ggf. eines oder mehrere Schwefelatome und/oder ggf. eine oder mehrere -N(H)-Gruppen als Kettenglied(er) aufweisenden C₁₋₁₀ Alkyl-Rest, für einen linearen oder verzweigten, ggf. eines oder mehrere Sauerstoffatome und/oder ggf. eines oder mehrere Schwefelatome und/oder ggf. eine oder mehrere -N(H)-Gruppen als Kettenglied(er) aufweisenden C₂₋₁₀ AlkenylRest, für einen linearen oder verzweigten, ggf. eines oder mehrere Sauerstoffatome und/oder ggf. eines oder mehrere Schwefelatome und/oder ggf. eine oder mehrere-N(H)-Gruppen als Kettenglied(er) aufweisenden C₂₋₁₀ Alkinyl-Rest,
für einen unsubstituierten oder wenigstens einfach substituierten, gesättigten oder ungesättigen, ggf. eines oder mehrere Heteroatome, unabhängig voneinander, ausgewählt aus der Gruppe bestehend aus Sauerstoff, Schwefel und Stickstoff als Ringglied(er) aufweisenden 3- bis 8-gliedrigen cycloaliphatischen Rest, der über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -C(CH₃)₂- oder -C(H)(CH₃)-Gruppe gebunden und/oder mit einem unsubstituierten oder wenigstens einfach substituierten, 5- oder 6-gliedrigen monozyklischen Ringssystem kondensiert sein kann,
für einen unsubstituierten oder wenigstens einfach substituierten, 5- oder 6-gliedrigen Aryl- oder Heteroaryl-Rest, der über eine -(CH₂)-, -(CH₂)₂-, -(CH₂)₃-, -(CH₂)₄-,-C(C₂H₅)(H)-, -(CH₂)-O-, -(CH₂)₂-O-, -(CH₂)₃-O-, -(CH₂)₄-O-, -C-(CH₂)-, -O-(CH₂)₂-,-O-(CH₂)₃-, -O-(CH₂)₄-, -C(C₂H₅)(H)-O-, -O-C(C₂H₅)(H)-, -CH₂-O-CH₂-, -CH₂-S-CH₂-,-C(CH₃)₂-, -C(H)(CH₃)- oder -CH=CH-Gruppe gebunden und/oder mit einem unsubstituierten oder wenigstens einfach substituierten 5- oder 6-gliedrigen, monozyklischen Ringsystem kondensiert sein kann,
für einen -C(=O)-R⁷-Rest, der über eine -(CH₂)-, -(CH₂)₂- oder -(CH₂)₃-Gruppe gebunden sein kann,
für einen -C(=O)-O-R⁸-Rest, der über eine lineare oder verzweigte, unsubstituierte oder einfach oder mehrfach mit einem Phenyl-Rest substituierte C₁₋₃-Alkylen-Gruppe gebunden sein kann, oder
für einen -N(H)-C(=O)-O-R⁹-Rest, der über eine lineare oder verzweigte, unsubstituierte oder einfach oder mehrfach mit einem Phenyl-Rest substituierte, ggf. wenigstens eine -N(H)-C(=O)- oder wenigstens eine -C(=O)-N(H)-Gruppierung als Kettenglied aufweisende C₁₋₈-Alkylen-Gruppe gebunden sein kann, steht,
bevorzugt
für einen Rest ausgewählt aus der Gruppe bestehend aus Methyl, Ethyl, n-Propyl, iso-Propyl, n-Butyl, sec-Butyl, tert-Butyl, n-Pentyl, neo-Pentyl, iso-Pentyl,-C(H)(C₂H₅)₂, -C(H)(n-C₃H₇)₂-, -CH=CH-CH=CH-CH₃, -CH₂-CH₂-CH=CH₂, -CH₂-CH₂-C(H)(CH₃)-(CH₂)₃-CH₃, -CH₂-O-CH₃ und -CH₂-O-CH₂-CH₂-O-CH₃,
für einen Rest ausgewählt aus der Gruppe bestehend aus Cyclopropyl, Cyclobutyl, Cyclopentyl, Cyclohexyl, Cycloheptyl, Cyclooctyl, Piperidinyl, Piperazinyl, Morpholinyl, Tetrahydrofuranyl (Tetrahydrofuryl), Dithiolanyl, 1,2,3,4-Tetrahydroindolyl, 1,2,3,4-Tetrahydronaphthyl, 1,3-Dihydroisoindolyl, Benzooxazolyl und Imidazolidinyl, wobei der zyklische Rest jeweils über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-,-CH₂-O-CH₂-, -CH₂-S-CH₂-, -C(CH₃)₂-, oder -C(H)(CH₃)-Gruppe gebunden und/oder einfach oder mehrfach, gleich oder verschieden, mit einem Substituenten ausgewählt aus der Gruppe bestehend aus Phenyl, -C(=O)-O-tert.-Butyl, oxo (=O), -S(=O)₂-Methyl und -S(=O)₂-Phenyl substituiert sein kann, wobei die vorstehend genannten Phenyl-Reste jeweils einfach oder mehrfach, gleich oder verschieden, mit einem Substituenten ausgewählt aus der Gruppe bestehend aus F, Cl, Br und I substituiert sein können,
für einen Rest ausgewählt aus der Gruppe bestehend aus Phenyl, Naphthyl, Furanyl (Furyl), Thiophenyl (Thienyl), Pyridinyl, Isoxazolyl, Triazolyl, Pyrazolyl, Thiazolyl, Benzo[1,2,5]-oxadiazolyl, 2,3-Dihydrobenzofuranyl, Chinolinyl, Chromanyl, Chromenyl, Benzo[b]furanyl, Benzo[b]thiophenyl, Benzo[1,3]-dioxinyl, Indolyl, 2,3-Dihydro-indolyl, 3,4-Dihydro-benzo[1,4]oxazinyl und 1,2,3,4-Tetrahydroisochinolinyl steht, wobei der zyklische Rest jeweils über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-,-O-(CH₂)₃-, -O-(CH₂)₄-, -C(C₂H₅)(H)-O-, -O-C(C₂H₅)(H)-, -CH₂-O-CH₂-, -CH₂-S-CH₂-,-C(CH₃)₂-, -C(H)(CH₃)- oder -CH=CH-Gruppe gebunden und/oder einfach oder mehrfach, gleich oder verschieden, mit einem Substituenten ausgewählt aus der Gruppe bestehend aus F, Cl, Br, I, -CN, -NO₂, -OH, -SH, Methyl, Ethyl, n-Propyl, iso-Propyl, n-Butyl, iso-Butyl, sec-Butyl, tert.-Butyl, n-Pentyl, iso-Pentyl, neo-Pentyl, Methoxy, Ethoxy, n-Propoxy, iso-Propoxy, n-Butyloxy, iso-Butyloxy, sec-Butyloxy, tert.-Butyloxy, -S-Methyl, -S-Ethyl, -CF₃, -CHF₂, -CH₂F, -O-CF₃, -O-CHF₂, -O-CH₂F,-S-CF₃, -S-CHF₂, -S-CH₂F, -S(=O)₂-Phenyl, Pyrazolyl, Phenyl, -N(CH₃)₂, -N(C₂H₅)₂,-CH₂-O-C(=O)-Phenyl, -O-C(=O)-Phenyl, Furyl (Furanyl), Thiadiazolyl, Thiophenyl (Thienyl), Phenoxy und Benzyl substituiert sein kann, und wobei die zyklischen Substituenten selbst jeweils einfach oder mehrfach, gleich oder verschieden, mit einem Substituenten ausgewählt aus der Gruppe bestehend aus F, Cl, Br, I substituiert sein können,
für einen -C(=O)-R⁷-Rest, der über eine -(CH₂)-, -(CH₂)₂- oder -(CH₂)₃-Gruppe gebunden sein kann, für einen -C(=O)-O-R⁸-Rest, der über eine -(CH₂)-, -(CH₂)₂-, -(CH₂)₃- oder -C(H)(Phenyl)-Gruppe gebunden sein kann, oder
für einen -N(H)-C(=O)-O-R⁹-Rest, der über eine -(CH₂)-, -(CH₂)₂-, -(CH₂)₃-, -(CH₂)₄-, -(CH₂)₅-, -C(H)(CH₂-Phenyl)-, -C(H)(Phenyl), -C(H)(C(H)(CH₃)₂)- oder -C(H)(CH₂-CH(CH₃)₂)-NH-C(=O)-CH₂-Gruppe gebunden ist, steht,
und jeweils die übrigen Reste X, R¹, R², R^{2a}, R³, R⁴ und R⁶ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-pyrido[4,3-d]pyrimidin-2-yl- und 5,6,7,8-Tetrahydro-chinazolin-2-yl-Verbindungen der vorstehend angegebenen allgemeinen Formel I, in denen R⁶
für eine Gruppe -NR¹⁰R¹¹,
für einen linearen oder verzweigten, unsubstituierten C₁₋₁₀ Alkyl-Rest, einen linearen oder verzweigten, unsubstituierten C₂₋₁₀-Alkenyl-Rest, für einen linearen oder verzweigten, unsubstituierten, C₂₋₁₀-Alkinyl-Rest,
für einen unsubstituierten oder wenigstens einfach substituierten, gesättigten oder ungesättigten 3-, 4-, 5-, 6-, 7- oder 8-gliedrigen cycloaliphatischen Rest, der über eine lineare oder verzweigte C₁₋₃-Alkylen-Gruppe gebunden und/oder mit einer linearen oder verzweigten C₁₋₃ Alkylen-Gruppe überbrückt sein kann,
für einen unsubstituierten oder wenigstens einfach substituierten, 5- oder 6-gliedrigen Aryl- oder Heteroaryl-Rest steht, der über eine lineare oder verzweigte C₁₋₆-Alkylen-Gruppe gebunden und/oder mit einem unsubstituierten oder wenigstens einfach substituierten, 5- oder 6-gliedrigen, monozyklischen Ringsystem kondensiert sein kann, steht, bevorzugt
für eine Gruppe -NR¹⁰R¹¹,
für einen linearen oder verzweigten, unsubstituierten C₁₋₅ Alkyl-Rest, einen linearen oder verzweigten, unsubstituierten C₂₋₅-Alkenyl-Rest, für einen linearen oder verzweigten, unsubstituierten, C₂₋₅-Alkinyl-Rest,
für einen unsubstituierten oder wenigstens einfach substituierten, gesättigten oder ungesättigten, 5-, 6- oder 7-gliedrigen cycloaliphatischen Rest, der über eine -(CH₂)-, -(CH₂)₂, oder -(CH₂)₃-Gruppe gebunden und/oder mit einer -(C(CH₃)₂)-Gruppe überbrückt sein kann, oder
für einen unsubstituierten oder wenigstens einfach substituierten, 5- oder 6-gliedrigen Aryl- oder Heteroaryl-Rest, der über eine -(CH₂)-, -(CH₂)₂-, oder -(CH₂)₃-Gruppe gebunden und/oder mit einem unsubstituierten oder wenigstens einfach substituierten, 5- oder 6-gliedrigen, ggf. eines oder mehrere Sauerstoffatome als Ringglied(er) und/oder eines oder mehrere Stickstoffatome als Ringglied(er) enthaltenen monozyklischen Ringsystem kondensiert sein kann, steht,
besonders bevorzugt für
für eine Gruppe -NR¹⁰R¹¹,
für einen Methyl-, Ethyl-, n-Propyl-, iso-Propyl-, n-Butyl-, iso-Butyl-, sec-Butyl- oder tert.-Butyl-Rest,
für einen ggf. über eine -(CH₂)-, -(CH₂)₂- oder -(CH₂)₃-Gruppe gebundenen 7,7-Dimethyl-2-oxo-bicyclo[2.2.1]heptyl-Rest, oder
für einen Phenyl-, Naphthyl-, Thiophenyl- (Thienyl-), Furanyl-, (Furyl-), Thiazolyl-, Pyrazolyl-, 2,3-Dihydro-benzo[1,4]-dioxinyl-, 1,2,3,4-Tetrahydroiso-chinolinyl- oder 3,4-Dihydrobenzo[1.4]oxazinyl-Rest steht, der über eine -(CH₂)-, -(CH₂)₂-, oder-(CH₂)₃-Gruppe gebunden und/oder einfach oder mehrfach, gleich oder verschieden, mit einem Substituenten ausgewählt aus der Gruppe bestehend aus F, Cl, Br, I, Methyl, Ethyl, n-Propyl, iso-Propyl, n-Butyl, iso-Butyl, sec-Butyl, tert.-Butyl, n-Pentyl, iso-Pentyl, neo-Pentyl, Methoxy, Ethoxy, -CN, -CF₃, -CF₂H, -CFH₂, -NO₂, -C(=O)-CF₃, -O-CF₃, -C(=O)-O-CH₃, -C(=O)-O-C₂H₅, -NH-C(=O)-CH₃, -S(=O)₂-CH₃ und Phenyl substituiert sein kann, steht,
und jeweils die übrigen Reste X, R¹, R², R^{2a}, R³-R⁵ und R⁷ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Des weiteren sind solche substituierten 5,6,7,8-Tetrahydro-pyrido[4,3-d]pyrimidin-2-yl- und 5,6,7,8-Tetrahydro-chinazolin-2-yl-Verbindungen der vorstehend angegebenen allgemeinen Formel I bevorzugt, in denen der Rest R⁷ für einen linearen oder verzweigten, unsubstituierten C₁₋₃-Alkyl-Rest, für einen unsubstituierten, 5- oder 6- gliedrigen Aryl- oder Heteroaryl-Rest, für einen unsubstituierten, gesättigten oder ungesättigten, ggf. ein Stickstoffatom als Ringglied aufweisenden 5-, 6- oder 7-gliedrigen cycloaliphatischen Rest, der mit einem unsubstituierten, 6-gliedrigen monozyklischen Ringsystem kondensiert sein kann, oder für einen -NR^{7a}R^{7b}-Rest steht, worin die Reste R^{7a} und R^{7b}, gleich oder verschieden, jeweils für einen linearen oder verzweigten C₁₋₃-Alkyl-Rest stehen,
bevorzugt für einen Methyl-, Ethyl-, n-Propyl-, Phenyl-, Indolyl-, 2,3-Dihydroindolyl-, Dimethylamino- oder Diethylamino-Rest steht,
und jeweils die übrigen Reste X, R¹, R², R^{2a}, R³ bis R⁶ und R⁸ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I, worin R⁸ für einen linearen oder verzweigten, unsubstituierten C₁₋₃-Alkyl-Rest oder für einen ggf. über eine lineare oder verzweigte C₁₋₃-Alkylen-Gruppe gebundenen Phenyl-Rest steht,
besonders bevorzugt für einen Methyl-, Ethyl-, Phenyl- oder Benzyl-Rest steht,
und jeweils die übrigen Reste X, R¹, R², R^{2a}, R³ bis R⁷ und R⁹ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind ferner auch substituierte 5,6,7,8-Tetrahydro-pyrido[4,3-d]pyrimidin-2-yl- und 5,6,7,8-Tetrahydro-chinazolin-2-yl-Verbindungen der vorstehend angegebenen allgemeinen Formel I, in denen R⁹ für einen linearen oder verzweigten, unsubstituierten C₁₋₃-Alkyl-Rest, für einen unsubstituierten, ggf. über eine lineare oder verzweigte C₁₋₃-Alkylen-Gruppe gebundenen, 5- oder 6-gliedrigen Aryl- oder Heteroaryl-Rest, oder für einen unsubstituierten oder wenigstens einfach substituierten, gesättigten oder ungesättigten, 5-oder 6-gliedrigen cycloaliphatischen Rest, der mit wenigstens einem unsubstituierten, 6-gliedrigen monozyklischen Ringsystem kondensiert sein kann, steht,
vorzugsweise für einen Methyl-, Ethyl-, n-Propyl-, iso-Propyl-, n-Butyl-, iso-Butyl-, sec-Butyl-, tert.-Butyl-, oder für einen ggf. über eine -(CH₂)-Gruppe gebundenen Phenyl- oder Fluorenyl-Rest steht,
und jeweils die übrigen Reste X, R¹, R², R^{2a}, R³ bis R⁸, R¹⁰ und R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I, worin die Reste R¹⁰ und R¹¹, gleich oder verschieden, jeweils für einen Methyl-, Ethyl-, n-Propyl- oder iso-Propyl-Rest stehen, und jeweils die übrigen Reste X, R¹, R², R^{2a} und R³ bis R⁹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 5,6,7,8-Tetrahydro-pyrido[4,3-d]pyrimidin-2-yl- und 5,6,7,8-Tetrahydro-chinazolin-2-yl-Verbindungen der allgemeinen Formel I, 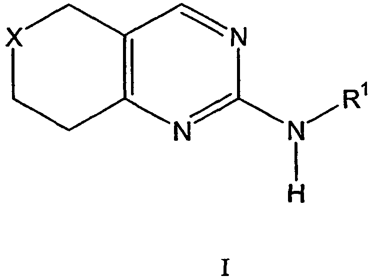 worin
- X: für eine C(H)(NHR²)-Gruppe oder für eine NR^{2a}-Gruppe steht,
- R¹: für eine -C(=O)-R³-Gruppe oder für eine -C(=O)-O-R⁴-Gruppe steht,
- R², R^{2a},: unabhängig voneinander, jeweils für eine -C(=O)-R⁵-Gruppe oder für eine -S(=O)₂-R⁶-Gruppe stehen,
- R³: für einen Methyl-, Ethyl-, n-Propyl-, iso-Propyl-, n-Butyl-, iso-Butyl-, sec-Butyl-oder tert.-Butyl-Rest, für einen unsubstituierten, gesättigten oder ungesättigten, ggf. eines oder mehrere Sauerstoffatome und/oder eines oder mehrere Stickstoffatome als Ringglied(er) aufweisenden, 5-, 6- oder 7-gliedrigen cycloaliphatischen Rest, der über eine -(CH₂)-, -(CH₂)₂- oder-(CH₂)₃-Gruppe gebunden sein kann, oder für einen Phenyl-Rest, Thiophenyl-Rest (Thienyl-Rest), Furanyl-Rest (Furyl-Rest), Pyridinyl-Rest oder Naphthyl-Rest, der über eine -(CH₂)-, -(CH₂)₂- oder -(CH₂)₃-Gruppe gebunden und/oder einfach oder mehrfach, gleich oder verschieden, mit einem Substituenten ausgewählt aus der Gruppe bestehend aus Methyl, Ethyl, n-Propyl, iso-Propyl, n-Butyl, iso-Butyl, sec-Butyl, tert.-Butyl, Methoxy, Ethoxy, n-Propoxy, iso-Propoxy, n-Butoxy, iso-Butoxy, sec-Butoxy, tert.-Butoxy, F, Cl, Br, I, -CN und-CF₃ substituiert sein kann, steht,
- R⁴: für einen unsubstituierten Benzyl-Rest steht,
- R⁵: für einen Rest ausgewählt aus der Gruppe bestehend aus Methyl, Ethyl, n-Propyl, iso-Propyl, n-Butyl, sec-Butyl, tert-Butyl, n-Pentyl, iso-Pentyl, neo-Pentyl, -C(H)(C₂H₅)₂, -C(H)(n-C₃H₇)₂, -CH=CH-CH=CH-CH₃, -CH₂-CH₂-CH=CH₂, -CH₂-CH₂-C(H)(CH₃)-(CH₂)₃-CH₃, -CH₂-O-CH₃ und -CH₂-O-CH₂-CH₂-O-CH₃,
für einen Rest ausgewählt aus der Gruppe bestehend aus Cyclopropyl, Cyclobutyl, Cyclopentyl, Cyclohexyl, Cycloheptyl, Cyclooctyl, Piperidinyl, Piperazinyl, Morpholinyl, Tetrahydrofuranyl (Tetrahydrofuryl), Dithiolanyl, 1,2,3,4-Tetrahydroindolyl, 1,2,3,4-Tetrahydronaphthyl, 1,3-Dihydroisoindolyl, Benzooxazolyl und Imidazolidinyl, wobei der zyklische Rest jeweils über eine-(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -C(CH₃)₂-, oder -C(H)(CH₃)-Gruppe gebunden und/oder einfach oder mehrfach, gleich oder verschieden, mit einem Substituenten ausgewählt aus der Gruppe bestehend aus Phenyl, -C(=O)-O-tert.-Butyl, oxo (=O), -S(=O)₂-Methyl und -S(=O)₂-Phenyl substituiert sein kann, und wobei die vorstehend genannten Phenyl-Reste jeweils einfach oder mehrfach, gleich oder verschieden, mit einem Substituenten ausgewählt aus der Gruppe bestehend aus F, Cl, Br und I substituiert sein können,
für einen Rest ausgewählt aus der Gruppe bestehend aus Phenyl, Naphthyl, Furanyl (Furyl), Thiophenyl (Thienyl), Pyridinyl, Isoxazolyl, Triazolyl, Pyrazolyl, Thiazolyl, Benzo[1,2,5]-oxadiazolyl, 2,3-Dihydrobenzofuranyl, Chinolinyl, Chromanyl, Chromenyl, Benzo[b]furanyl, Benzo[b]thiophenyl, Benzo[1,3]-dioxinyl, Indolyl, 2,3-Dihydro-indolyl, 3,4-Dihydro-benzo[1,4]oxazinyl und 1,2,3,4-Tetrahydroisochinolinyl steht, wobei der zyklische Rest jeweils über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-,-C(C₂H₅)(H)-O-, -O-C(C₂H₅)(H)-, -CH₂-O-CH₂-, -CH₂-S-CH₂-, -C(CH₃)₂-,-C(H)(CH₃)- oder -CH=CH-Gruppe gebunden und/oder einfach oder mehrfach, gleich oder verschieden, mit einem Substituenten ausgewählt aus der Gruppe bestehend aus F, Cl, Br, I, -CN, -NO₂, -OH, -SH, Methyl, Ethyl, n-Propyl, iso-Propyl, n-Butyl, iso-Butyl, sec-Butyl, tert.-Butyl, n-Pentyl, neo-Pentyl, Methoxy, Ethoxy, n-Propoxy, iso-Propoxy, n-Butyloxy, iso-Butyloxy, sec-Butyloxy, tert.-Butyloxy, -S-Methyl, -S-Ethyl, -CF₃, -CHF₂, -CH₂F, -O-CF₃, -O-CHF₂, -O-CH₂F, -S-CF₃, -S-CHF₂, -S-CH₂F, -S(=O)₂-Phenyl, Pyrazolyl, Phenyl, -N(CH₃)₂,-N(C₂H₅)₂, -CH₂O-C(=O)-Phenyl, -O-C(=O)-Phenyl, Furyl (Furanyl), Thiadiazolyl, Thiophenyl (Thienyl), Phenoxy und Benzyl substituiert sein kann, und wobei die zyklischen Substituenten selbst jeweils einfach oder mehrfach, gleich oder verschieden, mit einem Substituenten ausgewählt aus der Gruppe bestehend aus F, Cl, Br, I substituiert sein können,
für einen -C(=O)-R⁷-Rest, der über eine -(CH₂)-, -(CH₂)₂- oder -(CH₂)₃-Gruppe gebunden sein kann,
für einen -C(=O)-O-R⁸-Rest, der über eine -(CH₂)-, -(CH₂)₂-, -(CH₂)₃- oder-C(H)(Phenyl)-Gruppe gebunden sein kann, oder für einen -N(H)-C(=O)-O-R⁹-Rest, der über eine -(CH₂)-, -(CH₂)₂-, -(CH₂)₃-,-(CH₂)₄-, -(CH₂)₅-, -C(H)(CH₂-Phenyl)-, -C(H)(Phenyl), -C(H)(C(H)(CH₃)₂)-oder -C(H)(CH₂-CH(CH₃)₂)-NH-C(=O)-CH₂-Gruppe gebunden ist, steht,
- R⁶: für eine Gruppe -NR¹⁰R¹¹,
für einen Methyl-, Ethyl-, n-Propyl-, iso-Propyl-, n-Butyl-, iso-Butyl-, sec-Butyl- oder tert.-Butyl-Rest,
für einen ggf. über eine -(CH₂)-, -(CH₂)₂- oder -(CH₂)₃-Gruppe gebundenen 7,7-Dimethyl-2-oxo-bicyclo[2.2.1]heptyl-Rest, oder
für einen Phenyl-, Naphthyl-, Thiophenyl- (Thienyl-), Furanyl-, (Furyl-), Thiazolyl-, Pyrazolyl-, 2,3-Dihydro-benzo[1,4]-dioxinyl-, 1,2,3,4-Tetrahydroiso-chinolinyl- oder 3,4-Dihydrobenzo[1.4]oxazinyl-Rest, der über eine -(CH₂)-,(CH₂)₂-, oder -(CH₂)₃-Gruppe gebunden und/oder einfach oder mehrfach, gleich oder verschieden, mit einem Substituenten ausgewählt aus der Gruppe bestehend aus F, Cl, Br, I, Methyl, Ethyl, n-Propyl, iso-Propyl, n-Butyl, iso-Butyl, sec-Butyl, tert.-Butyl, n-Pentyl, iso-Pentyl, neo-Pentyl, Methoxy, Ethoxy, -CN, -CF₃, -CF₂H, -CFH₂, -NO₂, -C(=O)-CF₃, -O-CF₃, -C(=O)-O-CH₃, -C(=O)-O-C₂H₅, -NH-C(=O)-CH₃, -S(=O)₂-CH₃ und Phenyl substituiert sein kann, steht,
- R⁷: für einen Methyl-, Ethyl-, n-Propyl-, Phenyl-, Indolyl-, 2,3-Dihydro-indolyl-, Dimethylamino- oder Diethylamino-Rest steht
- R⁸: für einen Methyl-, Ethyl-, Phenyl- oder Benzyl-Rest steht,
- R⁹: für einen Methyl-, Ethyl-, n-Propyl-, iso-Propyl-, n-Butyl-, iso-Butyl-, sec-Butyl-, tert.-Butyl-, oder für einen ggf. über eine -(CH₂)-Gruppe gebundenen Phenyl- oder Fluorenyl-Rest steht,
- R¹⁰, R¹¹,: gleich oder verschieden, jeweils für einen Methyl-, Ethyl-, n-Propyl-oder iso-Propyl-Rest stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte 5,6,7,8-Tetrahydro-pyrido[4,3-d]pyrimidin-2-yl- und 5,6,7,8-Tetrahydro-chinazolin-2-yl-Verbindungen der allgemeinen Formel I ausgewählt aus der Gruppe bestehend aus
[1] 3-Fluor-N-[6-(4-fluor-benzoyl)-5,6,7,8-tetrahydro-pyrido[4,3-d]pyrimidin-2-yl]-benzamid,
[2] 3,5-Dichlor-N-[6-(3-fluor-4-methoxy-benzoyl)-5,6,7,8-tetrahydro-pyrido[4,3-d]pyrimidin-2-yl]-benzamid,
[3] 4-tert-Butyl-N-(6-hexanoyl-5,6,7,8-tetrahydro-pyrido[4,3-d]pyrimidin-2-yl)-benzamid,
[4] N-(6-Acetyl-5,6,7,8-tetrahydro-pyrido[4,3-d]pyrimidin-2-yl)-3,4-dichlor-benzamid,
[5] 3,5-Dichlor-N-[6-(3-trifluormethyl-benzoyl)-5,6,7,8-tetrahydro-pyrido[4,3-d]pyrimidin-2-yl]-benzamid,
[6] 3-Chlor-N-[6-(4-methoxy-2,3,6-trimethyl-benzolsulfonyl)-5,6,7,8-tetrahydro-pyrido[4,3-d]pyrimidin-2-yl]-benzamid,
[7] N-[6-(2-Ethoxy-benzoyl)-5,6,7,8-tetrahydro-pyrido[4,3-d]pyrimidin-2-yl]-3-fluor-benzamid,
[8] 3-Chlor-N-[6-(3-phenyl-propionyl)-5,6,7,8-tetrahydro-pyrido[4,3-d]pyrimidin-2-yl]-benzamid,
[9] 4-tert-Butyl-N-[6-(isoxazol-5-carbonyl)-5,6,7,8-tetrahydro-pyrido[4,3-d]pyrimidin-2-yl]-benzamid,
[10] N-[6-(2-Benzylsulfanyl-acetyl)-5,6,7,8-tetrahydro-pyrido[4,3-d]pyrimidin-2-yl]-2-methoxy-benzamid,
[11] Thiophen-2-carbonsäure {6-[2-(4-chlor-phenyl)-2-methyl-propionyl]-5,6,7,8-tetrahydro-pyrido[4,3-d]pyrimidin-2-yl}-amid,
[12] N-(6-Benzoyl-5,6,7,8-tetrahydro-pyrido[4,3-d]pyrimidin-2-yl)-3-methyl-benzamid,
[13] N-[6-(2,3-Dihydro-benzofuran-5-carbonyl)-5,6,7,8-tetrahydro-pyrido[4,3-, d]pyrimidin-2-yl]-2-fluor-benzamid,
[14] Thiophen-2-carbonsäure [6-(3,4,5-trimethoxy-benzoylamino)-5,6,7,8-tetrahydro-chinazolin-2-yl]-amid,
[15] Naphthalin-1-carbonsäure [6-(3-methyl-5-phenyl-isoxazol-4-carbonyl)-5,6,7,8-tetrahydro-pyrido[4,3-d]pyrimidin-2-yl]-amid,
[16] 3-Chlor-N-{6-[2-(5-methyl-2-phenyl-thiazol-4-yl)-acetyl]-5,6,7,8-tetrahydro-pyrido[4,3-d]pyrimidin-2-yl}-benzamid,
[17] N-[6-(4-Chlor-2,5-dimethyl-benzolsulfonyl)-5,6,7,8-tetrahydro-pyrido[4,3-d]pyrimidin-2-yl]-2-trifluormethyl-benzamid,
[18] N-[6-(1-Benzolsulfonyl-1H-indol-2-carbonyl)-5,6,7,8-tetrahydro-pyrido[4,3-d]pyrimidin-2-yl]-3-methoxy-benzamid,
[20] N-[6-(5-Methyl-1-phenyl-1H-pyrazol-4-sulfonyl)-5,6,7,8-tetrahydro-pyrdo[4,3-d]pyrimidin-2-yl]-2-trifluormethyl-benzamid,
[21] N-[6-(3-Chlor-2-methyl-benzolsulfonyl)-5,6,7,8-tetrahydro-pyrido[4,3-d]pyrimidin-2-yl]-3-fluor-benzamid,
[22] 3,5-Dichlor-N-[6-(3,5-dimethyl-isoxazol-4-carbonyl)-5,6,7,8-tetrahydro-pyrido[4,3-d]pyrimidin-2-yl]-benzamid,
[23] Thiophen-2-carbonsäure [6-(4-trifluormethoxy-benzolsulfonylamino)-5,6,7,8-tetrahydro-chinazolin-2-yl]-amid,
[25] N-[6-(Furan-2-carbonyl)-5,6,7,8-tetrahydro-pyrido[4,3-d]pyrimidin-2-yl]-3-methoxy-benzamid,
[26] N-{6-[4-(2,3-Dihydro-indol-1-yl)-4-oxo-butyryl]-5,6,7,8-tetrahydro-pyrido[4,3-d]pyrimidin-2-yl}-3-methyl-benzamid,
[27] N-{6-[2-(4-Methyl-cyclohexyl)-acetyl]-5,6,7,8-tetrahydro-pyrido[4,3-d]pyrimidin-2-yl}-2-trifluormethyl-benzamid,
[28] 3,4-Difluor-N-[6-(6-phenoxy-pyridin-3-carbonyl)-5,6,7,8-tetrahydro-pyrido[4,3-d]pyrimidin-2-yl]-benzamid,
[29] 4-tert-Butyl-N-[6-(2-phenyl-thiazol-4-carbonyl)-5,6,7,8-tetrahydro-pyrido[4,3-d]pyrimidin-2-yl]-benzamid,
[30] 3,5-Dichlor-N-[6-(2-trifluormethyl-benzolsulfonyl)-5,6,7,8-tetrahydro-pyrido[4,3-d]pyrimidin-2-yl]-benzamid,
[31] Thiophen-2-carbonsäure [6-(3-brom-benzolsulfonylamino)-5,6,7,8-tetrahydro-chinazolin-2-yl]-amid,
[32] N-[6-(2-Chlor-pyridin-4-carbonyl)-5,6,7,8-tetrahydro-pyrido[4,3-d]pyrimidin-2-yl]-3-methoxy-benzamid,
[33] 4-Fluor-N-[6-(2-methansulfonyl-benzolsulfonylamino)-5,6,7,8-tetrahydro-chinazolin-2-yl]-benzamid,
[35] N-(6-Dimethylsulfamoyl-5,6,7,8-tetrahydro-pyrido[4,3-d]pyrimidin-2-yl)-3-fluor-benzamid,
[36] 3-Fluor-N-{6-[2-(4-trifluormethyl-phenyl)-acetyl]-5,6,7,8-tetrahydro-pyrido[4,3-d]pyrimidin-2-yl}-benzamid,
[37] N-{6-[2-(1,3-Dioxo-1,3-dihydro-isoindol-2-yl)-acetyl]-5,6,7,8-tetrahydro-pyrido[4,3-d]pyrimidin-2-yl}-2-trifluormethyl-benzamid,
[38] Thiophen-2-carbonsäure {6-[4-(4-chlor-2-methyl-phenoxy)-butyrylamino]-5,6,7,8-tetrahydro-chinazolin-2-yl}-amid,
[39] N-[6-(Butan-1-sulfonyl)-5,6,7,8-tetrahydro-pyrido[4,3-d]pyrimidin-2-yl]-3-fluor-benzamid,
[40] 2-Methoxy-N-[6-(2-propyl-pentanoyl)-5,6,7,8-tetrahydro-pyrido[4,3-d]pyrimidin-2-yl]-benzamid,
[41] N-[6-(4,5-Dichlor-thiophen-2-sulfonylamino)-5,6,7,8-tetrahydro-chinazolin-2-yl]-4-fluor-benzamid,
[42] 4-Chlor-N-[6-(2-trifluormethyl-benzolsulfonyl)-5,6,7,8-tetrahydro-pyrido[4,3-d]pyrimidin-2-yl]-benzamid,
[43] Thiophen-2-carbonsäure [6-(4-methoxy-benzolsulfonylamino)-5,6,7,8-tetrahydro-chinazolin-2-yl]-amid,
[44] 5-[2-(3,4-Difluor-benzoylamino)-7,8-dihydro-5H-pyrido[4,3-d]pyrimidin-6-yl]-5-oxo-valeriansäure methyl ester,
[45] N-[6-(Benzo[b]thiophen-3-carbonyl)-5,6,7,8-tetrahydro-pyrido[4,3-d]pyrimidin-2-yl]-4-chlor-benzamid,
[46] N-[6-(5-Brom-2-methoxy-benzolsulfonylamino)-5,6,7,8-tetrahydro-chinazolin-2-yl]-4-fluor-benzamid,
[47] 4-Fluor-N-[6-(4-fluor-benzolsulfonylamino)-5,6,7,8-tetrahydro-chinazolin-2-yl]-benzamid,
[48] N-{6-[2-(1H-Indol-3-yl)-2-oxo-acetyl]-5,6,7,8-tetrahydro-pyrido[4,3-d]pyrimidin-2-yl}-2-trifluormethyl-benzamid,
[49] 5-tert-Butyl-2-methyl-furan-3-carbonsäure {2-[(thiophen-2-carbonyl)-amino]-5,6,7,8-tetrahydro-chinazolin-6-yl}-amid,
[50] N-[6-(2,5-Dimethoxy-benzolsulfonyl)-5,6,7,8-tetrahydro-pyrido[4,3-d]pyrimidin-2-yl]-3-methoxy-benzamid,
[51] Benzoesäure 2-{2-[(thiophen-2-carbonyl)-amino]-5,6,7,8-tetrahydro-chinazolin-6-ylcarbamoyl}-benzyl ester,
[52] 3,4-Difluor-N-(6-hexanoyl-5,6,7,8-tetrahydro-pyrido[4,3-d]pyrimidin-2-yl)-benzamid,
[53] 4-Ethyl-N-[6-(tetrahydro-furan-2-carbonyl)-5,6,7,8-tetrahydro-pyrido[4,3-d]pyrimidin-2-yl]-benzamid,
[54] N-[6-(2-Chlor-benzolsulfonyl)-5,6,7,8-tetrahydro-pyrido[4,3-d]pyrimidin-2-yl]-3-fluor-benzamid,
[55] 4-tert-Butyl-N-[6-(5-tert-butyl-2-methyl-2H-pyrazol-3-carbonyl)-5,6,7,8-tetrahydro-pyrido[4,3-d]pyrimidin-2-yl]-benzamid,
[56] 3-Methoxy-N-[6-(4-methoxy-benzolsulfonyl)-5,6,7,8-tetrahydro-pyrido[4,3-d]pyrimidin-2-yl]-benzamid,
[57] {2-[2-(3,4-Difluor-benzoylamino)-7,8-dihydro-5H-pyrido[4,3-d]pyrimidin-6-yl]-2-oxo-1-phenyl-ethyl}-carbaminsäure benzyl ester,
[58] 5-Phenyl-oxazol-4-carbonsäure {2-[(thiophen-2-carbonyl)-amino]-5,6,7,8-tetrahydro-chinazolin-6-yl}-amid,
[59] 4-Chlor-N-(6-dimethylsulfamoyl-5,6,7,8-tetrahydro-pyrido[4,3-d]pyrimidin-2-yl)-benzamid,
[60] 4-tert-Butyl-N-[6-(2-fluor-benzolsulfonyl)-5,6,7,8-tetrahydro-pyrido[4,3-d]pyrimidin-2-yl]-benzamid,
[61] 4-Chlor-N-[6-(3-cyclopentyl-propionyl)-5,6,7,8-tetrahydro-pyrido[4,3-d]pyrimidin-2-yl]-benzamid,
[62] 4-Chlor-N-[6-(4-phenyl-butyryl)-5,6,7,8-tetrahydro-pyrido[4,3-d]pyrimidin-2-yl]-benzamid,
[63] 4-tert-Butyl-N-[6-(5-methyl-2-phenyl-2H-[1,2,3]triazol-4-carbonyl)-5,6,7,8-tetrahydro-pyrido[4,3-d]pyrimidin-2-yl]-benzamid,
[64] 2-Chlor-N-{2-[(thiophen-2-carbonyl)-amino]-5,6,7,8-tetrahydro-chinazolin-6-yl}-isonicotinamid,
[66] N-[6-(5-Chlor-thiophen-2-sulfonyl)-5,6,7,8-tetrahydro-pyrido[4,3-d]pyrimidin-2-yl]-3-fluor-benzamid,
[67] N-[6-(4-Diethylamino-benzoyl)-5,6,7,8-tetrahydro-pyrido[4,3-d]pyrimidin-2-yl]-3-methoxy-benzamid,
[68] N-[6-(2,4-Dimethyl-thiazol-5-sulfonyl)-5,6,7,8-tetrahydro-pyrido[4,3-d]pyrimidin-2-yl]-3-methyl-benzamid,
[69] 4-Chlor-N-[6-(naphthalin-2-carbonyl)-5,6,7,8-tetrahydro-pyrido[4,3-d]pyrimidin-2-yl]-benzamid,
[70] N-[6-(2,4-Difluor-benzolsulfonyl)-5,6,7,8-tetrahydro-pyrido[4,3-d]pyrimidin-2-yl]-2-methoxy-benzamid,
[71] 3,4-Dichlor-N-[6-(4-chlor-benzolsulfonyl)-5,6,7,8-tetrahydro-pyrido[4,3-d]pyrimidin-2-yl]-benzamid,
[72] 3,4-Difluor-N-[6-(thiophen-2-sulfonyl)-5,6,7,8-tetrahydro-pyrido[4,3-d]pyrimidin-2-yl]-benzamid,
[74] 4-Ethyl-N-[6-(4-pyrazol-1-yl-benzoyl)-5,6,7,8-tetrahydro-pyrido[4,3-d]pyrimidin-2-yl]-benzamid,
[75] N-[6-(7,7-Dimethyl-2-oxo-bicyclo[2.2.1]hept-1-ylmethansulfonylamino)-5,6,7,8-tetrahydro-chinazolin-2-yl]-butyramid,
[76] 5-Oxo-5-phenyl-valeriansäure (2-butyrylamino-5,6,7,8-tetrahydro-chinazolin-6-yl)-amid,
[77] 3-[2-(4-Chlor-benzoylamino)-7,8-dihydro-5H-pyrido[4,3-d]pyrimidin-6-yl]-3-oxo-propionsäure methyl ester,
[78] N-{6-[5-(4-Chlor-phenyl)-2-methyl-furan-3-carbonyl]-5,6,7,8-tetrahydro-pyrido[4,3-d]pyrimidin-2-yl}-2-methoxy-benzamid,
[79] 4-Chlor-N-[6-(2,4-dimethyl-thiazol-5-sulfonyl)-5,6,7,8-tetrahydro-pyrido[4,3-d]pyrimidin-2-yl]-benzamid,
[80] N-[6-(2,3-Difluor-benzoyl)-5,6,7,8-tetrahydro-pyrido[4,3-d]pyrimidin-2-yl]-3-methoxy-benzamid,
[81] 4-Chlor-N-{6-[2-(2-methoxy-ethoxy)-acetyl]-5,6,7,8-tetrahydro-pyrido[4,3-d]pyrimidin-2-yl}-benzamid,
[82] 2-[2-(2-Ethoxy-benzoylamino)-7,8-dihydro-5H-pyrido[4,3-d]pyrimidin-6-sulfonyl]-benzoesäure methyl ester,
[83] 4-tert-Butyl-N-[6-(4-nitro-benzolsulfonyl)-5,6,7,8-tetrahydro-pyrido[4,3-d]pyrimidin-2-yl]-benzamid,
[86] N-[6-(5-Chlor-thiophen-2-sulfonyl)-5,6,7,8-tetrahydro-pyrido[4,3-d]pyrimidin-2-yl]-2-methoxy-benzamid,
[87] 4-Fluor-N-[6-(furan-2-carbonyl)-5,6,7,8-tetrahydro-pyrido[4,3-d]pyrimidin-2-yl]-benzamid,
[88] 4-{2-[(Thiophen-2-carbonyl)-amino]-5,6,7,8-tetrahydro-chinazolin-6-ylcarbamoyl}-piperidin-1-carbonsäure tert-butyl ester,
[89] 1-(4-Chlor-phenyl)-cyclopropancarbonsäure (2-butyrylamino-5,6,7,8-tetrahydro-chinazolin-6-yl)-amid,
[90] N-(6-Ethansulfonyl-5,6,7,8-tetrahydro-pyrido[4,3-d]pyrimidin-2-yl)-3-methoxy-benzamid,
[91] 5-Methyl-thiophen-2-carbonsäure (2-butyrylamino-5,6,7,8-tetrahydro-chinazolin-6-yl)-amid,
[92] 4-tert-Butyl-N-[6-(4-methyl-3-nitro-benzoyl)-5,6,7,8-tetrahydro-pyrido[4,3-d]pyrimidin-2-yl]-benzamid,
[94] 4-Fluor-N-[6-(3-phenyl-propionylamino)-5,6,7,8-tetrahydro-chinazolin-2-yl]-benzamid,
[95] 3,4-Dichlor-N-[6-(2,4,6-trimethyl-benzolsulfonyl)-5,6,7,8-tetrahydro-pyrido[4,3-d]pyrimidin-2-yl]-benzamid,
[96] N-[6-(4-Methyl-3,4-dihydro-2H-benzo[1,4]oxazin-7-sulfonyl)-5,6,7,8-tetrahydro-pyrido[4,3-d]pyrimidin-2-yl]-nicotinamid,
[97] Thiophen-2-carbonsäure [6-(3,5-difluor-benzoylamino)-5,6,7,8-tetrahydro-chinazolin-2-yl]-amid,
[98] N-[6-(2,4-Difluor-benzolsulfonylamino)-5,6,7,8-tetrahydro-chinazolin-2-yl]-butyramid,
[99] Thiophen-2-carbonsäure [6-(2,3,5,6-tetramethyl-benzolsulfonyl)-5,6,7,8-tetrahydro-pyrido[4,3-d]pyrimidin-2-yl]-amid,
[101] N-[6-(3,4-Dichlor-benzoyl)-5,6,7,8-tetrahydro-pyrido[4,3-d]pyrimidin-2-yl]-4-ethyl-benzamid,
[102] N-[6-(7,7-Dimethyl-2-oxo-bicyclo[2.2.1]hept-1-ylmethansulfonyl)-5,6,7,8-tetrahydro-pyrido[4,3-d]pyrimidin-2-yl]-3-fluor-benzamid,
[103] N-[6-(6-Fluor-4H-benzo[1,3]dioxin-8-carbonyl)-5,6,7,8-tetrahydro-pyrido[4,3-d]pyrimidin-2-yl]-2-trifluormethyl-benzamid,
[104] [5-(2-Butyrylamino-5,6,7,8-tetrahydro-chinazolin-6-ylcarbamoyl)-pentyl]-carbaminsäure benzyl ester,
[105] 2-Ethoxy-N-[6-(4-oxo-pentanoyl)-5,6,7,8-tetrahydro-pyrido[4,3-d]pyrimidin-2-yl]-benzamid,
[106] Naphthalin-1-carbonsäure [6-(propan-1-sulfonyl)-5,6,7,8-tetrahydro-pyrido[4,3-d]pyrimidin-2-yl]-amid,
[107] N-[6-(5-Fluor-2-methyl-benzolsulfonyl)-5,6,7,8-tetrahydro-pyrido[4,3-d]pyrimidin-2-yl]-4-methyl-benzamid,
[109] 3-Chlor-N-[6-(2-methyl-benzoyl)-5,6,7,8-tetrahydro-pyrido[4,3-d]pyrimidin-2-yl]-benzamid,
[110] Thiophen-2-carbonsäure [6-(3-chlor-benzo[b]thiophen-2-carbonyl)-5,6,7,8-tetrahydro-pyrido[4,3-d]pyrimidin-2-yl]-amid,
[112] Thiophen-2-carbonsäure [6-(2-cyclopentyl-acetylamino)-5,6,7,8-tetrahydro-chinazolin-2-yl]-amid,
[113] N-[6-(3,4-Dichlor-benzoyl)-5,6,7,8-tetrahydro-pyrido[4,3-d]pyrimidin-2-yl]-2-fluor-benzamid,
[114] N-[6-(2-Chlor-benzolsulfonyl)-5,6,7,8-tetrahydro-pyrido[4,3-d]pyrimidin-2-yl]-4-ethyl-benzamid,
[115] Thiophen-2-carbonsäure[6-(4-brom-3-methyl-benzoylamino)-5,6,7,8-tetrahydro-chinazolin-2-yl]-amid,
[116] 3-Methyl-N-[6-(toluen-3-sulfonyl)-5,6,7,8-tetrahydro-pyrido[4,3-d]pyrimidin-2-yl]-benzamid,
[117] Naphthalin-1-carbonsäure [6-(5-chlor-thiophen-2-sulfonyl)-5,6,7,8-tetrahydro-pyrido[4,3-d]pyrimidin-2-yl]-amid,
[118] N-(2-Butyrylamino-5,6,7,8-tetrahydro-chinazolin-6-yl)-2,6-difluorbenzamid,
[119] 3-Chlor-N-[6-(3,4-dimethoxy-benzoyl)-5,6,7,8-tetrahydro-pyrido[4,3-d]pyrimidin-2-yl]-benzamid,
[120] N-[6-(3-Chlor-benzo[b]thiophen-2-carbonyl)-5,6,7,8-tetrahydro-pyrido[4,3-d]pyrimidin-2-yl]-benzamid,
[121] 4-Ethyl-N-[6-(thiophen-3-sulfonyl)-5,6,7,8-tetrahydro-pyrido[4,3-d]pyrimidin-2-yl]-benzamid,
[123] 2-Ethoxy-N-[6-(2-ethylsulfanyl-pyridin-3-carbonyl)-5,6,7,8-tetrahydro-pyrido[4,3-d]pyrimidin-2-yl]-benzamid,
[124] 3-[2-(3-Chlor-benzoylamino)-7,8-dihydro-5H-pyrido[4,3-d]pyrimidin-6-yl]-3-oxo-propionsäure ethyl ester,
[125] N-[6-(3-Brom-benzolsulfonyl)-5,6,7,8-tetrahydro-pyrido[4,3-d]pyrimidin-2-yl]-3-chlor-benzamid,
[126] 3,4-Dichlor-N-{6-[2-(4-chlor-phenyl)-acetyl]-5,6,7,8-tetrahydro-pyrido[4,3-d]pyrimidin-2-yl}-benzamid,
[127] 3-Chlor-N-[6-(2-chlor-6-fluor-benzoyl)-5,6,7,8-tetrahydro-pyrido[4,3-d]pyrimidin-2-yl]-benzamid,
[128] 4-Fluor-N-(6-hexanoylamino-5,6,7,8-tetrahydro-chinazolin-2-yl)-benzamid,
[129] N-[6-(5-Brom-2-methoxy-benzolsulfonyl)-5,6,7,8-tetrahydro-pyrido[4,3-d]pyrimidin-2-yl]-3-methoxy-benzamid,
[132] N-[6-(3-Cyclopentyl-propionyl)-5,6,7,8-tetrahydro-pyrido[4,3-d]pyrimidin-2-yl]-isonicotinamid,
[133] 5-Benzyl-furan-2-carbonsäure (2-butyrylamino-5,6,7,8-tetrahydro-chinazolin-6-yl)-amid
[134] 3,4-Dichlor-N-[6-(4-trifluormethoxy-benzolsulfonyl)-5,6,7,8-tetrahydro-pyrido[4,3-d]pyrimidin-2-yl]-benzamid,
[135] [((2-Butyrylamino-5,6,7,8-tetrahydro-chinazolin-6-ylcarbamoyl)-phenyl-methyl]-carbaminsäure benzyl ester,
[136] N-[6-(3,4-Dimethoxy-benzolsulfonyl)-5,6,7,8-tetrahydro-pyrido[4,3-d]pyrimidin-2-yl]-4-fluor-benzamid,
[137] 2-Fluor-N-[6-(2-methyl-5-phenyl-furan-3-carbonyl)-5,6,7,8-tetrahydro-pyrido[4,3-d]pyrimidin-2-yl]-benzamid,
[138] Thiophen-2-carbonsäure(6-pent-4-enoylamino-5,6,7,8-tetrahydro-chinazolin-2-yl)-amid,
[139] ((2-Methyl-1-{2-[(thiophen-2-carbonyl)-amino]-5,6,7,8-tetrahydro-chinazolin-6-ylcarbamoyl}-propyl)-carbaminsäure 9H-fluoren-9-ylmethyl ester,
[140] ((5-{2-[(Thiophen-2-carbonyl)-amino]-5,6,7,8-tetrahydro-chinazolin-6-ylcarbamoyl}-pentyl)-carbaminsäure tert-butyl ester,
[141] 3-Fluor-N-[6-(1,2,3,4-tetrahydro-naphthalin-2-carbonyl)-5,6,7,8-tetrahydro-pyrido[4,3-d]pyrimidin-2-yl]-benzamid,
[142] 4-Chlor-N-{6-[2-(2,5-dioxo-imidazolidin-4-yl)-acetyl]-5,6,7,8-tetrahydro-pyrido[4,3-d]pyrimidin-2-yl}-benzamid,
[143] 3-Fluor-N-[6-(toluen-4-sulfonyl)-5,6,7,8-tetrahydro-pyrido[4,3-d]pyrimidin-2-yl]-benzamid,
[144] 3,5-Dichlor-N-[6-(4-thiophen-2-yl-benzoyl)-5,6,7,8-tetrahydro-pyrido[4,3-d]pyrimidin-2-yl]-benzamid,
[145] N-[6-(3-Chlor-thiophen-2-carbonyl)-5,6,7,8-tetrahydro-pyrido[4,3-d]pyrimidin-2-yl]-2-ethoxy-benzamid,
[146] N-[6-(Toluen-3-sulfonylamino)-5,6,7,8-tetrahydro-chinazolin-2-yl]-butyramid,
[147] Thiophen-2-carbonsäure {6-[2-(2,2,2-trifluor-acetyl)-1,2,3,4-tetrahydro-isochinolin-7-sulfonylamino]-5,6,7,8-tetrahydro-chinazolin-2-yl}-amid,
[148] 4-Methyl-N-[6-(4-trifluormethylsulfanyl-benzoyl)-5,6,7,8-tetrahydro-pyrido[4,3-d]pyrimidin-2-yl]-benzamid,
[149] ((2-{2-[(Thiophen-2-carbonyl)-amino]-5,6,7,8-tetrahydro-chinazolin-6-ylcarbamoyl}-ethyl)-carbaminsäure tert-butyl ester,
[150] 2-Fluor-N-[6-(2-methyl-5-nitro-benzolsulfonyl)-5,6,7,8-tetrahydro-pyrido[4,3-d]pyrimidin-2-yl]-benzamid,
[151] N-[6-(4-Methoxy-benzolsulfonylamino)-5,6,7,8-tetrahydro-chinazolin-2-yl]-butyramid,
[152] 4-Chlor-N-[6-(2,4-difluor-benzoyl)-5,6,7,8-tetrahydro-pyrido[4,3-d]pyrimidin-2-yl]-benzamid
[153] 4-Methyl-N-(6-pent-4-enoyl-5,6,7,8-tetrahydro-pyrido[4,3-d]pyrimidin-2-yl)-benzamid,
[154] Naphthalin-1-carbonsäure[6-(3-fluor-4-methyl-benzoyl)-5,6,7,8-tetrahydro-pyrido[4,3-d]pyrimidin-2-yl]-amid,
[155] N-{6-[2-(4-Trifluormethyl-phenyl)-acetyl]-5,6,7,8-tetrahydro-pyrido[4,3-d]pyrimidin-2-yl}-benzamid,
[156] N-[6-(3,4-Dichlor-benzoyl)-5,6,7,8-tetrahydro-pyrido[4,3-d]pyrimidin-2-yl]-nicotinamid,
[157] 4-Ethyl-N-{6-[2-(2,2,2-trifluor-acetyl)-2,3,4-tetrahydro-isochinolin-7-sulfonyl]-5,6,7,8-tetrahydro-pyrido[4,3-d]pyrimidin-2-yl}-benzamid,
[158] 2-Methoxy-N-[6-(4-methoxy-2,3,6-trimethyl-benzolsulfonyl)-5,6,7,8-tetrahydro-pyrido[4,3-d]pyrimidin-2-yl]-benzamid,
[159] 4-Ethyl-N-[6-(2-methoxy-acetyl)-5,6,7,8-tetrahydro-pyrido[4,3-d]pyrimidin-2-yl]-benzamid,
[160] 4-Fluor-N-[6-(propan-1-sulfonylamino)-5,6,7,8-tetrahydro-chinazolin-2-yl]-benzamid,
[162] N-[6-(7,7-Dimethyl-2-oxo-bicyclo[2.2.1]hept-1-ylmethansulfonylamino)-5,6,7,8-tetrahydro-chinazolin-2-yl]-butyramid,
[163] N-{6-[2-(2,5-Dimethyl-phenyl)-acetylamino]-5,6,7,8-tetrahydro-chinazolin-2-yl}-4-fluor-benzamid,
[165] Thiophen-2-carbonsäure (6-phenylmethansulfonylamino-5,6,7,8-tetrahydro-chinazolin-2-yl)-amid,
[166] N-[6-(3,4-Dimethoxy-benzolsulfonylamino)-5,6,7,8-tetrahydro-chinazolin-2-yl]-butyramid,
[167] N-{6-[4-(1,1-Dimethyl-propyl)-benzolsulfonylamino]-5,6,7,8-tetrahydro-chinazolin-2-yl}-butyramid,
[168] N-(2-Butyrylamino-5,6,7,8-tetrahydro-chinazolin-6-yl)-3-(3-trifluormethyl-phenyl)-acrylamid,
[169] N-[6-(Butan-1-sulfonylamino)-5,6,7,8-tetrahydro-chinazolin-2-yl]-4-fluor-benzamid,
[170] Naphthalin-1-carbonsäure (6-acetyl-5,6,7,8-tetrahydro-pyrido[4,3-d]pyrimidin-2-yl)-amid,
[171] 3,5-Dichlor-N-[6-(3-diethylcarbamoyl-propionyl)-5,6,7,8-tetrahydro-pyrido[4,3-d]pyrimidin-2-yl]-benzamid,
[172] 4-Ethyl-N-[6-(4-methoxy-benzolsulfonyl)-5,6,7,8-tetrahydro-pyrido[4,3-d]pyrimidin-2-yl]-benzamid,
[173] 4-Ethyl-N-[6-(chinolin-6-carbonyl)-5,6,7,8-tetrahydro-pyrido[4,3-d]pyrimidin-2-yl]-benzamid,
[174] N-[6-(2-Cyclopropyl-acetyl)-5,6,7,8-tetrahydro-pyrido[4,3-d]pyrimidin-2-yl]-2-fluor-benzamid,
[175] N-[6-(2H-Chromen-3-carbonyl)-5,6,7,8-tetrahydro-pyrido[4,3-d]pyrimidin-2-yl]-3,4-difluor-benzamid,
[176] N-{6-[2-(4-Chlor-phenyl)-propionyl]-5,6,7,8-tetrahydro-pyrido[4,3-d]pyrimidin-2-yl}-benzamid,
[177] Thiophen-2-carbonsäure [6-(2-naphthalin-1-yl-acetylamino)-5,6,7,8-tetrahydro-chinazolin-2-yl]-amid,
[178] 4-Fluor-N-(6-phenylmethansutfonyl-5,6,7,8-tetrahydro-pyrido[4,3-d]pyrimidin-2-yl)-benzamid,
[180] Thiophen-2-carbonsäure[6-(2,5-dichlor-thiophen-3-sulfonylamino)-5,6,7,8-tetrahydro-chinazolin-2-yl]-amid,
[181] {5-[2-(4-Fluor-benzoylamino)-5,6,7,8-tetrahydro-chinazolin-6-ylcarbamoyl]-pentyl}-carbaminsäure benzyl ester,
[182] N-[6-(2,5-Dimethyl-2H-pyrazol-3-carbonyl)-5,6,7,8-tetrahydro-pyrido[4,3-d]pyrimidin-2-yl]-4-ethyl-benzamid,
[183] Naphthalin-1-carbonsäure[6-(3-fluor-benzoyl)-5,6,7,8-tetrahydro-pyrido[4,3-d]pyrimidin-2-yl]-amid,
[184] N-[6-(Benzo[b]thiophen-3-carbonyl)-5,6,7,8-tetrahydro-pyrido[4,3-d]pyrimidin-2-yl]-4-tert-butyl-benzamid,
[185] 4-Fluor-N-[6-(4-methoxy-benzolsulfonyl)-5,6,7,8-tetrahydro-pyrido[4,3-d]pyrimidin-2-yl]-benzamid,
[186] 4-tert-Butyl-N-[6-(2,3-dihydro-benzo[1,4]dioxin-6-sulfonyl)-5,6,7,8-tetrahydro-pyrido[4,3-d]pyrimidin-2-yl]-benzamid,
[187] 3-Chlor-N-{6-[2-(2,6-dichlor-phenyl)-acetyl]-5,6,7,8-tetrahydro-pyrido[4,3-d]pyrimidin-2-yl}-benzamid,
[188] Thiophen-2-carbonsäure[6-(2,3-dihydro-benzo[1,4]dioxin-6-sulfonylamino)-5,6,7,8-tetrahydro-chinazolin-2-yl]-amid,
[189] N-{6-[2-(3-Chlor-phenoxy)-acetyl]-5,6,7,8-tetrahydro-pyrido[4,3-d]pyrimidin-2-yl}-4-methl-benzamid,
[190] N-[6-(5-Phenyl-pentanoyl)-5,6,7,8-tetrahydro-pyrido[4,3-d]pyrimidin-2-yl]-benzamid,
[191] 4-Ethyl-N-[6-(2-ethyl-butyryl)-5,6,7,8-tetrahydro-pyrido[4,3-d]pyrimidin-2-yl]-benzamid,
[192] 3,5-Dichlor-N-[6-(4-trifluormethoxy-benzoyl)-5,6,7,8-tetrahydro-pyrido[4,3-d]pyrimidin-2-yl]-benzamid,
[193] Naphthalin-1-carbonsäure [6-(4-methyl-octanoyl)-5,6,7,8-tetrahydro-pyrido[4,3-d]pyrimidin-2-yl]-amid,
[194] 4-[2-(4-Fluor-benzoylamino)-5,6,7,8-tetrahydro-chinazolin-6-ylcarbamoyl]-piperidin-1-carbonsäure tert-butyl ester,
[195] N-[6-(2-Benzyloxy-acetyl)-5,6,7,8-tetrahydro-pyrido[4,3-d]pyrimidin-2-yl]-2-methoxy-benzamid,
[196] N-[6-(2-Trifluormethyl-benzolsulfonylamino)-5,6,7,8-tetrahydro-chinazolin-2-yl]-butyramid,
[197] N-[2-(4-Fluor-benzoylamino)-5,6,7,8-tetrahydro-chinazolin-6-yl]-2-methyl-6-trifluormethyl-nicotinamid,
[198] N-[6-(3-Cyano-benzoyl)-5,6,7,8-tetrahydro-pyrido[4,3-d]pyrimidin-2-yl]-2-trifluormethyl-benzamid,
[199] 3-Methoxy-N-[6-(3-trifluormethoxy-benzoyl)-5,6,7,8-tetrahydro-pyrido[4,3-d]pyrimidin-2-yl]-benzamid,
[200] N-(6-Butyryl-5,6,7,8-tetrahydro-pyrido[4,3-d]pyrimidin-2-yl)-2-fluor-benzamid,
[201] N-(6-Benzolsulfonylamino-5,6,7,8-tetrahydro-chinazolin-2-yl)-4-fluor-benzamid,
[202] N-[6-(5-Chlor-thiophen-2-sulfonylamino)-5,6,7,8-tetrahydro-chinazolin-2-yl]-4-fluor-benzamid,
[203] Naphthalin-1-carbonsäure (6-hexanoyl-5,6,7,8-tetrahydro-pyrido[4,3-d]pyrimidin-2-yl)-amid,
[204] N-(6-Propionyl-5,6,7,8-tetrahydro-pyrido[4,3-d]pyrimidin-2-yl)-2-trifluormethyl-benzamid,
[205] N-[6-(2-Ethyl-butyryl)-5,6,7,8-tetrahydro-pyrido[4,3-d]pyrimidin-2-yl]-4-methyl-benzamid,
[206] 2-Fluor-N-[6-(3-trifluormethyl-benzolsulfonyl)-5,6,7,8-tetrahydro-pyrido[4,3-d]pyrimidin-2-yl]-benzamid,
[207] 5-Benzyl-furan-2-carbonsäure {2-[(thiophen-2-carbonyl)amino]-5,6,7,8-tetrahydro-chinazolin-6-yl}-amid,
[208] Thiophen-2-carbonsäure {6-[2-(2,5-dimethyl-phenyl)-acetylamino]-5,6,7,8-tetrahydro-chinazolin-2-yl}-amid,
[210] 4-Fluor-N-[6-(propan-1-sulfonyl)-5,6,7,8-tetrahydro-pyrido[4,3-d]pyrimidin-2-yl]-benzamid,
[211] 2-[2-(3-Fluor-benzoylamino)-7,8-dihydro-5H-pyrido[4,3-d]pyrimidin-6-sulfonyl]-benzoesäure methyl ester,
[212] 3,5-Dichlor-N-[6-(5-[1,2]dithiolan-3-yl-pentanoyl)-5,6,7,8-tetrahydro-pyrido[4,3-d]pyrimidin-2-yl]-benzamid,
[213] Thiophen-2-carbonsäure [6-(2-phenoxy-butyrylamino)-5,6,7,8-tetrahydro-chinazolin-2-yl]-amid,
[214] Thiophen-2-carbonsäure {6-[2-(4-methoxy-phenyl)acetylamino]-5,6,7,8-tetrahydro-chinazolin-2-yl}-amid,
[215] N-(6-Cyclohexancarbonyl-5,6,7,8-tetrahydro-pyrido[4,3-d]pyrimidin-2-yl)-2-trifluormethyl-benzamid, .
[216] N-[6-(2,4-Dimethyl-thiazol-5-sulfonyl)-5,6,7,8-tetrahydro-pyrido[4,3-d]pyrimidin-2-yl]-2-trifluormethyl-benzamid,
[217] 2-Thiophen-2-yl-thiazol-4-carbonsäure {2-[(thiophen-2-carbonyl)-amino]-5,6,7,8-tetrahydro-chinazolin-6-yl}-amid,
[219] Thiophen-2-carbonsäure {6-[3-(3-trifluormethyl-phenyl)-acryloylamino]-5,6,7,8-tetrahydro-chinazolin-2-yl}-amid,
[220] Thiophen-2-carbonsäure [6-(5-phenyl-pentanoylamino)-5,6,7,8-tetrahydro-chinazolin-2-yl]-amid,
[221] Thiophen-2-carbonsäure[6-(2-chlor-5-trifluormethy-benzoylamino)-5,6,7,8-tetrahydro-chinazolin-2-yl]-amid,
[222] 4-Fluor-N-[6-(2,3,5,6-tetramethyl-benzolsulfonylamino)-5,6,7,8-tetrahydro-chinazolin-2-yl]-benzamid,
[223] 4-Fluor-N-[6-(4-methyl-octanoyl)-5,6,7,8-tetrahydro-pyrido[4,3-d]pyrimidin-2-yl]-benzamid,
[224] 2-{2-[(Thiophen-2-carbonyl)-amino]-5,6,7,8-tetrahydro-chinazolin-6-ylsulfamoyl}-benzoesäure methyl ester,
[225] [((3-Methyl-1-{2-[(thiophen-2-carbonyl)-amino]-5,6,7,8-tetrahydro-chinazolin-6-ylcarbamoyl}-butylcarbamoyl)-methyl]-carbaminsäure benzyl ester,
[226] 3-Chlor-N-(6-pentanoyl-5,6,7,8-tetrahydro-pyrido[4,3-d]pyrimidin-2-yl)-benzamid,
[227] 4-Fluor-N-[6-(5-oxo-5-phenyl-pentanoylamino)-5,6,7,8-tetrahydro-chinazolin-2-yl]-benzamid,
[228] Thiophen-2-carbonsäure [6-(3-phenyl-acryloylamino)-5,6,7,8-tetrahydro-chinazolin-2-yl]-amid,
[229] N-[6-(5-[1,2]Dithiolan-3-yl-pentanoylamino)-5,6,7,8-tetrahydro-chinazolin-2-yl]-4-fluor-benzamid,
[230] Benzoesäure 2-(2-{2-[(thiophen-2-carbonyl)-amino]-5,6,7,8-tetrahydro-chinazolin-6-ylcarbamoyl}-ethyl)-phenyl ester,
[231] 4-Fluor-N-[6-(2-methyl-5-nitro-benzolsulfonylamino)-5,6,7,8-tetrahydro-chinazolin-2-yl]-benzamid,
[232] 4-Chlor-N-[6-(2-phenoxy-acetyl)-5,6,7,8-tetrahydro-pyrido[4,3-d]pyrimidin-2-yl]-benzamid,
[234] N-[6-(5-Brom-thiophen-2-carbonyl)-5,6,7,8-tetrahydro-pyrido[4,3-d]pyrimidin-2-yl]-2-fluor-benzamid,
[235] 4-Fluor-N-[6-(5-fluor-2-methyl-benzolsulfonylamino)-5,6,7,8-tetrahydro-chinazolin-2-yl]-benzamid,
[236] N-[6-(4-Acetylamino-benzolsulfonylamino)-5,6,7,8-tetrahydro-chinazolin-2-yl]-4-fluor-benzamid,
[237] Thiophen-2-carbonsäure[6-(2-trifluormethyl-benzoylamino)-5,6,7,8-tetrahydro-chinazolin-2-yl]-amid,
[238] 3,4-Difluor-N-[6-(chinolin-6-carbonyl)-5,6,7,8-tetrahydro-pyrido[4,3-d]pyrimidin-2-yl]-benzamid,
[239] {1-[2-(4-Fluor-benzoylamino)-5,6,7,8-tetrahydro-chinazolin-6-ylcarbamoyl]-2-phenyl-ethyl}-carbaminsäure tert-butyl ester,
[240] Thiophen-2-carbonsäure [6-(2-methyl-benzoylamino)-5,6,7,8-tetrahydro-chinazolin-2-yl]-amid,
[241] 3-Chlor-N-{6-[3-(2-oxo-benzooxazol-3-yl)propionyl]-5,6,7,8-tetrahydro-pyrido[4,3-d]pyrimidin-2-yl}-benzamid,
[242] 3-Chlor-N-[6-(5-chlor-thiophen-2-sulfonyl)-5,6,7,8-tetrahydro-pyrido[4,3-d]pyrimidin-2-yl]-benzamid,
[243] 3,5-Dichlor-N-[6-(4-methoxy-benzolsulfonyl)-5,6,7,8-tetrahydro-pyrido[4,3-d]pyrimidin-2-yl]-benzamid,
[244] 4-tert-Butyl-N-[6-(5-methyl-isoxazol-3-carbonyl)-5,6,7,8-tetrahydro-pyrido[4,3-d]pyrimidin-2-yl]-benzamid,
[245] N-[6-(5-Brom-2-methoxy-benzolsulfonyl)-5,6,7,8-tetrahydro-pyrido[4,3-d]pyrimidin-2-yl]-4-methyl-benzamid,
[246] 4-tert-Butyl-N-{6-[3-(2-hydroxy-phenyl)-propionyl]-5,6,7,8-tetrahydro-pyrido[4,3-d]pyrimidin-2-yl}-benzamid,
[247] 4-Fluor-N-[6-(2-phenyl-butyrylamino)-5,6,7,8-tetrahydro-chinazolin-2-yl]-benzamid,
[248] 4-tert-Butyl-N-[6-(4-propyl-benzoyl)-5,6,7,8-tetrahydro-pyrido[4,3-d]pyrimidin-2-yl]-benzamid,
[249] Thiophen-2-carbonsäure [6-(2-brom-benzoylamino)-5,6,7,8-tetrahydro-chinazolin-2-yl]-amid,
[250] 2-Methoxy-N-[6-(6-phenoxy-pyridin-3-carbonyl)-5,6,7,8-tetrahydro-pyrido[4,3-d]pyrimidin-2-yl]-benzamid,
[251] N-[6-(4-Acetylamino-benzolsulfonyl)-5,6,7,8-tetrahydro-pyrido[4,3-d]pyrimidin-2-yl]-4-ethyl-benzamid,
[252] 3,5-Dichlor-N-[6-(4-fluor-benzolsulfonyl)-5,6,7,8-tetrahydro-pyrido[4,3-d]pyrimidin-2-yl]-benzamid,
[253] N-[6-(1-Benzolsulfonyl-1H-indol-2-carbonyl)-5,6,7,8-tetrahydro-pyrido[4,3-d]pyrimidin-2-yl]-4-fluor-benzamid,
[255] N-[6-(3-Chlor-benzolsulfonylamino)-5,6,7,8-tetrahydro-chinazolin-2-yl]-4-fluor-benzamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung substituierter 5,6,7,8-Tetrahydro-pyrido[4,3-d]pyrimidin-2-yl-Verbindungen der vorstehend angegebenen allgemeinen Formel I, worin X für eine NR^{2a}-Gruppe steht, gemäß dem ein N-geschütztes Piperidin-4-on der allgemeinen Formel II, 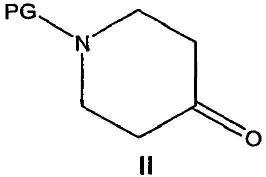 worin PG für eine Schutzgruppe steht, durch Umsetzung mit Dimethoxy-methyl-dimethyl-amin der Formel III 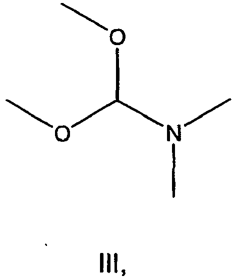 in einem organischen Reaktionsmedium in eine Verbindung der allgemeinen Formel IV, 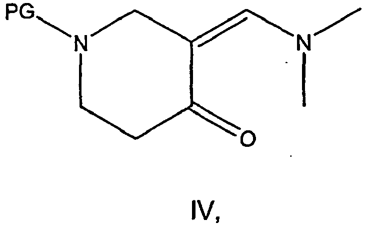 worin PG die vorstehend genannte Bedeutung hat, überführt wird, diese ggf. gereinigt und/oder isoliert wird, und durch Umsetzung mit Guanidin der Formel V, 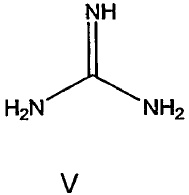 ggf. in Form eines entsprechenden Salzes, in einem organischen Reaktionsmedium, ggf. in Gegenwart wenigstens einer Base in eine Verbindung der allgemeinen Formel VI, 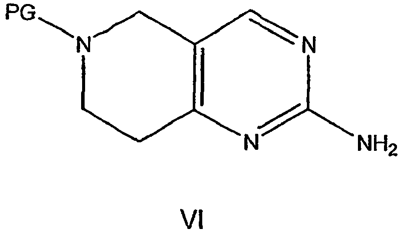 worin PG die vorstehend genannte Bedeutung hat, überführt wird, diese ggf. gereinigt und/oder isoliert wird, und in einem organischen Reaktionsmedium, ggf. in Gegenwart wenigstens einer Base und/oder wenigstens eines Katalysators, mit einer Verbindung der allgemeinen Formel R³-C(=O)-X¹ oder einer Verbindung der allgemeinen Formel (R³-C(=O))₂O, oder durch Umsetzung mit einer Verbindung der allgemeinen Formel R³-C(=O)-OH in einem organischen Reaktionsmedium in Gegenwart wenigstens eines Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Anwesenheit wenigstens einer Base, wobei R³ und R⁴ jeweils die vorstehend angegebene Bedeutung haben und X¹ und X^{1a} jeweils für eine Austrittsgruppe stehen, zu einer Verbindung der allgemeinen Formel VII, 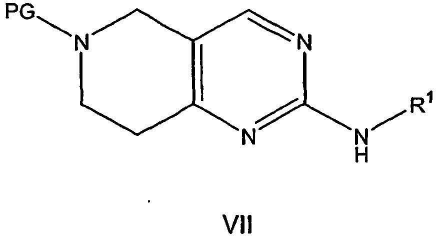 worin PG und R¹ die vorstehend genannte Bedeutung haben, umgesetzt wird, diese ggf. gereinigt und/oder isoliert wird, und durch Abspaltung der Schutzgruppe PG in eine entsprechend substituierte Verbindung der allgemeinen Formel VIII, 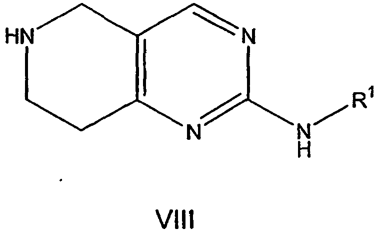 ggf. in Form eines entsprechenden Salzes überführt wird, diese ggf. gereinigt und/oder isoliert wird, und durch Umsetzung mit einer Verbindung der allgemeinen Formel R⁵-C(=O)-X² oder (R⁵-C(=O))₂O, worin R⁵ jeweils die vorstehend genannte Bedeutung hat und X² für eine Austrittsgruppe steht, in einem organischen Reaktionsmedium, ggf. in Gegenwart wenigstens einer Base und/oder in Gegenwart wenigstens eines Katalysators,
oder durch Umsetzung mit einer Verbindung der allgemeinen Formel R⁶-SO₂-X³, worin R⁶ die vorstehend genannte Bedeutung hat und X³ für eine Austrittsgruppe steht, in einem organischen Reaktionsmedium, ggf. in Gegenwart wenigstens einer Base, oder durch Umsetzung mit einer Verbindung der allgemeinen Formel R⁵-COOH, worin R⁵ die vorstehend genannte Bedeutung hat, in einem organischen Reaktionsmedium, in Gegenwart wenigstens eines geeigneten Kopplungsmittels, ggf. in Gegenwart wenigstens einer Base, in eine Verbindung der allgemeinen Formel IX 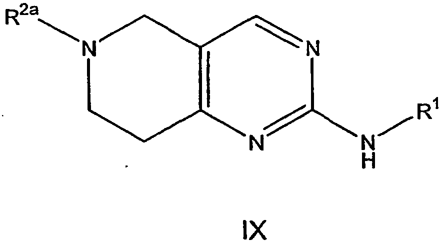 überführt, worin R¹ und R^{2a} die vorstehend genannte Bedeutung haben, diese ggf. reinigt und/oder isoliert, und ggf. in ein entsprechendes Salz überführt und dieses ggf. reinigt und/oder isoliert, oder Piperidin-4-on der allgemeinen Formel X 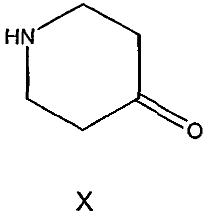 ggf. in Form eines entsprechenden Salzes, durch Umsetzung mit einer Verbindung der allgemeinen Formel R⁵-C(=O)-X² oder (R⁵-C(=O))₂O, worin R⁵ jeweils die vorstehend genannte Bedeutung hat und X² für eine Austrittsgruppe steht, in einem organischen Reaktionsmedium, ggf. in Gegenwart wenigstens einer Base und/oder in Gegenwart wenigstens eines Katalysators, oder durch Umsetzung mit einer Verbindung der allgemeinen Formel R⁶-SO₂-X³, worin R⁶ die vorstehend genannte hat und X³ für eine Austrittsgruppe steht, in einem organischen Reaktionsmedium, ggf. in Gegenwart wenigstens einer Base, oder durch Umsetzung mit einer Verbindung der allgemeinen Formel R⁵-COOH, worin R⁵ die vorstehend genannte hat, in einem organischen Reaktionsmedium, in Gegenwart eines geeigneten Kopplungsmittels, ggf. in Gegenwart wenigstens einer Base, in eine Verbindung der allgemeinen Formel XI, 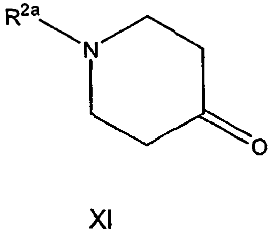 worin R^{2a} die vorstehend genannte Bedeutung hat, diese ggf. gereinigt und/oder isoliert wird, und durch Umsetzung mit Dimethoxy-methyl-dimethyl-amin der Formel III 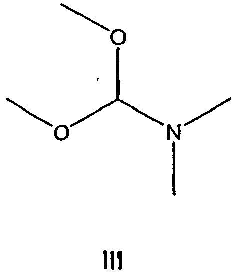 in einem organischen Reaktionsmedium in eine Verbindung der allgemeinen Formel XII, 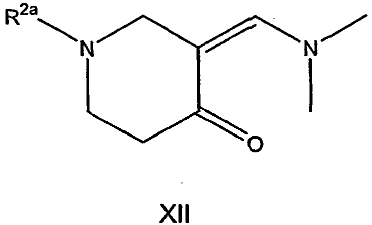 überführt wird, worin R^{2a} die vorstehend genannte Bedeutung hat, diese ggf. gereinigt und/oder isoliert wird, und mit Guanidin der Formel V, 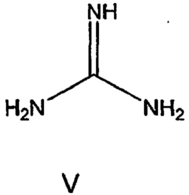 ggf. in Form eines entsprechenden Salzes, in einem organischen Reaktionsmedium, ggf. in Gegenwart wenigstens einer Base, in eine Verbindung der allgemeinen Formel XIII 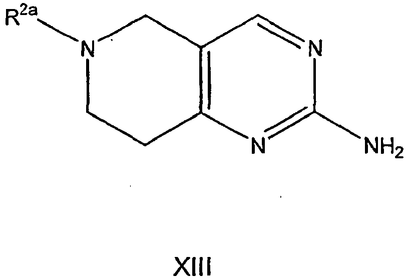 überführt wird, diese ggf. gereinigt und/oder isoliert wird, und in einem organischen Reaktionsmedium, ggf. in Gegenwart wenigstens einer Base und/oder wenigstens eines Katalysators, mit einer Verbindung der allgemeinen Formel R³-(C=O)-X¹ oder einer Verbindung der allgemeinen Formel (R³-C(=O))₂O, oder durch Umsetzung mit einer Verbindung der allgemeinen Formel R³-COOH in einem organischen Reaktionsmedium in Gegenwart wenigstens eines Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Anwesenheit wenigstens einer Base, wobei R³ und R⁴ jeweils die vorstehend genannte Bedeutung haben und X¹ und X^{1a} für eine Austrittsgruppe stehen, zu einer Verbindung der allgemeinen Formel XIV 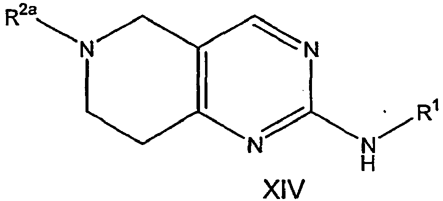 worin R¹ und R^{2a} die vorstehend genannte Bedeutung haben, umsetzt, und diese ggf. reinigt und/oder isoliert, und diese ggf. in ein entsprechendes Salz überführt und dieses Salz ggf. reinigt und/oder isoliert.

Das erfindungsgemäße Verfahren zur Herstellung substituierter 5,6,7,8-Tetrahydropyrido[4,3-d]pyrimidin-2-yl-Verbindungen der vorstehend angegebenen allgemeinen Formel I, worin X für eine -NR^{2a}-Gruppe steht ausgehend von N-geschütztem Piperidin-4-on ist auch im nachfolgenden Schema I wiedergegeben.

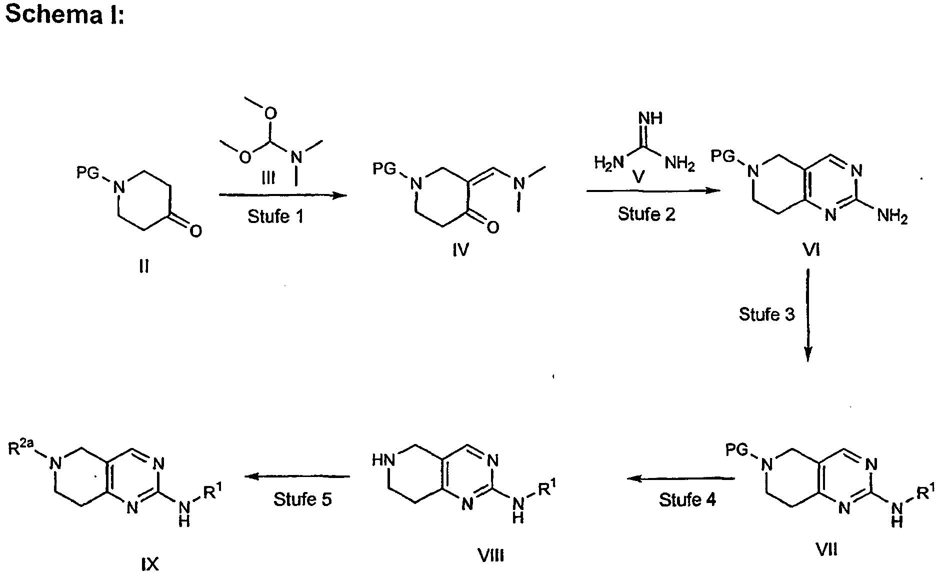

In Stufe 1 wird Piperidin-4-on, das am Stickstoffatom mit einer Schutzgruppe (PG) ausgerüstet ist, zunächst mit Dimethoxymethyl-dimethyl-amin in einem organischen Reaktionsmedium, vorzugsweise Toluol und/oder Benzol zu wenigstens einer Verbindung der vorstehend angegebenen allgemeinen Formel IV umgesetzt, welche vorzugsweise ohne weitere Aufarbeitung in Stufe 2 eingesetzt wird.

Dimethoxymethyl-dimethyl-amin, Piperidin-4-on sowie N-geschütztes Piperidon sind am Markt käuflich erhältlich, können aber auch nach üblichen, dem Fachmann bekannten Methoden hergestellt werden. Als geeignete Schutzgruppen für das Piperidon kommen beispielsweise Trifluoracetamid, Methoxycarbonyl, Ethoxycarbonyl, tert-Butoxycarbonyl, Benzyloxycarbonyl, Allyloxycarbonyl oder 9-Fluorenylmethoxycarbonyl in Betracht. Geeignete Reagenzien und Methoden zur Einführung dieser Schutzgruppen sind dem Fachmann bekannt.

In Stufe 2 wird die jeweilige Verbindung der allgemeinen Formel IV ggf. in Gegenwart wenigstens einer organischen Base und/oder wenigstens einer metallorganischen Base, vorzugsweise in Gegenwart von Natriumethanolat und/oder Natriummethanolat, in einem organischen Reaktionsmedium, vorzugsweise Methanol und/oder Ethanol, mit Guanidin der Formel V, ggf. in Form eines entsprechenden Salzes wie beispielsweise Guanidin-Hydrochlorid, zu der jeweiligen Verbindung der vorstehend angegebenen allgemeinen Formel VI umgesetzt.

Guanidin und dessen entsprechende Salze sind käuflich am Markt erhältlich, können aber auch nach üblichen, dem Fachmann bekannten Verfahren hergestellt werden.

In Stufe 3 wird die jeweilige Verbindung der allgemeinen Formel VI unter üblichen, dem Fachmann bekannten Bedingungen in einem organischen Reaktionsmedium wie beispielsweise Pyridin durch eine Acylierung mit wenigstens einer Verbindung der allgemeinen Formel R³-C(=O)-X¹ oder wenigstens einer Verbindung der allgemeinen Formel (R³-C(=O))₂O, ggf. in Gegenwart wenigstens einer organischen Base und/oder wenigstens einer anorganischen Base, die vorzugsweise ausgewählt werden kann aus der Gruppe bestehend aus Diisopropylethylamin, Triethylamin, Pyridin und Diethylamin, ggf. in Anwesenheit wenigstens eines Katalysators, vorzugsweise in Gegenwart von Dimethylaminopyridin (DMAP), zu der jeweiligen Verbindung der allgemeinen Formel VII umgesetzt.

Alternativ kann die Umsetzung der jeweiligen Verbindung der allgemeinen Formel VI auch mit wenigstens einer Carbonsäure der allgemeinen Formel R³-COOH, worin R³ jeweils die vorstehend genannte Bedeutung hat, in einem organischen Reaktionsmedium, vorzugsweise ausgewählt aus der Gruppe bestehend aus Dichlormethan, DMF, THF und Acetonitril, ggf. in Gegenwart wenigstens eines üblichen, dem Fachmann bekannten Kopplungsmittels, vorzugsweise ausgewählt aus der Gruppe bestehend aus N,N'-Carbonyldiimidazol (CDI), N,N'-Dicyclohexylcarbodiimid (DCC) und N-Ethyl-N'-[3-(dimethylamino)-propyl]-carbodiimid (EDCI), oder in Gegenwart von 1-Hydroxy-7-azabenzotriazol (HOAt) und wenigstens einer organischen Base wie beispielsweise Diisopropylethylamin (DIPEA) erfolgen.

Als weitere Alternative kommt die Umsetzung mit wenigstens einer Verbindung der allgemeinen Formel R⁴-O-C(=O)-X^{1a}, worin R⁴ die vorstehend genannte Bedeutung hat und X^{1a} für eine Austrittsgruppe steht, in einem organischen Reaktionsmedium wie beispielsweise Acetonitril, DMF oder Dichlormethan, ggf. in Gegewart wenigstens einer Base beispielsweise ausgewählt aus der Gruppe bestehend aus wässrigem Kaliumcarbonat, Natriumhydrogencarbonat, Natronlauge, Diisopropylethylamin, Triethylamin, Pyridin und Diethylamin in Betracht.

Die jeweilige Verbindung der allgemeinen Formel VII wird durch Abspaltung der jeweiligen Schutzgruppe (PG) in Stufe 4 unter üblichen, dem Fachmann bekannten Bedingungen in die jeweilige Verbindung der vorstehend angegebenen allgemeinen Formel VIII, ggf. in Form eines entsprechenden Salzes wie beispielsweise des Trifluoracetates überführt.

Beispielsweise kann die Abspaltung der vorstehend genannten Schutzgruppen in Gegenwart wenigstens einer anorganischen Base, Säure oder Lewis-Säure, wie beispielsweise Kaliumcarbonat, Lithiumhydroxid, Kaliumhydroxid, Schwefelsäure, Bromwasserstoffsäure, Flusssäure, Salzsäure, Bortrifluoridetherat oder Bortrichlorid, wenigstens einer organischen Säure wie beispielsweise Trifluoressigsäure, Trifluormethansulfonsäure oder Essigsäure, oder in Gegenwart wenigstens einer organischen Base, wie beispielsweise Morpholin, Triethylamin, Diethylamin, Diisopropylethylamin oder Pyridin oder durch Hydrierung erfolgen.

Die jeweilige Verbindung der vorstehend angegebenen allgemeinen Formel VIII wird in Stufe 5 unter üblichen, dem Fachmann bekannten Bedingungen in einem organischen Reaktionsmedium wie beispielsweise Pyridin durch Acylierung mit wenigtens einer Verbindung der allgemeinen Formel R⁵-C(=O)-X² oder wenigstens einer Verbindung der allgemeinen Formel (R⁵-C(=O))₂O, worin R⁵ jeweils die vorstehend genannte Bedeutung hat und X² für eine übliche, dem Fachmann geläufige Austrittsgruppe, vorzugsweise für ein Halogen wie beispielsweise Chlor, steht, ggf. in Gegenwart wenigstens einer organischen Base und/oder wenigstens einer anorganischen Base, vorzugsweise wenigstens einer Base ausgewählt aus der Gruppe bestehend aus Diisopropylethylamin, Triethylamin, Pyridin und Diethylamin, gegebenenfalls in Anwesenheit wenigstens eines Katalysators, vorzugsweise Dimethylaminopyridin (DMAP), oder durch Sulfonylierung mit wenigstens einer Sulfonylverbindung der allgemeinen Formel R⁶-S(=O)₂-X³, worin R⁶ jeweils die vorstehend genannte Bedeutung hat und X³ für eine übliche, dem Fachmann geläufige Austrittsgruppe, vorzugsweise für ein Halogen wie beispielsweise Chlor, steht, ggf. in Gegenwart wenigstens einer organischen Base und/oder wenigstens einer anorganischen Base, vorzugsweise wenigstens einer Base ausgewählt aus der Gruppe bestehend aus Natriumhydrogencarbonat, Diisopropylethylamin, Triethylamin, Pyridin und Diethylamin, oder durch Umsetzung mit wenigstens einer Carbonsäure der allgemeinen Formel R⁵-COOH, worin R⁵ jeweils die vorstehend genannte Bedeutung hat, in einem organischen Reaktionsmedium, vorzugsweise ausgewählt aus der Gruppe bestehend aus Dichlormethan, DMF, THF und Acetonitril, ggf. in Gegenwart wenigstens eines üblichen, dem Fachmann bekannten Kopplungsmittels, vorzugsweise ausgewählt aus der Gruppe bestehend aus N,N'-Carbonyldiimidazol (CDI), N,N'-Dicyclohexylcarbodiimid (DCC) und N-Ethyl-N'-[3-(dimethylamino)-propyl]-carbodiimid (EDCI), oder in Gegenwart von 1-Hydroxy-7-azabenzotriazol (HOAt) und wenigstens einer organischen Base wie beispielsweise Diisopropylethylamin (DIPEA) zu der jeweiligen substituierter 5,6,7,8-Tetrahydro-pyrido[4,3-d]pyrimidin-2-yl-Verbindung der vorstehend angegebenen allgemeinen Formel I umgesetzt.

Alternativ werden die erfindungsgemäßen substituierten 5,6,7,8-Tetrahydro-pyrido[4,3-d]pyrimidin-2-yl-Verbindungen der vorstehend angegebenen allgemeinen Formel I, in denen X für eine -NR^{2a}-Gruppe steht, ausgehend von Piperidin-4-on hergestellt. Das entsprechende Verfahren ist in Schema II dargestellt.

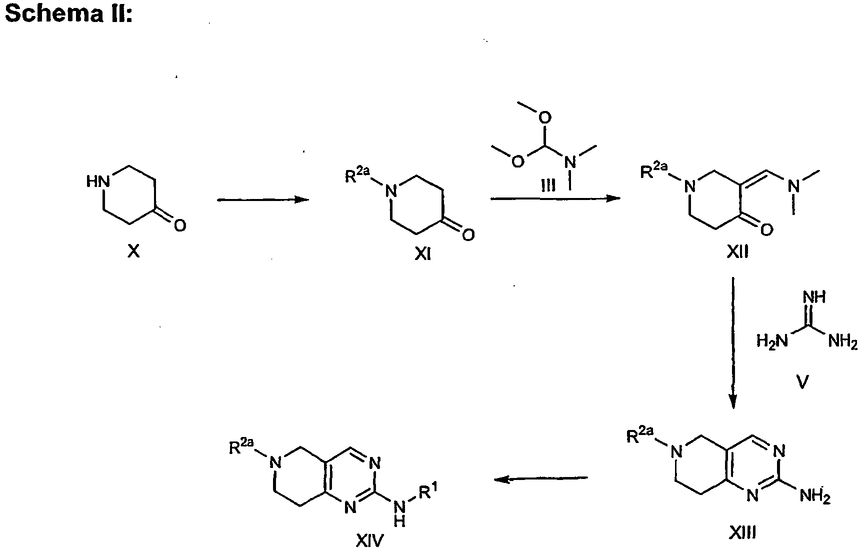

Die jeweilige Verbindung der vorstehend angegebenen allgemeinen Formel XI wird ausgehend von Piperidin-4-on der allgemeinen Formel X, ggf. in Form eines entsprechenden Salzes wie beispielweise des Hydrochlorids, unter üblichen, dem Fachmann bekannten Bedingungen in einem organischen Reaktionsmedium wie beispielsweise Pyridin durch Acylierung mit wenigstens einer Verbindung der allgemeinen Formel R⁵-C(=O)-X² oder (R⁵-C(=O))₂O worin R⁵ jeweils die vorstehend genannte Bedeutung hat und X² für eine übliche, dem Fachmann geläufige Austrittsgruppe, vorzugsweise für ein Halogen wie beispielsweise Chlor, steht, ggf. in Gegenwart wenigstens einer organischen Base und/oder wengistens einer anorganischen Base, vorzugsweise wenigstens einer Base ausgewählt aus der Gruppe bestehend aus Diisopropylethylamin, Triethylamin, Pyridin und Diethylamin, gegebenenfalls in Anwesenheit wenigstens eines Katalysators, vorzugsweise DMAP, oder durch Sulfonylierung mit wenigstens einer Verbindung der allgemeinen Formel R⁶-S(=O)₂-X³, worin R⁶ die vorstehend genannte Bedeutung hat und X³ für eine übliche, dem Fachmann geläufige Austrittsgruppe, vorzugsweise für ein Halogen wie beispielsweise Chlor, steht, in Gegenwart wenigstens einer organischen Base und/oder wenigstens einer anorganischen Base, vorzugsweise wenigstens einer Base ausgewählt aus der Gruppe bestehend aus Natriumhydrogencarbonat, Diisopropylethylamin, Triethylamin, Pyridin und Diethylamin, oder durch Umsetzung mit einer Carbonsäure der allgemeinen Formel R⁵-COOH in einem organischen Reaktionsmedium, vorzugsweise ausgewählt aus der Gruppe bestehend aus Dichlormethan, DMF, THF und Acetonitril, in Gegenwart eines üblichen, dem Fachmann bekannten Kopplungsmittels, vorzugsweise ausgewählt aus der Gruppe bestehend aus CDI, DCC und EDCI, oder ggf. in Gegenwart von HOAt und wenigstens einer organischen Base wie beispielsweise DIPEA erhalten.

Die so erhaltene Verbindung der vorstehend angegebenen allgemeinen Formel XI wird anschließend mit Dimethoxy-methyl-dimethyl-amin der Formel III in einem organischen Reaktionsmedium, vorzugsweise Toluol und/oder Benzol, zu der jeweiligen Verbindung der vorstehend angegebenen allgemeinen Formel XII umgesetzt, die vorzugsweise ohne weitere Aufarbeitung in Gegenwart wenigstens einer organischen Base und/oder wenigstens einer metallorganischen Base, vorzugsweise Natriumethanolat und/oder Natriummethanolat, in einem organischen Reaktionsmedium, vorzugsweise Methanol und/oder Ethanol, mit Guanidin der vorstehend angegebenen Formel V, ggf. in Form eines entsprechenden Salzes wie beispielsweise Guanidin-Hydrochlorid, zu der jeweiligen Verbindung der vorstehend angegebenen allgemeinen Formel XIII überführt wird.

Die Umsetzung der jeweiligen Verbindung der allgemeinen Formel XIII mit wenigstens einer Verbindung der allgemeinen Formel R⁴-O-(C=O)-X^{1a} oder wenigstens einer Verbindung der allgemeinen Formel (R³-C(=O))₂O oder einer Verbindung der allgemeinen Formel R³-C(=O)-X¹, worin R³ und R⁴ jeweils die vorstehend genannte Bedeutung haben und X¹ und X^{1a} für eine übliche, dem Fachmann geläufige Austrittsgruppe, vorzugsweise für ein Halogen wie beispielsweise Chlor, in Gegenwart wengistens einer Base, vorzugsweise ausgewählt aus der Gruppe bestehend aus wässrigem Kaliumcarbonat, Natriumhydrogencarbonat, Natronlauge, Diisopropylethylamin, Triethylamin, Pyridin, Diethylamin, ggf. in einem organischen Reaktionsmedium, vorzugsweise ausgewählt aus der Gruppe bestehend aus Acetonitril, DMF und Dichlormethan führt dann zu der jeweiligen substituierten 5,6,7,8-Tetrahydro-pyrido[4,3-d]pyrimidin-2-yl-Verbindungen der vorstehend angegebenen allgemeinen Formel I, worin X für eine -NR^{2a}-Gruppe steht und R¹ die vorstehend genannte Bedeutung hat.

Alternativ kann die Umsetzung der jeweiligen Verbindung der allgemeinen Formel XIII auch mit wenigstens einer Carbonsäure der allgemeinen Formel R³-COOH, worin R³ jeweils die vorstehend genannte Bedeutung hat, in einem organischen Reaktionsmedium, vorzugsweise ausgewählt aus der Gruppe bestehend aus Dichlormethan, DMF, THF und Acetonitril, ggf. in Gegenwart wenigstens eines üblichen, dem Fachmann bekannten Kopplungsmittels, vorzugsweise ausgewählt aus der Gruppe bestehend aus N,N'-Carbonyldiimidazol (CDI), N,N'-Dicyclohexylcarbodiimid (DCC) und N-Ethyl-N'-[3-(dimethylamino)propyl]-carbodiimid (EDCI), oder in Gegenwart von 1-Hydroxy-7-azabenzotriazol (HOAt) und wenigstens einer organischen Base wie beispielsweise Diisopropylethylamin (DIPEA) erfolgen.

Ebenfalls Gegenstand der vorliegenden Erfindung ist ein Verfahren zur Herstellung substituierter 5,6,7,8-Tetrahydro-chinazolin-2-yl-Verbindungen der vorstehend angegebenen allgemeinen Formel I, worin X für eine C(H)(NHR²)-Gruppe steht, gemäß dem 1,4-Dioxa-spiro[4.5]dec-8-yl-amin der Formel C 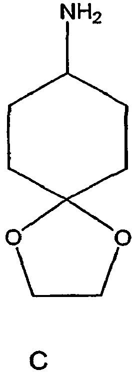 durch Umsetzung mit einer Verbindung der allgemeinen Formel R⁵-C(=O)-X⁴ oder (R⁵-C(=O))₂O, worin R⁵ jeweils die vorstehend genannte Bedeutung hat und X⁴ für eine Austrittsgruppe steht, in einem organischen Reaktionsmedium, ggf. in Gegenwart wenigstens einer Base und/oder in Gegenwart wenigstens eines Katalysators, oder durch Umsetzung mit einer Verbindung der allgemeinen Formel R⁶-SO₂-X⁵, worin R⁶ die vorstehend genannte Bedeutung hat und X⁵ für eine Austrittsgruppe steht, in einem organischen Reaktionsmedium, ggf. in Gegenwart wenigstens einer Base, oder durch Umsetzung mit einer Verbindung der allgemeinen Formel R⁵-COOH, worin R⁵ die vorstehend genannte Bedeutung hat, in einem organischen Reaktionsmedium, in Gegenwart eines geeigneten Kopplungsmittels, ggf. in Gegenwart wenigstens einer Base, in eine Verbindung der allgemeinen Formel D 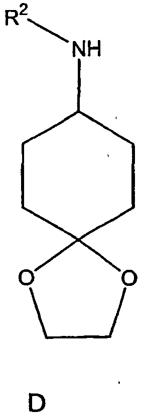 überführt wird, worin R² die vorstehend genannte Bedeutung hat, diese ggf. gereinigt und/oder isoliert wird, und diese durch Abspaltung der Acetal-Gruppe in einem organischen Reaktionsmedium in Gegenwart von Wasser und wenigstens einer Säure in eine Verbindung der allgemeinen Formel E, 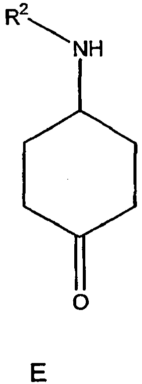 worin R² die vorstehend genannte Bedeutung hat, überführt wird, und diese durch Einführung einer Schutzgruppe (PG¹) in einem organischen Reaktionsmedium in die Verbindung der allgemeinen Formel F 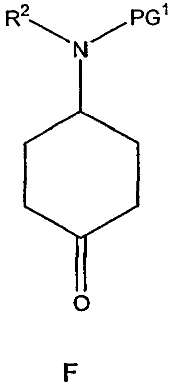 überführt wird, worin R² die vorstehend genannte Bedeutung hat und PG¹ für eine Schutzgruppe steht, diese ggf. gereinigt und/oder isoliert wird, und diese durch Umsetzung mit Dimethoxymethyl-dimethyl-amin der allgemeinen Formel III 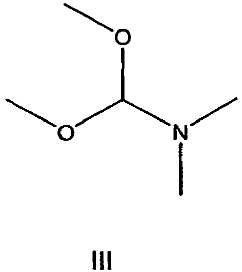 in einem organischen Reaktionsmedium in eine Verbindung der allgemeinen Formel G 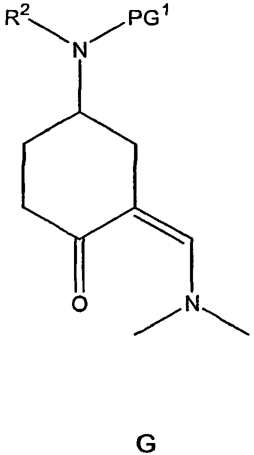 worin R² und PG¹ die vorstehend genannte Bedeutung haben, überführt wird und diese ggf. gereinigt und/oder isoliert wird, und ggf. in Gegenwart wenigstens einer Base in einem organischen Reaktionsmedium mit einer Guanidin-Verbindung der Formel V, ggf. in Form eines entsprechenden Salzes, 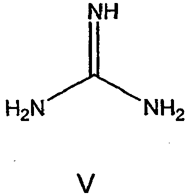 in eine Verbindung der allgemeinen Formel H 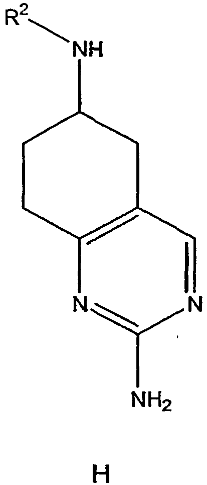 überführt wird, diese ggf. gereinigt und/oder isoliert wird, und diese durch Einführung einer Schutzgruppe (PG2) in einem organischen Reaktionsmedium in eine Verbindung der allgemeinen Formel I 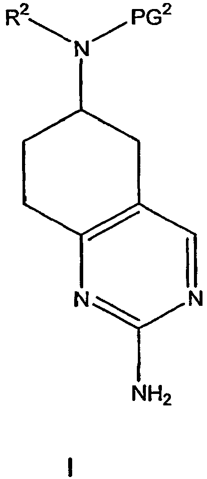 überführt wird, worin R² und PG² die vorstehend genannte Bedeutung haben, diese ggf. gereinigt und/oder isoliert wird, und diese durch Umsetzung mit einer Verbindung der allgemeinen Formel R³-C(=O)-X⁶ oder einer Verbindung der allgemeinen Formel (R³-C(=O))₂O, worin R³ jeweils die vorstehend genannte Bedeutung hat und X⁶ für eine Austrittsgruppe steht, oder durch Umsetzung mit einer Verbindung der allgemeinen Formel R³COOH, worin R³ die vorstehend genannte Bedeutung hat, in einem organischen Reaktionsmedium in Gegenwart eines Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Gegenwart einer Base, in eine Verbindung der allgemeinen Formel J 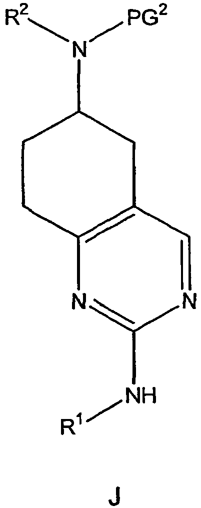 überführt, worin R² und PG² die vorstehend genannte Bedeutung haben und R¹ die vorstehend genannte Bedeutung hat, diese ggf. reinigt und/oder isoliert, und diese durch Abspaltung der Schutzgruppe in eine 5,6,7,8-Tetrahydro-chinazolin-2-yl-Verbindung der allgemeinen Formel I, worin X für eine C(H)(NHR²)-Gruppe steht, überführt, diese ggf. reinigt und/oder isoliert und anschließend ggf. in ein entsprechendes Salz überführt, und dieses ggf. reinigt und/oder isoliert.

Das erfindungsgemäße Verfahren zur Herstellung substituierter 5,6,7,8-Tetrahydro-chinazolin-2-yl-Verbindungen der vorstehend angegebenen allgemeinen Formel I, worin X für eine C(H)(NHR²)-Gruppe steht, ist auch im nachfolgenden Schema III wiedergegeben.

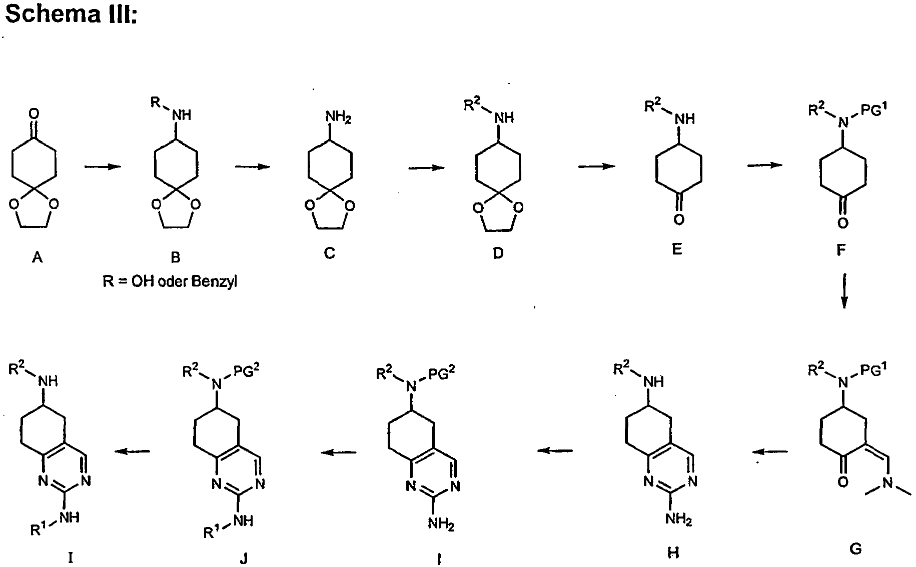

1,4-Dioxa-spiro[4.5]dec-8-yl-amin der Formel C kann nach üblichen, dem Fachmann bekannten Methoden hergestellt werden, beispielsweise ausgehend von 1,4-Dioxaspiro[4.5]decan-8-on, das über eine reduktive Aminierung mit Benzylamin in einem organischen Reaktionsmedium wie beispielsweise Dichlormethan in Gegenwart von Natriumcyanoborhydrid oder Natriumtriacetoxyborhydrid oder durch Umsetzung mit Hydroxylaminhydrochlorid in Gegenwart einer organischen Base wie Triethylamin oder Amberlyst in einem organischen Reaktionsmedium wie Ethanol in die Verbindung der allgemeinen Formel B überführt wird, worin R für eine Hydroxy-Gruppe oder einen Benzyl-Rest steht, die ggf. gereinigt und/oder isoliert wird, und in Gegenwart wenigstens eines Katalysators wie Palladium auf Aktivkohle (Pd(C)) durch Hydriderung oder durch Reduktion mit wenigstens einem Metallhydrid wie beispielsweise Lithiumaluminiumhydrid in einem organischen Reaktionsmedium, vorzugsweise Ethanol und/oder THF zu 1,4-Dioxa-spiro[4.5]dec-8-yl-amin der Formel C umgesetzt wird.

Die Verbindung 1,4-Dioxa-spiro[4.5]dec-8-yl-amin der Formel C wird dann unter üblichen, dem Fachmann bekannten Bedingungen in einem organischen Reaktionsmedium wie beispielsweise Pyridin durch Acylierung mit einer Verbindung der allgemeinen Formel R⁵-C(=O)-X⁴ oder (R⁵-C(=O))₂O, worin R⁵ die vorstehend genannte Bedeutung hat, und X⁴ für eine übliche, dem Fachmann bekannte Austrittsgruppe, vorzugsweise für Halogen, besonders bevorzugt für Chlor, steht, ggf. in Gegenwart wenigstens einer organischen Base und/oder wenigstens einer anorganischen Base, vorzugsweise wenigstens einer Base ausgewählt aus der Gruppe bestehend aus Diisopropylethylamin, Triethylamin, Pyridin und Diethylamin, gegebenenfalls in Anwesenheit wenistens eines Katalysators, vorzugsweise DMAP, oder durch Sulfonylierung der Verbindung 1,4-Dioxa-spiro[4.5]dec-8-yl-amin der Formel C mit wenigstens einer Sulfonylverbindung der allgemeinen Formel R⁶-SO₂-X⁵, worin R⁶ die vorstehend genannte Bedeutung hat, und X⁵ für eine übliche, dem Fachmann bekannte Austrittsgruppe, vorzugsweise für Halogen, besonders bevorzugt für Chlor, steht, in einem organischen Reaktionsmedium, vorzugsweise Acetonitril und/oder Dichlormethan, in Gegenwart wenigstens einer organischen Base und/oder wenigstens einer anorganischen Base, vorzugsweise in Gegenwart wenigstens einer Base ausgewählt aus der Gruppe bestehend aus Kaliumcarbonat, Natriumhydrogencarbonat, Diisopropylethylamin, Triethylamin, Pyridin und Diethylamin, oder durch Umsetzung der Verbindung 1,4-Dioxa-spiro[4.5]dec-8-yl-amin der Formel C mit wenigstens einer Carbonsäure der allgemeinen Formel R⁵-COOH, worin R⁵ die vorstehend genannte Bedeutung hat, in wenigstens einem organischen Reaktionsmedium, vorzugsweise ausgewählt aus der Gruppe bestehend aus Dichlormethan, DMF, THF und Acetonitril, in Gegenwart wenigstens eines üblichen, dem Fachmann bekannten Kopplungsmittels zu der entsprechenden Verbindung der allgemeinen Formel D umgesetzt.

Entsprechende Kopplungsmittel, die zur Ausbildung einer Amidgruppierung führen sind beispielsweise CDI, DCC oder EDCI. Alternativ kann kommt auch HOAt in Verbindung mit wenigstens einer organischen Base, wie beispielsweise DIPEA in Betracht.

Die jeweilige Verbindung der allgemeinen Formel D wird dann durch Abspaltung der Acetalschutzgruppe unter üblichen, dem Fachmann bekannten Bedingungen in eine Verbindung der allgemeinen Formel E überführt, vorzugsweise durch Abspaltung der Acetalschutzgruppe in einem organischen Reaktionsmedium, vorzugsweise Dichlormethan und/oder Methanol, in Gegenwart von Wasser mit wenigstens einer anorganischen Säure und/oder wenigstens einer organischen Säure, vorzugsweise ausgewählt aus der Gruppe bestehend aus methanolischer Salzsäure, Salzsäure, p-Toluolsulfonsäure, Trifluoressigsäure, Essigsäure, und Citronensäure, oder in Gegenwart eine Lewis-Säure.

Ausgehend von einer Verbindung der allgemeinen Formel E wird die jeweilige Verbindung F durch Einführung einer Schutzgruppe (PG1) erhalten.

Geeignete Schutzgruppen (PG1) sind beispielsweise Trifluoracetamid, Methoxycarbonyl, Ethoxycarbonyl, tert-Butoxycarbonyl, Benzyloxycarbonyl, Allyloxycarbonyl und 9-Fluorenylmethoxycarbonyl. Geeignete Reagenzien und Verfahren zur Einführung dieser Schutzgruppen sind dem Fachmann bekannt

Die jeweilige Verbindung der allgemeinen Formel F wird anschließend mit Dimethoxymethyl-dimethyl-amin der allgemeinen Formel III in einem organischen Reaktionsmedium, vorzugsweise Toluol und/oder Benzol, zu der entsprechenden Verbindung der allgemeinen Formel G umgesetzt, die vorzugsweise ohne weitere Aufarbeitung in Gegenwart wenigstens einer organischen Base oder wenigstens einer metallorganischen Base, vorzugsweise in Gegenwart von Natriumethanolat und/oder Natriummethanolat, in einem organischen Reaktionsmedium, vorzugsweise Methanol und/oder Ethanol, mit Guanidin der allgemeinen Formel V, vorzugsweise in Form eines entsprechenden Salzes wie beispielsweise Guanidin-Hydrochlorid zu der jeweiligen Verbindung der allgemeinen Formel H umgesetzt.

Hierbei kann es gegebenenfalls zu einer teilweisen Abspaltung der Schutzgruppe kommen, deren Einführung unter üblichen, dem Fachmann bekannten Bedingungen erneut durchgeführt wird, so daß die jeweilige Verbindung der Formel I erhalten wird. Hierbei kann die eingeführte Schutzgruppe (PG2) gleich oder verschieden, vorzugsweise gleich, der vorstehend angeführten Schutzgruppe (PG1) sein.

Die jeweilige Verbindung der vorstehend angegebenen allgemeinen Formel I wird dann unter üblichen, dem Fachmann bekannten Bedingungen in einem organischen Reaktionsmedium wie beispielsweise Pyridin durch einer Acylierung mit wenigstens einer Verbindung der allgemeinen Formel R³-C(=O)-X⁶ oder einer Verbindung der allgemeinen Formel (R³-C(=O))₂O, worin R³ jeweils die vorstehend genannte Bedeutung hat und X⁶ für eine übliche, dem Fachmann bekannte Austrittsgruppe, vorzugsweise für Halogen, besonders bevorzugt für Chlor, steht, in einem organischen Reaktionsmedium, ggf. in Gegenwart wenigstens einer organischen Base und/oder wenigstens einer anorganischen Base, vorzugsweise einer Base ausgewählt aus der Gruppe bestehend aus Diisopropylethylamin, Triethylamin, Pyridin und Diethylamin, gegebenenfalls in Anwesenheit wenigstens eines Katalysators, vorzugsweise DMAP, oder durch Umsetzung mit wenigstens einer Carbonsäure R³-COOH, worin R³ die vorstehend genannte Bedeutung hat, in einem organischen Reaktionsmedium, vorzugsweise ausgewählt aus der Gruppe bestehend aus Dichlormethan, DMF, THF und Acetonitril, in Gegenwart wenigstens eines üblichen Kopplungsmittels, vorzugsweise ausgewählt aus der Gruppe bestehend aus CDI, DCC und EDCI, oder in Gegenwart von HOAt und wenigstens einer organischen Base wie beispielsweise DIPEA zu der entsprechenden Verbindung der allgemeinen Formel J umgesetzt.

Als weitere Alternative kommt die Umsetzung mit wenigstens einer Verbindung der allgemeinen Formel R⁴-O-C(=O)-X^{1a}, worin R⁴ die vorstehend genannte Bedeutung hat und X^{1a} für eine Austrittsgruppe steht, in einem organischen Reaktionsmedium wie beispielsweise Acetonitril, DMF oder Dichlormethan, ggf. in Gegenwart wenigstens einer Base beispielsweise ausgewählt aus der Gruppe bestehend aus wässrigem Kaliumcarbonat, Natriumhydrogencarbonat, Natronlauge, Diisopropylethylamin, Triethylamin, Pyridin und Diethylamin in Betracht.

Die jeweilige Verbindung der allgemeinen Formel J wird anschließend durch Abspaltung der Schutzgruppe (PG2) unter üblichen, dem Fachmann bekannten Bedingungen in die jeweilige substituierte 5,6,7,8-Tetrahydro-chinazolin-2-yl-Verbindung der vorstehend angegebenen allgemeinen Formel I, worin X für eine C(H)(NHR²)-Gruppe steht, überführt.

Die Abspaltung der vorstehend genannten Schutzgruppe (PG2) kann beispielsweise in Gegenwart einer anorganischen Base, Säure oder Lewis-Säure, wie Kaliumcarbonat, Lithiumhydroxid, Kaliumhydroxid, Schwefelsäure, Bromwasserstoffsäure, Flusssäure, Salzsäure, Bortrifluoridetherat, Bortrichlorid, einer organischen Säure wie Trifluoressigsäure, Trifluormethansulfonsäure, Essigsäure, oder in Gegenwart einer organischen Base, wie Morpholin, Triethylamin, Diethylamin, Diisopropylethylamin, Pyridin oder durch Hydrierung erfolgen.

Die Verbindungen der allgemeinen Formeln R³-C(=O)-X¹, R⁵-C(=O)-X², R⁵-C(=O)-X⁴, R³-C(=O)-X⁶, (R³-C(=O))₂O, (R⁵-C(=O))₂O, R³-COOH, R⁵-COOH, R⁶-SO₂-X³, R⁶-SO₂-X⁵ und R⁴-O-(C=O)-X^{1a} sind jeweils am Markt käuflich erhältlich und/oder können nach üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Temperatur, Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reingungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Sofern die erfindungsgemäßen substituierten 5,6,7,8-Tetrahydro-pyrido[4,3-d]pyrimidin-2-yl- und 5,6,7,8-Tetrahydro-chinazolin-2-yl-Verbindungen der vorstehend angegebenen allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I sowie ggf. jeweils entsprechende Stereoisomere können nach üblichen, dem Fachmann bekannten Verfahren in Form entsprechender Salze, insbesondere in Form entsprechender physiologisch verträglicher Salze erhalten werden, erhalten werden, wobei das erfindungsgemäße Arzneimittel eines oder mehrere Salze einer oder mehrerer dieser Verbindungen aufweisen kann.

Die jeweiligen Salze der erfindungsgemäßen substituierten 5,6,7,8-Tetrahydro-pyrido[4,3-d]pyrimidin-2-yl- und 5,6,7,8-Tetrahydro-chinazolin-2-yl-Verbindungen der vorstehend angegebenen allgemeinen Formel I sowie entsprechender Stereoisomeren können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, α-Liponsäure, Acetylglycin, Hippursäure, Phosphorsäure, Maleinsäure, Malonsäure und Asparaginsäure.

Die erfindungsgemäßen substituierten 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I sowie ggf. entsprechende Stereoisomere und jeweils deren physiologisch verträgliche Salze können nach üblichen, dem Fachmann bekannten Verfahren auch in Form ihrer Solvate, insbesondere in Form ihrer Hydrate erhalten werden.

Es wurde überraschenderweise gefunden, daß sich die erfindungsgemäßen substituierten 5,6,7,8-Tetrahydro-pyrido[4,3-d]pyrimidin-2-yl- und 5,6,7,8-Tetrahydro-chinazolin-2-yl-Verbindungen zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Die erfindungsgemäßen substituierten 5,6,7,8-Tetrahydro-pyrido[4,3-d]pyrimidin-2-yl-und 5,6,7,8-Tetrahydro-chinazolin-2-yl-Verbindungen der vorstehend angegebenen allgemeinen Formel I und ggf. entsprechende Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße substituierte 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation.

Bevorzugt eignet sich das erfindungsgemäße Arzneimittel zur Prophylaxe und/oder Behandlung von Störungen und/oder Krankheiten, die zumindest teilweise durch Noradrenalin-Rezeptoren, 5-HT-Rezeptoren, mGluR5-Rezeptoren und/oder Batrachotoxin-(BTX)-Rezeptoren vermittelt werden.

Besonders bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz und/oder zur Prophylaxe und/oder Behandlung von Migräne, Depressionen, Harninkontinenz, Husten, neurodegenerativen Erkrankungen, vorzugsweise ausgewählt aus der Gruppe bestehend aus Morbus Parkinson, Morbus Huntington, Morbus Alzheimer und multipler Sklerose, Störungen der Nahrungsmittelaufnahme, vorzugsweise ausgewählt aus der Gruppe bestehend aus Bulimie, Anorexie, Fettsucht und Kachexie, kognitiven Dysfunktionen, vorzugsweise Gedächtnisstörungen, kognitive Mangelzustände (attention deficit syndrom, ADS), Störungen des nervösen Systems, Epilepsie, Schizophrenie, cerebralen Ischämien, Muskelspasmen, Krämpfen, Diarrhoe, Pruritus, Magen-Ösophagus-Reflux-Syndrom, Panikzuständen, Alkohol- und/oder Drogen-(insbesondere Nikotin- und/oder Cocain-) und/oder Medikamentenmißbrauch, Alkohol- und/oder Drogen-(insbesondere Nikotin- und/oder Cocain-) und/oder Medikamentenabhängigkeit, vorzugsweise zur Prophylaxe und/oder Verminderung von Entzugserscheinungen bei Alkohol- und/oder Drogen- und/oder Medikamentenabhängigkeit, zur Prophylaxe und/oder Verminderung einer Toleranzentwicklung gegenüber Medikamenten, insbesondere Medikamenten auf Basis von Opioiden, zur Regulation der Nahrungsaufnahme, zur Modulation der Bewegungsaktivität, zur Regulation des kardiovaskulären Systems, zur Lokalanästhesie, zur Anxiolyse, zur Vigilanzsteigerung, zur Libidosteigerung, zur Diurese und/oder zur Antinatriurese.

Ganz besonders bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Ein weiterer Gegenstand der vorliegenden Erfindung ist die Verwendung wenigstens einer erfindungsgemäßen substituierten 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation.

Bevorzugt ist die Verwendung wenigstens einer erfindungsgemäßen substituierten 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch Noradrenalin-Rezeptoren, 5-HT-Rezeptoren, mGluR5-Rezeptoren und/oder Batrachotoxin-(BTX)-Rezeptoren vermittelt werden. Besonders bevorzugt ist die Verwendung wenigstens einer erfindungsgemäßen substituierten 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz, und/oder zur Prophylaxe und/oder Behandlung von Migräne, Depressionen, Harninkontinenz, Husten, neurodegenerativen Erkrankungen, vorzugsweise ausgewählt aus der Gruppe bestehend aus Morbus Parkinson, Morbus Huntington, Morbus Alzheimer und multipler Sklerose, Störungen der Nahrungsmittelaufnahme, vorzugsweise ausgewählt aus der Gruppe bestehend aus Bulimie, Anorexie, Fettsucht und Kachexie, kognitiven Dysfunktionen, vorzugsweise Gedächtnisstörungen, kognitive Mangelzustände (attention deficit syndrom, ADS), Störungen des nervösen Systems, Epilepsie, Schizophrenie, cerebralen Ischämien, Muskelspasmen, Krämpfen, Diarrhoe, Pruritus, Magen-Ösophagus-Reflux-Syndrom, Panikzuständen, Alkohol-und/oder Drogen- und/oder Medikamentenmißbrauch, Alkohol- und/oder Drogen-und/oder Medikamentenabhängigkeit, vorzugsweise zur Prophylaxe und/oder Verminderung von Entzugserscheinungen bei Alkohol- und/oder Drogen- und/oder Medikamentenabhängigkeit, zur Prophylaxe und/oder Verminderung einer Toleranzentwicklung gegenüber Medikamenten, insbesondere Medikamenten auf Basis von Opioiden, zur Regulation der Nahrungsaufnahme, zur Modulation der Bewegungsaktivität, zur Regulation des kardiovaskulären Systems, zur Lokalanästhesie, zur Anxiolyse, zur Vigilanzsteigerung, zur Libidosteigerung, zur Diurese und/oder zur Antinatriurese.

Ganz besonders ist die Verwendung wenigstens einer erfindungsgemäßen substituierten 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerzen, vorzugsweise von akuten Schmerzen, chronischen Schmerzen, neuropathischen Schmerzen oder visceralen Schmerzen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigtens einer erfindungsgemäßen substituierten 5,6,7,8-Tetrahydro-pyrido[4,3-d]pyrimidin-2-yl- oder 5,6,7,8-Tetrahydro-chinazolin-2-yl-Verbindung der vorstehend angegebenen allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten 5,6,7,8-Tetrahydro-pyrido[4,3-d]pyrimidin-2-yl- bzw. 5,6,7,8-Tetrahydro-chinazolin-2-yl-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 5,6,7,8-Tetrahydro-pyrido[4,3-d]pyrimidin-2-yl- bzw. 5,6,7,8-Tetrahydro-chinazolin-2-yl-Verbindungen auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen substituierten 5,6,7,8-Tetrahydro-pyrido[4,3-d]pyrimidin-2-yl- bzw. 5,6,7,8-Tetrahydro-chinazolin-2-yl-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg, besonders bevorzugt 0,05 bis 75 mg/kg Körpergewicht des Patienten wenigstens einer solchen Verbindung appliziert.

### Pharmakologische Methoden:

### I. Methode zur Bestimmung der Noradrenalin- und der 5HT-Uptake-Inhibierung:

Für in vitro Studien werden Synaptosomen aus Rattenhimarealen frisch isoliert, wie in der Veröffentlichung "The isolation of nerve endings from brain" von E.G. Gray.und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1mL) in einem Glas-Homogenisierer mit Teflonstößel homogenisiert, indem fünf volle Auf- und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert werden.

Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:
NA-Uptake : Km = 0,32 ± 0,11 µM
5HT-Uptake: Km = 0,084 ± 0,011 µM

### II. Methode zur Bestimmung der Affinität zur Batrachotoxin-(BTX)-Bindungsstelle des Natriumkanals:

Die Bindungsstelle 2 des Natriumkanals ist die sogenannte Batrachotoxin-(BTX) Bindungsstelle. Als Ligand wird [³H]-Batrachotoxinin A20 α-Benzoat (10 nM im Ansatz) eingesetzt. Die lonenkanal-Partikel (Synaptosomen) werden aus dem Ratten Cerebrocortex angereichert, wie in der Veröffentlichung von Gray und Whittaker, 1962, J. Anat. 76, 79-88 beschrieben. Die entsprechende Beschreibung wird hiermit als Referenz eingeführt und gilt als Teil der vorliegenden Offenbarung. Als unspezifische Bindung ist die Radioaktivität definiert, die in Gegenwart von Veratridin (3 x 10⁻⁴ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben. Die entsprechende Beschreibung wird hiermit als Referenz eingeführt und gilt als Teil der vorliegenden Offenbarung.

Abweichend von dieser Vorschrift wird der Gesamtansatz auf 250 µl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

Folgende Kenndaten wurden für den K_{D}-Wert der Bindungsstelle ermittelt:
K_{D}: 24,63 ± 1,56 nM.

### III. Methode zur Bestimmung der Affinität zum mGluR5-Rezeptor

Schweinehirnhomogenat wird durch Homogenisation (Polytron PT 3000, Kinematica AG, 10.000 UPM (Umdrehungen pro Minute) für 90 Sekunden) von Schweinehimhälften ohne Medulla, Cerebellum und Pons in einem Puffer von pH 8.0 (30mM Hepes, Sigma, Bestellnummer H3375 + 1 Tablette Complete Roche Diagnostics, Bestellnummer. 1836145 auf 100ml) im Verhältnis 1:20 (Hirngewicht/Volumen) und Differentialzentrifugation bei 900 g und 40.000 g hergestellt. In 250 µl Inkubationsansätzen in 96 Well Mikrotiterplattten werden jeweils 450 µg Protein aus Hirnhomogenat mit 15nM ³[H]-MPEP (Tocris, Bestellnummer R1212) und die zu untersuchenden Verbindungen (jeweils 10µM im Test) in vorstehend angegebenem Puffer bei Raumtemperatur für 60 Minuten inkubiert.

Danach werden die Ansätze mit Hilfe eines Brandel Cell Harvesters (Brandel, TYP Robotic 9600) auf Unifilterplatten mit Glasfiltermatten (Perkin Elmer, Bestellnummer 6005177) filtriert und anschließend mit Puffer der vorstehend angegebenen Zusammensetzung 3 mal mit je 250 µl pro Probe nachgewaschen. Die Filterplatten werden anschließend für 60 Minuten bei 55 C° getrocknet. Anschließend werden pro Well 30µL Ultima Gold Szintillator (Packard BioScience, Bestellnummer 6013159) zugegeben und nach 3 Stunden werden die Proben am ß-Counter (Mikrobeta, Perkin Elmer) gemessen. Die unspezifische Bindung wird durch Zugabe von 10µM 2-Methyl-6-(phenylethinyl)-pyridin (Tocris, Bestellnummer 1212) bestimmt.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Ether" bedeutet Diethylether, "EE" Ethylacetat, "DCM" Dichlormethan, "DMF" Dimethylformamid, "DME" Dimethoxyethan, "DMSO" Dimethylsulfoxid und "THF" Tetrahydrofuran. Die Angabe "Äquivalente" bedeutet Stoffmengenäquivalente, "Smp." Schmelzpunkt bzw. Schmelzbereich, "Zers." Zersetzung, "RT" Raumtemperatur, d.h. ca 20 °C, "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Weitere Abkürzungen:
- DIPEA: Diisopropytlethylamin
- HOAt: 1-Hydroxy-7-azabenzotriazol
- EDCI: N-Ethyl-N'-[3-(dimethylamino)propyl]-carbodiimid
- DCC: N, N'-Dicyclohexylcarbodiimid
- DMA: mit Dimethoxymethyl-dimethyl-amin
- DMAP: Dimethylaminopyridin
- CDI: N,N'-Carbonyldiimidazole
- Pd(C): Palladium auf Aktivkohle

Die eingesetzten Chemikalien und Lösungsmittel wurden kommerziell bei den herkömmlichen Anbietern (Acros, Acocado, Aldrich, Bachem, Fluka, Lancaster, Maybridge, Merck, Sigma, TCi etc.) erworben oder nach üblichen, dem Fachmann geläufigen Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die Analytik erfolgte über NMR und HPLC-MS.

### Allgemeine Vorschrift für die Herstellung beispielgemäßer substituierter 5,6,7,8-Tetrahydro-pyrido[4,3-d]pyrimidin-2-yl-Verbindungen:

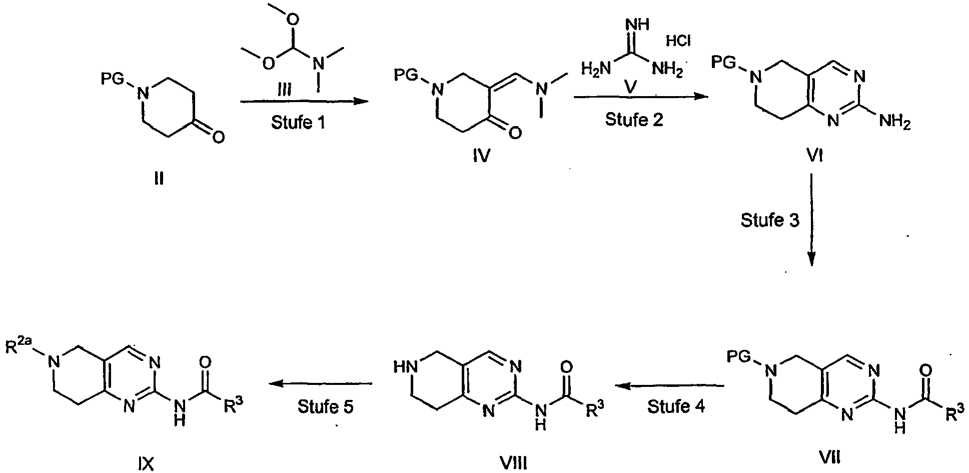

Die Synthese von 2-Amino-7,8-dihydro-5H-pyrido[4,3-d]pyrimidin-6-carbonsäure tert-butylester der Formel VI (PG = tert.-Butyloxycarbonyl, BOC) erfolgte wie nachstehend detailliert beschrieben.

In Stufe 3 wurde die Verbindung VI durch Umsetzung mit Carbonsäurechloriden der allgemeinen Formel R³-(C=O)-Cl oder Carbonsäurebromiden der allgemeinen Formel R³-(C=O)-Br acyliert, wobei das jeweilige Carbonsäurechlorid ggf. aus der entsprechenden Carbonsäure R³-(C=O)-OH unmittelbar hergestellt wurde.

Hierzu wurde in Stufe 3 zu einer Lösung der jeweiligen Carbonsäure (1 Äquivalent) in CH₂Cl₂ Oxalylchlorid (5 Äquivalente) und wenige Tropfen DMF gegeben und für 1 h bei RT unter Stickstoff als Inertgas gerührt. Nach Entfernung des Lösungsmittels wurde das jeweilige Carbonsäurechlorid in Pyridin gelöst. Sofern ein Carbonsäurechlorid bzw. Carbonsäurebromid eingesetzt wurde, wurde dies (1 Äquivalent) unmittelbar in Pyridin gelöst.

Zu dieser Lösung wurde bei 0 °C 2-Amino-7,8-dihydro-5*H*-pyrido[4,3-d]pyrimidin-6-carbonsäure tert-butylester der Formel VI (0,85 Äquivalente) gegeben und für 2 h bei 0 °C und anschließend für 2 h bei RT unter Stickstoff als Inertgas gerührt. Nach Zugabe von wässriger 1 M NaOH und Rühren für 20 Minuten wurde CH₂Cl₂ zugegeben und die wässrige Phase mit CH₂Cl₂ extrahiert. Die vereinigten organischen Phasen wurden über Na₂SO₄ getrocknet. Nach Filtration und Entfernung des Lösungsmittels wurde mittels Säulenchromatographie aufgereinigt und man erhielt die jeweilige Verbindung der allgemeinen Formel VII.

In Stufe 4 gab man zu einer Lösung der Boc-geschützten Verbindung der allgemeinen Formel VII (1 Äquivalent) in CH₂Cl₂ unter Stickstoff als Inertgas Trifluoressigsäure (65 Äquivalente) und rührte für 2 h bei RT. Nach Entfernung des Lösungsmittels erhielt man die ungeschützten Verbindungen der allgemeinen Formel VIII, die ohne weitere Aufarbeitung in Stufe 5 eingesetzt wurden.

In Stufe 5 erfolgte die Acylierung der Verbindungen der allgemeinen Formel VIII mit Carbonsäurechloriden der allgemeinen Formel R⁵-C(=O)-Cl, Carbonsäurebromiden der allgemeinen Formel R⁵-C(=O)-Br oder durch Umsetzung mit Carbonsäuren der allgemeinen Formel R⁵-C(=O)-OH.

Zur Acylierung mittels einer Carbonsäure in Stufe 5 wurde die jeweilige Verbindung VIII in CH₂Cl₂ gelöst und DIPEA und die jeweilige Carbonsäure (1 Äquivalent) hinzugegeben. Nach Abkühlung der Reaktionsmischung auf 0 °C wurde EDCI (1 Äquivalent) und HOAt (20 mg, 0,15 Äquivalente) zugegeben. Die Reaktionsmischung wurde für 1 h bei 0°C und anschließend über Nacht bei RT gerührt. Nach Entfernung des Lösungsmittels erfolgte die Reinigung über präparative HPLC oder Säulenchromatographie und man erhielt die jeweilige Verbindung der allgemeinen Formel IX.

Zur Acylierung mittels einem Carbonsäurehalogenid wurde die jeweilige Verbindung der allgemeinen Formel VIII (1 Äquivalent) in CH₂Cl₂ gelöst, Triethylamin (2 Äquivalente), eine katalytische Menge DMAP und das jeweilige Carbonsäurehalogenid (1 Äquivalent) hinzugegeben und anschließend über Nacht bei RT gerührt. Nach Entfernung des Lösungsmittels erfolgte die Reinigung über präparative HPLC oder Säulenchromatographie und man erhielt die jeweilige Verbindung der allgemeinen Formel IX.

In Stufe 5 erfolgte die Sulfonylierung der Verbindungen der allgemeinen Formel VIII mit Sulfonsäurechloriden der allgemeinen Formel R⁶-S(=O)₂-Cl.

Zur Sulfonylierung wurde die jeweilige Verbindung der allgemeinen Formel VIII (1 Äquivalent) in CH₂Cl₂ gelöst und bei 0°C Triethylamin (2 Äquivalente) und das jeweilige Sulfonsäurechlorid (1 Äquivalent) hinzugegeben. Die Reaktionsmischung wurde über Nacht bei RT gerührt. Nach Entfernung des Lösungsmittels erfolgte die Reinigung über präparative HPLC oder Säulenchromatographie und man erhielt die jeweilige Verbindung der allgemeinen Formel IX.

Im folgenden wird die vorstehend beschriebene Vorschrift für die Herstellung substituierter 5,6,7,8-Tetrahydro-pyrido[4,3-d]pyrimidin-2-yl-Verbindungen anhand einiger Beispielverbindungen detailliert erläutert:

### Herstellung von 2-Amino-7,8-dihydro-5H-pyrido[4,3-d]pyrimidin-6-carbonsäure tert-butylester

Zu einer Lösung von 4-Oxo-piperidin-1-carbonsäure tert-butylester 10.59 g (53.14 mmol) in Toluol (50 ml) wurde unter Rühren und Stickstoff als Inertgas Dimethoxymethyl-dimethyl-amin (DMA) 35.5 ml (266 mmol) zugegeben und für 3.5 h unter Rückfluß erhitzt. Nach Entfernung des Lösungsmittels erhielt man das Produkt 3-Dimethylaminomethylen-4-oxo-piperidin-1-carbonsäure tert-butylester, welches ohne weitere Aufreinigung in der nachfolgenden Umsetzung eingesetzt wurde. Zu einer Lösung von Natrium 1.65 g (74 mmol) in absolutem Ethanol wurde bei 0 °C Guanidin Hydrochlorid 7.07 g (74 mmol) gegeben und für 2 h bei RT unter Stickstoff als Inertgas gerührt. 60 ml (60 mmol) dieser Reaktionsmischung wurden zu der Lösung von 3-Dimethylaminomethylen-4-oxo-piperidin-1-carbonsäure tert-butylester in 100 ml absolutem Ethanol gegeben und für 5 h unter Rückfluß erhitzt. Nach Entfernung des Lösungsmittels und Aufreinigung über Säulenchromathographie erhielt man 2-Amino-7,8-dihydro-5*H*-pyrido[4,3-*d*]pyrimidin-6-carbonsäure tert-butylester 5.72 g (43 % der Theorie).

### Beispiel 5:

### 3,5-Dichlor-N-[6-(3-trifluormethyl-benzoyl)-5,6,7,8-tetrahydro-pyrido[4,3-d]pyrimidin-2-yl]-benzamid

### a) Herstellung von 2-(3,5-Dichlor-benzoylamino)-7,8-dihydro-5H-pyrido[4,3-d]pyrimidin-6-carbonsäure tert-butylester

Zu einer Lösung von 3,5-Dichlorbenzoesäure 527 mg (2.76 mmol) in CH₂Cl₂ wurden Oxalylchlorid 1184 µl (13.8 mmol) und zwei Tropfen DMF gegeben und für 1 h bei RT unter Stickstoff als Inertgas gerührt. Nach Entfernung des Lösungsmittels wurde das 3,5-Dichlorbenzoylchlorid in Pyridin (30 ml) gelöst. Zu dieser Lösung wurde bei 0 °C 2-Amino-7,8-dihydro-5*H*-pyrido[4,3-*d*]pyrimidin-6-carbonsäure tert-butylester 599 mg (2.39 mmol) gegeben und für 2 h bei 0 °C und anschließend für 2 h bei RT unter Stickstoff als Inertgas gerührt. Nach Zugabe von wässriger 1 M NaOH (40 ml) und Rühren für 20 Minuten wurde CH₂Cl₂ (50 ml) zugegeben und die wässrige Phase mit CH₂Cl₂ (3 x 20 ml) extrahiert. Die vereinigten organischen Phasen wurden über Na₂SO₄ getrocknet. Nach Filtration und Entfernung des Lösungsmittels wurde über Säulenchromathographie aufgereinigt und man erhielt das Produkt 2-(3,5-Dichlor-benzoylamino)-7,8-dihydro-5*H*-pyrido[4,3-d]pyrimidin-6-carbonsäure tert-butylester 573 mg (1.35 mmol, 57 % der Theorie).

### b) Herstellung von 3,5-Dichlor-N-[6-(3-trifluormethyl-benzoyl)-5,6,7,8-tetrahydro-pyrido[4,3-d]pyrimidin-2-yl]-benzamid

Zu einer Lösung von 2-(3,5-Dichlor-benzoylamino)-7,8-dihydro-5*H*-pyrido[4,3-**d]pyrimidin-6-carbonsäure** tert-butylester 404 mg (0.954 mmol) in CH₂Cl₂ (7 ml) wurde unter Stickstoff als Inertgas Trifluoressigsäure 5.0 ml (65 mmol) gegeben und für 2 h bei RT gerührt. Nach Entfernung des Lösungsmittels wurde das Intermediat (3,5-Dichlor-*N*-(5,6,7,8-tetrahydro-pyrido[4,3-d]pyrimidin-2-yl)-benzamid Trifluoracetat) in CH₂Cl₂ (10 ml) gelöst und DIPEA 340 µl und 3-Trifluormethylbenzoesäure 200 mg (1.05 mmol) hinzugegeben. Nach Abkühlung der Reaktionsmischung auf 0 °C wurde EDCI 201 mg (1.05 mmol) und HOAt 20 mg (0.15 mmol) zugegeben. Die Reaktionsmischung wurde für 1 h bei 0 °C und anschließend über Nacht bei RT gerührt. Nach Entfernung des Lösungsmittels wurde über Säulenchromathographie gereinigt und man erhielt das Produkt 3,5-Dichlor-*N-*[6-(3-trifluormethyl-benzoyl)-5,6,7,8-tetrahydro-pyrido[4,3-d]pyrimidin-2-yl]-benzamid 260 mg (0.53 mmol, 55 % der Theorie).

### Beispiel 145: N-[6-(3-Chlor-thiophen-2-carbonyl)-5,6,7,8-tetrahydro-pyrido[4,3-d]pyrimidin-2-yl]-2-ethoxy-benzamid

### a) Herstellung von 2-(2-Ethoxy-benzoylamino)-7,8-dihydro-5H-pyrido[4,3-d]pyrimidin-6-carbonsäure tert-butylester

Zu einer Lösung von 2-Ethoxybenzoesäurechlorid 390 mg (2.11 mmol) in Pyridin (25 ml) wurde bei 0 °C 2-Amino-7,8-dihydro-5*H*-pyrido[4,3-*d*]pyrimidin-6-carbonsäure tert-butylester 528 mg (2.11 mmol) gegeben und für 2 h bei 0 °C und anschließend für 72 h bei RT unter Stickstoff als Inertgas gerührt. Nach Zugabe von wässriger 1 M NaOH (40 ml) und Rühren für 20 Minuten wurde CH₂Cl₂ (50 ml) zugegeben und die wässerige Phase mit CH₂Cl₂ (3 x 20 ml) extrahiert. Die vereinigten organischen Phasen wurden über Na₂SO₄ getrocknet. Nach Filtration und Entfernung des Lösungsmittels wurde über Säulenchromathographie aufgereinigt und man erhielt das Produkt 2-(2-Ethoxy-benzoylamino)-7,8-dihydro-5*H*-pyrido[4,3-d]pyrimidin-6-carbonsäure tert-butylester 600 mg (1.51 mmol, 72 % der Theorie).

### b) Herstellung von N-[6-(3-Chlor-thiophen-2-carbonyl)-5,6,7,8-tetrahydro-pyrido[4,3-d]pyrimidin-2-yl]-2-ethoxy-benzamid

Zu einer Lösung von 2-(2-Ethoxy-benzoylamino)-7,8-dihydro-5H-pyrido[4,3-d]pyrimidin-6-carbonsäure tert-butylester 300 mg (0.753 mmol) in CH₂Cl₂ (10 ml) wurde unter Stickstoff als Inertgas Trifluoressigsäure 3.6 ml (49 mmol) gegeben und für 2 h bei RT gerührt. Nach Entfernung des Lösungsmittels wurde das Intermediat (2-Ethoxy-*N*-(5,6,7,8-tetrahydro-pyrido[4,3-*d*]pyrimidin-2-yl)-benzamid Trifluoracetat) in CH₂Cl₂ (10 ml) gelöst und DIPEA 225 µl und 3-Chlorthiophen-2-carbonsäure 129 mg (0.791 mmol) hinzugegeben. Nach Abkühlung der Reaktionsmischung auf 0 °C wurde EDCI 158 mg (0.828 mmol) und HOAt 15 mg (0.11 mmol) zugegeben. Die Reaktionsmischung wurde für 1 h bei 0 °C und anschließend über Nacht bei RT gerührt. Nach Entfernung des Lösungsmittels wurde über Säulenchromathographie gereinigt und man erhielt das Produkt *N*-[6-(3-Chlor-thiophen-2-carbonyl)-5,6,7,8-tetrahydro-pyrido[4,3-*d*]pyrimidin-2-yl]-2-ethoxy-benzamid 170 mg (0.38 mmol, 51 % der Theorie).

### Beispiel 109: 3-Chlor-N-[6-(2-methyl-benzoyl)-5,6,7,8-tetrahydro-pyrido[4,3-d]pyrirnidin-2-yl]-benzamid

### a) Herstellung von 2-(3-Chlor-benzoylamino)-7,8-dihydro-5H-pyrido[4,3-d]pyrimidin-6-carbonsäure tert-butylester

Zu einer Lösung von 3-Chlorbenzoesäurechlorid 615 µl (4.8 mmol) in Pyridin (25 ml) wurde bei 0 °C 2-Amino-7,8-dihydro-5*H*-pyrido[4,3-*d*]pyrimidin-6-carbonsäure tert-butylester 1202 mg (4.8 mmol) gegeben und für 2 h bei 0 °C und anschließend über Nacht bei RT unter Stickstoff als Inertgas gerührt. Nach Zugabe von wässriger 1 M NaOH (40 ml) und Rühren für 20 Minuten wurde CH₂Cl₂ (50 ml) zugegeben und die wässerige Phase mit CH₂Cl₂ (3 x 20 ml) extrahiert. Die vereinigten organischen Phasen wurden über Na₂SO₄ getrocknet. Nach Filtration und Entfernung des Lösungsmittels wurde über Säulenchromathographie aufgereinigt und man erhielt das Produkt 2-(3-Chlor-benzoylamino)-7,8-dihydro-5*H*-pyrido[4,3-d]pyrimidin-6-carbonsäure tert-butylester 790 mg (2.55 mmol, 53 % der Theorie).

### b) Herstellung von 3-Chlor-N-[6-(2-methyl-benzoyl)-5,6,7,8-tetrahydro-pyrido[4,3-d]pyrimidin-2-yl]-benzamid

Zu einer Lösung von 2-(3-Chlor-benzoylamino)-7,8-dihydro-5H-pyrido[4,3-**d]pyrimidin-6-carbonsäure** tert-butylester 307 mg (0.789 mmol) in CH₂Cl₂ (8 ml) wurde unter Stickstoff als inertgas Trifluoressigsäure 4.0 ml (54 mmol) gegeben und für 2 h bei RT gerührt. Nach Entfernung des Lösungsmittels wurde das Intermediat (3-Chlor-*N*-(5,6,7,8-tetrahydro-pyrido[4,3-*d*]pyrimidin-2-yl)-benzamid Trifluoacetat) in CH₂Cl₂ (10 ml) gelöst und DIPEA 380 µl und 2-Methylbenzoesäure 118 mg (0.868 mmol) hinzugegeben. Nach Abkühlung der Reaktionsmischung auf 0 °C wurde EDCI 166 mg (0.868 mmol) und HOAt 16 mg (0.12 mmol) zugegeben. Die Reaktionsmischung wurde für 1 h bei 0 °C und anschließend über Nacht bei RT gerührt. Nach Entfernung des Lösungsmittels wurde über Säulenchromathographie gereinigt und man erhielt das Produkt 3-Chlor-*N*-[6-(2-methyl-benzoyl)-5,6,7,8-tetrahydro-pyrido[4,3-*d*]pyrimidin-2-yl]-benzamid 260 mg (0.64 mmol, 81 % der Theorie).

### Beispiel 121: 4-Ethyl-N-[6-(thiophen-3-sulfonyl)-5,6,7,8-tetrahydro-pyrido[4,3-d]pyrimidin-2-yl]-benzamid

### a) Herstellung von 2-(4-Ethyl-benzoylamino)-7,8-dihydro-5H-pyrido[4,3-d]pyrimidin-6-carbonsäure tert-butylester

Zu einer Lösung von 4-Ethylbenzoesäurechlorid 296 µl (2.0 mmol) in Pyridin (25 ml) wurde bei 0 °C 2-Amino-7,8-dihydro-5*H*-pyrido[4,3-*d*]pyrimidin-6-carbonsäure tert-butylester 501 mg (2.0 mmol) gegeben und für 2 h bei 0 °C und anschließend über Nacht bei RT unter Stickstoff als Inertgas gerührt. Nach Zugabe von wässriger 1 M NaOH (40 ml) und Rühren für 20 Minuten wurde CH₂Cl₂ (50 ml) zugegeben und die wässerige Phase mit CH₂Cl₂ (3 x 20 ml) extrahiert. Die vereinigten organischen Phasen wurden über Na₂SO₄ getrocknet. Nach Filtration und Entfernung des Lösungsmittels wurde über Säulenchromathographie aufgereinigt und man erhielt das Produkt 2-(4-Ethyl-benzoylamino)-7,8-dihydro-5*H*-pyrido[4,3-*d*]pyrimidin-6-carbonsäure tert-butylester 640 mg (1.67 mmol, 84 % der Theorie).

### b) Herstellung von 4-Ethyl-N-[6-(thiophen-3-sulfonyl)-5,6,7,8-tetrahydro-pyrido[4,3-d]pyrimidin-2-yl]-benzamid

Zu einer Lösung von 2-(4-Ethyl-benzoylamino)-7,8-dihydro-5*H*-pyrido[4,3-*d*]pyrimidin-6-carbonsäure tert-butylester 567 mg (1.48 mmol) in CH₂Cl₂ (10 ml) wurde unter Stickstoff als Inertgas Trifluoressigsäure 7.3 ml (96 mmol) gegeben und für 1.5 h bei RT gerührt. Nach Entfernung des Lösungsmittels wurde das Intermediat (4-Ethyl-*N-*(5,6,7,8-tetrahydro-pyrido[4,3-*d*]pyrimidin-2-yl)-benzamid Trifluoracetat) in CH₂Cl₂ (10 ml) gelöst. Nach Zugabe von Triethylamin 1150 µl (8.20 mmol) wurde die Lösung auf 0 °C abgekühlt und 3-Thiophensulfonylchlorid 253 mg (1.39 mmol) zugegeben. Die Reaktionsmischung wurde für 1 h bei 0 °C und anschließend über Nacht bei RT gerührt. Nach Entfernung des Lösungsmittels wurde über Säulenchromathographie gereinigt und man erhielt das Produkt 4-Ethyl-*N*-[6-(thiophen-3-sulfonyl)-5,6,7,8-tetrahydro-pyrido[4,3-*d*]pyrimidin-2-yl]-benzamid 400 mg (0.93 mmol, 63 % der Theorie).

### Beispiel 125: N-[6-(3-Brom-benzolsulfonyl)-5,6,7,8-tetrahydro-pyrido[4,3-d]pyrimidin-2-yl]-3-chlor-benzamid

### a) Herstellung von 2-(3-Chlor-benzoylamino)-7,8-dihydro-5H-pyrido[4,3-d]pyrimidin-6-carbonsäure tert-butylester

Zu einer Lösung von 3-Chlorbenzoesäurechlorid 615 µl (4.8 mmol) in Pyridin (25 ml) wurde bei 0 °C 2-Amino-7,8-dihydro-5*H*-pyrido[4,3-*d*]pyrimidin-6-carbonsäure tert-butylester 1202 mg (4.8 mmol) gegeben und für 2 h bei 0 °C und anschließend über Nacht bei RT unter Stickstoff als inertgas gerührt. Nach Zugabe von wässriger 1 M NaOH (40 ml) und Rühren für 20 Minuten wurde CH₂Cl₂ (50 ml) zugegeben und die wässerige Phase mit CH₂Cl₂ (3 x 20 ml) extrahiert. Die vereinigten organischen Phasen wurden über Na₂SO₄ getrocknet. Nach Filtration und Entfernung des Lösungsmittels wurde über Säulenchromathographie aufgereinigt und man erhielt das Produkt 2-(3-Chlor-benzoylamino)-7,8-dihydro-5*H*-pyrido[4,3-d]pyrimidin-6-carbonsäure tert-butylester 790 mg (2.55 mmol, 53 % der Theorie).

### b) Herstellung von N-[6-(3-Brom-benzolsulfonyl)-5,6,7,8-tetrahydro-pyrido[4,3-d]pyrimidin-2-yl]-3-chlor-benzamid

Zu einer Lösung von 2-(3-Chlor-benzoylamino)-7,8-dihydro-5*H*-pyrido[4,3-d]pyrimidin-6-carbonsäure tert-butylester 487 mg (1.25 mmol) in CH₂Cl₂ (8 ml) wurde unter Stickstoff als Inertgas Trifluoressigsäure 6.2 ml (81 mmol) gegeben und für 1.5 h bei RT gerührt. Nach Entfernung des Lösungsmittels wurde das Intermediat (3-Chlor-*N*-(5,6,7,8-tetrahydro-pyrido[4,3-*d*]pyrimidin-2-yl)-benzamid Trifluoracetat) in CH₂Cl₂ (15 ml) gelöst. Nach Zugabe von Triethylamin 820 µl (5.84 mmol) wurde die Lösung auf 0 °C abgekühlt und 3-Brombenzolsulfonylchlorid 311 mg (1.37 mmol) zugegeben. Die Reaktionsmischung wurde für 1 h bei 0 °C und anschließend über Nacht bei RT gerührt. Nach Entfernung des Lösungsmittels wurde über Säulenchromathographie gereinigt und man erhielt das Produkt *N*-[6-(3-Brom-benzolsulfonyl)-5,6,7,8-tetrahydro-pyrido[4,3-*d*]pyrimidin-2-yl]-3-chlor-benzamid 525 mg (81 % der Theorie).

### Allgemeine Vorschrift für die Herstellung substituierter 5,6,7,8-Tetrahydro-chinazolin-2-yl-Verbindungen:

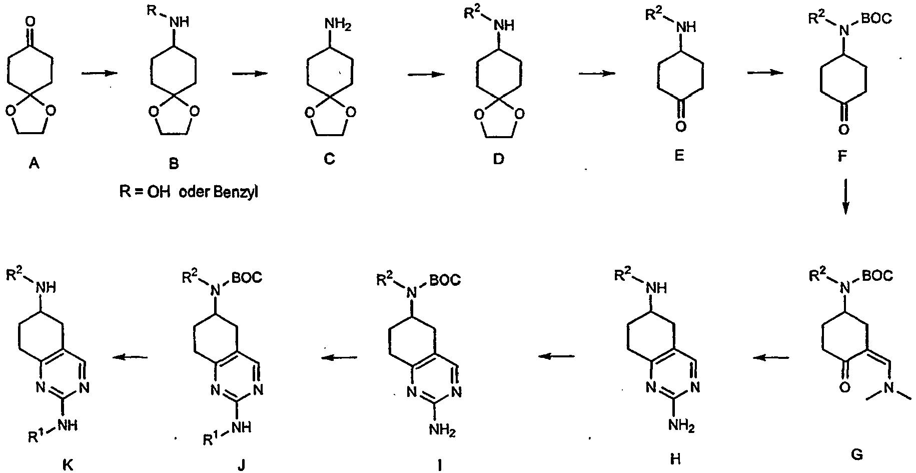

Die Synthese von 1,4-Dioxa-spiro[4.5]dec-8-ylamin der Formel C erfolgte wie nachstehend detailliert beschrieben.

Die Sulfonylierung von 1,4-Dioxa-spiro[4.5]dec-8-ylamin der Formel C erfolgte mittels Sulfonsäurechloriden der allgemeinen Formel R^{s}-S(=O)₂-Cl.

Zur Sulfonylierung wurde zu einer Lösung von 1,4-Dioxa-spiro[4.5]dec-8-ylamin der Formel C (1 Äquivalent) in CH₃CN K₂CO₃ (5 Äquivalente) zugegeben. Eine Lösung des jeweiligen Sulfonsäurechlorids (1 Äquivalent) in CH₃CN wurde zugegeben und bei RT für 2 h unter Stickstoff als inertgas gerührt. Die Reaktionsmischung wurde filtriert und der Rückstand mit CH₂Cl₂ gewaschen. Nach Reinigung durch Säulenchromatographie erhielt man die jeweilige Verbindung der allgemeinen Formel D.

Alternativ wurden zur Sulfonylierung zu einer Lösung von 1,4-Dioxa-spiro[4.5]dec-8-ylamin der Formel C (1 Äquivalent) in CH₂Cl₂ bei 0°C Triethylamin (2 Äquivalente) und das jeweilige Sulfonsäurechlorid (1 Äquivalent) hinzugegeben. Die Reaktionsmischung wurde über Nacht bei RT gerührt. Man erhielt die jeweilige Verbindung der allgemeinen Formel D nach Entfernung des Lösungsmittels und Reinigung über präparative HPLC oder Säulenchromatographie.

Die Acylierung der Verbindung C erfolgte mit Carbonsäurechloriden der allgemeinen Formel R⁵-(C=O)-Cl, Carbonsäurebromiden der allgemeinen Formel R⁵-(C=O)-Br oder durch Umsetzung mit Carbonsäuren der allgemeinen Formel R⁵-(C=O)-OH.

Zur Acylierung mittels einer Carbonsäure wurde die Verbindung C (1 Äquivalent) in CH₂Cl₂ gelöst und DIPEA und die jeweilige Carbonsäure (1 Äquivalent) hinzugegeben. Nach Abkühlung der Reaktionsmischung auf 0°C wurde EDCI (1 Äquivalent) und HOAt (0,15 Äquivalente) zugegeben. Die Reaktionsmischung wurde für 1 h bei 0°C und anschließend über Nacht bei RT gerührt. Nach Entfernung des Lösungsmittels wurde die jeweilige Verbindung der Formel D über Säulenchromatographie gereinigt.

Zur Acylierung mittels eines Carbonsäurehalogenids wurde die jeweilige Verbindung der allgemeinen Formel D (1 Äquivalent) in CH₂Cl₂ gelöst, Triethylamin (2 Äquivalente), eine katalytische Menge DMAP und das jeweilige Carbonsäurehalogenid (1 Äquivalent) hinzugegeben und anschließend über Nacht bei RT gerührt. Nach Entfernung des Lösungsmittels und Reinigung über Säulenchromatographie wurde die jeweilige Verbindung der allgemeinen Formel D erhalten.

Die Umsetzung zu den Verbindungen der Formeln D bis I erfolgte wie nachstehend detailliert beschrieben.

Die Umsetzung der jeweiligen Verbindung I zu den Verbindungen J erfolgte in einer Acylierung mit Carbonsäurechloriden der allgemeinen Formel R³-(C=O)-Cl, Carbonsäurebromiden der allgemeinen Formel R³-(C=O)-Br oder durch Umsetzung mit Carbonsäuren der allgemeinen Formel R³-(C=O)-OH.

Zur Acylierung mittels einer Carbonsäure wurde die jeweilige Verbindung I (1 Äquivalent) in CH₂Cl₂ gelöst und DIPEA und die jeweilige Carbonsäure (1 Äquivalent) hinzugegeben. Nach Abkühlung der Reaktionsmischung auf 0°C wurde EDCI (1 Äquivalent) und HOAt (0,15 Äquivalente) zugegeben. Die Reaktionsmischung wurde für 1 h bei 0°C und anschließend über Nacht bei RT gerührt. Nach Entfernung des Lösungsmittels wurde die jeweilige Verbindung der allgemeinen Formel J über Säulenchromatographie gereinigt.

Zur Acylierung mittels eines Carbonsäurehalogenids wurde die jeweilige Verbindung der allgemeinen Formel I (1 Äquivalent) in CH₂Cl₂ gelöst, Triethylamin (2 Äquivalente), eine katalytische Menge DMAP und das jeweilige Carbonsäurehalogenid (1 Äquivalent) hinzugegeben und anschließend über Nacht bei RT gerührt. Nach Entfernung des Lösungsmittels und Reinigung über Säulenchromatographie wurde die jeweilige Verbindung der allgemeinen Formel J erhalten.

Die abschließende Abspaltung der BOC-Schutzgruppe erfolgte wie nachstehend detailliert beschrieben.

### Beispiel 180:

### Thiophen-2-carbonsäure [6-(2,5-dichlor-thiophen-3-sulfonylamino)-5,6,7,8-tetrahydro-chinazolin-2-yl]-amid

### a) Herstellung von Benzyl-(1,4-dioxa-spiro[4.5]dec-8-yl)-amin

Zu einer Lösung von 1,4-Dioxa-spiro[4.5]decan-8-on 1.0 g (6.4 mmol) und Benzylamin 0.69 g (6.4 mmol) in CH₂Cl₂ (20 ml) wurde unter Stickstoff als Inertgas Na(OAc)₃BH 2.03 g, 9.6 mmol) gegeben und für 2 h bei RT gerührt. Nach der Zugabe von CH₂Cl₂ (80 ml) wurde die organische Phase mit wässriger gesättigter NaHCO₃-Lösung gewaschen. Nach Trocknung der separierten organischen Phase über Na₂SO₄ und Filtration wurde das Lösungsmittel abdestilliert und man erhielt das Produkt Benzyl-(1,4-dioxa-spiro[4.5]dec-8-yl)-amin 1.6 g (100 % der Theorie).

### b) Herstellung von 1,4-Dioxa-spiro[4.5]dec-8-ylamin

Benzyl-(1,4-dioxa-spiro[4.5]dec-8-yl)-amin (6.4 mmol) wurde in EtOH (10 ml) gelöst und unter Stickstoff mit Pd(C) 340 mg (0.32 mmol) unter Rühren über Nacht bei RT hydriert. Die Reaktionsmischung wurde über Celite abfiltriert und mit CH₂Cl₂ gewaschen. Nach Entfernung des Lösungsmittels erhielt man das Produkt 1,4-Dioxa-spiro[4.5]dec-8-ylamin 824 mg (82 % der Theorie).

### c) Herstellung von 2,5-Dichlor-thiophen-3-sulfonsäure (1,4-dioxa-spiro[4.5]dec-8-yl)-amid

Zu einer Lösung von 1,4-Dioxa-spiro[4.5]dec-8-ylamin 571 mg (3.63 mmol) in CH₃CN (10 ml) wurde K₂CO₃ 2.51 g (18.16 mmol) zugegeben. Eine Lösung von 3,5-Dichlorthiophen-3-sulfonylchlorid (914 mg, 3,63 mmol) in CH₃CN (10 ml) wurde zugegeben und bei RT für 100 min unter Stickstoff als Inertgas gerührt. Die Reaktionsmischung wurde filtriert und der Rückstand mit CH₂Cl₂ gewaschen. Nach Reinigung durch Säulenchromatographie erhielt man das 2,5-Dichlor-thiophen-3-sulfonsäure (1,4-dioxa-spiro[4.5]dec-8-yl)-amid 914 mg (68 % der Theorie).

### d) Herstellung von 2,5-Dichloro-thiophen-3-sulfonsäure (4-oxo-cyclohexyl)-amid

Zu einer Lösung von 2,5-Dichlor-thiophen-3-sulfonsäure-(1,4-dioxa-spiro[4.5]dec-8-yl)-amid 894 mg (2.40 mmol) in Aceton (10 ml) und Wasser (1 ml) wurde p-TosOH x H₂O 4 g (21.0 mmol) gegeben und für 6 h bei RT gerührt. Nach Zugabe von wässriger 1 M NaOH (100 ml) und wässriger gesättigter NaCl-Lösung (100 ml) wurde mit CH₂Cl₂ (3 x 200 ml) extrahiert. Nach Separation der organischen Phasen, Trocknung über Na₂SO₄ und Filtration wurde das Lösungsmittel abdestilliert. Nach Reinigung über Säulenchromathographie erhielt man das Produkt 2,5-Dichloro-thiophen-3-sulfonsäure **(4-oxo-cyclohexyl)-amid** 634 mg (80 % der Theorie).

### e) Herstellung von N-Boc geschütztem 2,5-Dichloro-thiophen-3-sulfonsäure (4-oxo-cyclohexyl)-amid

Zu einer Lösung von **2,5-Dichloro-thiophen-3-sulfonsäure (4-oxo-cyclohexyl)-amid** 388 mg (1.18 mmol) in CH₂Cl₂ (10 ml) wurde Et₃N 179 mg (1.77 mmol) und DMAP 29 mg (0.24 mmol) zugegeben. Nach Abkühlung auf 0 °C wurde Boc₂O 386 mg (1.77 mmol) zugegeben und für 2 h unter Stickstoff als Inertgas gerührt. Nach Entfernung des Lösungsmittels und Reinigung über Säulenchromathographie erhielt man das Produkt N-Boc geschützte 2,5-Dichloro-thiophen-3-sulfonsäure (4-oxo-cyclohexyl)-amid 419 mg (83 % der Theorie).

### f) Herstellung von 2,5-Dichlor-thiophen-3-sulfonsäure (2-amino-5,6,7,8-tetrahydro-chinazolin-6-yl)-amid

Eine Reaktionslösung von N-Boc geschütztem 2,5-Dichloro-thiophen-3-sulfonsäure (4-oxo-cyclohexyl)-amid 383 mg (0.89 mmol) und Dimethoxymethyl-dimethyl-amin 533 mg (4.47 mmol) in Toluol (10 ml) wurde unter Rühren und Stickstoff als inertgas für 4 h unter Rückfluß erhitzt. Nach Entfernung des Lösungsmittels erhielt man das Rohprodukt N-Boc geschütztem 2,5-Dichlor-thiophen-3-sulfonsäure (3-dimethylaminomethylen-4-oxo-cyclohexyl)-amid, welches ohne weitere Aufreinigung in die nächste Stufe eingesetzt wurde.

Zu einer Lösung von Natrium 59 mg (2.6 mmol) in absolutem Ethanol (26 ml) wurde Guanidin Hydrochlorid 245 mg (2.56 mmol) gegeben und für 30 min bei RT unter Stickstoff als Inertgas gerührt. 9 ml (60 mmol) dieser Reaktionsmischung wurde zu N-Boc geschütztem 2,5-Dichlor-thiophen-3-sulfonsäure (3-dimethylaminomethylen-4-oxo-cyclohexyl)-amid (0.89 mmol) gegeben und für 8 h unter Rückfluß und unter Stickstoff als Inertgas erhitzt. Nach Zugabe von CH₂Cl₂ (5 ml) und TFA (5 ml) und Rühren für 1 h bei RT und Entfernung des Lösungsmittels wurde über Säulenchromathographie aufgereinigt und man erhielt man das Produkt 2,5-Dichlor-thiophen-3-sulfonsäure (2-amino-5,6,7,8-tetrahydro-chinazolin-6-yl)-amid 133 mg (39 % der Theorie).

### g) Herstellung von N-Boc geschütztem 2,5-Dichlor-thiophen-3-sulfonsäure (2-amino-5,6,7,8-tetrahydro-chinazolin-6-yl)-amid

Zu einer Lösung von 2,5-Dichlor-thiophen-3-sulfonsäure (2-amino-5,6,7,8-tetrahydro-chinazolin-6-yl)-amid 63 mg (0.17 mmol) in CH₂Cl₂ (10 ml) wurde bei 0 °C Boc₂O 41 mg (0.188 mmol) und DMAP 2 mg (0.016 mmol) gegeben und unter Stickstoff als Inertgas für 3.5 h bei 0 °C gerührt. Nach Zugabe von Boc₂O 20 mg und Rühren für 30 min bei 0 °C wurde das Lösungsmittel abdestilliert. Nach Reiniung über Säulenchromathograhie erhielt man das Produkt N-Boc geschütztes 2,5-Dichlor-thiophen-3-sulfonsäure (2-amino-5,6,7,8-tetrahydro-chinazolin-6-yl)-amid 58 mg (73 % der Theorie).

### h) Herstellung von N-Boc geschütztem Thiophen-2-carbonsäure [6-(2,5-dichlor-thiophen-3-sulfonylamino)-5,6,7,8-tetrahydro-chinazolin-2-yl]-amid

Zu einer Lösung von N-Boc geschütztem 2,5-Dichlor-thiophen-3-sulfonsäure (2-amino-5,6,7,8-tetrahydro-chinazolin-6-yl)-amid 54 mg (0.11 mmol) in CH₂Cl₂ (4 ml) und Pyridin (5 ml) wurde bei 0 °C Thiophen-2-carbonsäurechlorid 25 mg (0.17 mmol) gelöst in CH₂Cl₂ (1 ml) zugegeben und für 1 h bei 0 °C und über Nacht bei RT und unter Stickstoff als inertgas gerührt. Nach Entfernung des Lösungsmittels und Reinigung über Säulenchromathographie erhielt man das Produkt N-Boc geschütztes Thiophen-2-carbonsäure [6-(2,5-dichlor-thiophen-3-sulfonylamino)-5,6,7,8-tetrahydro-chinazolin-2-yl]-amid 21 mg (32 % der Theorie).

### i) Herstellung von Thiophen-2-carbonsäure [6-(2,5-dichlor-thiophen-3-sulfonylamino)-5,6,7,8-tetrahydro-chinazolin-2-yl]-amid

Zu einer Lösung von N-Boc geschütztem Thiophen-2-carbonsäure [6-(2,5-dichlor-thiophen-3-sulfonylamino)-5,6,7,8-tetrahydro-chinazolin-2-yl]-amid 21 mg (36 µmol) in CH₂Cl₂ (1 ml) wurde TFA (2 ml) zugegeben und für 2 h bei RT gerührt. Nach Entfernung des Lösungsmittels und Reinigung über Säulenchromathographie erhielt man das Produkt Thiophen-2-carbonsäure [6-(2,5-dichlor-thiophen-3-sulfonylamino)-5,6,7,8-tetrahydro-chinazolin-2-yl]-amid 12 mg (69 % der Theorie).

Die zuvor nicht detailliert beschriebene Herstellung der übrigen Verbindungen gemäß den nachstehend angegebenen Beispielen erfolgte ebenfalls entsprechend den vorstehend angegebenen Herstellungsvorschriften, wobei dem Fachmann aufgrund dieser Vorschriften die jeweils eingesetzten Edukte bekannt sind.

| Beispiel | Verbindung |
|---|---|
| 1 | 3-Fluor-N-[6-(4-fluor-benzoyl)-5,6,7,8-tetrahydro-pyrido[4,3-d]pyrimidin-2-yl]-benzamid |
| 2 | 3,5-Dichlor-N-[6-(3-fluor-4-methoxy-benzoyl)-5,6,7,8-tetrahydro-pyrido[4,3-d]pyrimidin-2-yl]-benzamid |
| 3 | 4-tert-Butyl-N-(6-hexanoyl-5,6,7,8-tetrahydro-pyrido[4,3-d]pyrimidin-2-yl)-benzamid |
| 4 | N-(6-Acetyl-5,6,7,8-tetrahydro-pyrido[4,3-d]pyrimidin-2-yl)-3,4-dichlor-benzamid |
| 5 | 3,5-Dichlor-N-[6-(3-trifluormethyl-benzoyl)-5,6,7,8-tetrahydro-pyrido[4,3-d]pyrimidin-2-yl]-benzamid |
| 6 | 3-Chlor-N-[6-(4-methoxy-2,3,6-trimethyl-benzolsulfonyl)-5,6,7,8-tetrahydro-pyrido[4,3-d]pyrimidin-2-yl]-benzamid |
| 7 | N-[6-(2-Ethoxy-benzoyl)-5,6,7,8-tetrahydro-pyrido[4,3-d]pyrimidin-2-yl]-3-fluor-benzamid |
| 8 | 3-Chlor-N-[6-(3-phenyl-propionyl)-6,6,7,8-tetrahydro-pyrido[4,3-d]pyrimidin-2-yl]-benzamid |
| 9 | 4-tert-Butyl-N-[6-(isoxazol-5-carbonyl)-5,6,7,8-tetrahydro-pyrido[4,3-d]pyrimidin-2-yl]-benzamid |
| 10 | N-[6-(2-Benzylsulfanyl-acetyl)-5,6,7,8-tetrahydro-pyrido[4,3-d]pyrimidin-2-yl]-2-methoxy-benzamid |
| 11 | Thiophen-2-carbonsäure {6-[2-(4-chlor-phenyl)-2-methyl-propionyl]-5,6,7,8-tetrahydro-pyrido[4,3-d]pyrimidin-2-yl}-amid |
| 12 | N-(6-Benzoyl-5,6,7,8-tetrahydro-pyrido[4,3-d]pyrimidin-2-yl)-3-methyl-benzamid |
| 13 | N-[6-(2,3-Dihydro-benzofuran-5-carbonyl)-5,6,7,8-tetrahydro-pyrido[4,3-d]pyrimidin-2-yl]-2-fluor-benzamid |
| 14 | Thiophen-2-carbonsäure [6-(3,4,5-trimethoxy-benzoylamino)-5,6,7,8-tetrahydro-chinazolin-2-yl]-amid |
| 15 | Naphthalin-1-carbonsäure [6-(3-methyl-5-phenyl-isoxazol-4-carbonyl)-5,6,7,8-tetrahydro-pyrido[4,3-d]pyrimidin-2-yl]-amid |
| 16 | 3-Chlor-N-{6-[2-(5-methyl-2-phenyl-thiazol-4-yl)-acetyl]-5,6,7,8-tetrahydro-pyrido[4,3-d]pyrimidin-2-yl}-benzamid |
| 17 | N-[6-(4-Chlor-2,5-dimethyl-benzolsulfonyl)-5,6,7,8-tetrahydro-pyrido[4,3-d]pyrimidin-2-yl]-2-trifluormethyl-benzamid |
| 18 | N-[6-(1-Benzolsulfonyl-1H-indol-2-carbonyl)-5,6,7,8-tetrahydro-pyrido[4,3-d]pyrimidin-2-yl]-3-methoxy-benzamid |
| 20 | N-[6-(5-Methyl-1-phenyl-1H-pyrazol-4-sulfonyl)-5,6,7,8-tetrahydro-pyrido[4,3-d]pyrimidin-2-yl]-2-trifluormethyl-benzamid |
| 21 | N-[6-(3-Chlor-2-methyl-benzolsulfonyl)-5,6,7,8-tetrahydro-pyrido[4,3-d]pyrimidin-2-yl]-3-fluor-benzamid |
| 22 | 3,5-Dichlor-N-[6-(3,5-dimethyl-isoxazol-4-carbonyl)-5,6,7,8-tetrahydro-pyrido[4,3-d]pyrimidin-2-yl]-benzamid |
| 23 | Thiophen-2-carbonsäure [6-(4-trifluormethoxy-benzolsulfonylamino)-5,6,7,8-tetrahydro-chinazolin-2-yl]-amid |
| 25 | N-[6-(Furan-2-carbonyl)-5,6,7,8-tetrahydro-pyrido[4,3-d]pyrimidin-2-yl]-3-methoxy-benzamid |
| 26 | N-{6-[4-(2,3-Dihydro-indol-1-yl)-4-oxo-butyryl]-5,6,7,8-tetrahydro-pyrido[4,3-d]pyrimidin-2-yl}-3-methyl-benzamid |
| 27 | N-{6-[2-(4-Methyl-cyclohexyl)-acetyl]-5,6,7,8-tetrahydro-pyrido[4,3-d]pyrimidin-2-yl}-2-trifluormethyl-benzamid |
| | yl}-2-trifluormethyl-benzamid |
| 28 | 3,4-Difluor-N-[6-(6-phenoxy-pyridin-3-carbonyl)-5,6,7,8-tetrahydro-pyrido[4,3-d]pyrimidin-2-yl]-benzamid |
| 29 | 4-tert-Butyl-N-[6-(2-phenyl-thiazol-4-carbonyl)-5,6,7,8-tetrahydro-pyrido[4,3-d]pyrimidin-2-yl]-benzamid |
| 30 | 3,5-Dichlor-N-[6-(2-trifluormethyl-benzolsulfonyl)-5,6,7,8-tetrahydro-pyrido[4,3-d]pyrimidin-2-yl]-benzamid |
| 31 | Thiophen-2-carbonsäure [6-(3-brom-benzolsulfonylamino)-5,6,7,8-tetrahydro-chinazolin-2-yl]-amid |
| 32 | N-[6-(2-Chlor-pyridin-4-carbonyl)-5,6,7,8-tetrahydro-pyrido[4,3-d]pyrimidin-2-yl]-3-methoxy-benzamid |
| 33 | 4-Fluor-N-[6-(2-methansulfonyl-benzolsulfonylamino)-5,6,7,8-tetrahydro-chinazolin-2-yl]-benzamid |
| 35 | N-(6-Dimethylsulfamoyl-5,6,7,8-tetrahydro-pyrido[4,3-d]pyrimidin-2-yl)-3-fluor-benzamid |
| 36 | 3-Fluor-N-{6-[2-(4-trifluormethyl-phenyl)-acetyl]-5,6,7,8-tetrahydro-pyrido[4,3-d]pyrimidin-2-yl}-benzamid |
| 37 | N-{6-[2-(1,3-Dioxo-1,3-dihydro-isoindol-2-yl)-acetyl]-5,6,7,8-tetrahydro-pyrido[4,3-d]pyrimidin-2-yl}-2-trifluormethyl-benzamid |
| 38 | Thiophen-2-carbonsäure {6-[4-(4-chlor-2-methyl-phenoxy)-butyrylamino]-5,6,7,8-tetrahydro-chinazolin-2-yl}-amid |
| 39 | N-[6-(Butan-1-sulfonyl)-5,6,7,8-tetrahydro-pyrido[4,3-d]pyrimidin-2-yl]-3-fluor-benzamid |
| 40 | 2-Methoxy-N-[6-(2-propyl-pentanoyl)-5,6,7,8-tetrahydro-pyrido[4,3-d]pyrimidin-2-yl]-benzamid |
| 41 | N-[6-(4,5-Dichlor-thiophen-2-sulfonylamino)-5,6,7,8-tetrahydro-chinazolin-2-yl]-4-fluor-benzamid |
| 42 | 4-Chlor-N-[6-(2-trifluormethyl-benzolsulfonyl)-5,6,7,8-tetrahydro-pyrido[4,3-d]pyrimidin-2-yl]-benzamid |
| 43 | Thiophen-2-carbonsäure [6-(4-methoxy-benzolsulfonylamino)-5,6,7,8-tetrahydro-chinazolin-2-yl]-amid |
| 44 | 5-[2-(3,4-Difluor-benzoylamino)-7,8-dihydro-5H-pyrido[4,3-d]pyrimidin-6-yl]-5-oxo-valeriansäure methyl ester |
| 45 | N-[6-(Benzo[b]thiophen-3-carbonyl)-5,6,7,8-tetrahydro-pyrido[4,3-d]pyrimidin-2-yl]-4-chlor-benzamid |
| 46 | N-[6-(5-Brom-2-methoxy-benzolsulfonylamino)-5,6,7,8-tetrahydro-chinazolin-2-yl]-4-fluor-benzamid |
| 47 | 4-Fluor-N-[6-(4-fluor-benzolsulfonylamino)-5,6,7,8-tetrahydro-chinazolin-2-yl]-benzamid |
| 48 | N-{6-[2-(1H-Indol-3-yl)-2-oxo-acetyl]-5,6,7,8-tetrahydro-pyrido[4,3-d]pyrimidin-2-yl}-2-trifluormethyl-benzamid |
| 49 | 5-tert-Butyl-2-methyl-furan-3-carbonsäure {2-[(thiophen-2-carbonyl)-amino]-5,6,7,8-tetrahydro-chinazolin-6-yl}-amid |
| 50 | N-[6-(2,5-Dimethoxy-benzolsulfonyl)-5,6,7,8-tetrahydro-pyrido[4,3-d]pyrimidin-2-yl]-3-methoxy-benzamid |
| 51 | Benzoesäure 2-{2-[(thiophen-2-carbonyl)-amino]-5,6,7,8-tetrahydro-chinazolin-6-ylcarbamoyl}-benzyl ester |
| 52 | 3,4-Difluor-N-(6-hexanoyl-5,6,7,8-tetrahydro-pyrido[4,3-d]pyrimidin-2-yl)-benzamid |
| 53 | 4-Ethyl-N-[6-(tetrahydro-furan-2-carbonyl)-5,6,7,8-tetrahydro-pyrido[4,3-d]pyrimidin-2-yl]-benzamid |
| 54 | N-[6-(2-Chlor-benzolsulfonyl)-5,6,7,8-tetrahydro-pyrido[4,3-d]pyrimidin-2-yl]-3-fluor-benzamid |
| 55 | 4-tert-Butyl-N-[6-(5-tert-butyl-2-methyl-2H-pyrazol-3-carbonyl)-5,6,7,8-tetrahydro-pyrido[4,3-*d*]pyrimidin-2-yl]-benzamid |
| 56 | 3-Methoxy-N-[6-(4-methoxy-benzolsulfonyl)-5,6,7,8-tetrahydro-pyrido[4,3-d)pyrimidin-2-yl)-benzamid |
| 57 | {2-[2-(3,4-Difluor-benzoylamino)-7,8-dihydro-5H-pyrido[4,3-d]pyrimidin-6-yl]-2-oxo-1-phenyl-ethyl}-carbaminsäure benzyl ester |
| 58 | 5-Phenyl-oxazol-4-carbonsäure {2-[(thiophen-2-carbonyl)-amino]-5,6,7,8-tetrahydro-chinazolin-6-yl}-amid |
| 59 | 4-Chlor-N-(6-dimethylsulfamoyl-5,6,7,8-tetrahydro-pyrido[4,3-d]pyrimidin-2-yl)-benzamid |
| 60 | 4-tert-Butyl-N-[6-(2-fluor-benzolsulfonyl)-5,6,7,8-tetrahydro-pyrido[4,3-d]pyrimidin-2-yl]-benzamid |
| 61 | 4-Chlor-N-[6-(3-cyclopentyl-propionyl)-5,6,7,8-tetrahydro-pyrido[4,3-d]pyrimidin-2-yl]-benzamid |
| 62 | 4-Chlor-N-[6-(4-phenyl-butyryl)-5,6,7,8-tetrahydro-pyrido[4,3-d]pyrimidin-2-yl]-benzamid |
| 63 | 4-tert-Butyl-N-[6-(5-methyl-2-phenyl-2H-[1,2,3]triazol-4-carbonyl)-5,6,7,8-trahydro-pyrido[4,3-d]pyrimidin-2-yl]-benzamid |
| 64 | 2-Chlor-N-{2-[(thiophen-2-carbonyl)-amino]-5,6,7,8-tetrahydro-chinazolin-6-yl}-isonicotinamid |
| 66 | N-[6-(5-Chlor-thiophen-2-sulfonyl)-5,6,7,8-tetrahydro-pyrido[4,3-d]pyrimidin-2-yl]-3-fluor-benzamid |
| 67 | N-[6-(4-Diethylamino-benzoyl)-5,6,7,8-tetrahydro-pyrido[4,3-d]pyrimidin-2-yl]-3-methoxy-benzamid |
| 68 | N-[6-(2,4-Dimethyl-thiazol-5-sulfonyl)-5,6,7,8-tetrahydro-pyrido[4,3-d]pyrimidin-2-yl]-3-methyl-benzamid |
| 69 | 4-Chlor-N-[6-(naphthalin-2-carbonyl)-5,6,7,8-tetrahydro-pyrido[4,3-d]pyrimidin-2-yl]-benzamid |
| 70 | N-[6-(2,4-Difluor-benzolsulfonyl)-5,6,7,8-tetrahydro-pyrido[4,3-d]pyrimidin-2-yl]-2-methoxy-benzamid |
| 71 | 3,4-Dichlor-N-[6-(4-chlor-benzolsulfonyl)-5,6,7,8-tetrahydro-pyrido[4,3-d]pyrimidin-2-yl]-benzamid |
| 72 | 3,4-Difluor-N-[6-(thiophen-2-sulfonyl)-5,6,7,8-tetrahydro-pyrido[4,3-d]pyrimidin-2-yl]-benzamid |
| 74 | 4-Ethyl-N-[6-(4-pyrazol-1-yl-benzoyl)-5,6,7,8-tetrahydro-pyrido[4,3-d]pyrimidin-2-yl]-benzamid |
| 75 | N-[6-(7,7-Dimethyl-2-oxo-bicyclo[2.2.1]hept-1-ylmethansulfonylamino)-5,6,7,8-tetrahydro-chinazolin-2-yl]-butyramid |
| 76 | 5-Oxo-5-phenyl-valeriansäure (2-butyrylamino-5,6,7,8-tetrahydro-chinazolin-6-yl)-amid |
| 77 | 3-[2-(4-Chlor-benzoylamino)-7,8-dihydro-5H-pyrido[4,3-d]pyrimidin-6-yl]-3-oxo-propionsäure methyl ester |
| 78 | N-{6-[5-(4-Chlor-phenyl)-2-methyl-furan-3-carbonyl]-5,6,7,8-tetrahydro-pyrido[4,3-d]pyrimidin-2-yl}-2-methoxy-benzamid |
| 79 | 4-Chlor-N-[6-(2,4-dimethyl-thiazol-5-sulfonyl)-5,6,7,8-tetrahydro-pyrido[4,3-d]pyrimidin-2-yl]-benzamid |
| 80 | N-[6-(2,3-Difluor-benzoyl)-5,6,7,8-tetrahydro-pyrido[4,3-d]pyrimidin-2-yl]-3-methoxy-benzamid |
| 81 | 4-Chlor-N-{6-[2-(2-methoxy-ethoxy)-acetyl]-5,6,7,8-tetrahydro-pyrido[4,3-d]pyrimidin-2-yl}-benzamid |
| 82 | 2-[2-(2-Ethoxy-benzoylamino)-7,8-dihydro-5H-pyrido[4,3-d]pyrimidin-6-sulfonyl]-benzoesäure methyl ester |
| 83 | 4-tert-Butyl-N-[6-(4-nitro-benzolsulfonyl)-5,6,7,8-tetrahydro-pyrido[4,3-d]pyrimidin-2-yl]-benzamid |
| 86 | N-[6-(5-Chlor-thiophen-2-sulfonyl)-5,6,7,8-tetrahydro-pyrido[4,3-d]pyrimidin-2-yl]-2-methoxy-benzamid |
| 87 | 4-Fluor-N-[6-(furan-2-carbonyl)-5,6,7,8-tetrahydro-pyrido[4,3-d]pyrimidin-2-yl]-benzamid |
| 88 | 4-{2-[(Thiophen-2-carbonyl)-amino]-5,6,7,8-tetrahydro-chinazolin-6-ylcarbamoyl}-piperidin-1-carbonsäure tert-butyl ester |
| 89 | 1-(4-Chlor-phenyl)-cyclopropancarbonsäure (2-butyrylamino-5,6,7,8-tetrahydro-chinazolin-6-yl)-amid |
| 90 | N-(6-Ethansulfonyl-5,6,7,8-tetrahydro-pyrido[4,3-d]pyrimidin-2-yl)-3-methoxy-benzamid |
| 91 | 5-Methyl-thiophen-2-carbonsäure (2-butyrylamino-5,6,7,8-tetrahydro-chinazolin-6-yl)-amid |
| 92 | 4-tert-Butyl-N-[6-(4-methyl-3-nitro-benzoyl)-5,6,7,8-tetrahydro-pyrido[4,3-d]pyrimidin-2-yl]-benzamid |
| 94 | 4-Fluor-N-[6-(3-phenyl-propionylamino)-5,6,7,8-tetrahydro-chinazolin-2-yl]-benzamid |
| 95 | 3,4-Dichlor-N-[6-(2,4,6-trimethyl-benzolsulfonyl)-5,6,7,8-tetrahydro-pyrido[4,3-d]pyrimidin-2-yl]-benzamid |
| 96 | N-[6-(4-Methyl-3,4-dihydro-2H-benzo[1,4]oxazin-7-sulfonyl)-5,6,7,8-tetrahydro-pyrido[4,3-d]pyrimidin-2-yl]-nicotinamid |
| 97 | Thiophen-2-carbonsäure [6-(3,5-difluor-benzoylamino)-5,6,7,8-tetrahydro-chinazolin-2-yl]-amid |
| 98 | N-[6-(2,4-Difluor-benzolsulfonylamino)-5,6,7,8-tetrahydro-chinazolin-2-yl]-butyramid |
| 99 | Thiophen-2-carbonsäure [6-(2,3,5,6-tetramethyl-benzolsulfonyl)-5,6,7,8-tetrahydro-pyrido[4,3-d]pyrimidin-2-yl]-amid |
| 101 | N-[6-(3,4-Dichlor-benzoyl)-5,6,7,8-tetrahydro-pyrido[4,3-d]pyrimidin-2-yl]-4-ethyl-benzamid |
| 102 | N-[6-(7,7-Dimethyl-2-oxo-bicyclo[2.2.1]hept-1-ylmethansulfonyl)-5,6,7,8-tetrahydro-pyrido[4,3-d]pyrimidin-2-yl]-3-fluor-benzamid |
| 103 | N-[6-(6-Fluor-4H-benzo[1,3]dioxin-8-carbonyl)-5,6,7,8-tetrahydro-pyrido[4,3-d]pyrimidin-2-yl]-2-trifluormethyl-benzamid |
| 104 | [5-(2-Butyrylamino-5,6,7,8-tetrahydro-chinazolin-6-ylcarbamoyl)-pentyl]-carbaminsäure benzyl ester |
| 105 | 2-Ethoxy-N-[6-(4-oxo-pentanoyl)-5,6,7,8-tetrahydro-pyrido[4,3-d]pyrimidin-2-yl]-benzamid |
| 106 | Naphthalin-1-carbonsäure [6-(propan-1-sulfonyl)-5,6,7,8-tetrahydro-pyrido[4,3-d]pyrimidin-2-yl]-amid |
| 107 | N-[6-(5-Fluor-2-methyl-benzolsulfonyl)-5,6,7,8-tetrahydro-pyrido[4,3-d]pyrimidin-2-yl]-4-methyl-benzamid |
| 109 | 3-Chlor-N-[6-(2-methyl-benzoyl)-5,6,7,8-tetrahydro-pyrido[4,3-d]pyrimidin-2-yl]-benzamid |
| 110 | Thiophen-2-carbonsäure [6-(3-chlor-benzo[b]thiophen-2-carbonyl)-5,6,7,8-tetrahydro-pyrido[4,3-d]pyrimidin-2-yl]-amid |
| 112 | Thiophen-2-carbonsäure [6-(2-cyclopentyl-acetylamino)-5,6,7,8-tetrahydro-chinazolin-2-yl]-amid |
| 113 | N-[6-(3,4-Dichlor-benzoyl)-5,6,7,8-tetrahydro-pyrido[4,3-d]pyrimidin-2-yl]-2-fluor-benzamid |
| 114 | N-[6-(2-Chlor-benzolsulfonyl)-5,6,7,8-tetrahydro-pyrido[4,3-d]pyrimidin-2-yl]-4-ethyl-benzamid |
| 115 | Thiophen-2-carbonsäure [6-(4-brom-3-methyl-benzoylamino)-5,6,7,8-tetrahydro-chinazolin-2-yl]-amid |
| 116 | 3-Methyl-N-[6-(toluen-3-sulfonyl)-5,6,7,8-tetrahydro-pyrido[4,3-d]pyrimidin-2-yl]-benzamid |
| 117 | Naphthalin-1-carbonsäure [6-(5-chlor-thiophen-2-sulfonyl)-5,6,7,8-tetrahydro-pyrido[4,3-d]pyrimidin-2-yl]-amid |
| 118 | N-(2-Butyrylamino-5,6,7,8-tetrahydro-chinazolin-6-yl)-2,6-difluor-benzamid |
| 119 | 3-Chlor-N-[6-(3,4-dimethoxy-benzoyl)-5,6,7,8-tetrahydro-pyrido[4,3-d]pyrimidin-2-yl]-benzamid |
| 120 | N-[6-(3-Chlor-benzo[b]thiophen-2-carbonyl)-5,6,7,8-tetrahydro-pyrido[4,3-d]pyrimidin-2-yl]-benzamid |
| 121 | 4-Ethyl-N-[6-(thiophen-3-sulfonyl)-5,6,7,8-tetrahydro-pyrido[4,3-d]pyrimidin-2-yl]-benzamid |
| 123 | 2-Ethoxy-N-[6-(2-ethylsulfanyl-pyridin-3-carbonyl)-5,6,7,8-tetrahydro-pyrido[4,3-d]pyrimidin-2-yl]-benzamid |
| 124 | 3-[2-(3-Chlor-benzoylamino)-7,8-dihydro-5H-pyrido[4,3-d]pyrimidin-6-yl]-3-oxo-propionsäure ethyl ester |
| 125 | N-[6-(3-Brom-benzolsulfonyl)-5,6,7,8-tetrahydro-pyrido[4,3-d]pyrimidin-2-yl]-3-chlor-benzamid |
| 126 | 3,4-Dichlor-N-{6-[2-(4-chlor-phenyl)-acetyl]-5,6,7,8-tetrahydro-pyrido[4,3-d]pyrimidin-2-yl)-benzamid |
| 127 | 3-Chlor-N-[6-(2-chlor-6-fluor-benzoyl)-5,6,7,8-tetrahydro-pyrido[4,3-d]pyrimidin-2-yl]-benzamid |
| 128 | 4-Fluor-N-(6-hexanoylamino-5,6,7,8-tetrahydro-chinazolin-2-yl)-benzamid |
| 129 | N-[6-(5-Brom-2-methoxy-benzolsulfonyl)-5,6,7,8-tetrahydro-pyrido[4,3-d]pyrimidin-2-yl]-3-methoxy-benzamid |
| 132 | N-[6-(3-Cyclopentyl-propionyl)-5,6,7,8-tetrahydro-pyrido[4,3-d]pyrimidin-2-yl]-isonicotinamid |
| 133 | 5-Benzyl-furan-2-carbonsäure (2-butyrylamino-5,6,7,8-tetrahydro-chinazolin-6-yl)-amid |
| 134 | 3,4-Dichlor-N-[6-(4-trifluormethoxy-benzolsulfonyl)-5,6,7,8-tetrahydro-pyrido[4,3-d]pyrimidin-2-yl]-benzamid |
| 135 | [(2-Butyrylamino-5,6,7,8-tetrahydro-chinazolin-6-ylcarbamoyl)-phenyl-methyl]-carbaminsäure benzyl ester |
| 136 | N-[6-(3,4-Dimethoxy-benzolsulfonyl)-5,6,7,8-tetrahydro-pyrido[4,3-d]pyrimidin-2-yl]-4-fluor-benzamid |
| 137 | 2-Fluor-N-[6-(2-methyl-5-phenyl-furan-3-carbonyl)-5,6,7,8-tetrahydro-pyrido[4,3-d]pyrimidin-2-yl]-benzamid |
| 138 | Thiophen-2-carbonsäure (6-pent-4-enoylamino-5,6,7,8-tetrahydro-chinazolin-2-yl)-amid |
| 139 | (2-Methyl-1-{2-[(thiophen-2-carbonyl)-aminol-5,6,7,8-tetrahydro-chinazolin-6-ylcarbamoyl}-propyl)-carbaminsäure 9H-fluoren-9-ylmethyl ester |
| 140 | (5-{2-[(Thiophen-2-carbonyl)-amino]-5,6,7,8-tetrahydro-chinazolin-6-ylcarbamoyl}-pentyl)-carbaminsäure tert-butyl ester |
| 141 | 3-Fluor-N-[6-(1,2,3,4-tetrahydro-naphthalin-2-carbonyl)-5,6,7,8-tetrahydro-pyrido[4,3-d]pyrimidin-2-yl]-benzamid |
| 142 | 4-Chlor-N-{6-[2-(2,5-dioxo-imidazolidin-4-yl)-acetyl]-5,6,7,8-tetrahydro-pyrido[4,3-d]pyrimidin-2-yl}-benzamid |
| 143 | 3-Fluor-N-[6-(toluen-4-sulfonyl)-5,6,7,8-tetrahydro-pyrido[4,3-d]pyrimidin-2-yl]-benzamid |
| 144 | 3,5-Dichlor-N-[6-(4-thiophen-2-yl-benzoyl)-5,6,7,8-tetrahydro-pyrido[4,3-d]pyrimidin-2-yl]-benzamid |
| 145 | N-[6-(3-Chlor-thiophen-2-carbonyl)-5,6,7,8-tetrahydro-pyrido[4,3-d]pyrimidin-2-yl]-2-ethoxy-benzamid |
| 146 | N-[6-(Toluen-3-sulfonylamino)-5,6,7,8-tetrahydro-chinazolin-2-yl]-butyramid |
| 147 | Thiophen-2-carbonsäure {6-[2-(2,2,2-trifluor-acetyl)-1,2,3,4-tetrahydro-isochinolin-7-sulfonylamino]-5,6,7,8-tetrahydro-chinazolin-2-yl}-amid |
| 148 | 4-Methyl-N-[6-(4-trifluormethylsulfanyl-benzoyl)-5,6,7,8-tetrahydro-pyrido[4,3-d]pyrimidin-2-yl]-benzamid |
| 149 | (2-{2-[(Thiophen-2-carbonyl)-amino]-5,6,7,8-tetrahydro-chinazolin-6-ylcarbamoyl}-ethyl)-carbaminsäure tert-butyl ester |
| 150 | 2-Fluor-N-[6-(2-methyl-5-nitro-benzolsulfonyl)-5,6,7,8-tetrahydro-pyrido[4,3-d]pyrimidin-2-yl]-benzamid |
| 151 | N-[6-(4-Methoxy-benzolsulfonylamino)-5,6,7,8-tetrahydro-chinazolin-2-yl]-butyramid |
| 152 | 4-Chlor-N-[6-(2,4-difluor-benzoyl)-5,6,7,8-tetrahydro-pyrido[4,3-d]pyrimidin-2-y1]-benzamid |
| 153 | 4-Methyl-N-(6-pent-4-enoyl-5,6,7,8-tetrahydro-pyrido[4,3-d]pyrimidin-2-yl)-benzamid |
| 154 | Naphthalin-1-carbonsäure [6-(3-fluor-4-methyl-benzoyl)-5,6,7,8-tetrahydro-pyrido[4,3-d]pyrimidin-2-yl]-amid |
| 155 | N-{6-[2-(4-Trifluormethyl-phenyl)-acetyl]-5,6,7,8-tetrahydro-pyrido[4,3-d]pyrimidin-2-yl}-benzamid |
| 156 | N-[6-(3,4-Dichlor-benzoyl)-5,6,7,8-tetrahydro-pyrido[4,3-d]pyrimidin-2-yl]-nicotinamid |
| 157 | 4-Ethyl-N-{6-[2-(2,2,2-trifluor-acetyl)-1,2,3,4-tetrahydro-isochinolin-7-sulfonyl]-5,6,7,8-tetrahydro-pyrido[4,3-d]pyrimidin-2-yl}-benzamid |
| 158 | 2-Methoxy-N-[6-(4-methoxy-2,3,6-trimethyl-benzolsulfonyl)-5,6,7,8-tetrahydro-pyrido[4,3-d]pyrimidin-2-yl]-benzamid |
| 159 | 4-Ethy)-N-[6-(2-methoxy-acetyl)-5,6,7,8-tetrahydro-pyrido[4,3-d]pyrimidin-2-yl]-benzamid |
| 160 | 4-Fluor-N-[6-(propan-1-sulfonylamino)-5,6,7,8-tetrahydro-chinazolin-2-yl]-benzamid |
| 162 | N-[6-(7,7-Dimethyl-2-oxo-bicyclo[2.2.1]hept-1-ylmethansulfonylamino)-5,6,7,8-tetrahydro-chinazolin-2-yl]-butyramid |
| 163 | N-{6-[2-(2,5-Dimethyl-phenyl)-acetylamino]-5,6,7,8-tetrahydro-chinazolin-2-yl}-4-fluor-benzamid |
| 165 | Thiophen-2-carbonsäure (6-phenylmethansulfonylamino-5,6,7,8-tetrahydro-chinazolin-2-yl)-amid |
| 166 | N-[6-(3,4-Dimethoxy-benzolsulfonylamino)-5,6,7,8-tetrahydro-chinazolin-2-yl]-butyramid |
| 167 | N-{6-[4-(1,1-Dimethyl-propyl)-benzolsulfonylamino]-5,6,7,8-tetrahydro-chinazolin-2-yl}-butyramid |
| 168 | N-(2-Butyrylamino-5,6,7,8-tetrahydro-chinazolin-6-yl)-3-(3-trifluormethyl-phenyl)-acrylamid |
| 169 | N-[6-(Butan-1-sulfonylamino)-5,6,7,8-tetrahydro-chinazolin-2-yl]-4-fluor-benzamid |
| 170 | Naphthalin-1-carbonsäure (6-acety)-5,6,7,8-tetrahydro-pyrido[4,3-d]pyrimidin-2-yl)-amid |
| 171 | 3,5-Dichlor-N-[6-(3-diethylcarbamoyl-propionyl)-5,6,7,8-tetrahydro-pyrido[4,3-d]pyrimidin-2-yl]-benzamid |
| 172 | 4-Ethyl-N-[6-(4-methoxy-benzolsulfonyl)-5,6,7,8-tetrahydro-pyrido[4,3-d]pyrimidin-2-yl]-benzamid |
| 173 | 4-Ethyl-N-[6-(chinolin-6-carbonyl)-5,6,7,8-tetrahydro-pyrido[4,3-d]pyrimidin-2-yl]-benzamid |
| 174 | N-[6-(2-Cyclopropyl-acetyl)-5,6,7,8-tetrahydro-pyrido[4,3-d]pyrimidin-2-yl]-2-fluor-benzamid |
| 175 | N-[6-(2H-Chromen-3-carbonyl)-5,6,7,8-tetrahydro-pyrido[4,3-d]pyrimidin-2-yl]-3,4-difluor-benzamid |
| 176 | N-{6-[2-(4-Chlor-phenyl)-propionyl]-5,6,7,8-tetrahydro-pyrido[4,3-d]pyrimidin-2-yl}-benzamid |
| 177 | Thiophen-2-carbonsäure [6-(2-naphthalin-1-yl-acetylamino)-5,6,7,8-tetrahydro-chinazolin-2-yl]-amid |
| 178 | 4-Fluor-N-(6-phenylmethansulfonyl-5,6,7,8-tetrahydro-pyrido[4,3-d]pyrimidin-2-yl)-benzamid |
| 180 | Thiophen-2-carbonsäure [6-(2,5-dichlor-thiophen-3-sulfonylamino)-5,6,7,8-tetrahydro-chinazolin-2-yl]-amid |
| 181 | {5-[2-(4-Fluor-benzoylamino)-5,6,7,8-tetrahydro-chinazolin-6-ylcarbamoyl]-pentyl}-carbaminsäure benzyl ester |
| 182 | N-[6-(2,5-Dimethyl-2H-pyrazol-3-carbonyl)-5,6,7,8-tetrahydro-pyrido[4,3-d]pyrimidin-2-yl)-4-ethyl-benzamid |
| 183 | Naphthalin-1-carbonsäure [6-(3-fluor-benzoyl)-5,6,7,8-tetrahydro-pyrido[4,3-d]pyrimidin-2-yl]-amid |
| 184 | N-[6-(Benzo[b]thiophen-3-carbonyl)-5,6,7,8-tetrahydro-pyrido[4,3-d]pyrimidin-2-yl]-4-tert-butyl-benzamid |
| 185 | 4-Fluor-N-[6-(4-methoxy-benzolsulfonyl)-5,6,7,8-tetrahydro-pyrido[4,3-d]pyrimidin-2-yl]-benzamid |
| 186 | 4-tert-Butyl-N-[6-(2,3-dihydro-benzo[1,4]dioxin-6-sulfonyl)-5,6,7,8-tetrahydro-pyrido[4,3-d]pyrimidin-2-yl]-benzamid |
| 187 | 3-Chlor-N-{6-[2-(2,6-dichlor-phenyl)-acetyl]-5,6,7,8-tetrahydro-pyrido[4,3-d]pyrimidin-2-yl}-benzamid |
| 188 | Thiophen-2-carbonsäure [6-(2,3-dihydro-benzo[1,4]dioxin-6-sulfonylamino)-5,6,7,8-tetrahydro-chinazolin-2-yl]-amid |
| 189 | N-{6-[2-(3-Chlor-phenoxy)-acetyl]-5,6,7,8-tetrahydro-pyrido[4,3-d]pyrimidin-2-yl}-4-methyl-benzamid |
| 190 | N-[6-(5-Phenyl-pentanoyl)-5,6,7,8-tetrahydro-pyrido[4,3-d]pyrimidin-2-yl]-benzamid |
| 191 | 4-Ethyl-N-[6-(2-ethyl-butyryl)-5,6,7,8-tetrahydro-pyrido[4,3-d]pyrimidin-2-yl]-benzamid |
| 192 | 3,5-Dichlor-N-[6-(4-trifluormethoxy-benzoyl)-5,6,7,8-tetrahydro-pyrido[4,3-d]pyrimidin-2-yl]-benzamid |
| 193 | Naphthalin-1-carbonsäure [6-(4-methyl-octanoyl)-5,6,7,8-tetrahydro-pyrido[4,3-d]pyrimidin-2-yl]-amid |
| 194 | 4-[2-(4-Fluor-benzoylamino)-5,6,7,8-tetrahydro-chinazolin-6-ylcarbamoyl]-piperidin-1-carbonsäure tert-butyl ester |
| 195 | N-[6-(2-Benzyloxy-acetyl)-5,6,7,8-tetrahydro-pyrido[4,3-d]pyrimidin-2-yl]-2-methoxy-benzamid |
| 196 | N-[6-(2-Trifluormethyl-benzolsulfonylamino)-5,6,7,8-tetrahydro-chinazolin-2-yl]-butyramid |
| 197 | N-[2-(4-Fluor-benzoylamino)-5,6,7,8-tetrahydro-chinazolin-6-yl]-2-methyl-6-trifluormethyl-nicotinamid |
| 198 | N-[6-(3-Cyano-benzoyl)-5,6,7,8-tetrahydro-pyrido[4,3-d]pyrimidin-2-yl]-2-trifluormethyl-benzamid |
| 199 | 3-Methoxy-N-[6-(3-trifluormethoxy-benzoyl)-5,6,7,8-tetrahydro-pyrido[4,3-d]pyrimidin-2-yl]-benzamid |
| 200 | N-(6-Butyryl-5,6,7,8-tetrahydro-pyrido[4,3-d]pyrimidin-2-yl)-2-fluor-benzamid |
| 201 | N-(6-Benzolsulfonylamino-5,6,7,8-tetrahydro-chinazolin-2-yl)-4-fluor-benzamid |
| 202 | N-[6-(5-Chlor-thiophen-2-sulfonylamino)-5,6,7,8-tetrahydro-chinazolin-2-yl]-4-fluor-benzamid |
| 203 | Naphthalin-1-carbonsäure (6-hexanoyl-5,6,7,8-tetrahydro-pyrido[4,3-d]pyrimidin-2-yl)-amid |
| 204 | N-(6-Propionyl-5,6,7,8-tetrahydro-pyrido[4,3-d]pyrimidin-2-yl)-2-trifluormethyl-benzamid |
| 205 | N-[6-(2-Ethyl-butyryl)-5,6,7,8-tetrahydro-pyrido[4,3-d]pyrimidin-2-yl]-4-methyl-benzamid |
| 206 | 2-Fluor-N-[6-(3-trifluormethyl-benzolsulfonyl)-5,6,7,8-tetrahydro-pyrido[4,3-d]pyrimidin-2-yl]-benzamid |
| 207 | 5-Benzyl-furan-2-carbonsäure {2-[(thiophen-2-carbonyl)-amino]-5,6,7,8-tetrahydro-chinazolin-6-yl}-amid |
| 208 | Thiophen-2-carbonsäure {6-[2-(2,5-dimethyl-phenyl)-acetylamino]-5,6,7,8-tetrahydro-chinazolin-2-yl}-amid |
| 210 | 4-Fluor-N-[6-(propan-1-sulfonyl)-5,6,7,8-tetrahydro-pyrido[4,3-d]pyrimidin-2-yl]-benzamid |
| 211 | 2-[2-(3-Fluor-benzoylamino)-7,8-dihydro-5H-pyrido[4,3-d]pyrimidin-6-sulfonyl)-benzoesäure methyl ester |
| 212 | 3,5-Dichlor-N-[6-(5-[1,2]dithiolan-3-yl-pentanoyl)-5,6,7,8-tetrahydro-pyrido[4,3-d]pyrimidin-2-yl]-benzamid |
| 213 | Thiophen-2-carbonsäure [6-(2-phenoxy-butyrylamino)-5,6,7,8-tetrahydro-chinazolin-2-yl]-amid |
| 214 | Thiophen-2-carbonsäure {6-[2-(4-methoxy-phenyl]-acetylamino]-5,6,7,8-tetrahydro-chinazolin-2-yl}-amid |
| 215 | N-(6-Cyclohexancarbonyl-5,6,7,8-tetrahydro-pyrido[4,3-d]pyrimidin-2-yl)-2-trifluormethyl-benzamid |
| 216 | N-[6-(2,4-Dimethyl-thiazol-5-sulfonyl)-5,6,7,8-tetrahydro-pyrido[4,3-d]pyrimidin-2-yl]-2-trifluormethyl-benzamid |
| 217 | 2-Thiophen-2-yl-thiazol-4-carbonsäure {2-[(thiophen-2-carbonyl)-amino]-5,6,7,8-tetrahydro-chinazolin-6-yl}-amid |
| 219 | Thiophen-2-carbonsäure {6-[3-(3-trifluormethyl-phenyl)-acryloylamino]-5,6,7,8-tetrahydro-chinazolin-2-yl}-amid |
| 220 | Thiophen-2-carbonsäure [6-(5-phenyl-pentanoylamino)-5,6,7,8-tetrahydro-chinazolin-2-yl]-amid |
| 221 | Thiophen-2-carbonsäure [6-(2-chlor-5-trifluormethyl-benzoylamino)-5,6,7,8-tetrahydro-chinazolin-2-yl]-amid |
| 222 | 4-Fluor-N-[6-(2,3,5,6-tetramethyl-benzolsulfonylamino)-5,6,7,8-tetrahydro-chinazolin-2-yl]-benzamid |
| 223 | 4-Fluor-N-[6-(4-methyl-octanoyl)-5,6,7,8-tetrahydro-pyrido[4,3-d]pyrimidin-2-yl]-benzamid |
| 224 | 2-{2-[(Thiophen-2-carbonyl)amino]-5,6,7,8-tetrahydro-chinazolin-6-ylsulfamoyl}-benzoesäure methyl ester |
| 225 | [(3-Methyl-1-{2-[(thiophen-2-carbonyl)-amino]-5,6,7,8-tetrahydro-chinazolin-6-ylcarbamoyl}-butylcarbamoyl)-methyl]-carbaminsäure benzyl ester |
| 226 | 3-Chlor-N-(6-pentanoyl-5,6,7,8-tetrahydro-pyrido[4,3-d]pyrimidin-2-yl)-benzamid |
| 227 | 4-Fluor-N-[6-(5-oxo-5-phenyl-pentanoylamino)-5,6,7,8-tetrahydro-chinazolin-2-yl]-benzamid |
| 228 | Thiophen-2-carbonsäure [6-(3-phenyl-acryloylamino)-5,6,7,8-tetrahydro-chinazolin-2-yl]-amid |
| 229 | N-[6-(5-[1,2]Dithiolan-3-yl-pentanoylamino)-5,6,7,8-tetrahydro-chinazolin-2-yl]-4-fluor-benzamid |
| 230 | Benzoesäure 2-(2-{2-[(thiophen-2-carbonyl)-amino]-5,6,7,8-tetrahydro-chinazolin-6-ylcarbamoyl}-ethyl)-phenyl ester |
| 231 | 4-Fluor-N-[6-(2-methyl-5-nitro-benzolsulfonylamino)-5,6,7,8-tetrahydro-chinazolin-2-yl]-benzamid |
| 232 | 4-Chlor-N-[6-(2-phenoxy-acetyl)-5,6,7,8-tetrahydro-pyrido[4,3-d]pyrimidin-2-yl]-benzamid |
| 234 | N-[6-(5-Brom-thiophen-2-carbonyl)-5,6,7,8-tetrahydro-pyrido[4,3-d]pyrimidin-2-yl]-2-fluor-benzamid |
| 235 | 4-Fluor-N-[6-(5-fluor-2-methyl-benzolsulfonylamino)-5,6,7,8-tetrahydro-chinazolin-2-yl]-benzamid |
| 236 | N-[6-(4-Acetylamino-benzolsulfonylamino)-5,6,7,8-tetrahydro-chinazolin-2-yl]-4-fluor-benzamid |
| 237 | Thiophen-2-carbonsäure [6-(2-trifluormethyl-benzoylamino)-5,6,7,8-tetrahydro-chinazolin-2-yl]-amid |
| 238 | 3,4-Difluor-N-[6-(chinolin-6-carbonyl)-5,6,7,8-tetrahydro-pyrido[4,3-d]pyrimidin-2-yl]-benzamid |
| 239 | {1-[2-(4-Fluor-benzoylamino)-5,6,7,8-tetrahydro-chinazolin-6-ylcarbamoyl]-2-phenyl-ethyl}-carbaminsäure tert-butyl ester |
| 240 | Thiophen-2-carbonsäure [6-(2-methyl-benzoylamino)-5,6,7,8-tetrahydro-chinazolin-2-yl]-amid |
| 241 | 3-Chlor-N-{6-[3-(2-oxo-benzooxazol-3-yl)-propionyl]-5,6,7,8-tetrahydro-pyrido[4,3-d]pyrimidin-2-yl}-benzamid |
| 242 | 3-Chlor-N-[6-(5-chlor-thiophen-2-sulfonyl)-5,6,7,8-tetrahydro-pyrido[4,3-d]pyrimidin-2-yl]-benzamid |
| 243 | 3,5-Dichlor-N-[6-(4-methoxy-benzolsulfonyl)-5,6,7,8-tetrahydro-pyrido[4,3-d]pyrimidin-2-yl]-benzamid |
| 244 | 4-tert-Butyl-N-[6-(5-methyl-isoxazol-3-carbonyl)-5,6,7,8-tetrahydro-pyrido[4,3-d]pyrimidin-2-yl]-benzamid |
| 245 | N-[6-(5-Brom-2-methoxy-benzolsulfonyl)-5,6,7,8-tetrahydro-pyrido[4,3-d]pyrimidin-2-yl]-4-methyl-benzamid |
| 246 | 4-tert-Butyl-N-{6-[3-(2-hydroxy-phenyl)-propionyl]-5,6,7,8-tetrahydro-pyrido[4,3-d]pyrimidin-2-yl}-benzamid |
| 247 | 4-Fluor-N-[6-(2-phenyl-butyrylamino)-5,6,7,8-tetrahydro-chinazolin-2-yl]-benzamid |
| 248 | 4-tert-Butyl-N-[6-(4-propyl-benzoyl)-5,6,7,8-tetrahydro-pyrido[4,3-d]pyrimidin-2-yl]-benzamid |
| 249 | Thiophen-2-carbonsäure [6-(2-brom-benzoylamino)-5,6,7,8-tetrahydro-chinazolin-2-yl]-amid |
| 250 | 2-Methoxy-N-[6-(6-phenoxy-pyridin-3-carbonyl)-5,6,7,8-tetrahydro-pyrido[4,3-d]pyrimidin-2-yl]-benzamid |
| 251 | N-[6-(4-Acetylamino-benzolsulfonyl)-5,6,7,8-tetrahydro-pyrido[4,3-d]pyrimidin-2-yl]-4-ethyl-benzamid |
| 252 | 3,5-Dichlor-N-[6-(4-fluor-benzolsulfonyl)-5,6,7,8-tetrahydro-pyrido[4,3-d]pyrimidin-2-yl]-benzamid |
| 253 | N-[6-(1-Benzolsulfonyl-1H-indol-2-carbonyl)-5,6,7,8-tetrahydro-pyrido[4,3-d]pyrimidin-2-yl]-4-fluor-benzamid |
| 255 | N-[6-(3-Chlorbenzolsulfonylamino)-5,6,7,8-tetrahydro-chinazolin-2-yl]-4-fluor-benzamid |

### Pharmakologische Daten:

Die Affinität der erfindungsgemäßen substituierten 5,6,7,8-Tetrahydro-pyrido[4,3-d]pyrimidin-2-yl- bzw. 5,6,7,8-Tetrahydro-chinazolin-2-yl-Verbindungen für die Batrachotoxin-(BTX)-Bindungsstelle, für den mGluR5-Rezeptor sowie die Hemmung der Noradrenalin- bzw. 5-HT-Wiederaufnahme, wurde wie vorstehend beschrieben bestimmt.

Die erfindungsgemäßen substituierten 5,6,7,8-Tetrahydro-pyrido[4,3-d]pyrimidin-2-yl-bzw. 5,6,7,8-Tetrahydro-chinazolin-2-yl-Verbindungen zeigen eine gute bis sehr gute Inhibierung der Noradrenalin-Wiederaufnahme sowie eine gute bis sehr gute Inhibierung der 5-Hydroxy-Tryptophan-Wiederaufnahme.

Darüber hinaus zeigen diese erfindungsgemäßen Verbindungen auch ausgezeichnete Affinitäten für die Batrachotoxin-(BTX)-Bindungsstelle des Nätriumkanals und den mGluR5-Rezeptor.

In der nachfolgenden Tabelle I sind die jeweiligen pharmakologischen Daten für einige beispielgemäße substituierte 5,6,7,8-Tetrahydro-pyrido[4,3-d]pyrimidin-2-yl-bzw. 5,6,7,8-Tetrahydro-chinazolin-2-yl-Verbindungen wiedergegeben.

**Tabelle I:**

| **Verbindung gemäß Beispiel** | **mGluR5 (MPEP), 10 µM, % Hemmung** | **BTX Inhierung** | **5-HT-Uptake, Ratte, 1µM, % Hemmung** | **Na-Uptake Ratte, 10 µM, % Hemmung** |
|---|---|---|---|---|
| 29 | 40,51 | | | |
| 68 | | 35 | | |
| 69 | | 64 | 70 | 73 |
| 71 | | 69 | 47 | 74 |
| 72 | | 31 | | 41 |
| 74 | | 66 | 54 | 43 |
| 78 | 41,06 | 31 | | |
| 79 | | 34 | | |
| 80 | | | | 54 |
| 82 | 36,33 | 38 | | 32 |
| 83 | | | 39 | 77 |
| | | | | |
| 86 | | 62 | | 32 |
| 92 | | 75 | 66 | 35 |
| 94 | | 47 | | |
| 95 | | 77 | 59 | 72 |
| 97 | | | 30 | |
| 99 | | 78 | 41 | 56 |
| 101 | | 68 | 65 | 84 |
| 102 | | | 63 | |
| 103 | | | 65 | |
| 107 | 38,23 | 73 | 36 | 39 |
| 109 | | | 30 | 92 |
| 110 | | 40 | 96 | 75 |
| 113 | 31,26 | 38 | 45 | 67 |
| 114 | | 82 | 50 | 73 |
| 115 | 34,26 | 82 | 56 | 49 |
| 116 | | 53 | 31 | 52 |
| 117 | | 37 | 32 | 31 |
| 119 | | | 34 | 72 |
| 120 | | 48 | 92 | 78 |
| 121 | | 57 | 52 | 92 |
| 124 | | | | 65 |
| 125 | | 66 | 43 | 70 |
| 126 | | 70 | 55 | 89 |
| 127 | | 30 | 35 | 71 |
| 128 | | 33 | | |
| 129 | 51,14 | 76 | | |
| 132 | 58,08 | 72 | | |
| 133 | | 39 | | |
| 134 | | 64 | 58 | 59 |
| 137 | | 43 | | |
| 139 | 31,02 | 62 | | |
| 141 | | 63 | 54 | 55 |
| 143 | 37,25 | 61 | | 53 |
| 144 | | 81 | 42 | 67 |
| 145 | 84,43 | 46 | | |
| 146 | 39,64 | | | |
| 147 | | 48 | | |
| 148 | | 85 | 32 | |
| 149 | | | 32 | |
| 150 | | 49 | 54 | |
| 152 | 34,01 | 55 | 33 | |
| 154 | 30,72 | | | 50 |
| 155 | | 62 | | |
| 156 | 45,96 | | | 50 |
| 157 | | 63 | 43 | 61 |
| 158 | | 74 | 55 | 36 |
| 160 | | | 40 | |
| 162 | | | 29 | |
| 163 | | 28 | | |
| 165 | | | 35 | |
| 167 | | 51 | 32 | |
| 168 | 33,45 | 39 | 46 | |
| 171 | | | 32 | |
| 172 | 70,12 | 57 | 57 | 85 |
| 173 | | | 69 | |
| 175 | | 49 | 32 | 46 |
| 176 | | | | 38 |
| 177 | 41,98 | | 39 | |
| 178 | 45,31 | | | |
| 180 | 86,74 | 46 | | 34 |
| 181 | 36,39 | | | |
| 183 | | 31 | | 63 |
| 184 | | 87 | 48 | 69 |
| 185 | | | | 36 |
| 186 | | 77 | 46 | 82 |
| 187 | | 43 | 29 | 100 |
| 189 | 39,49 | 55 | 46 | 53 |
| 190 | | 56 | 36 | 73 |
| 192 | | 87 | 47 | 73 |
| 193 | 51,83 | 84 | 38 | 43 |
| 194 | 45,64 | | | |
| 195 | | 35 | | 31 |
| 197 | 32,15 | | | |
| 199 | | 48 | | 52 |
| 202 | 33,07 | | | |

## Patentansprüche

1. Substituierte 5,6,7,8-Tetrahydro-pyrido[4,3-d]pyrimidin-2-yl- und 5,6,7,8-Tetrahydro-chinazolin-2-yl-Verbindungen der allgemeinen Formel I, 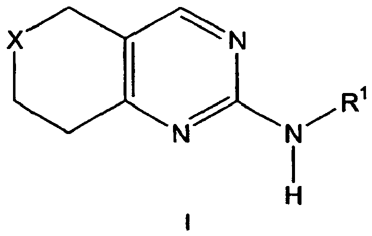 worin
X für eine C(H)(NHR²)-Gruppe oder für eine NR^{2a}-Gruppe steht,
R¹ für eine -C(=O)-R³-Gruppe oder für eine -C(=O)-O-R⁴-Gruppe steht,
R², R^{2a}, unabhängig voneinander, jeweils für eine -C(=O)-R⁵-Gruppe oder für eine -S(=O)₂-R⁶-Gruppe stehen,
R³ für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₈-Rest, für einen unsubstituierten oder wenigstens einfach substituierten, gesättigten oder ungesättigten, ggf. wenigstens ein Heteroatom als Ringglied aufweisenden, 3-bis 8-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₃-Alkylen-Gruppe gebunden sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₃-Alkylen-Gruppe gebunden sein kann, steht,
R⁴ für einen unsubstituierten oder wenigstens einfach substituierten 5- bis 14-gliedrigen Aryl- oder Heteroaryl-Rest steht, der über eine lineare oder verzweigte, unsubstituierte oder wenigstens einfach substituierte C₁₋₃-Alkylen-Gruppe gebunden sein kann,
R⁵ für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₁₀ Rest,
für einen unsubstituierten oder wenigstens einfach substituierten, gesättigten oder ungesättigten, ggf. wenigstens ein Heteroatom als Ringglied aufweisenden 3- bis 8-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₆-Alkylen-Gruppe gebunden und/oder mit einem unsubstituierten oder wenigstens einfach substituierten mono- oder polyzyklischen Ringssystem kondensiert sein kann,
für einen unsubstituierten oder wenigstens einfach substituierten, 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₆-Alkylen-, C₂₋₆-Alkenylen- oder C₂₋₆-Alkinylen-Gruppe gebunden und/oder mit einem unsubstituierten oder wenigstens einfach substituierten, mono- oder polyzyklischen Ringsystem kondensiert sein kann,
für einen -C(=O)-R⁷-Rest, der über eine lineare oder verzweigte, unsubstituierte oder wenigstens einfach substituierte C₁₋₃-Alkylen-Gruppe gebunden sein kann,
für einen -C(=O)-O-R⁸-Rest, der über eine lineare oder verzweigte, unsubstituierte oder wenigstens einfach substituierte C₁₋₃-Alkylen-Gruppe gebunden sein kann, oder
für einen -N(H)-C(=O)-O-R⁹-Rest, der über eine lineare oder verzweigte, unsubstituierte oder wenigstens einfach substituierte, ggf. wenigstens eine -N(H)-C(=O)- oder wenigstens eine -C(=O)-N(H)-Gruppierung als Kettenglied aufweisende C₁₋₈Alkylen-Gruppe gebunden sein kann, steht,
R⁶ für eine Gruppe -NR¹⁰R¹¹, für einen linearen oder verzweigten, unsubstituierten oder wenigstens einfach substituierten, gesättigten oder ungesättigten, ggf. wenigstens ein Heteroatom als Kettenglied aufweisenden aliphatischen C₁₋₁₀ Rest,
für einen unsubstituierten oder wenigstens einfach substituierten, gesättigten oder ungesättigten, ggf. wenigstens ein Heteroatom als Ringglied aufweisenden, ggf. mit einem unsubstituierten oder wenigstens einfach substituierten, mono- oder polyzyklischen Ringsystem kondensierten drei- bis acht-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₆-Alkylen-Gruppe gebunden und/oder mit einer linearen oder verzweigten C₁₋₆ Alkylen-Gruppe überbrückt sein kann, oder
für einen unsubstituierten oder wenigstens einfach substituierten 5- bis 14-gliedrigen Aryl- oder Heteroaryl-Rest steht, der über eine lineare oder verzweigte, unsubstituierte oder wenigstens einfach substituierte C₁₋₆-Alkylen-Gruppe gebunden und/oder mit einem unsubstituierten oder wenigstens einfach substituierten mono- oder polyzyklischen Ringsystem kondensiert sein kann, steht,
R⁷ für einen linearen oder verzweigten, unsubstituierten oder wenigstens einfach substituierten C₁₋₃-Alkyl-Rest, für einen unsubstituierten oder wenigtens einfach substituierten 5- bis 14- gliedrigen Aryl- oder Heteroaryl-Rest, für einen unsubstituierten oder wenigstens einfach substituierten, gesättigten oder ungesättigten, ggf. wenigstens ein Heteroatom als Ringglied aufweisenden 5-, 6- oder 7-gliedrigen cycloaliphatischen Rest, der mit einem unsubstituierten oder wenigstens einfach substituierten monozyklischen Ringsystem kondensiert sein kann, oder für einen -NR^{7a}R^{7b}-Rest steht, worin die Reste R^{7a} und R^{7b}, gleich oder verschieden, jeweils für einen linearen oder verzweigten C₁₋₅-Alkyl-Rest stehen,
R⁸ für einen linearen oder verzweigten, unsubstituierten oder wenigstens einfach substituierten C₁₋₃-Alkyl-Rest oder für einen unsubstituierten oder wenigstens einfach substituierten, ggf. über eine lineare oder verzweigte C₁₋₃-Alkylen-Gruppe gebundenen 5- oder 6-gliedrigen Aryl- oder Heteroaryl-Rest steht,
R⁹ für einen linearen oder verzweigten, unsubstituierten oder wenigstens einfach substituierten C₁₋₃-Alkyl-Rest, für einen unsubstituierten oder wenigtens einfach substituierten, ggf. über eine lineare oder verzweigte C₁₋₃-Alkylen-Gruppe gebundenen, 5- oder 6-gliedrigen Aryl- oder Heteroaryl-Rest, oder für einen unsubstituierten oder wenigstens einfach substituierten, gesättigten oder ungesättigten, 5-oder 6-gliedrigen cycloaliphatischen Rest, der mit wenigstens einem unsubstituierten oder wenigstens einfach substituierten monozyklischen Ringsystem kondensiert sein kann, steht,
R¹⁰ und R¹¹, gleich oder verschieden, jeweils für einen linearen oder verzweigten C₁₋₅-Alkyl-Rest stehen,
wobei
sofern einer oder mehrere der Substituenten R³ und R⁵-R⁹ für einen gesättigten oder ungesättigten aliphatischen Rest, d.h. für einen Alkyl-, Alkenyl- oder Alkinyl-Rest, stehen, der 1-, 2-, 3-, 4-, 5-, 6-, 7-, 8- oder 9-fach substituiert ist, dessen Substituenten, jeweils unabhängig voneinander, ausgewählt sind aus der Gruppe bestehend aus F, Cl, Br, -OH, -SH und -NH₂;
sofern einer oder mehrere der Substituenten R³, R⁵ und R⁶ für einen gesättigten oder ungesättigten aliphatischen Rest, d.h. für einen Alkyl-, Alkenyl- oder Alkinyl-Rest, stehen, der 1, 2, 3, 4 oder 5 Heteroatome als Kettenglied(er) aufweist, diese Heteroatome, jeweils unabhängig voneinander, ausgewählt sind aus der Gruppe bestehend aus Sauerstoff, Schwefel und Stickstoff (NH);
sofern einer oder mehrere der Substituenten R³, R⁵-R⁷ und R⁹ für einen cycloaliphatischen Rest steht oder einen cycloaliphatischen Rest aufweist, der 1-, 2-, 3-, 4- oder 5-fach substituiert ist, dessen Substituenten, jeweils unabhängig voneinander, ausgewählt sind aus der Gruppe bestehend aus -C₁₋₅-Alkyl, -C(=O)-O-C₁₋₅-Alkyl, -C(=O)-CF₃, -S(=O)₂-C₁₋₅-Alkyl, -S(=O)₂-Pheny), oxo (=O) und Phenyl, wobei die C₁₋₅-Alkyl-Reste jeweils linear oder verzweigt und die Phenyl-Reste jeweils 1-, 2-, 3-, 4- oder 5-fach, gleich oder verschieden, mit einem Substituenten ausgewählt aus der Gruppe bestehend aus F, Cl, Br und I substituiert sein können;
sofern die cycloaliphatischen Reste 1, 2 oder 3 Heteroatome als Ringglieder aufweisen, diese, jeweils unabhängig voneinander, ausgewählt sind aus der Gruppe bestehend aus Stickstoff, Sauerstoff und Schwefel;
sofern einer oder mehrere der Substituenten R³-R⁹ für einen Aryl- oder Heteroaryl-Rest stehen oder einen Aryl- oder Heteroaryl-Rest aufweisen, der 1-, 2-, 3-, 4- oder 5-fach substituiert ist, dessen Substituenten, jeweils unabhängig voneinander, ausgewählt sind aus der Gruppe bestehend aus Halogen, -CN, -NO₂, -OH, -SH, C₁₋₅-Alkyl, C₁₋₅-Alkoxy, -S-C₁₋₅-Alkyl, -CF₃, -CHF₂, -CH₂F, -O-CF₃, -O-CHF₂, -O-CH₂F,-C(=O)-CF₃, -S-CF₃, -S-CHF₂, -S-CH₂F, -S(=O)₂-Phenyl, -S(=O)₂-C₁₋₅-Alkyl, N(C₁₅Alkyl)(C₁₋₅-Alkyl), -C(=O)-O-C₁₋₅-Alkyl, -CH₂-O-C(=O)-Phenyl, -O-C(=O)-Phenyl, -NH-C(=O)-C₁₋₅-Alkyl, -C≡C-Phenyl, -C≡C-Naphthyl, -C≡C-Pyrrolyl, -C≡C-Indolyl, -C=C-Furyl (-C≡C-Furanyl), -C≡C-Benzo[b]furanyl, -C≡C-Thienyl (-C≡C-Thiophenyl), -C≡C-Benzo[b]thienyl, -C≡C-Pyrazolyl, -C≡C-Imidazolyl, -C≡C-Thiazolyl, -C≡C-Thiadiazolyl, -C≡C-Triazolyl, -C≡C-Oxazolyl, -C≡C-Isoxazolyl, -C≡C-Pyridinyl, -C≡C-Pyridazinyl,-C≡C-Pyrimidinyl, -C≡C-Pyrazinyl, -C≡C-Pyranyl, -C≡C-Indazolyl, -C≡C-Purinyl, -C≡C-Indolizinyl, -C≡C-Chinolinyl, -C≡C-Isochinolinyl, -C≡C-Chinazolinyl, Pyrazolyl, Phenyl, Furyl (Furanyl), Thiadiazolyl, Thiophenyl (Thienyl), Phenoxy und Benzyl, wobei die zyklischen Substituenten selbst jeweils 1-, 2-, 3-, 4- oder 5-fach, gleich oder verschieden, mit einem Substituenten ausgewählt aus der Gruppe bestehend aus F, Cl, Br, I substituiert sein können;
sofern einer oder mehrere der Substituenten R³-R⁹ für einen Heteroaryl-Rest stehen oder einen Heteroaryl-Rest aufweisen, dessen 1, 2, 3, 4 oder 5 Heteroatome, jeweils unabhängig voneinander, ausgewählt sind aus der Gruppe bestehend aus Sauerstoff, Schwefel und Stickstoff;
sofern einer der vorstehend genannten Substituenten R³-R⁶ eine lineare oder verzweigte Alkylen-, Alkenylen- oder Alkinylen-Gruppe aufweist, die 1-, 2-, 3-, 4- oder 5-fach substituiert ist, deren Substituenten, jeweils unabhängig voneinander, ausgewählt sind aus der Gruppe bestehend aus F, Cl, Br, Hydroxy und unsubstituiertem Phenyl;
sofern die Alkylen-, Alkenylen- oder Alkinylen-Gruppe 1, 2, 3, 4 oder 5 Heteroatome als Kettenglied(er) aufweist, diese ausgewählt sind aus der Gruppe bestehend aus Sauerstoff, Schwefel und Stickstoff (NH);
und wobei
unter einem mono- bzw. polyzyklischen Ringsystem mono- bzw. polyzyklische Kohlenwasserstoffreste verstanden werden, die gesättigt, ungesättigt oder aromatisch sein und ggf. 1, 2, 3, 4 oder 5 Heteroatome als Ringglieder aufweisen können, wobei die verschiedenen Ringe, jeweils unabhängig voneinander, einen unterschiedlichen Sättigungsgrad aufweisen können, d.h. gesättigt, ungesättigt oder aromatisch sein können; und
sofern ein polyzyklisches Ringsystem vorliegt, die verschiedenen Ringe, jeweils unabhängig voneinander, einen unterschiedlichen Sättigungsgrad aufweisen können, d.h. gesättigt, ungesättigt oder aromatisch sein können und die Heteroatome jedes Ringes, jeweils gleich oder verschieden, ausgewählt werden können aus der Gruppe bestehend aus Sauerstoff, Stickstoff und Schwefel; und
sofern einer oder mehrere der Substituenten R⁵, R⁶, R⁷ und R⁹ ein monozyklisches oder polyzyklisches Ringsystem aufweisen, das 1-, 2-, 3-, 4- oder 5-fach substituiert ist, dessen Substituenten, jeweils unabhängig voneinander, ausgewählt sind aus der Gruppe bestehend aus Halogen, -CN, -NO₂, -OH, -SH, C₁₋₅-Alkyl, C₁₋₅-Alkoxy, -S-C₁₋₅-Alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, N(C₁₋₅Alkyl)(C₁₋₅-Alkyl), -C(=O)-O-C₁₋₅-Alkyl, CH₂-O-C(=O)-Phenyl, -O-C(=O)-Phenyl, -NH-C(=O)-C₁₋₅-Alkyl, -C≡C-Phenyl,-C≡C-Naphthyl, -C≡C-Pyrrolyl, -C≡C-Indolyl, -C≡C-Furyl (-C≡C-Furanyl), -C≡C-Benzo[b]furanyl, -C≡C-Thienyl (-C≡C-Thiophenyl), -C≡C-Benzo[b]thienyl, -C≡C-Pyrazolyl, -C≡C-Imidazolyl, -C≡C-Thiazolyl, -C≡C-Thiadiazolyl, -C≡C-Triazotyl, -C≡C-Oxazolyl, -C≡C-Isoxazolyl, -C≡C-Pyridinyl, -C≡C-Pyridazinyl, -C≡C-Pyrimidinyl, -C≡C-Pyrazinyl, -C≡C-Pyranyl, -C≡C-Indazolyl, -C≡C-Purinyl, -C≡C-Indolizinyl, -C≡C-Chinolinyl, -C≡C-Isochinofinyl, -C≡C-Chinazolinyl Pyrazolyl, Phenyl, Furyl (Furanyl), Thiadiazolyl, Thiophenyl (Thienyl), Phenoxy und Benzyl, wobei die zyklischen Substituenten selbst jeweils 1-, 2-, 3-, 4- oder 5-fach, gleich oder verschieden, mit einem Substituenten ausgewählt aus der Gruppe bestehend aus F, Cl, Br, I substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, daß** R³ für einen linearen oder verzweigten, unsubstituierten oder wenigstens einfach substituierten, ggf. eines oder mehrere Sauerstoffatome und/oder eine oder mehrere NH-Gruppen als Kettenglied(er) aufweisenden C₁₋₈-Alkyl-Rest, für einen unsubstituierten oder wenigstens einfach substituierten, gesättigten oder ungesättigten, ggf. wenigstens ein Heteroatom als Ringglied aufweisenden, 5-, 6- oder 7-gliedrigen cycloaliphatischen Rest, der über eine lineare oder verzweigte, unsubstituierte oder wenigstens einfach substituierte, ggf. wenigstens ein Heteroatom als Kettenglied aufweisende C₁₋₃-Alkylen-Gruppe gebunden sein kann, oder für einen unsubstituierten oder wenigstens einfach substituierten Phenyl-Rest, Thiophenyl-Rest (Thienyl-Rest), Furanyl-Rest (Furyl-Rest), Pyridinyl-Rest oder Naphthyl-Rest, der über eine lineare oder verzweigte, unsubstituierte oder wenigstens einfach substituierte C₁₋₃-Alkylen-Gruppe gebunden sein kann, steht.

3. Verbindungen gemäß Anspruche 1 oder 2, **dadurch gekennzeichnet, daß** der Rest R⁴ für einen gegebenenfalls über eine -(CH₂)-, -(CH₂)₂- oder -CH₂)₃-Brücke gebundenen Phenyl-Rest steht, wobei der Phenyl-Ring unsubstituiert oder wenigstens einfach, gleich oder verschieden, mit einem Substituenten ausgewählt aus der Gruppe bestehend Methyl, Ethyl, n-Propyl, iso-Propyl, n-Butyl, iso-Butyl, sec-Butyl, tert.-Butyl, Methoxy, Ethoxy, n-Propoxy, iso-Propoxy, n-Butoxy, iso-Butoxy, sec-Butoxy, tert.-Butoxy, F, Cl, Br, I, -CN und -CF₃ substituiert sein kann.

4. Verbindungen gemäß einem oder mehreren der Ansprüche 1-3, **dadurch gekennzeichnet, daß**
R⁵ für einen linearen oder verzweigten, ggf. eines oder mehrere Sauerstoffatome und/oder ggf. eines oder mehrere Schwefelatome und/oder ggf. eine oder mehrere-N(H)-Gruppen als Kettenglied(er) aufweisenden C₁₋₁₀Alkyl-Rest, für einen linearen oder verzweigten, ggf. eines oder mehrere Sauerstoffatome und/oder ggf. eines oder mehrere Schwefelatome und/oder ggf. eine oder mehrere -N(H)-Gruppen als Kettenglied(er) aufweisenden C₂₋₁₀ Alkenyl-Rest, für einen linearen oder verzweigten, ggf. eines oder mehrere Sauerstoffatome und/oder ggf. eines oder mehrere Schwefelatome und/oder ggf. eine oder mehrere -N(H)-Gruppen als Kettenglied(er) aufweisenden C₂₋₁₀Alkinyl-Rest,
für einen unsubstituierten oder wenigstens einfach substituierten, gesättigten oder ungesättigen, ggf. eines oder mehrere Heteroatome, unabhängig voneinander, ausgewählt aus der Gruppe bestehend aus Sauerstoff, Schwefel und Stickstoff als Ringglied(er) aufweisenden 3- bis 8-gliedrigen cycloaliphatischen Rest, der über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-,-(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -C(CH₃)₂- oder -C(H)(CH₃)-Gruppe gebunden und/oder mit einem unsubstituierten oder wenigstens einfach substituierten, 5- oder 6-gliedrigen monozyklischen Ringssystem kondensiert sein kann,
für einen unsubstituierten oder wenigstens einfach substituierten, 5- oder 6-gliedrigen Aryl- oder Heteroaryl-Rest, der über eine -(CH₂)-, -(CH₂)₂-, -(CH₂)₃-, -(CH₂)₄-,-C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-,-C(CH₃)₂-, -C(H)(CH₃)- oder -CH=CH-Gruppe gebunden und/oder mit einem unsubstituierten oder wenigstens einfach substituierten 5- oder 6-gliedrigen, monozyklischen Ringsystem kondensiert sein kann,
für einen -C(=O)-R⁷-Rest, der über eine -(CH₂)-, -(CH₂)₂- oder -(CH₂)₃-Gruppe gebunden sein kann,
für einen -C(=O)-O-R⁸-Rest, der über eine lineare oder verzweigte, unsubstituierte oder einfach oder mehrfach mit einem Phenyl-Rest substituierte C₁₋₃-Akylen-Gruppe gebunden sein kann, oder
für einen -N(H)-C(=O)-O-R⁹-Rest, der über eine lineare oder verzweigte, unsubstituierte oder einfach oder mehrfach mit einem Phenyl-Rest substituierte, ggf. wenigstens eine -N(H)-C(=O)- oder wenigstens eine -C(=O)-N(H)-Gruppierung als Kettenglied aufweisende C₁₋₈-Alkylen-Gruppe gebunden sein kann, steht.

5. Verbindungen gemäß einem oder mehreren der Ansprüche 1-4, **dadurch gekennzeichnet, daß** R⁶ für eine Gruppe -NR¹⁰R¹¹,
für einen linearen oder verzweigten, unsubstituierten C₁₋₁₀Alkyl-Rest, einen linearen oder verzweigten, unsubstituierten C₂₋₁₀-Alkenyl-Rest, für einen linearen oder verzweigten, unsubstituierten, C₂₋₁₀-Alkinyl-Rest,
für einen unsubstituierten oder wenigstens einfach substituierten, gesättigten oder ungesättigten 3-, 4-, 5-, 6-, 7- oder 8-gliedrigen cycloaliphatischen Rest, der über eine lineare oder verzweigte C₁₋₃-Alkylen-Gruppe gebunden und/oder mit einer linearen oder verzweigten C₁₋₃Alkylen-Gruppe überbrückt sein kann,
für einen unsubstituierten oder wenigstens einfach substituierten, 5- oder 6-gliedrigen Aryl- oder Heteroaryl-Rest steht, der über eine lineare oder verzweigte C₁₋₆-Alkylen-Gruppe gebunden und/oder mit einem unsubstituierten oder wenigstens einfach substituierten, 5- oder 6-gliedrigen, monozyklischen Ringsystem kondensiert sein kann, steht.

6. Verbindungen gemäß einem oder mehreren der Ansprüche 1-5, **dadurch gekennzeichnet, daß** der Rest R⁷ für einen linearen oder verzweigten, unsubstituierten C₁₋₃-Alkyl-Rest, für einen unsubstituierten, 5- oder 6- gliedrigen Aryl- oder Heteroaryl-Rest, für einen unsubstituierten, gesättigten oder ungesättigten, ggf. ein Stickstoffatom als Ringglied aufweisenden 5-, 6- oder 7-gliedrigen cycloaliphatischen Rest, der mit einem unsubstituierten, 6-gliedrigen monozyklischen Ringsystem kondensiert sein kann, oder für einen -NR^{7a}R^{7b}-Rest steht, worin die Reste R^{7a} und R^{7b}, gleich oder verschieden, jeweils für einen linearen oder verzweigten C₁₋₃-Alkyl-Rest stehen.

7. Verbindungen gemäß einem oder mehreren der Ansprüche 1-6, **dadurch gekennzeichnet, daß** R⁸ für einen linearen oder verzweigten, unsubstituierten C₁₋₃-Alkyl-Rest oder für einen ggf. über eine lineare oder verzweigte C₁₋₃-Alkylen-Gruppe gebundenen Phenyl-Rest steht.

8. Verbindungen gemäß einem oder mehreren der Ansprüche 1-7, **dadurch gekennzeichnet, daß** R⁹ für einen linearen oder verzweigten, unsubstituierten C₁₋₃-Alkyl-Rest, für einen unsubstituierten, ggf. über eine lineare oder verzweigte C₁₋₃-Alkylen-Gruppe gebundenen, 5- oder 6-gliedrigen Aryl- oder Heteroaryl-Rest, oder für einen unsubstituierten oder wenigstens einfach substituierten, gesättigten oder ungesättigten, 5-oder 6-gliedrigen cycloaliphatischen Rest, der mit wenigstens einem unsubstituierten, 6-gliedrigen monozyklischen Ringsystem kondensiert sein kann, steht.

9. Verbindungen gemäß einem oder mehreren der Ansprüche 1-8, **dadurch gekennzeichnet, daß** R¹⁰ und R¹¹, gleich oder verschieden, jeweils für einen Methyl-, Ethyl-, n-Propyl- oder iso-Propyl-Rest stehen.

10. Verbindungen gemäß einem oder mehreren der Ansprüche 1-9, **dadurch gekennzeichnet, daß**
X für eine C(H)(NHR²)-Gruppe oder für eine NR^{2a}-Gruppe steht,
R¹ für eine -C(=O)-R³-Gruppe oder für eine -C(=O)-O-R⁴-Gruppe steht,
R², R^{2a}, unabhängig voneinander, jeweils für eine -C(=O)-R⁵-Gruppe oder für eine -S(=O)₂-R⁶-Gruppe stehen,
R³ für einen Methyl-, Ethyl-, n-Propyl-, iso-Propyl-, n-Butyl-, iso-Butyl-, sec-Butyl- oder tert-Butyl-Rest, für einen unsubstituierten, gesättigten oder ungesättigten, ggf. eines oder mehrere Sauerstoffatome und/oder eines oder mehrere Stickstoffatome als Ringglied(er) aufweisenden, 5-, 6- oder 7-gliedrigen cycloaliphatischen Rest, der über eine -(CH₂)-, -(CH₂)₂- oder-(CH₂)₃-Gruppe gebunden sein kann, oder für einen Phenyl-Rest, Thiophenyl-Rest (Thienyl-Rest), Furanyl-Rest (Furyl-Rest), Pyridinyl-Rest oder Naphthyl-Rest steht, der über eine -(CH₂)-, -(CH₂)₂- oder -(CH₂)₃-Gruppe gebunden und/oder einfach oder mehrfach, gleich oder verschieden, mit einem Substituenten ausgewählt aus der Gruppe bestehend aus Methyl, Ethyl, n-Propyl, iso-Propyl, n-Butyl, iso-Butyl, sec-Butyl, tert.-Butyl, Methoxy, Ethoxy, n-Propoxy, iso-Propoxy, n-Butoxy, iso-Butoxy, sec-Butoxy, tert.-Butoxy, F, Cl, Br, I, -CN und -CF₃ substituiert sein kann, steht,
R⁴ für einen unsubstituierten Benzyl-Rest steht,
R⁵ für einen Rest ausgewählt aus der Gruppe bestehend aus Methyl, Ethyl, n-Propyl, iso-Propyl, n-Butyl, sec-Butyl, tert-Butyl, n-Pentyl, iso-Pentyl, neoPentyl, -C(H)(C₂H₅)₂, -C(H)(n-C₃H₇)₂-, -CH=CH-CH=CH-CH₃, -CH₂-CH₂-CH=CH₂, -CH₂-CH₂-C(H)(CH₃)-(CH₂)₃-CH₃, -CH₂-O-CH₃ und -CH₂-O-CH₂-CH₂-O-CH₃,
für einen Rest ausgewählt aus der Gruppe bestehend aus Cyclopropyl, Cyclobutyl, Cyclopentyl, Cyclohexyl, Cycloheptyl, Cyclooctyl, Piperidinyl, Piperazinyl, Morpholinyl, Tetrahydrofuranyl (Tetrahydrofuryl), Dithiolanyl, 1,2,3,4-Tetrahydroindolyl, 1,2,3,4-Tetrahydronaphthyl, 1,3-Dihydroisoindolyl, Benzooxazolyl und Imidazolidinyl, wobei der zyklische Rest jeweils über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-,-CH₂-O-CH₂-, -CH₂-S-CH₂-, -C(CH₃)₂-, oder -C(H)(CH₃)-Gruppe gebunden und/oder einfach oder mehrfach, gleich oder verschieden, mit einem Substituenten ausgewählt aus der Gruppe bestehend aus Phenyl, -C(=O)-tert.-Butyl, oxo (=O), -S(=O)₂-Methyl und -S(=O)₂-Phenyl substituiert sein kann, und wobei die vorstehend genannten Phenyl-Reste jeweils einfach oder mehrfach, gleich oder verschieden, mit einem Substituenten ausgewählt aus der Gruppe bestehend aus F, Cl, Br und I substituiert sein können,
für einen Rest ausgewählt aus der Gruppe bestehend aus Phenyl, Naphthyl, Furanyl (Furyl), Thiophenyl (Thienyl), Pyridinyl, Isoxazolyl, Triazolyl, Pyrazolyl, Thiazolyl, Benzo[1,2,5]-oxadiazolyl, 2,3-Dihydrobenzofuranyl, Chinolinyl, Chromanyl, Chromenyl, Benzo[b]furanyl, Benzo[b]thiophenyl, Benzo[1,3]-dioxinyl, Indolyl, 2,3-Dihydro-indolyl, 3,4-Dihydro-benzo[1,4]oxazinyl und 1,2,3,4-Tetrahydroisochinolinyl steht, wobei der zyklische Rest jeweils über eine -(CH₂)-, -(CH₂)₂-, -(CH₂)₃-, -(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-,-C(C₂H₅)(H)-O-, -O-C(C₂H₅)(H)-, -CH₂-O-CH₂-, -CH₂-S-CH₂-, -C(CH₃)₂-,-C(H)(CH₃)- oder -CH=CH-Gruppe gebunden und/oder einfach oder mehrfach, gleich oder verschieden, mit einem Substituenten ausgewählt aus der Gruppe bestehend aus F, Cl, Br, I, -CN, -NO₂, -OH, -SH, Methyl, Ethyl, n-Propyl, isoPropyl, n-Butyl, iso-Butyl, sec-Butyl, tert.-Butyl, n-Pentyl, neo-Pentyl, Methoxy, Ethoxy, n-Propoxy, iso-Propoxy, n-Butyloxy, iso-Butyloxy, sec-Butyloxy, tert.-Butyloxy, -S-Methyl, -S-Ethyl, -CF₃, -CHF₂, -CH₂F, -O-CF₃, -O-CHF₂, -O-CH₂F, -S-CF₃, -S-CHF₂, -S-CH₂F, -S(=O)₂-Phenyl, Pyrazolyl, Phenyl, -N(CH₃)₂,-N(C₂H₅)₂, -CH₂O-C(=O)-Phenyl, -O-C(=O)-Phenyl, Furyl (Furanyl), Thiadiazolyl, Thiophenyl (Thienyl), Phenoxy und Benzyl substituiert sein kann, und wobei die zyklischen Substituenten selbst jeweils einfach oder mehrfach, gleich oder verschieden, mit einem Substituenten ausgewählt aus der Gruppe bestehend aus F, Cl, Br, I substituiert sein können, für einen -C(=O)-R⁷-Rest, der über eine -(CH₂)-, -(CH₂)₂- oder -(CH₂)₃-Gruppe gebunden sein kann,
für einen -C(=O)-O-R⁸-Rest, der über eine -(CH₂)-, -(CH₂)₂-, -(CH₂)₃- oder-C(H)(Phenyl)-Gruppe gebunden sein kann, oder
für einen -N(H)-C(=O)-O-R⁹-Rest, der über eine -(CH₂)-, -(CH₂)₂-, -(CH₂)₃-,-(CH₂)₄-, -(CH₂)₅-, -C(H)(CH₂-Phenyl)-, -C(H)(Phenyl), -C(H)(C(H)(CH₃)₂)-oder -C(H)(CH₂-CH(CH₃)₂)-NH-C(=O)-CH₂-Gruppe gebunden ist, steht,
R⁶ für eine Gruppe -NR¹⁰R¹¹,
für einen Methyl-, Ethyl-, n-Propyl-, iso-Propyl-, n-Butyl-, iso-Butyl-, sec-Butyl- oder tert.-Butyl-Rest,
für einen ggf. über eine -(CH₂)-, -(CH₂)₂- oder -(CH₂)₃-Gruppe gebundenen 7,7-Dimethyl-2-oxo-bicyclo[2.2.1]heptyl-Rest, oder
für einen Phenyl-, Naphthyl-, Thiophenyl- (Thienyl-), Furanyl-, (Furyl-), Thiazolyl-, Pyrazolyl-, 2,3-Dihydro-benzo[1,4]-dioxinyl-, 1,2,3,4-Tetrahydroisochinolinyl- oder 3,4-Dihydrobenzo[1.4]oxazinyl-Rest, der über eine -(CH₂)-,-(CH₂)₂-, oder -(CH₂)₃-Gruppe gebunden und/oder einfach oder mehrfach, gleich oder verschieden, mit einem Substituenten ausgewählt aus der Gruppe bestehend aus F, Cl, Br, I, Methyl, Ethyl, n-Propyl, iso-Propyl, n-Butyl, isoButyl, sec-Butyl, tert.-Butyl, n-Pentyl, iso-Pentyl, neo-Pentyl, Methoxy, Ethoxy, -CN, -CF₃, -CF₂H, -CFH₂, -NO₂, -C(=O)-CF₃, -O-CF₃, -C(=O)-O-CH₃, -C(=O)-O-C₂H₅, -NH-C(=O)-CH₃, -S(=O)₂-CH₃ und Phenyl substituiert sein kann, steht,
R⁷ für einen Methyl-, Ethyl-, n-Propyl-, Phenyl-, Indolyl-, 2,3-Dihydro-indolyl-, Dimethylamino- oder Diethylamino-Rest steht,
R⁸ für einen Methyl-, Ethyl-, Phenyl- oder Benzyl-Rest steht,
R⁹ für einen Methyl-, Ethyl-, n-Propyl-, iso-Propyl-, n-Butyl-, iso-Butyl-, sec-Butyl-, tert.-Butyl-, oder für einen ggf. über eine -(CH₂)-Gruppe gebundenen Phenyl- oder Fluorenyl-Rest steht,
R¹⁰, R¹¹, gleich oder verschieden, jeweils für einen Methyl-, Ethyl-, n-Propyl- oder iso-Propyl-Rest stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

11. Verbindungen gemäß einem oder mehreren der Ansprüche 1-10, **dadurch gekennzeichnet, daß**
X für eine C(H)(NHR²)-Gruppe oder für eine NR^{2a}-Gruppe steht,
R¹ für eine -C(=O)-R³-Gruppe oder für eine -C(=O)-O-R⁴-Gruppe steht,
R², R^{2a}, unabhängig voneinander, jeweils für eine -C(=O)-R⁵-Gruppe oder für eine -S(=O)₂-R⁶-Gruppe stehen,
R³ für einen linearen oder verzweigten, ggf. substituierten, ggf. 1, 2 oder 3 Heteroatom(e) ausgewählt aus der Gruppe bestehend aus Sauerstoff, Schwefel und Stickstoff (NH) als Kettenglied(er) aufweisenden C₁₋₁₀Alkyl-Rest, C₂₋₁₀Alkenyl-Rest oder C₂₋₁₀Alkinyl-Rest;
für einen ungesättigten oder gesättigten, ggf. substituierten 3-, 4-, 5-, 6-, 7-, 8- oder 9-gliedrigen cycloaliphatischen Rest;
für einen ggf. substituierten 5- bis 14-gliedrigen Aryl- oder Heteroaryl-Rest; für -(CHR¹²)-Uₐ-(CH₂)_{b}-V_{c}-(CH₂)_{d}-R¹³ mit a = 0 oder 1, b = 0 oder 1, c = 0 oder 1 und d = 0 oder 1 steht, worin U und V, unabhängig voneinander, jeweils für O, S und NH steht;
oder für -(CHR¹⁴)-(CH₂)ₑ-R¹⁵ mit e = 0, 1, 2 oder 3 steht;
R⁴ für einen ggf. substituierten 5- bis 14-gliedrigen Aryl- oder Heteroaryl-Rest steht;
oder für -(CHR¹⁶)-(CH₂)_{f}-R¹⁷ mit f = 0, 1, 2 oder 3 steht;
R⁵ für einen linearen oder verzweigten, ggf. substituierten, ggf. 1, 2 oder 3 Heteroatom(e) ausgewählt aus der Gruppe bestehend aus Sauerstoff, Schwefel und Stickstoff (NH) als Kettenglied(er) aufweisenden C₁₋₁₀Alkyl-Rest, C₂₋₁₀Alkenyl-Rest oder C₂₋₁₀Alkinyl-Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR¹⁸)_{g}-(CH₂)ₕ-C(=O)-R⁷ mit g = 0 oder 1 und h = 0, 1, 2 oder 3;
für -(CHR¹⁹)ⱼ-(CH₂)ₖ-C(=O)-O-R⁸ mit j = 0 oder 1 und k = 0, 1, 2 oder 3;
für -(CHR²⁰)ₘ-(CH₂)ₖ-[NH-C(=O)]ₚ-(CH₂)_{q}-NH-C(=O)-O-R⁹ mit m = 0 oder 1; n = 0, 1, 2, 3, 4 oder 5; p = 0 oder 1 und q = 0, 1 oder 2;
für -(CR²¹R²²)-Wᵣ-(CH₂)ₛ-Xₜ-(CH₂)ᵤ-Yᵥ-R²³ mit r = 0 oder 1, s = 0 oder 1, t = 0 oder 1, u = 0, 1 oder 2 und v = 0 oder 1, worin W, X und Y, unabhängig voneinander, jeweils für O, S und NH steht;
oder für -CH=CH-R²⁴ steht;
R⁶ für eine Gruppe -NR¹⁰R¹¹ steht;
für einen linearen oder verzweigten, ggf. substituierten, ggf. 1, 2 oder 3 Heteroatom(e) ausgewählt aus der Gruppe bestehend aus Sauerstoff, Schwefel und Stickstoff (NH) als Kettenglied(er) aufweisenden C₁₋₁₀Alkyl-Rest, C₂₋₁₀ Alkenyl-Rest oder C₂₋₁₀ Alkinyl-Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert und/oder mit einer linearen oder verzweigten C₁₋₅-Alkylen-Gruppe überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR²⁵)-Z_{w}-(CH₂)ₓ-A_{y}-(CH₂)_{z}-R²⁸ mit w = 0 oder 1, x = 0 oder 1, y = 0 oder 1 und z = 0 oder 1, worin Z und A, unabhängig voneinander, jeweils für O, S und NH steht;
oder für -(CHR²⁷)-(CH₂)ₐₐ-R²⁸ mit aa = 0, 1, 2 oder 3 steht;
R⁷ für einen linearen oder verzweigten, ggf. substituierten C₁₋₁₀ Alkyl-Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest;
oder für einen -NR^{7a}R^{7b}-Rest steht, worin die Reste R^{7a} und R^{7b}, unabhängig voneinander, jeweils für einen linearen oder verzweigten C₁₋₁₀Alkyl-Rest stehen;
R⁸ für einen linearen oder verzweigten, ggf. substituierten C₁₋₁₀ Alkyl-Rest;
oder für einen ggf. substituierten 5- bis 14-gliedrigen Aryl- oder Heteroaryl-Rest steht, der über eine lineare oder verzweigte C₁₋₅-Alkylen-Gruppe gebunden sein kann;
R⁹ für einen linearen oder verzweigten, ggf. substituierten C₁₋₁₀ Alkyl-Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert und/oder über eine lineare oder verzweigte C₁₋₅-Alkylen-Gruppe gebunden sein kann;
oder für einen ggf. substituierten 5- bis 14-gliedrigen Aryl- oder Heteroaryl-Rest steht, der über eine lineare oder verzweigte C₁₋₅-Alkylen-Gruppe gebunden sein kann;
R¹⁰ und R¹¹, unabhängig voneinander, jeweils für einen linearen oder verzweigten, ggf. substituierten C₁₋₁₀Alkyl-Rest stehen;
R¹², R¹⁴, R¹⁶, R¹⁸, R²¹, R²², R²⁵ und R²⁷ unabhängig voneinander, jeweils
für einen Wasserstoff-Rest
oder für einen linearen oder verzweigten, ggf. substituierten C₁₋₁₀ Alkyl-Rest stehen;
R¹³ für einen ungesättigten oder gesättigten, ggf. substituierten 3-, 4-, 5-, 6-, 7-, 8- oder 9-gliedrigen cycloaliphatischen Rest steht;
R¹⁵ und R¹⁷, unabhängig voneinander, jeweils für einen ggf. substituierten 5- bis 14-gliedrigen Aryl- oder Heteroaryl-Rest stehen;
R¹⁹ für einen Wasserstoff-Rest;
für einen jeweils linearen oder verzweigten, ggf. substituierten C₁₋₁₀ Alkyl-Rest
oder für einen Phenyl-Rest steht;
R²⁰ für einen Wasserstoff-Rest;
für einen jeweils linearen oder verzweigten, ggf. substituierten C₁₋₁₀ Alkyl-Rest
oder für einen Phenyl-Rest, der über eine lineare oder verzweigte C₁₋₅-Alkylen-Gruppe gebunden sein kann, steht;
R²³ für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest steht, der mit einem gesättigten oder ungesättigten, ggf. substituierten mono- oder polyzyklischen Ringsystem kondensiert sein kann;
R²⁴ und R²⁸, unabhängig voneinander, jeweils
für einen ggf. substituierten 5- bis 14-gliedrigen Aryl- oder Heteroaryl-Rest stehen, der mit einem gesättigten oder ungesättigten, ggf. substituierten mono- oder polyzyklischen Ringsystem kondensiert sein kann;
und
R²⁶ für einen ungesättigten oder gesättigten, ggf. substituierten 3-, 4-, 5-, 6-, 7-, 8- oder 9-gliedrigen cycloaliphatischen Rest steht, der mit einem gesättigten, ungesättigten oder aromatischen, ggf. substituierten mono- oder polyzyklischen Ringsystem kondensiert und/oder mit einer linearen oder verzweigten C₁₋₅-Alkylen-Gruppe überbrückt sein kann;
wobei
die vorstehend genannten C₁₋₁₀Alkyl-Reste unsubstituiert oder jeweils ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein können;
die vorstehend genannten C₂₋₁₀Alkenyl-Reste unsubstituiert oder ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein können;
die vorstehend genannten C₂₋₁₀ Alkinyl-Reste unsubstituiert oder ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein können;
die vorstehend genannten cycloaliphatischen Reste unsubstituiert oder ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H,-C(=O)-C₁₋₅-Alkyl, -C(=O)-C₁₋₅-Perflouralkyl, -C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl,-S(=O)₂-Phenyl, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -S(=O)₂-NH-Phenyl, -S(=O)₂-Phenyl, -O-Benzyl, Phenyl,-(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel aufweisen können;
und die vorstehend genannten Aryl- oder Heteroaryl-Reste unsubstituiert oder ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -0-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, - C(=O)-C₁₋₅-Perfluoralkyl, -C(=O)-NH₂, -C(=O)-NH-C₁₋₅-Alkyl, -C(=O)-N-(C₁₋₅-Alkyl)₂, - S(=O)₂-NH₂, -S(=O)₂-NH-C₁₋₅-Alkyl, -S(=O)₂-NH-Phenyl, -S(=O)₂-C₁₋₅-Alkyl, -S(=O)₂-Phenyl, -(CH₂)-Benzo[b]furanyl, -O-Phenyl, -S-Phenyl, -S-Benzyl, -O-Benzyl, Phenyl, Furanyl, Thienyl (Thiophenyl), Pyrazolyl, Thiadiazolyl und Benzyl substituiert sein können, wobei jeweils der zyklische Teil der Reste -O-C(=O)-Phenyl, -(CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl, Furanyl, Thienyl (Thiophenyl), Pyrazolyl, Thiadiazolyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -O-C₁₋₅-Alkyl, -C₁₋₅-Alkyl, -O-CF₃, -S-CF₃, Phenyl und -O-Benzyl substituiert sein kann,
und die vorstehend genannten Heteroaryl-Reste jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
die Ringe der vorstehend genannten mono- oder polyzyklischen Ringsysteme unsubstituiert oder ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl, -S(=O)₂-Phenyl, -(CH₂)-Benzo[b]furanyl, -O-Phenyl, -S-Phenyl, -S-Benzyl, -O-Benzyl, Phenyl, Furanyl, Thienyl (Thiophenyl), Pyrazolyl, Thiadiazolyl und Benzyl substituiert sein können, wobei jeweils der zyklische Teil der Reste -O-C(=O)-Phenyl, -(CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl, Furanyl, Thienyl (Thiophenyl), Pyrazolyl, Thiadiazolyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

12. Verbindungen gemäß Anspruch 11, **dadurch gekennzeichnet, dass**
X für eine C(H)(NHR²)-Gruppe oder für eine NR²-Gruppe steht,
R¹ für eine -C(=O)-R³-Gruppe oder für eine -C(=O)-O-R⁴-Gruppe steht,
R², R^{2a}, unabhängig voneinander, jeweils für eine -C(=O)-R⁵-Gruppe oder für eine -S(=O)₂-R⁶-Gruppe stehen,
R³ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl, -(CH₂)--(CH₂)-(CH)(CH₃)-(CH₂)-(CH₂)-(CH₂)-(CH₃), Vinyl, 1-Propenyl, 2-Propenyl, 1-Butenyl, 2-Butenyl, 3-Butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH_{3,} -CH₂-NH-CH₃ und - CH₂-NH-CH₂-CH₃ steht;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, - OH, -O-C₁₋₅-Alkyl, -NH₂, -NO₂, -O-CF₃, -S-CF₃, -SH, -S-C₁₋₅-Alkyl, -C₁₋₅-Alkyl, - C(=O)-H, -C(=O)-C₁₋₅-Alkyl, -C(=O)-C₁₋₅-Perflouralkyl, -C(=O)-OH, -C(=O)-O-C₁₋₅-Alkyl, -(CH₂)-C(=O)-OH, -(CH₂)C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, - NO₂, -O-C₁₋₅-Alkyl, -C₁₋₅-Alkyl, -O-CF₃, -S-CF₃, Phenyl und -O-Benzyl substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, - O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl. -_{C}(₌₀)-_{H}. -C(=O)-C₁₋₅-Alkyl, -C(=O)-C₁₋₅-Perflouralkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -S(=O)2-NH-Phenyl, -S(=O)₂-C₁₋₅-Alkyl -S(=O)₂-Phenyl, -O-Phenyl, -S-Phenyl, -S-Benzyl, - O-Benzyl, Phenyl und Benzyl substituiert sein kann, und wobei jeweils der zyklische Teil der Reste -O-C(=O)-Phenyl, -(CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -O-C₁₋₅-Alkyl, -C₁₋₅-Alkyl, -O-CF₃, -S-CF₃, Phenyl und -0-Benzyl substituiert sein kann;
für -(CHR¹²)-R¹³, -(CHR¹²)-(CH₂)-R¹³, -(CHR¹²)-O-R¹³, -(CHR¹²)-S-R¹³, - (CHR¹²)-NH-R¹³, -(CHR¹²)-(CH₂)-(CH₂)-R¹³, -(CHR¹²)-(CH₂)-O-R¹³, -(CHR¹²)-(CH₂)-S-R¹³, -(CHR¹²)-(CH₂)-NH-R¹³, -(CHR¹²)-(CH₂)-O-(CH₂)-R¹³, -(CHR¹²)-(CH₂)-S-(CH₂)-R¹³ oder -(CHR¹²)-(CH₂)-NH-R¹³ steht;
oder für -(CHR¹⁴)-R¹⁵, -(CHR¹⁴)-(CH₂)-R¹⁵ oder -(CHR¹⁴)-(CH₂)-(CH₂)-R¹⁵ steht;
R⁴ für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl,-O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perfluoralkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl, -S(=O)₂-Phenyl, -O-Phenyl, -S-Phenyl, -S-Benzyl, - O-Benzyl, Phenyl und Benzyl substituiert sein kann, und wobei jeweils der zyklische Teil der Reste -O-C(=O)-Phenyl, -(CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für -(CHR¹⁶)-R¹⁷, -(CHR¹⁶)-(CH₂)-R¹⁷ oder -(CHR¹⁶)-(CH₂)-(CH₂)-R¹⁷ steht;
R⁵ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl, -(CH₂)-(CH₂)-(CH)(CH₃)-(CH₂)-(CH₂)-(CH₂)-(CH₃), Vinyl, 1-Propenyl, 2-Propenyl, 1-Butenyl, 2-Butenyl, 3-Butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ und -CH₂-NH-CH₂-CH₃ steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl und 3,4-Dihydro-2H-benzo[1.4]oxazinyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-C₁₋₅-Perflouralkyl, - C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, (3,4)-Dihydro-2H-benzo[1.4]oxazinyl, (2,3)-Dihydro-benzo[1,4]dioxinyl, Benzo[1,3]dioxinyl, (2,3)-Dihydrobenzofuranyl, 2H-Chromenyl, Chromanyl und (1,2,3,4)-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, - O-C₁₋₅-Alkyl, -O-C₂₋₅-Alkenyl, -NH₂, -NO₂, -O-CF₃, -O-CHF₂, -O-CH₂F, -S-CF₃, - S-CHF₂, -S-CH₂F, -SH, -S-C₁₋₅-Alkyl, -C₁₋₅-Alkyl, -C(=O)-OH, -C(=O)O-C₁₋₅-Alkyl, -O-C(=O)-C₁₋₅-Alkyl, -O-C(=O)-Phenyl, -(CH₂)-O-C(=O)-C₁₋₅-Alkyl, - (CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl, - C(=O)-NH₂, -C(=O)-NH-C₁₋₅-Alkyl, -C(=O)-N-(C₁₋₅-Alkyl)₂, -S(=O)₂-NH₂, - S(=O)₂-NH-C₁₋₅-Alkyl, -S(=O)₂-NH-Phenyl, -S(=O)₂-C₁₋₅-Alkyl, -S(=O)₂-Phenyl, -O-Phenyl, -S-Phenyl, -S-Benzyl, -O-Benzyl, Phenyl, Pyrazolyl, Thienyl, Furanyl, Thiadiazolyl und Benzyl substituiert sein kann, und wobei jeweils der zyklische Teil der Reste -O-C(=O)-Phenyl, -(CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl, Pyrazolyl, Thienyl, Furanyl, Thiadiazolyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅;-Alkyl, -O-CF₃, -S-CF₃, Phenyl und -O-Benzyl substituiert sein kann;
für -C(=O)-R⁷, -(CHR¹⁸)-C(=O)-R⁷, -(CHR¹⁸)-(CH₂)-C(=O)-R⁷ oder -(CHR¹⁸)-(CH₂)-(CH₂)-C(=O)-R⁷ steht;
für -C(=O)-O-R⁸, -(CHR¹⁹)-C(=O)-O-R⁸, -(CHR¹⁹)-(CH₂)-C(=O)-O-R⁸ oder-(CHR¹⁹)-(CH₂)-(CH₂)-C(=O)-O-R⁸ steht;
für -(CHR²⁰)-NH-C(=O)-O-R⁹, -(CHR²⁰)-(CH₂)-NH-C(=O)-O-R⁹, -(CHR²⁰)-NH-C(=O)-(CH₂)-NH-C(=O)-O-R⁹, -(CHR²⁰)-(CH₂)-(CH₂)-NH-C(=O)-O-R⁹, - (CHR²⁰)-(CH₂)-(CH₂)-(CH₂)-NH-C(=O)-O-R⁹, -(CHR²⁰)-(CH₂)-(CH₂)-(CH₂)-NH-C(=O)-O-R⁹ oder -(CHR²⁰)-(CH₂)-(CH₂)-(CH₂)-(CH₂)-NH-C(=O)-O-R⁹ steht;
für -(CR²¹R²²)-R²³, -(CR²¹R²²)-(CH₂)-R²³, -(CR²¹R²²)-O-R²³, -(R²¹R²²)-S-R²³, -(CR²¹R²²)-NH-R²³, -(CR²¹R²²)-(CH₂)-(CH₂)-R²³, -(CR²¹R²²)-O-(CH₂)-R²³, - (CR²¹R²²)-S-(CH₂)-R²³, -(CR²¹R²²)-NH-(CH₂)-R²³, -(CR²¹R²²)-(CH₂)-(CH₂)-O-R²³, -(CR²¹R²²)-(CH₂)-(CH₂)-NH-R²³, -(CR²¹R²²)-(CH₂)-(CH₂)-S-R²³ oder-(CR²¹R²²)-(CH₂)-(CH₂)-(CH₂)-R²³ steht;
oder für -(CH=CH)-R²⁴ steht;
R⁶ für eine Gruppe -NR¹⁰R¹¹ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl, -(CH₂)-(CH₂)-(CH)(CH₃)-(CH₂)-(CH₂)-(CH₂)-(CH₃), Vinyl, 1-Propenyl, 2-Propenyl, 1-Butenyl, 2-Butenyl, 3-Butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ und -CH₂-NH-CH₂-CH₃ steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl, 3,4-Dihydro-2H-benzo[1.4]oxazinyl und Bicyclo[2.2.1]heptyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, - C(=O)-C₁₋₅-Perflouralkyl, -C(=O)-OH, -C(=O)-O-C₁₋₅-Alkyl, -(CH₂)-C(=O)-OH, - (CH₂)-C(=O)-O-C₁₋₅-Alkyl, -NH-C₁₋₅-Alkyl, -N(C₁₋₅-Alkyl)₂, -O-Phenyl, -0-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, (3,4)-Dihydro-2H-benzo[1.4]oxazinyl, (2,3)-Dihydro-benzo[1,4]dioxinyl, Benzo[1,3]dioxinyl, (2,3)-Dihydrobenzofuranyl, 2H-Chromenyl, Chromanyl und (1,2,3,4)-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, - O-C₁₋₅-Alkyl, -O-C₂₋₅-Alkenyl, -NH₂, -NO₂, -O-CF₃, -O-CHF₂, -O-CH₂F, -S-CF₃, - S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -O-C(=O)-Phenyl, -(CH₂)-O-C(=O)-C₁₋₅-Alkyl, - (CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₆-Perflouralkyl, - C(=O)-NH₂, -C(=O)-NH-C₁-₅₋Alkyl, -C(=O)-N-(C₁₋₅-Alkyl)₂, -S(=O)₂-NH₂, - S(=O)₂-NH-C₁₋₅-Alkyl, -S(=O)₂-NH-Phenyl, -S(=O)₂C₁₋₅-Alkyl, -S(=O)₂-Phenyl, -O-Phenyl, -S-Phenyl, -S-Benzyl, -O-Benzyl, Phenyl und Benzyl substituiert sein kann, und wobei jeweils der zyklische Teil der Reste -O-C(=O)-Phenyl, - (CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
für -(CHR²⁵)-R²⁶, -(CHR²⁵)-(CH₂)-R²⁶, -(CHR²⁵)-O-R²⁶, -(CHR²⁵)-S-R²⁶, - (CHR²⁵)-NH-R²⁶, -(CHR²⁵)-(CH₂)-(CH₂)-R²⁶, -(CHR²⁵)-O-(CH₂)-R²⁶, -(CHR²⁵)-S-(CH₂)-R²⁶, -(CHR²⁵)-NH-(CH₂)-R²⁶, -(CHR²⁵)-(CH₂)-(CH₂)-O-R²⁶, -(CHR²⁵)-(CH₂)-(CH₂)-NH-R²⁶ oder -(CHR²⁵)-(CH₂)-(CH₂)-S-R²⁶ steht;
oder für -(CHR²⁷)-R²⁸, -(CHR²⁷)-(CH₂)-R²⁸ oder -(CHR²⁷)-(CH₂)-(CH₂)-R²⁸ steht;
R⁷ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl und 3,4-Dihydro-2H-benzo[1.4]oxazinyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-C₁₋₅-Perflouralkyl, - C(=O)-OH, -C(=O)-O-C₁₋₅-Alkyl, -(CH₂)-C(=O)-OH. -(CH₂)C(=O)-O-C₁₋₅-Alkyl, -NH-C₁₋₅-Alkyl, -N(C₁₋₅Alkyl)₂, -O-Phenyl, -O-Benzyl, Phenyl und Benzyl substituiert sein kann, und wobei jeweils der zyklische Teil der Reste -0-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl,-O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)C₁₋₅-Alkyl, -C(=O)-H, -C(=O)-C₁₋₅-Alkyl, -C(=O)-C₁₋₅-Perflouralkyl, -C(=O)-NH₂, -C(=O)-NH-C₁₋₅-Alkyl, -C(=O)-N-(C₁₋₅-Alkyl)₂, -O-Phenyl, -S-Phenyl, -S-Benzyl, -O-Benzyl, Phenyl und Benzyl substituiert sein kann, und wobei jeweils der zyklische Teil der Reste -O-C(=O)-Phenyl, -(CH₂)-O-C(=O)-Phenyl, -O-Phenyl, -S-Phenyl,-S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen -NR^{7a}R^{7b}-Rest steht, worin R^{7a} und R^{7b}, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl und n-Pentyl stehen;
R⁸ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) steht;
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, - O-C(=O)-Phenyl, -(CH₂)-O-C(=O)-C₁₋₅-Alkyl, -NH-C₁₋₅-Alkyl, -N(C₁₋₅-Alkyl)₂, - NH-C(=O)-O-C₁₋₅-Alkyl, -NH-C(=O)-C₁₋₅-Alkyl, -C(=O)-H, -C(=O)-C₁₋₅-Alkyl, - C(=O)-C₁₋₅-Perflouralkyl, -C(=O)-NH₂, -C(=O)-NH-C₁₋₅-Alkyl, -C(=O)-N-(C₁₋₅-Alkyl)₂, -S(=O)₂-NH₂ und -S(=O)₂-NH-C₁₋₅-Alkyl substituiert und/oder über eine -(CH₂)-, -(CH₂)₂- oder -(CH₂)₃-Gruppe gebunden sein kann;
R⁹ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl, 3,4-Dihydro-2H-benzo[1.4]oxazinyl und 9H-Fluorenyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, - C(=O)-C₁₋₅-Perflouralkyl, -C(=O)-OH, -C(=O)-O-C₁₋₅-Alkyl, -(CH₂)-C(=O)-OH, - (CH₂)-C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert und/oder über eine -(CH₂)-, -(CH₂)₂- oder -(CH₂)₃-Gruppe gebunden sein kann;
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl,-O-C(=O)-Phenyl, -(CH₂)-O-C(=O)-C₁₋₅-Alkyl, -NH-C₁₋₅-Alkyl, -N(C₁₋₅-Alkyl)₂,-NH-C(=O)-O-C₁₋₅-Alkyl, -NH-C(=O)-C₁₋₅-Alkyl, -C(=O)-H, -C(=O)-C₁₋₅-Alkyl,-C(=O)-C₁₋₅-Perfouralkyl, -C(=O)-NH₂, -C(=O)-NH-C₁₋₅-Alkyl, -C(=O)-N-(C₁₋₅-Alkyl)₂, -S(=O)₂-NH₂ und -S(=O)₂-NH-C₁₋₅-Alkyl substituiert und/oder über eine -(CH₂)-, -(CH₂)₂- oder -(CH₂)₃-Gruppe gebunden sein kann;
R¹⁰ und R¹¹, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und - (CH₂)-(CH₂)-(CH)(CH₃)-(CH₂)-(CH₂)-(CH₂)-(CH₃) stehen;
R¹², R¹⁴, R¹⁶, R¹⁸, R²¹, R²², R²⁵ und R²⁷, unabhängig voneinander, jeweils
für einen Wasserstoff-Rest stehen
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) stehen;
R¹³ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, - OH, -O-C₁₋₅-Alkyl, -NH₂, -NO₂, -O-CF₃, -S-CF₃, -SH, -S-C₁₋₅-Alkyl, -C₁₋₅-Alkyl,-C(=O)-H, -C(=O)-C₁₋₅-Alkyl, -C(=O)-C₁₋₅-Perflouralkyl, -C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₅-Alkyl)₂, -O-Phenyl, -O-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, - NO₂, -O-C₁₋₅-Alkyl, -C₁₋₅-Alkyl, -O-CF₃, -S-CF₃, Phenyl und -O-Benzyl substituiert sein kann;
R¹⁵ und R¹⁷, unabhängig voneinander, jeweils
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, - (CH₂)-O-C(=O)-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl, -C(=O)-NH₂, -C(=O)-NH-C₁₋₅-Alkyl, -C(=O)-N-(C₁₋₅-Alkyl)₂,-S(=O)₂-NH₂, -S(=O)₂-NH-C₁₋₅-Alkyl, -S(=O)₂-C₁₋₅-Alkyl, -O-Phenyl, -O-Benzyl, Phenyl und Benzyl substituiert sein kann, und wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃ und -S-CF₃ substituiert sein kann;
R¹⁹ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) steht;
oder für einen Phenyl-Rest steht;
R²⁰ für einen Wasserstoff-Rest steht; für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl und -(CH₂)-(CH₂)-(CH)(CH₃)-(CH₂)-(CH₂)-(CH₂)-(CH₃) steht;
oder für einen Phenyl-Rest, der über eine -(CH₂)- oder -(CH₂)₂-Gruppe gebunden sein kann, steht;
R²³ für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl und 3,4-Dihydro-2H-benzo[1.4]oxazinyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-C₁₋₅-Perflouralkyl,-C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein kann, und wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, (3,4)-Dihydro-2H-benzo[1.4]oxazinyl, (2,3)-Dihydro-benzo[1,4]dioxinyl, Benzo[1,3]dioxinyl, (2,3)-Dihydrobenzofuranyl, 2H-Chromenyl, Chromanyl und (1,2,3,4)-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, - O-C₁₋₅-Alkyl, -O-C₂₋₅-Alkenyl, -NH₂, -NO₂, -O-CF₃, -O-CHF₂, -O-CH₂F, -S-CF₃,-S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -O-C(=O)-Phenyl, -(CH₂)-O-C(=O)-C₁₋₅-Alkyl, - (CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl,-C(=O)-NH₂, -C(=O)-NH-C₁₋₅-Alkyl, -C(=O)-N-(C₁₋₅-Alkyl)₂, -S(=O)₂-NH₂,-S(=O)₂-NH-C₁₋₅-Alkyl, -S(=O)₂-NH-Phenyl, -S(=O)₂-C₁₋₅-Alkyl, -S(=O)₂-Phenyl, -O-Phenyl, -S-Phenyl, -S-Benzyl, -O-Benzyl, Phenyl und Benzyl substituiert sein kann, und wobei jeweils der zyklische Teil der Reste -O-C(=O)-Phenyl, - (CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
R²⁴ und R²⁸, unabhängig voneinander, jeweils
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, (3,4)-Dihydro-2H-benzo[1,4]oxazinyl, (2,3)-Dihydro-benzo[1,4]dioxinyl, Benzo[1,3]dioxinyl, (2,3)-Dihydrobenzofuranyl, 2H-Chromenyl, Chromanyl und (1,2,3,4)-Tetrahydroisochinolinyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl,-O-C(=O)-Phenyl, -(CH₂)-O-C(=O)-C₁₋₅-Alkyl, -(CH₂)-O-C(=O)-Phenyl, -NH-C₁₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perfouralkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl, -S(=O)₂-Phenyl, -O-Phenyl, -S-Phenyl, -S-Benzyl,-O-Benzyl, Phenyl und Benzyl substituiert sein kann, und wobei jeweils der zyklische Teil der Reste -O-C(=O)-Phenyl, -CH₂)-O-C(=O)-Phenyl, -S(=O)₂-NH-Phenyl, -S(=O)₂-Phenyl, -O-Phenyl, -S-Phenyl, -S-Benzyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃,-SF₅, -CN, -NO₂, -O-C₁₋₅-Alkyl, -C₁₋₅-Alkyl, -O-CF₃, -S-CF₃, Phenyl und -O-Benzyl substituiert sein kann; und
R²⁶ für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (1,2,3,4)-Tetrahydronaphthyl, (1,3)-Dihydroisoindolyl, (2,3)-Dihydroindolyl, 3,4-Dihydro-2H-benzo[1.4]oxazinyl und Bicyclo[2.2.1]heptyl steht, wobei der (hetero)cycloaliphatische Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl,-C(=O)-C₁₋₅-Perflouralkyl, -C(=O)-OH, -C(=O)-O-C₁₋₅-Alkyl, -(CH₂)-C(=O)-OH,-(CH₂)-C(=O)-O-C₁₋₅-Alkyl, -NH-C₁₋₅-Alkyl, -N(C₁₋₅-Alkyl)₂, -O-Phenyl, -0-Benzyl, Phenyl und Benzyl substituiert sein kann, wobei jeweils der zyklische Teil der Reste -O-Phenyl, -O-Benzyl, Phenyl und Benzyl, mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann.

13. Verbindungen gemäß Anspruch 11 oder 12, **dadurch gekennzeichnet, dass**
X für eine C(H)(NHR²)-Gruppe oder für eine NR^{2a}-Gruppe steht,
R¹ für eine -C(=O)-R³-Gruppe oder für eine -C(=O)-O-R⁴-Gruppe steht,
R², R^{2a}, unabhängig voneinander, jeweils für eine -C(=O)-R⁵-Gruppe oder für eine -S(=O)₂-R⁶-Gruppe stehen,
R³ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl und n-Heptyl steht;
für einen (hetero)cycloaliphatischen Rest ausgewählt aus der Gruppe bestehend aus Cyclopentyl, Cyclohexyl, Cycloheptyl, Tetrahydrofuranyl, Pyrrolidinyl, Piperidinyl, Morpholinyl, Piperazinyl, Azepanyl und Diazepanyl steht;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl und Oxadiazolyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, Methoxy, Ethoxy, n-Propoxy, Isopropoxy, n-Butoxy, Isobutoxy, tert-Butoxy, Methyl, Ethyl, n-Propyl, iso-Propyl, n-Butyl, iso-Butyl, sec-Butyl, tert-Butyl und n-Pentyl substituiert sein kann;
für -(CHR¹²)-R¹³, -(CHR¹²)-(CH₂)-R¹³ oder -(CHR¹²)-(CH₂)-(CH₂)-R¹³ steht;
oder für -(CHR¹⁴)-R¹⁵, -(CHR¹⁴)-(CH₂)-R¹⁵ oder -(CHR¹⁴)-(CH₂)-(CH₂)-R¹⁵ steht;
R⁴ für -(CHR¹⁶)-R¹⁷ oder -(CHR¹⁶)-(CH₂)-R¹⁷ steht;
R⁵ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl, 3-Hexyl, -(CH₂)-(CH₂)-(C(CH₃)₃), n-Heptyl, 3-Heptyl, 4-Heptyl, n-Octyl, -(CH₂)-(CH₂)-(CH)(CH₃)-(CH₂)-(CH₂)-(CH₂)-(CH₃), Vinyl, 1-Propenyl, 2-Propenyl, 1-Butenyl, 2-Butenyl, 3-Butenyl, -CH=CH-CH=CH-CH₃, -CH₂-O-CH₃ und -CH₂-O-CH₂-CH₂-O-CH₃ steht;
für einen Rest ausgewählt aus der Gruppe bestehend aus Cyclopropyl, Cyclobutyl, Cyclopentyl, Cyclohexyl, Cycloheptyl, Tetrahydrofuranyl, Piperidinyl, Piperazinyl und (1,2,3,4)-Tetrahydronaphthyl steht, wobei der (hetero)cycloaliphatische Rest jeweils ggf. mit 1, 2, 3, 4 oder 5 Substituenten unabhängig voneinander ausgewählt aus der Gruppe bestehend aus -C(=O)-O-CH₃, -C(=O)-O-C₂H₅, -C(=O)-C(CH₃)₃ und Phenyl substituiert sein können, wobei jeweils der zyklische Teil des Phenyl-Restes mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Indolyl, Benzo[b]thiophenyl, Thiazolyl, Thiadiazolyl, Oxazolyl, Isoxazolyl, Chinolinyl, Isochinolinyl, Benzo[1,3]dioxinyl, (2,3)-Dihydrobenzofuranyl, Benzo[1,2,5]oxadiazolyl, 2H-Chromenyl und Chromanyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, Methoxy, Ethoxy, n-Propoxy, Isopropoxy, n-Butoxy, Isobutoxy, tert-Butoxy, -NO₂, -O-CF₃, -O-CHF₂, -O-CH₂F, -S-CF₃, -S-CHF₂, -S-CH₂F, -S-CH₃, -S-C₂H₅, -S-n-C₃H₇, -S-C(CH₃)₃, Methyl, Ethyl, n-Propyl, iso-Propyl, n-Butyl, iso-Butyl, sec-Butyl, tert-Butyl, n-Pentyl, -O-C(=O)-Phenyl, -(CH₂)-O-C(=O)-Phenyl, -N(CH₃)₂, -N(C₂H₅)₂, -S(=O)₂-Phenyl, -O-Phenyl, Phenyl, Furanyl, Thienyl, Pyrazolyl, Thiadiazolyl und Benzyl substituiert sein kann, wobei jeweils der zyklische Teil der Reste -O-C(=O)-Phenyl, -(CH₂)-O-C(=O)-Phenyl, Furanyl, Thienyl, Pyrazolyl, Thiadiazolyl, -S(=O)₂-Phenyl, -O-Phenyl, Phenyl und Benzyl mit 1, 2, 3, 4 oder 5 Substituenten unabhängig voneinander ausgewählt aus der Gruppe bestehend aus F, Cl und Br substituiert sein kann;
für -C(=O)-R⁷, -(CHR¹⁸)-C(=O)-R⁷, -(CHR¹⁸)-(CH₂)-C(=O)-R⁷ oder -(CHR¹⁸)-(CH₂)-(CH₂)-C(=O)-R⁷ steht;
für -C(=O)-O-R⁸, -(CHR¹⁹)-C(=O)-O-R⁸, -(CHR¹⁹)-(CH₂)-C(=O)-O-R⁸ oder - (CHR¹⁹)-(CH₂)-(CH₂)-C(=O)-O-R⁸ steht;
für -(CHR²⁰)-NH-C(=O)-O-R⁹, -(CHR²⁰)-(CH₂)-NH-C(=O)-O-R⁹, -(CHR²⁰)-NH-C(=O)-(CH₂)-NH-C(=O)-O-R⁹, -(CHR²⁰)-(CH₂)-(CH₂)-NH-C(=O)-O-R⁹,-(CHR²⁰)-(CH₂)-(CH₂)-(CH₂)-NH-C(=O)-O-R⁹, -(CHR²⁰)-(CH₂)-(CH₂)-(CH₂)-NH-C(=O)-O-R⁹ oder -(CHR²⁰)-(CH₂)-(CH₂)-(CH₂)-(CH₂)-NH-C(=O)-O-R⁹ steht;
für -(CR²¹R²²)-R²³, -(CR²¹R²²)-(CH₂)-R²³, -(CR²¹R²²)-O-R²³, -(CR²¹R²²)-S-R²³, -(CR²¹R²²)-(CH₂)-(CH₂)-R²³, -(CR²¹R²²)-O-(CH₂)-R²³, -(CR²¹R²²)-S-(CH₂)-R²³, -(CR²¹R²²)-(CH₂)-(CH₂)-O-R²³, -(CR²¹R²²)-(CH₂)-(CH₂)-S-R²³ oder -(CR²¹R²²)-(CH₂)-(CH₂)-(CH₂)-R²³ steht;
oder für -(CH=CH)-R²⁴ steht;
R⁶ für eine Gruppe -NR¹⁰R¹¹ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl steht;
für einen Rest ausgewählt aus der Gruppe bestehend aus Phenyl, Naphthyl, Thiophenyl, Furanyl, Pyrazolyl, Thiazolyl, (3,4)-Dihydro-2H-benzo[1.4]oxazinyl, (2,3)-Dihydro-benzo[1,4]dioxinyl und (1,2,3,4)-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, Methoxy, Ethoxy, n-Propoxy, Isopropoxy, n-Butoxy, Isobutoxy, tert-Butoxy, - NO₂, -O-CF₃, -O-CHF₂, -O-CH₂F, Methyl, Ethyl, n-Propyl, iso-Propyl, n-Butyl, iso-Butyl, sec-Butyl, tert-Butyl, n-Pentyl, -C(CH₃)₂-CH₂-CH₃, -C(=O)-O-CH₃,-C(=O)-O-C₂H₅, -C(=O)-O-C(CH₃)₃, -NH-C(=O)-CH₃, -NH-C(=O)-C₂H₅, -NH-C(=O)-C(CH₃)₃, -C(=O)-CF₃, -C(=O)-C₂F₅, -S(=O)₂-CH₃, -S(=O)₂-C₂H₅, -S(=O)-C(CH₃)₃, Phenyl und Benzyl substituiert sein können;
für -(CHR²⁵)-R²⁶ oder -(CHR²⁵)-(CH₂)-R²⁶ steht;
oder für -(CHR²⁷)-R²⁸ oder -(CHR²⁷)-(CH₂)-R²⁸ steht;
R⁷ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl steht;
für einen (2,3)-Dihydroindolyl-Rest steht;
für einen Rest ausgewählt aus der Gruppe bestehend aus Phenyl, Naphthyl und Indolyl steht;
oder für einen -NR^{7a}R^{7b}-Rest steht, worin R^{7a} und R^{7b}, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl und n-Pentyl stehen;
R⁸ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl und n-Hexyl steht;
oder für einen Phenyl-Rest steht, der über eine -(CH₂)-Gruppe gebunden sein kann;
R⁹ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl steht;
für einen 9H-Fluorenyl-Rest steht, der über eine -(CH₂)-Gruppe gebunden sein kann;
oder für einen Phenyl-Rest steht, der über eine -(CH₂)-Gruppe gebunden sein kann;
R¹⁰ und R¹¹, unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
R¹², R¹⁴, R¹⁶, R¹⁸, R²¹, R²², R²⁵ und R²⁷, unabhängig voneinander, jeweils
für einen Wasserstoff-Rest stehen
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl stehen;
R¹³ für einen (hetero)cycloaliphatischen Rest ausgewählt aus der Gruppe bestehend aus Cyclopropyl, Cyclobutyl, Cyclopentyl, Cyclohexyl, Cycloheptyl, Pyrrolidinyl, Piperidinyl, Morpholinyl und Piperazinyl stehen;
R¹⁵ und R¹⁷, unabhängig voneinander, jeweils
für einen Phenyl-Rest stehen;
R¹⁹ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl steht;
oder für einen Phenyl-Rest steht;
R²⁰ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, 2-Hexyl und 3-Hexyl steht;
oder für einen Phenyl-Rest, der über eine -(CH₂)-Gruppe gebunden sein kann, steht;
R²³ für einen Rest ausgewählt aus der Gruppe bestehend aus Cyclopropyl, Cyclobutyl, Cyclopentyl, Cyclohexyl, Cycloheptyl, Pyrrolidinyl, Piperidinyl, Morpholinyl, Piperazinyl, Dithiolanyl, Isoindol-1,3-dionyl, Benzoxazolin-2-onyl und 2,5-Dioxo-imidazolidinyl steht, wobei der Rest jeweils mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl und n-Pentyl substituiert sein kann;
für einen Rest ausgewählt aus der Gruppe bestehend aus Phenyl, Naphthyl, Thiophenyl, Furanyl und Thiazolyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -OH, Methoxy, Ethoxy, n-Propoxy, Isopropoxy, n-Butoxy, Isobutoxy, tert-Butoxy, Methyl, Ethyl, n-Propyl, iso-Propyl, n-Butyl, iso-Butyl, sec-Butyl, tert-Butyl, n-Pentyl, -O-C(=O)-Phenyl, Phenyl und Benzyl substituiert sein kann, wobei jeweils der zyklische Teil der Reste -O-C(=O)-Phenyl, Phenyl und Benzyl mit 1, 2, 3, 4 oder 5 Substituenten unabhängig voneinander ausgewählt aus der Gruppe bestehend aus F, Cl und Br substituiert sein kann;
R²⁴ und R²⁸, unabhängig voneinander, jeweils
für einen Phenyl-Rest stehen, wobei der Rest jeweils mit 1, 2 oder 3 Substituenten unabhängig voneinander ausgewählt aus der Gruppe bestehend aus F, Cl, Br und -CF₃ substituiert sein kann; und
R²⁶ für einen 7,7-Dimethyl-2-oxo-bicyclo[2.2.1]hept-1-yl-Rest steht,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

14. Verbindungen gemäß einem oder mehreren der Ansprüche 1-13 ausgewählt aus der Gruppe bestehend aus
[1] 3-Fluor-N-[6-(4-fluor-benzoyl)-5,6,7,8-tetrahydro-pyrido[4,3-d]pyrimidin-2-yl]-benzamid,
[2] 3,5-Dichlor-N-[6-(3-fluor-4-methoxy-benzoyl)-5,6,7,8-tetrahydro-pyrido[4,3-d]pyrimidin-2-yl]-benzamid,
[3] 4-tert-Butyl-N-(6-hexanoyl-5,6,7,8-tetrahydro-pyrido[4,3-d]pyrimidin-2-yl)-benzamid,
[4] N-(6-Acetyl-5,6,7,8-tetrahydro-pyrido[4,3-d]pyrimidin-2-yl)-3,4-dichlor-benzamid,
[5] 3,5-Dichlor-N-[6-(3-trifluormethyl-benzoyl)-5,6,7,8-tetrahydro-pyrido[4,3-d]pyrimidin-2-yl]-benzamid,
[6] 3-Chlor-N-[6-(4-methoxy-2,3,6-trimethyl-benzolsulfonyl)-5,6,7,8-tetrahydro-pyrido[4,3-d]pyrimidin-2-yl]-benzamid,
[7] N-[6-(2-Ethoxy-benzoyl)-5,6,7,8-tetrahydro-pyrido[4,3-d]pyrimidin-2-yl]-3-fluor-benzamid,
[8] 3-Chlor-N-[6-(3-phenyl-propionyl)-5,6,7,8-tetrahydro-pyrido[4,3-d]pyrimidin-2-yl]-benzamid,
[9] 4-tert-Butyl-N-[6-(isoxazol-5-carbonyl)-5,6,7,8-tetrahydro-pyrido[4,3-d]pyrimidin-2-yl]-benzamid,
[10] N-[6-(2-Benzylsulfanyl-acetyl)-5,6,7,8-tetrahydro-pyrido[4,3-d]pyrimidin-2-yl]-2-methoxy-benzamid,
[11] Thiophen-2-carbonsäure {6-[2-(4-chlor-phenyl)-2-methyl-propionyl]-5,6,7,8-tetrahydro-pyrido[4,3-d]pyrimidin-2-yl}-amid,
[12] N-(6-Benzoyl-5,6,7,8-tetrahydro-pyrido[4,3-d]pyrimidin-2-yl)-3-methyl-benzamid,
[13] N-[6-(2,3-Dihydro-benzofuran-5-carbonyl)-5,6,7,8-tetrahydro-pyrido[4,3-d]pyrimidin-2-yl]-2-fluor-benzamid,
[14] Thiophen-2-carbonsäure [6-(3,4,5-trimethoxy-benzoylamino)-5,6,7,8-tetrahydro-chinazolin-2-yl]-amid,
[15] Naphthalin-1-carbonsäure [6-(3-methyl-5-phenyl-isoxazol-4-carbonyl)-5,6,7,8-tetrahydro-pyrido[4,3-d]pyrimidin-2-yl]-amid,
[16] 3-Chlor-N-{6-[2-(5-methyl-2-phenyl-thiazol-4-yl)-acetyl]-5,6,7,8-tetrahydro-pyrido[4,3-d]pyrimidin-2-yl}-benzamid,
[17] N-[6-(4-Chlor-2,5-dimethyl-benzolsulfonyl)-5,6,7,8-tetrahydro-pyrido[4,3-d]pyrimidin-2-yl]-2-trifluormethyl-benzamid,
[18] N-[6-(1-Benzolsulfonyl-1H-indol-2-carbonyl)-5,6,7,8-tetrahydro-pyrido[4,3-d]pyrimidin-2-yl]-3-methoxy-benzamid,
[20] N-[6-(5-Methyl-1-phenyl-1H-pyrazol-4-sulfonyl)-5,6,7,8-tetrahydro-pyrido[4,3-d]pyrimidin-2-yl]-2-trifluormethyl-benzamid,
[21] N-[6-(3-Chlor-2-methyl-benzolsulfonyl)-5,6,7,8-tetrahydro-pyrido[4,3-d]pyrimidin-2-yl]-3-fluor-benzamid,
[22] 3,5-Dichlor-N-[6-(3,5-dimethyl-isoxazol-4-carbonyl)-5,6,7,8-tetrahydro-pyrido[4,3-d]pyrimidin-2-yl]-benzamid,
[23] Thiophen-2-carbonsäure [6-(4-trifluormethoxy-benzolsulfonylamino)-5,6,7,8-tetrahydro-chinazolin-2-yl]-amid,
[25] N-[6-(Furan-2-carbonyl)-5,6,7,8-tetrahydro-pyrido[4,3-d]pyrimidin-2-yl]-3-methoxy-benzamid,
[26] N-{6-[4-(2,3-Dihydro-indol-1-yl)-4-oxo-butyryl]-5,6,7,8-tetrahydro-pyrido[4,3-d]pyrimidin-2-yl}-3-methyl-benzamid,
[27] N-{6-[2-(4-Methyl-cyclohexyl)-acetyl]-5,6,7,8-tetrahydro-pyrido[4,3-d]pyrimidin-2-yl}-2-trifluormethyl-benzamid,
[28] 3,4-Difluor-N-[6-(6-phenoxy-pyridin-3-carbonyl)-5,6,7,8-tetrahydro-pyrido[4,3-d]pyrimidin-2-yl]-benzamid,
[29] 4-tert-Butyl-N-[6-(2-phenyl-thiazol-4-carbonyl)-5,6,7,8-tetrahydro-pyrido[4,3-d]pyrimidin-2-yl]-benzamid,
[30] 3,5-Dichlor-N-[6-(2-trifluormethyl-benzolsulfonyl)-5,6,7,8-tetrahydro-pyrido[4,3-d]pyrimidin-2-yl]-benzamid,
[31] Thiophen-2-carbonsäure [6-(3-brom-benzolsulfonylamino)-5,6,7,8-tetrahydro-chinazolin-2-yl]-amid,
[32] N-[6-(2-Chlor-pyridin-4-carbonyl)-5,6,7,8-tetrahydro-pyrido[4,3-d]pyrimidin-2-yl]-3-methoxy-benzamid,
[33] 4-Fluor-N-[6-(2-methansulfonyl-benzolsulfonylamino)-5,6,7,8-tetrahydro-chinazolin-2-yl]-benzamid,
[35] N-(6-Dimethylsulfamoyl-5,6,7,8-tetrahydro-pyrido[4,3-d]pyrimidin-2-yl)-3-fluor-benzamid,
[36] 3-Fluor-N-{6-[2-(4-trifluormethyl-phenyl)-acetyl]-5,6,7,8-tetrahydro-pyrido[4,3-d]pyrimidin-2-yl}-benzamid,
[37] N-{6-[2-(1,3-Dioxo-1,3-dihydro-isoindol-2-yl)-acetyl]-5,6,7,8-tetrahydro-pyrido[4,3-d]pyrimidin-2-yl}-2-trifluormethyl-benzamid,
[38] Thiophen-2-carbonsäure {6-[4-(4-chlor-2-methyl-phenoxy)-butyrylamino]-5,6,7,8-tetrahydro-chinazolin-2-yl}-amid,
[39] N-[6-(Butan-1-sulfonyl)-5,6,7,8-tetrahydro-pyrido[4,3-d]pyrimidin-2-yl]-3-fluor-benzamid,
[40] 2-Methoxy-N-[6-(2-propyl-pentanoyl)-5,6,7,8-tetrahydro-pyrido[4,3-d]pyrimidin-2-yl]-benzamid,
[41] N-[6-(4,5-Dichlor-thiophen-2-sulfonylamino)-5,6,7,8-tetrahydro-chinazolin-2-yl]-4-fluor-benzamid,
[42] 4-Chlor-N-[6-(2-trifluormethyl-benzolsulfonyl)-5,6,7,8-tetrahydro-pyrido[4,3-d]pyrimidin-2-yl]-benzamid,
[43] Thiophen-2-carbonsäure [6-(4-methoxy-benzolsulfonylamino)-5,6,7,8-tetrahydro-chinazolin-2-yl]-amid,
[44] 5-[2-(3,4-Difluor-benzoylamino)-7,8-dihydro-5H-pyrido[4,3-d]pyrimidin-6-yl]-5-oxo-valeriansäure methyl ester,
[45] N-[6-(Benzo[b]thiophen-3-carbonyl)-5,6,7,8-tetrahydro-pyrido[4,3-d]pyrimidin-2-yl]-4-chlor-benzamid,
[46] N-[6-(5-Brom-2-methoxy-benzolsulfonylamino)-5,6,7,8-tetrahydro-chinazolin-2-yl]-4-fluor-benzamid,
[47] 4-Fluor-N-[6-(4-fluor-benzolsulfonylamino)-5,6,7,8-tetrahydro-chinazolin-2-yl]-benzamid,
[48] N-{6-[2-(1H-Indol-3-yl)-2-oxo-acetyl]-5,6,7,8-tetrahydro-pyrido[4,3-d]pyrimidin-2-yl}-2-trifluormethyl-benzamid,
[49] 5-tert-Butyl-2-methyl-furan-3-carbonsäure {2-[(thiophen-2-carbonyl)-amino]-5,6,7,8-tetrahydro-chinazolin-6-yl}-amid,
[50] N-[6-(2,5-Dimethoxy-benzolsulfonyl)-5,6,7,8-tetrahydro-pyrido[4,3-d]pyrimidin-2-yl]-3-methoxy-benzamid,
[51] Benzoesäure 2-{2-[(thiophen-2-carbonyl)-amino]-5,6,7,8-tetrahydro-chinazolin-6-ylcarbamoyl}-benzyl ester,
[52] 3,4-Difluor-N-(6-hexanoyl-5,6,7,8-tetrahydro-pyrido[4,3-d]pyrimidin-2-yl)-benzamid,
[53] 4-Ethyl-N-[6-(tetrahydro-furan-2-carbonyl)-5,6,7,8-tetrahydro-pyrido[4,3-d]pyrimidin-2-yl]-benzamid,
[54] N-[6-(2-Chlor-benzolsulfonyl)-5,6,7,8-tetrahydro-pyrido[4,3-d]pyrimidin-2-yl]-3-fluor-benzamid,
[55] 4-tert-Butyl-N-[6-(5-tert-butyl-2-methyl-2H-pyrazol-3-carbonyl)-5,6,7,8-tetrahydro-pyrido[4,3-d]pyrimidin-2-yl]-benzamid,
[56] 3-Methoxy-N-[6-(4-methoxy-benzolsulfonyl)-5,6,7,8-tetrahydro-pyrido[4,3-d]pyrimidin-2-yl]-benzamid,
[57] {2-[2-(3,4-Difluor-benzoylamino)-7,8-dihydro-5H-pyrido[4,3-d]pyrimidin-6-yl]-2-oxo-1-phenyl-ethyl}-carbaminsäure benzyl ester,
[58] 5-Phenyl-oxazol-4-carbonsäure {2-[(thiophen-2-carbonyl)-amino]-5,6,7,8-tetrahydro-chinazolin-6-yl}-amid,
[59] 4-Chlor-N-(6-dimethylsulfamoyl-5,6,7,8-tetrahydro-pyrido[4,3-d]pyrimidin-2-yl)-benzamid,
[60] 4-tert-Butyl-N-[6-(2-fluor-benzolsulfonyl)-5,6,7,8-tetrahydro-pyrido[4,3-d]pyrimidin-2-yl]-benzamid,
[61] 4-Chlor-N-[6-(3-cyclopentyl-propionyl)-5,6,7,8-tetrahydro-pyrido[4,3-d]pyrimidin-2-yl]-benzamid,
[62] 4-Chlor-N-[6-(4-phenyl-butyryl)-5,6,7,8-tetrahydro-pyrido[4,3-d]pyrimidin-2-yl]-benzamid,
[63] 4-tert-Butyl-N-[6-(5-methyl-2-phenyl-2H-[1,2,3]triazol-4-carbonyl)-5,6,7,8-tetrahydro-pyrido[4,3-d]pyrimidin-2-yl]-benzamid,
[64] 2-Chlor-N-{2-[(thiophen-2-carbonyl)-amino]-5,6,7,8-tetrahydro-chinazolin-6-yl}-isonicotinamid,
[66] N-[6-(5-Chlor-thiophen-2-sulfonyl)-5,6,7,8-tetrahydro-pyrido[4,3-d]pyrimidin-2-yl]-3-fluor-benzamid,
[67] N-[6-(4-Diethylamino-benzoyl)-5,6,7,8-tetrahydro-pyrido[4,3-d]pyrimidin-2-yl]-3-methoxy-benzamid,
[68] N-[6-(2,4-Dimethyl-thiazol-5-sulfonyl)-5,6,7,8-tetrahydro-pyrido[4,3-d]pyrimidin-2-yl]-3-methyl-benzamid,
[69] 4-Chlor-N-[6-(naphthalin-2-carbonyl)-5,6,7,8-tetrahydro-pyrido[4,3-d]pyrimidin-2-yl]-benzamid,
[70] N-[6-(2,4-Difluor-benzolsulfonyl)-5,6,7,8-tetrahydro-pyrido[4,3-d]pyrimidin-2-yl]-2-methoxy-benzamid,
[71] 3,4-Dichlor-N-[6-(4-chlor-benzolsulfonyl)-5,6,7,8-tetrahydro-pyrido[4,3-d]pyrimidin-2-yl]-benzamid,
[72] 3,4-Difluor-N-[6-(thiophen-2-sulfonyl)-5,6,7,8-tetrahydro-pyrido[4,3-d]pyrimidin-2-yl]-benzamid,
[74] 4-Ethyl-N-[6-(4-pyrazol-1-yl-benzoyl)-5,6,7,8-tetrahydro-pyrido[4,3-d]pyrimidin-2-yl]-benzamid,
[75] N-[6-(7,7-Dimethyl-2-oxo-bicyclo[2.2.1]hept-1-ylmethansulfonylamino)-5,6,7,8-tetrahydro-chinazolin-2-yl]-butyramid,
[76] 5-Oxo-5-phenyl-valeriansäure (2-butyrylamino-5,6,7,8-tetrahydro-chinazolin-6-yl)-amid,
[77] 3-[2-(4-Chlor-benzoylamino)-7,8-dihydro-5H-pyrido[4,3-d]pyrimidin-6-yl]-3-oxo-propionsäure methyl ester,
[78] N-{6-[5-(4-Chlor-phenyl)-2-methyl-furan-3-carbonyl]-5,6,7,8-tetrahydro-pyrido[4,3-d]pyrimidin-2-yl}-2-methoxy-benzamid,
[79] 4-Chlor-N-[6-(2,4-dimethyl-thiazol-5-sulfonyl)-5,6,7,8-tetrahydro-pyrido[4,3-d]pyrimidin-2-yl]-benzamid,
[80] N-[6-(2,3-Difluor-benzoyl)-5,6,7,8-tetrahydro-pyrido[4,3-d]pyrimidin-2-yl]-3-methoxy-benzamid,
[81] 4-Chlor-N-{6-[2-(2-methoxy-ethoxy)-acetyl]-5,6,7,8-tetrahydro-pyrido[4,3-d]pyrimidin-2-yl}-benzamid,
[82] 2-[2-(2-Ethoxy-benzoylamino)-7,8-dihydro-5H-pyrido[4,3-d]pyrimidin-6-sulfonyl]-benzoesäure methyl ester,
[83] 4-tert-Butyl-N-[6-(4-nitro-benzolsulfonyl)-5,6,7,8-tetrahydro-pyrido[4,3-d]pyrimidin-2-yl]-benzamid,
[86] N-[6-(5-Chlor-thiophen-2-sulfonyl)-5,6,7,8-tetrahydro-pyrido[4,3-d]pyrimidin-2-yl]-2-methoxy-benzamid,
[87] 4-Fluor-N-[6-(furan-2-carbonyl)-5,6,7,8-tetrahydro-pyrido[4,3-d]pyrimidin-2-yl]-benzamid,
[88] 4-{2-[(Thiophen-2-carbonyl)-amino]-5,6,7,8-tetrahydro-chinazolin-6-ylcarbamoyl}-piperidin-1-carbonsäure tert-butyl ester,
[89] 1-(4-Chlor-phenyl)-cyclopropancarbonsäure (2-butyrylamino-5,6,7,8-tetrahydro-chinazolin-6-yl)-amid,
[90] N-(6-Ethansulfonyl-5,6,7,8-tetrahydro-pyrido[4,3-d]pyrimidin-2-yl)-3-methoxy-benzamid,
[91] 5-Methyl-thiophen-2-carbonsäure (2-butyrylamino-5,6,7,8-tetrahydro-chinazolin-6-yl)-amid,
[92] 4-tert-Butyl-N-[6-4-methyl-3-nitro-benzoyl)-5,6,7,8-tetrahydro-pyrido[4,3-d]pyrimidin-2-yl]-benzamid,
[94] 4-Fluor-N-[6-(3-phenyl-propionylamino)-5,6,7,8-tetrahydro-chinazolin-2-yl]-benzamid,
[95] 3,4-Dichlor-N-[6-(2,4,6-trimethyl-benzolsulfonyl)-5,6,7,8-tetrahydro-pyrido[4,3-d]pyrimidin-2-yl]-benzamid,
[96] N-[6-(4-Methyl-3,4-dihydro-2H-benzo[1,4]oxazin-7-sulfonyl)-5,6,7,8-tetrahydro-pyrido[4,3-d]pyrimidin-2-yl]-nicotinamid,
[97] Thiophen-2-carbonsäure [6-(3,5-difluor-benzoylamino)-5,6,7,8-tetrahydro-chinazolin-2-yl]-amid,
[98] N-[6-(2,4-Difluor-benzolsulfonylamino)-5,6,7,8-tetrahydro-chinazolin-2-yl]-butyramid,
[99] Thiophen-2-carbonsäure [6-(2,3,5,6-tetramethyl-benzolsulfonyl)-5,6,7,8-tetrahydro-pyrido[4,3-d]pyrimidin-2-yl]-amid,
[101] N-[6-(3,4-Dichlor-benzoyl)-5,6,7,8-tetrahydro-pyrido[4,3-d]pyrimidin-2-yl]-4-ethyl-benzamid,
[102] N-[6-(7,7-Dimethyl-2-oxo-bicyclo[2.2.1]hept-1-ylmethansulfonyl)-5,6,7,8-tetrahydro-pyrido[4,3-d]pyrimidin-2-yl]-3-fluor-benzamid,
[103] N-[6-(6-Fluor-4H-benzo[1,3]dioxin-8-carbonyl)-5,6,7,8-tetrahydro-pyrido[4,3-d]pyrimidin-2-yl]-2-trifluormethyl-benzamid,
[104] [5-(2-Butyrylamino-5,6,7,8-tetrahydro-chinazolin-6-ylcarbamoyl)-pentyl]-carbaminsäure benzyl ester,
[105] 2-Ethoxy-N-[6-(4-oxo-pentanoyl)-5,6,7,8-tetrahydro-pyrido[4,3-d]pyrimidin-2-yl]-benzamid,
[106] Naphthalin-1-carbonsäure [6-(propan-1-sulfonyl)-5,6,7,8-tetrahydro-pyrido[4,3-d]pyrimidin-2-yl]-amid,
[107] N-[6-(5-Fluor-2-methyl-benzolsulfonyl)-5,6,7,8-tetrahydro-pyrido[4,3-d]pyrimidin-2-yl]-4-methyl-benzamid,
[109] 3-Chlor-N-[6-(2-methyl-benzoyl)-5,6,7,8-tetrahydro-pyrido[4,3-d]pyrimidin-2-yl]-benzamid,
[110] Thiophen-2-carbonsäure [6-(3-chlor-benzo[b]thiophen-2-carbonyl)-5,6,7,8-tetrahydro-pyrido[4,3-d]pyrimidin-2-yl]-amid,
[112] Thiophen-2-carbonsäure [6-(2-cyclopentyl-acetylamino)-5,6,7,8-tetrahydro-chinazolin-2-yl]-amid,
[113] N-[6-(3,4-Dichlor-benzoyl)-5,6,7,8-tetrahydro-pyrido[4,3-d]pyrimidin-2-yl]-2-fluor-benzamid,
[114] N-[6-(2-Chlor-benzolsulfonyl)-5,6,7,8-tetrahydro-pyrido[4,3-d]pyrimidin-2-yl]-4-ethyl-benzamid,
[115] Thiophen-2-carbonsäure [6-(4-brom-3-methyl-benzoylamino)-5,6,7,8-tetrahydro-chinazolin-2-yl]-amid,
[116] 3-Methyl-N-[6-(toluen-3-sulfonyl)-5,6,7,8-tetrahydro-pyrido[4,3-d]pyrimidin-2-yl]-benzamid,
[117] Naphthalin-1-carbonsäure [6-(5-chlor-thiophen-2-sulfonyl)-5,6,7,8-tetrahydro-pyrido[4,3-d]pyrimidin-2-yl]-amid,
[118] N-(2-Butyrylamino-5,6,7,8-tetrahydro-chinazolin-6-yl)-2,6-difluor-benzamid,
[119] 3-Chlor-N-[6-(3,4-dimethoxy-benzoyl)-5,6,7,8-tetrahydro-pyrido[4,3-d]pyrimidin-2-yl]-benzamid,
[120] N-[6-(3-Chlor-benzo[b]thiophen-2-carbonyl)-5,6,7,8-tetrahydro-pyrido[4,3-d]pyrimidin-2-yl]-benzamid,
[121] 4-Ethyl-N-[6-(thiophen-3-sulfonyl)-5,6,7,8-tetrahydro-pyrido[4,3-d]pyrimidin-2-yl]-benzamid,
[123] 2-Ethoxy-N-[6-(2-ethylsulfanyl-pyridin-3-carbonyl)-5,6,7,8-tetrahydro-pyrido[4,3-d]pyrimidin-2-yl]-benzamid,
[124] 3-[2-(3-Chlor-benzoylamino)-7,8-dihydro-5H-pyrido[4,3-d]pyrimidin-6-yl]-3-oxo-propionsäure ethyl ester,
[125] N-[6-(3-Brom-benzolsulfonyl)-5,6,7,8-tetrahydro-pyrido[4,3-d]pyrimidin-2-yl]-3-chlor-benzamid,
[126] 3,4-Dichlor-N-{6-[2-(4-chlor-phenyl)-acetyl]-5,6,7,8-tetrahydro-pyrido[4,3-d]pyrimidin-2-yl}-benzamid,
[127] 3-Chlor-N-[6-(2-chlor-6-fluor-benzoyl)-5,6,7,8-tetrahydro-pyrido[4,3-d]pyrimidin-2-yl]-benzamid,
[128] 4-Fluor-N-(6-hexanoylamino-5,6,7,8-tetrahydro-chinazolin-2-yl)-benzamid,
[129] N-[6-(5-Brom-2-methoxy-benzolsulfonyl)-5,6,7,8-tetrahydro-pyrido[4,3-d]pyrimidin-2-yl]-3-methoxy-benzamid,
[132] N-[6-(3-Cyclopentyl-propionyl)-5,6,7,8-tetrahydro-pyrido[4,3-d]pyrimidin-2-yl]-isonicotinamid,
[133] 5-Benzyl-furan-2-carbonsäure (2-butyrylamino-5,6,7,8-tetrahydro-chinazolin-6-yl)-amid
[134] 3,4-Dichlor-N-[6-(4-trifluormethoxy-benzolsulfonyl)-5,6,7,8-tetrahydro-pyrido[4,3-d]pyrimidin-2-yl]-benzamid,
[135] [((2-Butyrylamino-5,6,7,8-tetrahydro-chinazolin-6-ylcarbamoyl)-phenyl-methyl]-carbaminsäure benzyl ester,
[136] N-[6-(3,4-Dimethoxy-benzolsulfonyl)-5,6,7,8-tetrahydro-pyrido[4,3-d]pyrimidin-2-yl]-4-fluor-benzamid,
[137] 2-Fluor-N-[6-(2-methyl-5-phenyl-furan-3-carbonyl)-5,6,7,8-tetrahydro-pyrido[4,3-d]pyrimidin-2-yl]-benzamid,
[138] Thiophen-2-carbonsäure (6-pent-4-enoylamino-5,6,7,8-tetrahydro-chinazolin-2-yl)-amid,
[139] ((2-Methyl-1-{2-[(thiophen-2-carbonyl)-amino]-5,6,7,8-tetrahydro-chinazolin-6-ylcarbamoyl}-propyl)-carbaminsäure 9H-fluoren-9-ylmethyl ester,
[140] ((5-{2-[(Thiophen-2-carbonyl)-amino]-5,6,7,8-tetrahydro-chinazolin-6-ylcarbamoyl}-pentyl)-carbaminsäure tert-butyl ester,
[141] 3-Fluor-N-[6-(1,2,3,4-tetrahydro-naphthalin-2-carbonyl)-5,6,7,8-tetrahydro-pyrido[4,3-d]pyrimidin-2-yl]-benzamid,
[142] 4-Chlor-N-{6-[2-(2,5-dioxo-imidazolidin-4-yl)-acetyl]-5,6,7,8-tetrahydro-pyrido[4,3-d]pyrimidin-2-yl}-benzamid,
[143] 3-Fluor-N-[6-(toluen-4-sulfonyl)-5,6,7,8-tetrahydro-pyrido[4,3-d]pyrimidin-2-yl]-benzamid,
[144] 3,5-Dichlor-N-[6-(4-thiophen-2-yl-benzoyl)-5,6,7,8-tetrahydro-pyrido[4,3-d]pyrimidin-2-yl]-benzamid,
[145] N-[6-(3-Chlor-thiophen-2-carbonyl)-5,6,7,8-tetrahydro-pyrido[4,3-d]pyrimidin-2-yl]-2-ethoxy-benzamid,
[146] N-[6-(Toluen-3-sulfonylamino)-5,6,7,8-tetrahydro-chinazolin-2-yl]-butyramid,
[147] Thiophen-2-carbonsäure {6-[2-(2,2,2-trifluor-acetyl)-1,2,3,4-tetrahydro-isochinolin-7-sulfonylamino]-5,6,7,8-tetrahydro-chinazolin-2-yl}-amid,
[148] 4-Methyl-N-[6-(4-trifluormethylsulfanyl-benzoyl)-5,6,7,8-tetrahydro-pyrido[4,3-d]pyrimidin-2-yl]-benzamid,
[149] ((2-{2-[(Thiophen-2-carbonyl)-amino]-5,6,7,8-tetrahydro-chinazolin-6-ylcarbamoyl}-ethyl)-carbaminsäure tert-butyl ester,
[150] 2-Fluor-N-[6-(2-methyl-5-nitro-benzolsulfonyl)-5,6,7,8-tetrahydro-pyrido[4,3-d]pyrimidin-2-yl]-benzamid,
[151] N-[6-(4-Methoxy-benzolsulfonylamino)-5,6,7,8-tetrahydro-chinazolin-2-yl]-butyramid,
[152] 4-Chlor-N-[6-(2,4-difluor-benzoyl)-5,6,7,8-tetrahydro-pyrido[4,3-d]pyrimidin-2-yl]-benzamid
[153] 4-Methyl-N-(6-pent-4-enoyl-5,6,7,8-tetrahydro-pyrido[4,3-d]pyrimidin-2-yl)-benzamid,
[154] Naphthalin-1-carbonsäure [6-(3-fluor-4-methyl-benzoyl)-5,6,7,8-tetrahydro-pyrido[4,3-d]pyrimidin-2-yl]-amid,
[155] N-{6-[2-(4-Trifluormethyl-phenyl)-acetyl]-5,6,7,8-tetrahydro-pyrido[4,3-d]pyrimidin-2-yl}-benzamid,
[156] N-[6-(3,4-Dichlor-benzoyl)-5,6,7,8-tetrahydro-pyrido[4,3-d]pyrimidin-2-yl]-nicotinamid,
[157] 4-Ethyl-N-{6-[2-(2,2,2-trifluor-acetyl)-1,2,3,4-tetrahydro-isochinolin-7-sulfonyl]-5,6,7,8-tetrahydro-pyrido[4,3-d]pyrimidin-2-yl}-benzamid,
[158] 2-Methoxy-N-[6-(4-methoxy-2,3,6-trimethyl-benzolsulfonyl)-5,6,7,8-tetrahydro-pyrido[4,3-d]pyrimidin-2-yl]-benzamid,
[159] 4-Ethyl-N-[6-(2-methoxy-acetyl)-5,6,7,8-tetrahydro-pyrido[4,3-d]pyrimidin-2-yl]-benzamid,
[160] 4-Fluor-N-[6-(propan-1-sulfonylamino)-5,6,7,8-tetrahydro-chinazolin-2-yl]-benzamid,
[162] N-[6-(7,7-Dimethyl-2-oxo-bicyclo[2.2.1]hept-1-ylmethansulfonylamino)-5,6,7,8-tetrahydro-chinazolin-2-yl]-butyramid,
[163] N-{6-[2-(2,5-Dimethyl-phenyl)-acetylamino]-5,6,7,8-tetrahydro-chinazolin-2-yl}-4-fluor-benzamid,
[165] Thiophen-2-carbonsäure (6-phenylmethansulfonylamino-5,6,7,8-tetrahydro-chinazolin-2-yl)-amid,
[166] N-[6-(3,4-Dimethoxy-benzolsulfonylamino)-5,6,7,8-tetrahydro-chinazolin-2-yl]-butyramid,
[167] N-{6-[4-(1,1-Dimethyl-propyl)-benzolsulfonylamino]-5,6,7,8-tetrahydro-chinazolin-2-yl}-butyramid,
[168] N-(2-Butyrylamino-5,6,7,8-tetrahydro-chinazolin-6-yl)-3-(3-trifluormethyl-phenyl)-acrylamid,
[169] N-[6-(Butan-1-sulfonylamino)-5,6,7,8-tetrahydro-chinazolin-2-yl]-4-fluor-benzamid,
[170] Naphthalin-1-carbonsäure (6-acetyl-5,6,7,8-tetrahydro-pyrido[4,3-d]pyrimidin-2-yl)-amid,
[171] 3,5-Dichlor-N-[6-(3-diethylcarbamoyl-propionyl)-5,6,7,8-tetrahydro-pyrido[4,3-d]pyrimidin-2-yl]-benzamid,
[172] 4-Ethyl-N-[6-(4-methoxy-benzolsulfonyl)-5,6,7,8-tetrahydro-pyrido[4,3-d]pyrimidin-2-yl]-benzamid,
[173] 4-Ethyl-N-[6-(chinolin-6-carbonyl)-5,6,7,8-tetrahydro-pyrido[4,3-d]pyrimidin-2-yl]-benzamid,
[174] N-[6-(2-Cyclopropyl-acetyl)-5,6,7,8-tetrahydro-pyrido[4,3-d]pyrimidin-2-yl]-2-fluor-benzamid,
[175] N-[6-(2H-Chromen-3-carbonyl)-5,6,7,8-tetrahydro-pyrido[4,3-d]pyrimidin-2-yl]-3,4-difluor-benzamid,
[176] N-{6-[2-(4-Chlor-phenyl)-propionyl]-5,6,7,8-tetrahydro-pyrido[4,3-d]pyrimidin-2-yl}-benzamid,
[177] Thiophen-2-carbonsäure [6-(2-naphthalin-1-yl-acetylamino)-5,6,7,8-tetrahydro-chinazolin-2-yl]-amid,
[178] 4-Fluor-N-(6-phenylmethansulfonyl-5,6,7,8-tetrahydro-pyrido[4,3-d]pyrimidin-2-yl)-benzamid,
[180] Thiophen-2-carbonsäure [6-(2,5-dichlor-thiophen-3-sulfonylamino)-5,6,7,8-tetrahydro-chinazolin-2-yl]-amid,
[181] {5-[2-(4-Fluor-benzoylamino)-5,6,7,8-tetrahydro-chinazolin-6-ylcarbamoyl]-pentyl}-carbaminsäure benzyl ester,
[182] N-[6-(2,5-Dimethyl-2H-pyrazol-3-carbonyl)-5,6,7,8-tetrahydro-pyrido[4,3-d]pyrimidin-2-yl]-4-ethyl-benzamid,
[183] Naphthalin-1-carbonsäure [6-(3-fluor-benzoyl)-5,6,7,8-tetrahydro-pyrido[4,3-d]pyrimidin-2-yl]-amid,
[184] N-[6-(Benzo[b]thiophen-3-carbonyl)-5,6,7,8-tetrahydro-pyrido[4,3-d]pyrimidin-2-yl]-4-tert-butyl-benzamid,
[185] 4-Fluor-N-[6-(4-methoxy-benzolsulfonyl)-5,6,7,8-tetrahydro-pyrido[4,3-d]pyrimidin-2-yl]-benzamid,
[186] 4-tert-Butyl-N-[6-(2,3-dihydro-benzo[1,4]dioxin-6-sulfonyl)-5,6,7,8-tetrahydro-pyrido[4,3-d]pyrimidin-2-yl]-benzamid,
[187] 3-Chlor-N-{6-[2-(2,6-dichlor-phenyl)-acetyl]-5,6,7,8-tetrahydro-pyrido[4,3-d]pyrimidin-2-yl}-benzamid,
[188] Thiophen-2-carbonsäure [6-(2,3-dihydro-benzo[1,4]dioxin-6-sulfonylamino)-5,6,7,8-tetrahydro-chinazolin-2-yl]-amid,
[189] N-{6-[2-(3-Chlor-phenoxy)-acetyl]-5,6,7,8-tetrahydro-pyrido[4,3-d]pyrimidin-2-yl}-4-methyl-benzamid,
[190] N-[6-(5-Phenyl-pentanoyl)-5,6,7,8-tetrahydro-pyrido[4,3-d]pyrimidin-2-yl]-benzamid,
[191] 4-Ethyl-N-[6-(2-ethyl-butyryl)-5,6,7,8-tetrahydro-pyrido[4,3-d]pyrimidin-2-yl]-benzamid,
[192] 3,5-Dichlor-N-[6-(4-trifluormethoxy-benzoyl)-5,6,7,8-tetrahydro-pyrido[4,3-d]pyrimidin-2-yl]-benzamid,
[193] Naphthalin-1-carbonsäure [6-(4-methyl-octanoyl)-5,6,7,8-tetrahydro-pyrido[4,3-d]pyrimidin-2-yl]-amid,
[194] 4-[2-(4-Fluor-benzoylamino)-5,6,7,8-tetrahydro-chinazolin-6-ylcarbamoyl]-piperidin-1-carbonsäure tert-butyl ester,
[195] N-[6-(2-Benzyloxy-acetyl)-5,6,7,8-tetrahydro-pyrido[4,3-d]pyrimidin-2-yl]-2-methoxy-benzamid,
[196] N-[6-(2-Trifluormethyl-benzolsulfonylamino)-5,6,7,8-tetrahydro-chinazolin-2-yl]-butyramid,
[197] N-[2-(4-Fluor-benzoylamino)-5,6,7,8-tetrahydro-chinazolin-6-yl]-2-methyl-6-trifluormethyl-nicotinamid,
[198] N-[6-(3-Cyano-benzoyl)-5,6,7,8-tetrahydro-pyrido[4,3-d]pyrimidin-2-yl]-2-trifluormethyl-benzamid,
[199] 3-Methoxy-N-[6-(3-trifluormethoxy-benzoyl)-5,6,7,8-tetrahydro-pyrido[4,3-d]pyrimidin-2-yl]-benzamid,
[200] N-(6-Butyryl-5,6,7,8-tetrahydro-pyrido[4,3-d]pyrimidin-2-yl)-2-fluor-benzamid,
[201] N-(6-Benzolsulfonylamino-5,6,7,8-tetrahydro-chinazolin-2-yl)-4-fluor-benzamid,
[202] N-[6-(5-Chlor-thiophen-2-sulfonylamino)-5,6,7,8-tetrahydro-chinazolin-2-yl]-4-fluor-benzamid,
[203] Naphthalin-1-carbonsäure (6-hexanoyl-5,6,7,8-tetrahydro-pyrido[4,3-d]pyrimidin-2-yl)-amid,
[204] N-(6-Propionyl-5,6,7,8-tetrahydro-pyrido[4,3-d]pyrimidin-2-yl)-2-trifluormethyl-benzamid,
[205] N-[6-(2-Ethyl-butyryl)-5,6,7,8-tetrahydro-pyrido[4,3-d]pyrimidin-2-yl]-4-methyl-benzamid,
[206] 2-Fluor-N-[6-(3-trifluormethyl-benzolsulfonyl)-5,6,7,8-tetrahydro-pyrido[4,3-d]pyrimidin-2-yl]-benzamid,
[207] 5-Benzyl-furan-2-carbonsäure {2-[(thiophen-2-carbonyl)-amino]-5,6,7,8-tetrahydro-chinazolin-6-yl}-amid,
[208] Thiophen-2-carbonsäure {6-[2-(2,5-dimethyl-phenyl)-acetylamino]-5,6,7,8-tetrahydro-chinazolin-2-yl}-amid,
[210] 4-Fluor-N-[6-(propan-1-sulfonyl)-5,6,7,8-tetrahydro-pyrido[4,3-d]pyrimidin-2-yl]-benzamid,
[211] 2-[2-(3-Fluor-benzoylamino)-7,8-dihydro-5H-pyrido[4,3-d]pyrimidin-6-sulfonyl]-benzoesäure methyl ester,
[212] 3,5-Dichlor-N-[6-(5-[1,2]dithiolan-3-yl-pentanoyl)-5,6,7,8-tetrahydro-pyrido[4,3-d]pyrimidin-2-yl]-benzamid,
[213] Thiophen-2-carbonsäure [6-(2-phenoxy-butyrylamino)-5,6,7,8-tetrahydro-chinazolin-2-yl]-amid,
[214] Thiophen-2-carbonsäure {6-[2-(4-methoxy-phenyl)-acetylamino]-5,6,7,8-tetrahydro-chinazolin-2-yl}-amid,
[215] N-(6-Cyclohexancarbonyl-5,6,7,8-tetrahydro-pyrido[4,3-d]pyrimidin-2-yl)-2-trifluormethyl-benzamid,
[216] N-[6-(2,4-Dimethyl-thiazol-5-sulfonyl)-5,6,7,8-tetrahydro-pyrido[4,3-d]pyrimidin-2-yl]-2-trifluormethyl-benzamid,
[217] 2-Thiophen-2-yl-thiazol-4-carbonsäure {2-[(thiophen-2-carbonyl)-amino]-5,6,7,8-tetrahydro-chinazolin-6-yl}-amid,
[219] Thiophen-2-carbonsäure {6-[3-(3-trifluormethyl-phenyl)-acryloylamino]-5,6,7,8-tetrahydro-chinazolin-2-yl}-amid,
[220] Thiophen-2-carbonsäure [6-(5-phenyl-pentanoylamino)-5,6,7,8-tetrahydro-chinazolin-2-yl]-amid,
[221] Thiophen-2-carbonsäure [6-(2-chlor-5-trifluormethyl-benzoylamino)-5,6,7,8-tetrahydro-chinazolin-2-yl]-amid,
[222] 4-Fluor-N-[6-(2,3,5,6-tetramethyl-benzolsulfonylamino)-5,6,7,8-tetrahydro-chinazolin-2-yl]-benzamid,
[223] 4-Fluor-N-[6-(4-methyl-octanoyl)-5,6,7,8-tetrahydro-pyrido[4,3-d]pyrimidin-2-yl]-benzamid,
[224] 2-{2-[(Thiophen-2-carbonyl)-amino]-5,6,7,8-tetrahydro-chinazolin-6-ylsulfamoyl}-benzoesäure methyl ester,
[225] [((3-Methyl-1-{2-[(thiophen-2-carbonyl)-amino]-5,6,7,8-tetrahydro-chinazolin-6-ylcarbamoyl}-butylcarbamoyl)-methyl]-carbaminsäure benzyl ester,
[226] 3-Chlor-N-(6-pentanoyl-5,6,7,8-tetrahydro-pyrido[4,3-d]pyrimidin-2-yl)-benzamid,
[227] 4-Fluor-N-[6-(5-oxo-5-phenyl-pentanoylamino)-5,6,7,8-tetrahydro-chinazolin-2-yl]-benzamid,
[228] Thiophen-2-carbonsäure [6-(3-phenyl-acryloylamino)-5,6,7,8-tetrahydro-chinazolin-2-yl]-amid,
[229] N-[6-(5-[1,2]Dithiolan-3-yl-pentanoylamino)-5,6,7,8-tetrahydro-chinazolin-2-yl]-4-fluor-benzamid,
[230] Benzoesäure 2-(2-{2-[(thiophen-2-carbonyl)-amino]-5,6,7,8-tetrahydro-chinazolin-6-ylcarbamoyl}-ethyl)-phenyl ester,
[231] 4-Fluor-N-[6-(2-methyl-5-nitro-benzolsulfonylamino)-5,6,7,8-tetrahydro-chinazolin-2-yl]-benzamid,
[232] 4-Chlor-N-[6-(2-phenoxy-acetyl)-5,6,7,8-tetrahydro-pyrido[4,3-d]pyrimidin-2-yl]-benzamid,
[234] N-[6-(5-Brom-thiophen-2-carbonyl)-5,6,7,8-tetrahydro-pyrido[4,3-d]pyrimidin-2-yl]-2-fluor-benzamid,
[235] 4-Fluor-N-[6-(5-fluor-2-methyl-benzolsulfonylamino)-5,6,7,8-tetrahydro-chinazolin-2-yl]-benzamid,
[236] N-[6-(4-Acetylamino-benzolsulfonylamino)-5,6,7,8-tetrahydro-chinazolin-2-yl]-4-fluor-benzamid,
[237] Thiophen-2-carbonsäure [6-(2-trifluormethyl-benzoylamino)-5,6,7,8-tetrahydro-chinazolin-2-yl]-amid,
[238] 3,4-Difluor-N-[6-(chinolin-6-carbonyl)-5,6,7,8-tetrahydro-pyrido[4,3-d]pyrimidin-2-yl]-benzamid,
[239] {1-[2-(4-Fluor-benzoylamino)-5,6,7,8-tetrahydro-chinazolin-6-ylcarbamoyl]-2-phenyl-ethyl}-carbaminsäure tert-butyl ester,
[240] Thiophen-2-carbonsäure [6-(2-methyl-benzoylamino)-5,6,7,8-tetrahydro-chinazolin-2-yl]-amid,
[241] 3-Chlor-N-{6-[3-(2-oxo-benzooxazol-3-yl)-propionyl]-5,6,7,8-tetrahydro-pyrido[4,3-d]pyrimidin-2-yl}-benzamid,
[242] 3-Chlor-N-[6-(5-chlor-thiophen-2-sulfonyl)-5,6,7,8-tetrahydro-pyrido[4,3-d]pyrimidin-2-yl]-benzamid,
[243] 3,5-Dichlor-N-[6-(4-methoxy-benzolsulfonyl)-5,6,7,8-tetrahydro-pyrido[4,3-d]pyrimidin-2-yl]-benzamid,
[244] 4-tert-Butyl-N-[6-(5-methyl-isoxazol-3-carbonyl)-5,6,7,8-tetrahydro-pyrido[4,3-d]pyrimidin-2-yl]-benzamid,
[245] N-[6-(5-Brom-2-methoxy-benzolsulfonyl)-5,6,7,8-tetrahydro-pyrido[4,3-d]pyrimidin-2-yl]-4-methyl-benzamid,
[246] 4-tert-Butyl-N-{6-[3-(2-hydroxy-phenyl)-propionyl]-5,6,7,8-tetrahydro-pyrido[4,3-d]pyrimidin-2-yl}-benzamid,
[247] 4-Fluor-N-[6-(2-phenyl-butyrylamino)-5,6,7,8-tetrahydro-chinazolin-2-yl]-benzamid,
[248] 4-tert-Butyl-N-[6-(4-propyl-benzoyl)-5,6,7,8-tetrahydro-pyrido[4,3-d]pyrimidin-2-yl]-benzamid,
[249] Thiophen-2-carbonsäure [6-(2-brom-benzoylamino)-5,6,7,8-tetrahydro-chinazolin-2-yl]-amid,
[250] 2-Methoxy-N-[6-(6-phenoxy-pyridin-3-carbonyl)-5,6,7,8-tetrahydro-pyrido[4,3-d]pyrimidin-2-yl]-benzamid,
[251] N-[6-(4-Acetylamino-benzolsulfonyl)-5,6,7,8-tetrahydro-pyrido[4,3-d]pyrimidin-2-yl]-4-ethyl-benzamid,
[252] 3,5-Dichlor-N-[6-(4-fluor-benzolsulfonyl)-5,6,7,8-tetrahydro-pyrido[4,3-d]pyrimidin-2-yl]-benzamid,
[253] N-[6-(1-Benzolsulfonyl-1H-indol-2-carbonyl)-5,6,7,8-tetrahydro-pyrido[4,3-d]pyrimidin-2-yl]-4-fluor-benzamid und
[255] N-[6-(3-Chlor-benzolsulfonylamino)-5,6,7,8-tetrahydro-chinazolin-2-yl]-4-fluor-benzamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

15. Verfahren zur Herstellung substituierter 5,6,7,8-Tetrahydro-pyrido[4,3-d]pyrimidin-2-yl-Verbindungen gemäß einem oder mehreren der Ansprüche 1-14, in denen X für eine NR^{2a}-Gruppe steht, **dadurch gekennzeichnet, daß** ein geschütztes Piperidin-4-on der allgemeinen Formel II, 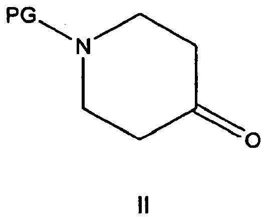 worin PG für eine Schutzgruppe steht, durch Umsetzung mit Dimethoxy-methyl-dimethyl-amin der Formel III 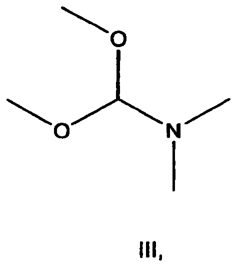 in einem organischen Reaktionsmedium in eine Verbindung der allgemeinen Formel IV, 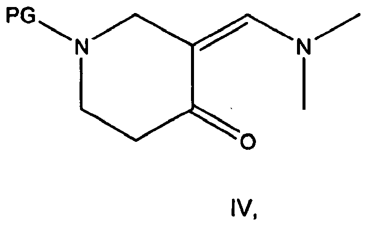 worin PG die vorstehend genannte Bedeutung hat, überführt wird, diese ggf. gereinigt und/oder isoliert wird, und durch Umsetzung mit Guanidin der Formel V, 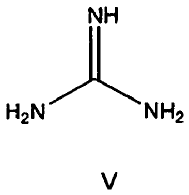 ggf. in Form eines entsprechenden Salzes, in einem organischen Reaktionsmedium, ggf. in Gegenwart wenigstens einer Base, in eine Verbindung der allgemeinen Formel VI, 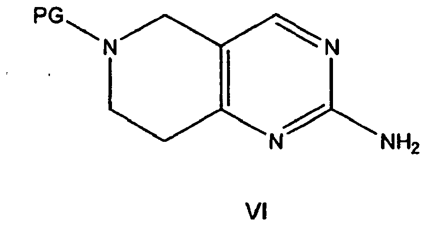 worin PG die vorstehend genannte Bedeutung hat, überführt wird, diese ggf. gereinigt und/oder isoliert wird, und in einem organischen Reaktionsmedium, ggf. in Gegenwart wenigstens einer Base und/oder wenigstens eines Katalysators, mit einer Verbindung der allgemeinen Formel R³-C(=O)-X¹ oder einer Verbindung der allgemeinen Formel (R³-C(=O))₂O, oder durch Umsetzung mit einer Verbindung der allgemeinen Formel R³-COOH in einem organischen Reaktionsmedium in Gegenwart eines geeigneten Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Anwesenheit einer Base, wobei R³ und R⁴ jeweils die Bedeutung gemäß einem oder mehreren der Ansprüche 1-15 haben und X¹ und X^{1a} jeweils für eine Austrittsgruppe stehen, zu einer Verbindung der allgemeinen Formel VII, 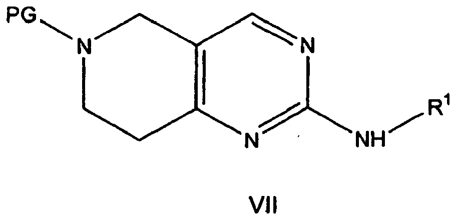 worin PG die vorstehend genannte Bedeutung hat und R¹ die Bedeutung gemäß einem oder mehreren der Ansprüche 1-15 hat, umgesetzt wird, diese ggf. gereinigt und/oder isoliert wird, und durch Abspaltung der Schutzgruppe PG in eine Verbindung der allgemeinen Formel VIII, 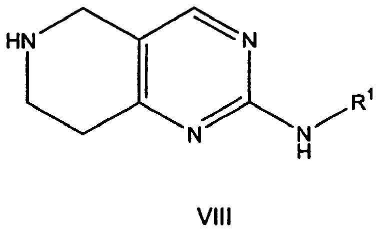 ggf. in Form eines entsprechenden Salzes überführt wird, diese ggf. gereinigt und/oder isoliert wird, und durch Umsetzung mit einer Verbindung der allgemeinen Formel R⁵-C(=O)-X² oder (R⁵-C(=O))₂O, worin R⁵ jeweils die Bedeutung gemäß einem oder mehreren der Ansprüche 1-15 hat und X² für eine Austrittsgruppe steht, in einem organischen Reaktionsmedium, ggf. in Gegenwart wenigstens einer Base und/oder in Gegenwart wenigstens eines Katalysators,
oder durch Umsetzung mit einer Verbindung der allgemeinen Formel R⁶-SO₂-X³, worin R⁶ die Bedeutung gemäß einem oder mehreren der Ansprüche 1-15 hat und X³ für eine Austrittsgruppe steht, in einem organischen Reaktionsmedium, ggf. in Gegenwart wenigstens einer Base, oder durch Umsetzung mit einer Verbindung der allgemeinen Formel
R⁵-COOH, worin R⁵ die Bedeutung gemäß einem oder mehreren der Ansprüche 1-15 hat, in einem organischen Reaktionsmedium, in Gegenwart eines geeigneten Kopplungsmittels, ggf. in Gegenwart wenigstens einer Base, in eine Verbindung der allgemeinen Formel IX 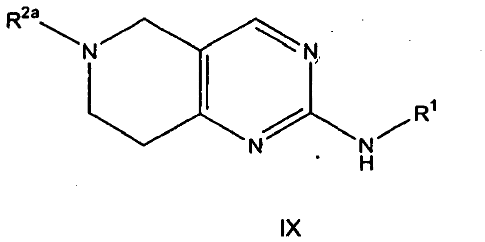 überführt, worin R¹ und R^{2a} die Bedeutung gemäß einem oder mehreren der Ansprüche 1-15 haben, diese ggf. reinigt und/oder isoliert, und ggf. in ein entsprechendes Salz überführt und dieses ggf. reinigt und/oder isoliert,
oder Piperidin-4-on der allgemeinen Formel X 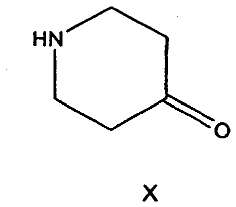 ggf. in Form eines entsprechenden Salzes, durch Umsetzung mit einer Verbindung der allgemeinen Formel R⁵-C(=O)-X² oder (R⁵-C(=O))₂O, worin R⁵ jeweils die Bedeutung gemäß einem oder mehreren der Ansprüche 1-15 hat und X² für eine Austrittsgruppe steht, in einem organischen Reaktionsmedium, ggf. in Gegenwart wenigstens einer Base und/oder in Gegenwart wenigstens eines Katalysators, oder durch Umsetzung mit einer Verbindung der allgemeinen Formel R⁶-SO₂-X³, worin R⁶ die Bedeutung gemäß einem oder mehreren der Ansprüche 1-15 hat und X³ für eine Austrittsgruppe steht, in einem organischen Reaktionsmedium, ggf. in Gegenwart wenigstens einer Base, oder durch Umsetzung mit einer Verbindung der allgemeinen Formel
R⁵-COOH, worin R⁵ die Bedeutung gemäß einem oder mehreren der Ansprüche 1-15 hat, in einem organischen Reaktionsmedium, in Gegenwart eines geeigneten Kopplungsmittels, ggf. in Gegenwart wenigstens einer Base, in eine Verbindung der allgemeinen Formel XI, 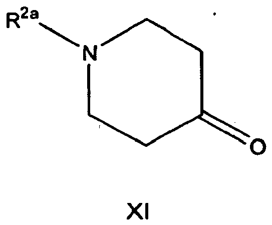 worin R^{2a} die Bedeutung gemäß einem oder mehreren der Ansprüche 1-15 hat, diese ggf. gereinigt und/oder isoliert wird, und durch Umsetzung mit Dimethoxy-methyl-dimethyl-amin der Formel III 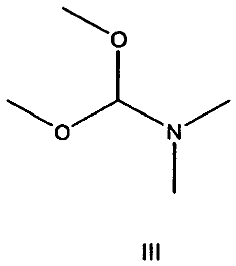 in einem organischen Reaktionsmedium in eine Verbindung der allgemeinen Formel XII, 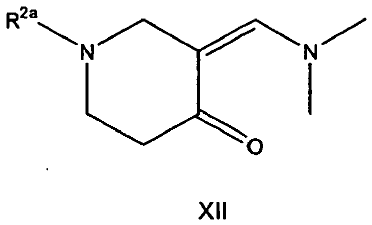 überführt wird, worin R^{2a} die vorstehend genannte Bedeutung hat, diese ggf. gereinigt und/oder isoliert wird, und mit Guanidin der Formel V, 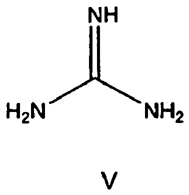 ggf. in Form eines entsprechenden Salzes, in einem organischen Reaktionsmedium, ggf. in Gegenwart wenigstens einer Base, in eine Verbindung der allgemeinen Formel XIII 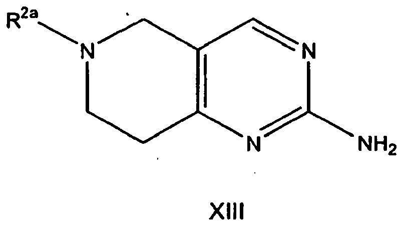 überführt wird, diese ggf. gereinigt und/oder isoliert wird, und in einem organischen Reaktionsmedium, ggf. in Gegenwart wenigstens einer Base und/oder wenigstens eines Katalysators, mit einer Verbindung der allgemeinen Formel (R³-C(=O))₂O oder einer Verbindung der allgemeinen Formel R³-C(=O)-X¹ oder durch Umsetzung mit einer Verbindung der allgemeinen Formel R³-COOH in einem organischen Reaktionsmedium, in Gegenwart eines geeigneten Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Anwesenheit einer Base, wobei R³ und R⁴ jeweils die Bedeutung gemäß einem oder mehreren der Ansprüche 1-15 haben und X¹ und X^{1a} jeweils für eine Austrittsgruppe stehen, zu einer Verbindung der allgemeinen Formel XIV 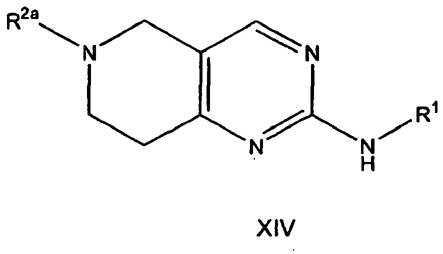 worin R¹ und R^{2a} die Bedeutung gemäß einem oder mehreren der Ansprüche 1-15 haben, umsetzt, und diese ggf. reinigt und/oder isoliert, und diese ggf. in ein entsprechendes Salz überführt und dieses Salz ggf. reinigt und/oder isoliert.

16. Verfahren zur Herstellung substituierter 5,6,7,8-Tetrahydro-chinazolin-2-yl-Verbindungen gemäß einem oder mehreren der Ansprüche 1-14, worin X für eine C(H)(NHR²)-Gruppe steht, **dadurch gekennzeichnet, daß** 1,4-Dioxa-spiro[4.5]dec-8-yl-amin der Formel C 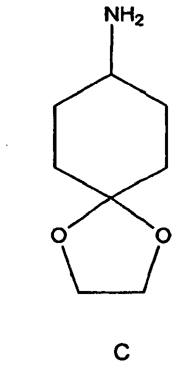 durch Umsetzung mit einer Verbindung der allgemeinen Formel R⁵-C(=O)-X⁴ oder (R⁵-C(=O))₂O, worin R⁵ jeweils die Bedeutung gemäß einem oder mehreren der Ansprüche 1-15 hat und X⁴ für eine Austrittsgruppe steht, in einem organischen Reaktionsmedium, ggf. in Gegenwart wenigstens einer Base und/oder in Gegenwart wenigstens eines Katalysators,
oder durch Umsetzung mit einer Verbindung der allgemeinen Formel R⁶-SO₂-X⁵, worin R⁶ die Bedeutung gemäß einem oder mehreren der Ansprüche 1-15 hat und X⁵ für eine Austrittsgruppe steht, in einem organischen Reaktionsmedium, ggf. in Gegenwart wenigstens einer Base, oder durch Umsetzung mit einer Verbindung der allgemeinen Formel
R⁵-COOH, worin R⁵ die Bedeutung gemäß einem oder mehreren der Ansprüche 1-15 hat, in einem organischen Reaktionsmedium, in Gegenwart wenigstens eines geeigneten Kopplungsmittels, ggf. in Gegenwart wenigstens einer Base, in eine Verbindung der allgemeinen Formel D 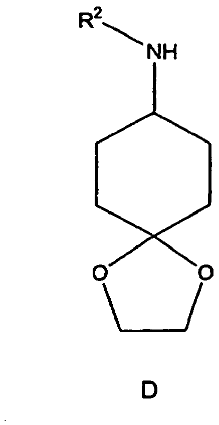 überführt wird, worin R² die Bedeutung gemäß einem oder mehreren der Ansprüche 1 bis 15 hat, diese ggf. gereinigt und/oder isoliert wird, und diese durch Abspaltung der Acetal-Gruppe in einem organischen Reaktionsmedium in Gegenwart von Wasser und wenigstens einer Säure in eine Verbindung der allgemeinen Formel E, 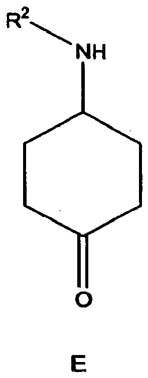 worin R² die vorstehend genannte Bedeutung hat, überführt wird, und diese durch Einführung einer Schutzgruppe (PG¹) in einem organischen Reaktionsmedium in eine Verbindung der allgemeinen Formel F 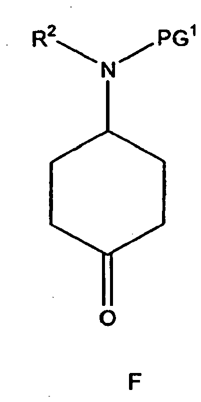 überführt wird, worin R² die vorstehend genannte Bedeutung hat und PG¹ für eine Schutzgruppe steht, diese ggf. gereinigt und/oder isoliert wird, und diese durch Umsetzung mit Dimethoxymethyl-dimethyl-amin der allgemeinen Formel III 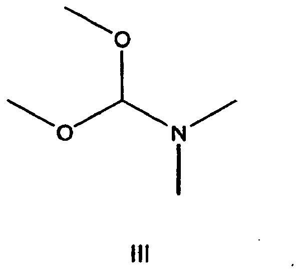 in einem organischen Reaktionsmedium in eine Verbindung der allgemeinen Formel G 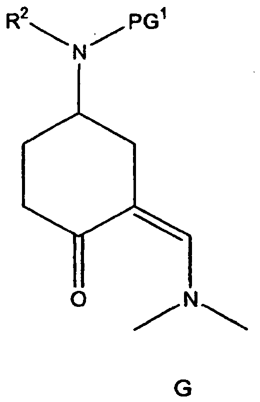 worin R² und PG¹ die vorstehend genannte Bedeutung haben, überführt wird und diese ggf. gereinigt und/oder isoliert wird, und ggf. in Gegenwart wenigstens einer Base in einem organischen Reaktionsmedium mit einer Guanidin-Verbindung der Formel V, ggf. in Form eines entsprechenden Salzes, 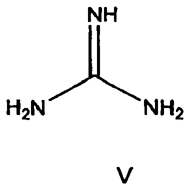 in eine Verbindung der allgemeinen Formel H 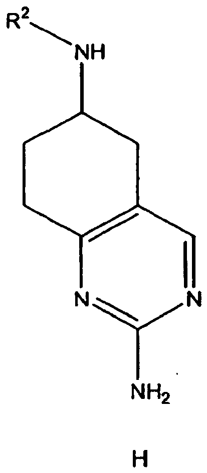 überführt wird, diese ggf. gereinigt und/oder isoliert wird, und diese durch Einführung einer Schutzgruppe (PG2) in einem organischen Reaktionsmedium in eine Verbindung der allgemeinen Formel I 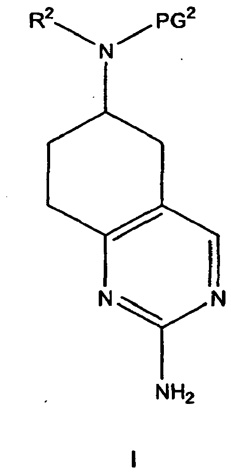 überführt wird, worin R² und PG² die vorstehend genannte Bedeutung haben, diese ggf. gereinigt und/oder isoliert wird, und diese durch Umsetzung mit einer Verbindung der allgemeinen Formel R³-C(=O)-X⁶ oder einer Verbindung der allgemeinen Formel (R³-C(=O))₂O, worin R³ jeweils die Bedeutung gemäß einem oder mehreren der Ansprüche 1-15 hat und X⁶ für eine Austrittsgruppe steht, oder durch Umsetzung mit einer Verbindung der allgemeinen Formel R³COOH, worin R³ die vorstehend genannte Bedeutung hat, in einem organischen Reaktionsmedium in Gegenwart eines Kopplungsmittels, ggf. in Gegenwart wenigstens einer Base, oder durch Umsetzung mit einer Verbindung der allgemeinen Formel R⁴-O-C(=O)-X^{1a} in einem organischen Reaktionsmedium, ggf. in Gegenwart einer Base, in eine Verbindung der allgemeinen Formel J 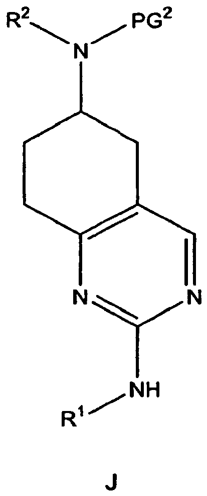 überführt, worin R² und PG² die vorstehend genannte Bedeutung haben und R¹ die Bedeutung gemäß einem oder mehreren der Ansprüche 1-15 hat, diese ggf. reinigt und/oder isoliert, und diese durch Abspaltung der Schutzgruppe in eine entsprechend substituierte 5,6,7,8-Tetrahydro-chinazolin-2-yl-Verbindung gemäß einem oder mehreren der Ansprüche 1-15, worin X für eine C(H)(NHR²)-Gruppe steht, überführt, diese ggf. reinigt und/oder isoliert und anschließend ggf. in ein entsprechendes Salz überführt, und dieses ggf. reinigt und/oder isoliert.

17. Arzneimittel enthaltend wenigstens eine Verbindung gemäß einem oder mehreren der Ansprüche 1-14 und ggf. einen oder mehrere pharmazeutisch verträgliche Hilfsstoffe.

18. Arzneimittel gemäß Anspruch 17 zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation.

19. Arzneimittel gemäß Anspruch 17 zur Prophylaxe und/oder Behandlung von Störungen und/oder Krankheiten, die zumindest teilweise durch einen oder mehreren der Rezeptoren ausgewählt aus der Gruppe bestehend aus Noradrenalin-Rezeptoren, 5-HT-Rezeptoren, mGluR5-Rezeptoren und Batrachotoxin-(BTX)-Rezeptoren vermittelt werden.

20. Arzneimittel gemäß einem oder mehreren der Ansprüche 17-19 zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

21. Arzneimittel gemäß einem oder mehreren der Ansprüche 17-19 zur Prophylaxe und/oder Behandlung von Migräne, Depressionen, Harninkontinenz, Husten, neurodegenerativen Erkrankungen, vorzugsweise ausgewählt aus der Gruppe bestehend aus Morbus Parkinson, Morbus Huntington, Morbus Alzheimer und multipler Sklerose, Störungen der Nahrungsmittelaufnahme, vorzugsweise ausgewählt aus der Gruppe bestehend aus Bulimie, Anorexie, Fettsucht und Kachexie, kognitiven Dysfunktionen, vorzugsweise Gedächtnisstörungen, kognitive Mangelzustände (attention deficit syndrom, ADS), Störungen des nervösen Systems, Epilepsie, Schizophrenie, cerebralen Ischämien, Muskelspasmen, Krämpfen, Diarrhoe, Pruritus, Magen-Ösophagus-Reflux-Syndrom, Panikzuständen, Alkohol- und/oder Drogen- und/oder Medikamentenmißbrauch, Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit, zur Prophylaxe und/oder Verminderung einer Toleranzentwicklung gegenüber Medikamenten, insbesondere Medikamenten auf Basis von Opioiden, zur Regulation der Nahrungsaufnahme, zur Modulation der Bewegungsaktivität, zur Regulation des kardiovaskulären Systems, zur Lokalanästhesie, zur Anxiolyse, zur Vigilanzsteigerung, zur Libidosteigerung, zur Diurese und/oder zur Antinatriurese.

22. Verwendung wenigstens einer Verbindung gemäß einem oder mehreren der Ansprüche 1-14 sowie ggf. einen oder mehrere pharmazeutisch verträgliche Hilfsstoffe zur Herstellung eines Arzneimittels zur Noradrenalin-Rezeptor-Regulation, insbesondere zur Hemmung der Noradrenalin-Wiederaufnahme (Noradrenalin-Uptake), zur 5-HT-Rezeptor-Regulation, insbesondere zur Hemmung der 5-Hydroxy-Tryptophan-Wiederaufnahme (5-HT-Uptake), zur mGluR5-Rezeptor-Regulation und/oder zur Batrachotoxin-(BTX)-Rezeptor-Regulation.

23. Verwendung wenigstens einer Verbindung gemäß einem oder mehreren der Ansprüche 1-14 sowie ggf. einen oder mehrere pharmazeutisch verträgliche Hilfsstoffe zur Herstellung eines Arzneimittels zur Prophylaxe und/oder Behandlung von Störungen und/oder Krankheiten, die zumindest teilweise durch einen oder mehrere Rezeptoren ausgewählt aus der Gruppe bestehend aus Noradrenalin-Rezeptoren, 5-HT-Rezeptoren, mGluR5-Rezeptoren und Batrachotoxin-(BTX)-Rezeptoren vermittelt werden.

24. Verwendung wenigstens einer Verbindung gemäß einem oder mehreren der Ansprüche 1-14 sowie ggf. einen oder mehrere pharmazeutisch verträgliche Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

25. Verwendung wenigstens einer Verbindung gemäß einem oder mehreren der Ansprüche 1-14 sowie ggf. einen oder mehrere pharmazeutisch verträgliche Hilfsstoffe zur Herstellung eines Arzneimittels zur zur Prophylaxe und/oder Behandlung von Migräne, Depressionen, Harninkontinenz, Husten, neurodegenerativen Erkrankungen, vorzugsweise ausgewählt aus der Gruppe bestehend aus Morbus Parkinson, Morbus Huntington, Morbus Alzheimer und multipler Sklerose, Störungen der Nahrungsmittelaufnahme, vorzugsweise ausgewählt aus der Gruppe bestehend aus Bulimie, Anorexie, Fettsucht und Kachexie, kognitiven Dysfunktionen, vorzugsweise Gedächtnisstörungen, kognitive Mangelzustände (attention deficit syndrom, ADS), Störungen des nervösen Systems, Epilepsie, Schizophrenie, cerebralen Ischämien, Muskelspasmen, Krämpfen, Diarrhoe, Pruritus, Magen-Ösophagus-Reflux-Syndrom, Panikzuständen, Alkohol- und/oder Drogen- und/oder Medikamentenmißbrauch, Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit, zur Prophylaxe und/oder Verminderung einer Toleranzentwicklung gegenüber Medikamenten, insbesondere Medikamenten auf Basis von Opioiden, zur Regulation der Nahrungsaufnahme, zur Modulation der Bewegungsaktivität, zur Regulation des kardiovaskulären Systems, zur Lokalanästhesie, zur Anxiolyse, zur Vigilanzsteigerung, zur Libidosteigerung, zur Diurese und/oder zur Antinatriurese.

## Claims

1. Substituted 5,6,7,8-tetrahydro-pyrido[4,3-d]pyrimidin-2-yl and 5,6,7,8-tetrahydro-quinazolin-2-yl compounds of the general formula I, 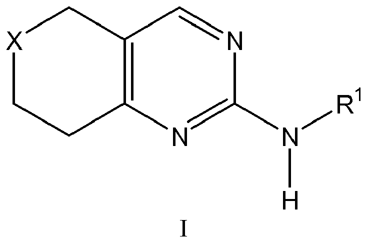 in which
X stands for a C(H)(NHR²) group or an NR^{2a} group,
R¹ stands for a -C(=O)-R³ group or a -C(=O)-O-R⁴ group,
R², R^{2a} independently of one another, respectively stand for a -C(=O)-R⁵ group or an -S(=O)₂-R⁶ group,
R³ stands for a linear or branched, unsubstituted or at least monosubstituted, saturated or unsaturated aliphatic C₁₋₈ radical, possibly comprising at least one heteroatom as chain member, an unsubstituted or at least monosubstituted, saturated or unsaturated 3- to 8-membered cycloaliphatic radical, possibly comprising at least one heteroatom as ring member, which radical may be attached via a linear or branched, unsubstituted or at least monosubstituted C₁₋₃ alkylene group, possibly comprising at least one heteroatom as chain member, or an unsubstituted or at least monosubstituted 5- to 14-membered aryl or heteroaryl radical, which may be attached via a linear or branched, unsubstituted or at least monosubstituted C₁₋₃ alkylene group,
R⁴ stands for an unsubstituted or at least monosubstituted 5- to 14-membered aryl or heteroaryl radical, which may be attached via a linear or branched, unsubstituted or at least monosubstituted C₁₋₃ alkylene group,
R⁵ stands for a linear or branched, unsubstituted or at least monosubstituted, saturated or unsaturated aliphatic C₁₋₁₀ radical, possibly comprising at least one heteroatom as chain member,
an unsubstituted or at least monosubstituted, saturated or unsaturated 3- to 8-membered cycloaliphatic radical, possibly comprising at least one heteroatom as ring member, which radical may be attached via a linear or branched, unsubstituted or at least monosubstituted C₁₋₆ alkylene group, possibly comprising at least one heteroatom as chain member and/or may be condensed with an unsubstituted or at least monosubstituted mono- or polycyclic ring system,
an unsubstituted or at least monosubstituted 5- to 14-membered aryl or heteroaryl radical, which may be attached via a linear or branched, unsubstituted or at least monosubstituted C₁₋₆ alkylene, C₂₋₆ alkenylene or C₂₋₆ alkynylene group, possibly comprising at least one heteroatom as chain member and/or may be condensed with an unsubstituted or at least monosubstituted mono- or polycyclic ring system,
a -C(=O)-R⁷ radical, which may be attached via a linear or branched, unsubstituted or at least monosubstituted C₁₋₃ alkylene group,
a -C(=O)-R⁸ radical, which may be attached via a linear or branched, unsubstituted or at least monosubstituted C₁₋₃ alkylene group, or
an -N(H)-C(=O)-O-R⁹ radical, which may be attached via a linear or branched, unsubstituted or at least monosubstituted C₁₋₈ alkylene group, possibly comprising at least one -N(H)-C(=O) or at least one -C(=O)-N(H) grouping as chain member,
R⁶ stands for a group -NR¹⁰R¹¹,
a linear or branched, unsubstituted or at least monosubstituted, saturated or unsaturated aliphatic C₁₋₁₀ radical, possibly comprising at least one heteroatom as chain member,
an unsubstituted or at least monosubstituted, saturated or unsaturated three- to eight-membered cycloaliphatic radical, possibly comprising at least one heteroatom as ring member, possibly condensed with an unsubstituted or at least monosubstituted mono- or polycyclic ring system, which radical may be attached via a linear or branched, unsubstituted or at least monosubstituted C₁₋₆ alkylene group, possibly comprising at least one heteroatom as chain member and/or may be bridged with a linear or branched C₁₋₆ alkylene group, or
an unsubstituted or at least monosubstituted 5- to 14-membered aryl or heteroaryl radical, which may be attached via a linear or branched, unsubstituted or at least monosubstituted C₁₋₆ alkylene group and/or may be condensed with an unsubstituted or at least monosubstituted mono- or polycyclic ring system,
R⁷ stands for a linear or branched, unsubstituted or at least monosubstituted C₁₋₃ alkyl radical, an unsubstituted or at least monosubstituted 5- to 14-membered aryl or heteroaryl radical, an unsubstituted or at least monosubstituted, saturated or unsaturated 5-, 6- or 7-membered cycloaliphatic radical, possibly comprising at least one heteroatom as ring member, which radical may be condensed with an unsubstituted or at least monosubstituted monocyclic ring system, or an -NR^{7a}R^{7b} radical, in which the radicals R^{7a} and R^{7b}, identical or different, respectively stand for a linear or branched C₁₋₅ alkyl radical,
R⁸ stands for a linear or branched, unsubstituted or at least monosubstituted C₁₋₃ alkyl radical or an unsubstituted or at least monosubstituted 5- or 6-membered aryl or heteroaryl radical, possibly attached via a linear or branched C₁₋₃ alkylene group,
R⁹ stands for a linear or branched, unsubstituted or at least monosubstituted C₁₋₃ alkyl radical, an unsubstituted or at least monosubstituted 5- or 6-membered aryl or heteroaryl radical, possibly attached via a linear or branched C₁₋₃ alkylene group, or an unsubstituted or at least monosubstituted, saturated or unsaturated 5- or 6-membered cycloaliphatic radical, which may be condensed with at least one unsubstituted or at least monosubstituted monocyclic ring system, and,
R¹⁰, R¹¹, identical or different, respectively stand for a linear or branched C₁₋₅ alkyl radical,
wherein
if one or more of the substituents R³ and R⁵-R⁹ stands for a saturated or unsaturated aliphatic radical, i.e. an alkyl, alkenyl or alkynyl radical, which is 1, 2, 3, 4, 5, 6, 7, 8 or 9 times substituted, the substituents thereof are respectively selected independently of one another from the group consisting of F, Cl, Br, -OH, -SH and -NH₂;
if one or more of the substituents R³, R⁵ and R⁶ stands for a saturated or unsaturated aliphatic radical, i.e. an alkyl, alkenyl or alkynyl radical, which comprises 1, 2, 3, 4 or 5 heteroatoms as chain member(s), these heteroatoms, are respectively selected independently of one another from the group consisting of oxygen, sulphur and nitrogen (NH);
if one or more of the substituents R³, R⁵-R⁷ and R⁹ stands for a cycloaliphatic radical or comprises a cycloaliphatic radical, which is 1, 2, 3, 4 or 5 times substituted, the substituents thereof are respectively selected independently of one another from the group consisting of -C₁₋₅ alkyl, -C(=O)-O-C₁₋₅-alkyl, -C(=O)-CF₃, -S(=O)₂-C₁₋₅-alkyl, -S(=O)₂-phenyl, oxo (=O) and phenyl, wherein the C₁₋₅ alkyl radical may respectively be linear or branched and the phenyl radical may respectively be 1, 2, 3, 4 or 5 times substituted, identically or differently, with a substituent selected from the group consisting of F, Cl, Br and I;
if the cycloaliphatic radicals comprise 1, 2 or 3 heteroatoms as ring members, these, are respectively selected independently of one another from the group consisting of nitrogen, oxygen and sulphur;
if one or more of the substituents R³-R⁹ stands for an aryl or heteroaryl radical or comprises an aryl or heteroaryl radical, which is 1, 2, 3, 4 or 5 times substituted, the substituents thereof may respectively be selected independently from one another from the group consisting of halogen, -CN, -NO₂, -OH, -SH, C₁₋₅ alkyl, C₁₋₅ alkoxy, -S-C₁₋₅-alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -S(=O)₂-C₁₋₅ alkyl, N(C₁₋₅alkyl)(C₁₋₅-alkyl), -C(=O)-O-C₁₋₅-alkyl, -CH₂-O-C(=O)-phenyl, -O-C(=O)-phenyl, -NH-C(=O)-C₁₋₅-alkyl, -C≡C-phenyl, -C≡C-naphthyl, -C≡C-pyrrolyl, -C≡C-indolyl, -C≡C-furyl (-C≡C-furanyl), -C≡C-benzo[b]furanyl, -C≡C-thienyl (-C≡C-thiophenyl), -C≡C-benzo[b]thienyl, -C≡C-pyrazolyl, -C≡C-imidazolyl, -C≡C-thiazolyl, -C≡C-thiadiazolyl, -C≡C-triazolyl, -C≡C-oxazolyl, -C≡C-isoxazolyl, -C≡C-pyridinyl, -C≡C-pyridazinyl, -C≡C-pyrimidinyl, -C≡C-pyrazinyl, -C≡C-pyranyl, -C≡C-indazolyl, -C≡C-purinyl, -C≡C-indolizinyl, -C≡C-quinolinyl, -C≡C-isoquinolinyl, -C≡C-quinazolinyl, pyrazolyl, phenyl, furyl (furanyl), thiadiazolyl, thiophenyl (thienyl), phenoxy and benzyl, wherein the cyclic substituents may themselves be respectively 1, 2, 3, 4 or 5 times substituted, identically or differently, with a substituent selected from the group consisting of F, Cl, Br, I;
if one or more of the substituents R³-R⁹ stands for a heteroaryl radical or comprises a heteroaryl radical, the heteroatom(s) thereof may respectively be selected independently of one another from the group consisting of oxygen, sulphur and nitrogen;
if one of the aforementioned substituents R³-R⁶ comprises a linear or branched alkylene, alkenylene or alkynylene group, which 1, 2, 3, 4 or 5 times substituted, the substituents thereof may respectively be selected independently of one another from the group consisting of F, Cl, Br, hydroxy and unsubstituted phenyl;
if the alkylene, alkenylene or alkynylene group comprises 1, 2, 3, 4 or 5 heteroatoms as chain member (s), these are selected from the group consisting of oxygen, sulphur and nitrogen (NH);
and wherein
a mono- or polycyclic ring system is understood to mean mono- or polycyclic hydrocarbon radicals, which may be saturated, unsaturated or aromatic and possibly may comprise 1, 2, 3, 4 or 5 heteroatoms as ring members, may respectively independently of one another exhibit a different degree of saturation, i.e. be saturated, unsaturated or aromatic; and
if a polycyclic ring system is present, the different rings may respectively independently of one another exhibit a different degree of saturation, i.e. be saturated, unsaturated or aromatic and the heteroatoms of each ring may, respectively identically or differently, be selected from the group consisting of oxygen, nitrogen and sulphur; and
if one or more of the substituents R⁵, R⁶, R⁷ and R⁹ comprises a monocyclic or polycyclic ring system which is 1, 2, 3, 4 or 5 times substituted, the substituents thereof may respectively be selected independently of one another from the group consisting of halogen, -CN, -NO₂, -OH, -SH, C₁₋₅ alkyl, C₁₋₅ alkoxy, -S-C₁₋₅-alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂ -phenyl, -S(=O)₂-C₁₋₅-alkyl, N(C₁₋₅alkyl)(C₁₋₅ alkyl), -C(=O)-O-C₁₋₅-alkyl, -CH₂-O-C(=O)-phenyl, -O-C(=O)-phenyl, -NH-C(=O)-C₁₋₅-alkyl, -C≡C-phenyl, -C≡C-naphthyl, -C≡C-pyrrolyl, -C≡C-indolyl, -C≡C-furyl (-C≡C-furanyl), -C≡C-benzo[b]furanyl, -C≡C-thienyl (-C≡C-thiophenyl), -C≡C-benzo[b]thienyl, -C≡C-pyrazolyl, -C≡C-imidazolyl, -C≡C-thiazolyl, -C≡C-thiadiazolyl, -C≡C-triazolyl, -C≡C-oxazolyl, -C≡C-isoxazolyl, -C≡C-pyridinyl, -C≡C-pyridazinyl, -C≡C-pyrimidinyl, -C≡C-pyrazinyl, -C≡C-pyranyl, -C≡C-indazolyl, -C≡C-purinyl, -C≡C-indolizinyl, -C≡C-quinolinyl, -C≡C-isoquinolinyl, -C≡C-quinazolinyl, pyrazolyl, phenyl, furyl (furanyl), thiadiazolyl, thiophenyl (thienyl), phenoxy and benzyl, wherein the cyclic substituents may themselves be respectively 1, 2, 3, 4 or 5 times substituted, identically or differently, with a substituent selected from the group consisting of F, Cl, Br, I;
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

2. Compounds according to claim 1, **characterised in that** R³ stands for a linear or branched, unsubstituted or at least monosubstituted C₁₋₈ alkyl radical, possibly comprising one or more oxygen atoms and/or possibly one or more NH groups as chain member(s), an unsubstituted or at least monosubstituted, saturated or unsaturated 5-, 6- or 7-membered cycloaliphatic radical possibly comprising at least one heteroatom as ring member, which radical may be attached via a linear or branched, unsubstituted or at least monosubstituted C₁₋₃ alkylene group possibly comprising at least one heteroatom as chain member, or an unsubstituted or at least monosubstituted phenyl radical, thiophenyl radical (thienyl radical), furanyl radical (furyl radical), pyridinyl radical or naphthyl radical, which may be attached via a linear or branched, unsubstituted or at least monosubstituted C₁₋₃ alkylene group.

3. Compounds according to claims 1 or 2, **characterised in that** the radical R⁴ stands for a phenyl radical possibly attached via a -(CH₂), -(CH₂)₂ or -(CH₂)₃ bridge, wherein the phenyl ring, unsubstituted or at least monosubstituted, identical or different, may be substituted with a substituent selected from the group consisting of methyl, ethyl, n-propyl, iso-propyl, n-butyl, iso-butyl, sec-butyl, tert-butyl, methoxy, ethoxy, n-propoxy, iso-propoxy, n-butoxy, iso-butoxy, sec-butoxy, tert-butoxy, F, Cl, Br, I, -CN and -CF₃,

4. Compounds according to one or more of claims 1-3, **characterised in that**
R⁵ stands for a linear or branched C₁₋₁₀ alkyl radical, possibly comprising one or more oxygen atoms and/or possibly one or more sulphur atoms and/or possibly one or more -N(H) groups as chain member(s), a linear or branched C₂₋₁₀ alkenyl radical, possibly comprising one or more oxygen atoms and/or possibly one or more sulphur atoms and/or possibly one or more -N(H) groups as chain member(s), a linear or branched C₂₋₁₀ alkynyl radical, possibly comprising one or more oxygen atoms and/or possibly one or more sulphur atom and/or possibly one or more -N(H) groups as chain member(s),
an unsubstituted or at least monosubstituted, saturated or unsaturated 3- to 8-membered cycloaliphatic radical, possibly comprising one or more heteroatoms, selected independently of one another from the group consisting of oxygen, sulphur and nitrogen as ring member(s), which radical may be attached via a -(CH₂), -(CH₂)₂, -(CH₂)₃, -(CH₂)₄, -C(C₂H₅)(H), -(CH₂)-O, -(CH₂)₂-O, -(CH₂)₃-0, -(CH₂)₄-O, -O-(CH₂), -O-(CH₂)₂, -O-(CH₂)₃, -O-(CH₂)₄, -C(C₂H₅)(H)-O-, -O-C(C₂H₅)(H), -CH₂-O-CH₂, -CH₂-S-CH₂, -C(CH₃)₂ or -C(H)(CH₃) group and/or may be condensed with an unsubstituted or at least monosubstituted, 5- or 6-membered monocyclic ring system,
an unsubstituted or at least monosubstituted, 5- or 6-membered aryl or heteroaryl radical, which may be attached via a -(CH₂)-, -(CH₂)₂, -(CH₂)₃, -(CH₂)₄, -C(C₂H₅)(H), -(CH₂)-O, -(CH₂)₂-O, -(CH₂)₃-0, -(CH₂)₄-O, -O-(CH₂), -O-(CH₂)₂, -O-(CH₂)₃, -O-(CH₂)₄, -C(C₂H₅)(H)-O, -O-C(C₂H₅)(H)-, -CH₂-O-CH₂, -CH₂-S-CH₂, -C(CH₃)₂, -C(H)(CH₃) or -CH=CH- group and/or may be condensed with an unsubstituted or at least monosubstituted 5- or 6-membered monocyclic ring system,
a -C(=O)-R⁷ radical, which may be attached via a -(CH₂), -(CH₂)₂ or -(CH₂)₃ group,
a -C(=O)-R⁸ radical, which may be attached via a linear or branched C₁₋₃ alkylene group which is unsubstituted or mono- or polysubstituted with a phenyl radical, or an -N(H)-C(=O)-O-R⁹ radical, which may be attached via a linear or branched C₁₋₈ alkylene group which is unsubstituted or mono- or polysubstituted with a phenyl radical and possibly comprises at least one -N(H)-C(=O) or at least one -C(=O)-N(H) grouping as chain member.

5. Compounds according to one or more of claims 1-4, **characterised in that** R⁶ stands for a group -NR¹⁰R¹¹,
a linear or branched, unsubstituted C₁₋₁₀ alkyl radical, a linear or branched, unsubstituted C₂₋₁₀ alkenyl radical, a linear or branched, unsubstituted C₂₋₁₀ alkynyl radical,
an unsubstituted or at least monosubstituted, saturated or unsaturated 3-, 4-, 5-, 6-, 7- or 8-membered cycloaliphatic radical, which may be attached via a linear or branched C₁₋₃ alkylene group and/or bridged with a linear or branched C₁₋₃ alkylene group,
an unsubstituted or at least monosubstituted 5- to 6-membered aryl or heteroaryl radical, which may be attached via a linear or branched C₁₋₆ alkylene group and/or may be condensed with an unsubstituted or at least monosubstituted 5- or 6-membered monocyclic ring system.

6. Compounds according to one or more of claims 1-5, **characterised in that** the radical R⁷ stands for a linear or branched, unsubstituted C₁₋₃ alkyl radical, an unsubstituted, 5- or 6-membered aryl or heteroaryl radical, an unsubstituted, saturated or unsaturated 5-, 6- or 7-membered cycloaliphatic radical, possibly comprising a nitrogen atom as ring member, which radical may be condensed with an unsubstituted 6-membered monocyclic ring system, or an -NR^{7a}R^{7b} radical, in which the radicals R^{7a} and R^{7b}, identical or different, respectively stand for a linear or branched C₁₋₃ alkyl radical.

7. Compounds according to one or more of claims 1-6, **characterised in that** R⁸ stands for a linear or branched, unsubstituted C₁₋₃ alkyl radical or a phenyl radical possibly attached via a linear or branched C₁₋₃ alkylene group.

8. Compounds according to one or more of claims 1-7, **characterised in that** R⁹ stands for linear or branched, unsubstituted C₁₋₃ alkyl radical, an unsubstituted 5- or 6-membered aryl or heteroaryl radical possibly attached via a linear or branched C₁₋₃ alkylene group, or an unsubstituted or at least monosubstituted, saturated or unsaturated, 5- or 6-membered cycloaliphatic radical, which may be condensed with at least one unsubstituted, 6-membered monocyclic ring system.

9. Compounds according to one or more of claims 1-8, **characterised in that** R¹⁰ and R¹¹, identical or different, respectively stand for a methyl, ethyl, n-propyl or iso-propyl radical.

10. Compounds according to one or more of claims 1-9, **characterised in that**
X stands for a C(H)(NHR²) group or an NR^{2a} group,
R¹ stands for a -C(=O)-R³ group or a -C(=O)-O-R⁴ group,
R², R^{2a}, independently of one another, respectively stand for a -C(=O)-R⁵ group or an -S(=O)₂-R⁶ group,
R³ stands for a methyl, ethyl, n-propyl, iso-propyl, n-butyl, iso-butyl, sec-butyl or tert-butyl radical, an unsubstituted, saturated or unsaturated 5-, 6- or 7-membered cycloaliphatic radical, possibly comprising one or more oxygen atoms and/or one or more nitrogen atoms as ring member(s), which radical may be attached via a -(CH₂), -(CH₂)₂ or -(CH₂)₃ group, or a phenyl radical, thiophenyl radical (thienyl radical), furanyl radical (furyl radical), pyridinyl radical or naphthyl radical, which may be attached via a -(CH₂), -(CH₂)₂ or -(CH₂)₃ group and/or may be mono- or polysubstituted, identically or differently, with a substituent selected from the group consisting of methyl, ethyl, n-propyl, iso-propyl, n-butyl, iso-butyl, sec-butyl, tert-butyl, methoxy, ethoxy, n-propoxy, iso-propoxy, n-butoxy, iso-butoxy, sec-butoxy, tert-butoxy, F, Cl, Br, I, -CN and -CF₃,
R⁴ stands for an unsubstituted benzyl radical,
R⁵ stands for a radical selected from the group consisting of methyl, ethyl, n-propyl, iso-propyl, n-butyl, sec-butyl, tert-butyl, n-pentyl, iso-pentyl, neopentyl, -C(H)(C₂H₅)₂, -C(H)(n-C₃H₇)₂-, -CH=CH-CH=CH-CH₃, -CH₂-CH₂-CH=CH₂, -CH₂-CH₂-C(H)(CH₃)-(CH₂)₃-CH₃, -CH₂-O-CH₃ and -CH₂-O-CH₂-CH₂-O-CH₃,
a radical selected from the group consisting of cyclopropyl, cyclobutyl, cyclopentyl, cyclohexyl, cycloheptyl, cyclooctyl, piperidinyl, piperazinyl, morpholinyl, tetrahydrofuranyl (tetrahydrofuryl), dithiolanyl, 1,2,3,4-tetrahydroindolyl, 1,2,3,4-tetrahydronaphthyl, 1,3-dihydroisoindolyl, benzooxazolyl and imidazolidinyl, wherein the cyclic radical may in each case be attached via a -(CH₂), -(CH₂)₂, -(CH₂)₃, -(CH₂)₄, -C(C₂H₅)(H), -(CH₂)-O, -(CH₂)₂-O, -(CH₂)₃-0, -(CH₂)₄-O, -O-(CH₂), -O-(CH₂)₂, -O-(CH₂)₃, -O-(CH₂)₄, -C(C₂H₅)(H)-O, -O-C(C₂H₅)(H), -CH₂-O-CH₂, -CH₂-S-CH₂, -C(CH₃)₂, or -C(H)(CH₃) group and/or be mono- or polysubstituted, identically or differently, with a substituent selected from the group consisting of phenyl, -C(=O)-O-tert-butyl, oxo (=O), -S(=O)₂-methyl and -S(=O)₂-phenyl, and wherein the aforementioned phenyl radicals may in each case be mono- or polysubstituted, identically or differently, with a substituent selected from the group consisting of F, Cl, Br and I,
a radical selected from the group consisting of phenyl, naphthyl, furanyl (furyl), thiophenyl (thienyl), pyridinyl, isoxazolyl, triazolyl, pyrazolyl, thiazolyl, benzo[1,2,5]-oxadiazolyl, 2,3-dihydrobenzofuranyl, quinolinyl, chromanyl, chromenyl, benzo[b]furanyl, benzo[b]thiophenyl, benzo[1,3] -dioxinyl, indolyl, 2,3-dihydroindolyl, 3,4-dihydro-benzo [1,4]oxazinyl and 1,2,3,4-tetrahydroisoquinolinyl, wherein the cyclic radical may in each case be attached via a -(CH₂)-, -(CH₂)₂, -(CH₂)₃, -(CH₂)₄, -C(C₂H₅)(H), -(CH₂)-O, -(CH₂)₂-O, -(CH₂)₃-0, -(CH₂)₄-O, -O-(CH₂), -O-(CH₂)₂, -O-(CH₂)₃, -O-(CH₂)₄, -C(C₂H₅)(H)-O, -O-C(C₂H₅)(H), -CH₂-O-CH₂, -CH₂-S-CH₂, -C(CH₃)₂, -C(H)(CH₃) or -CH=CH- group and/or be mono- or polysubstituted, identically or differently, with a substituent selected from the group consisting of F, Cl, Br, I, -CN, -NO₂, -OH, -SH, methyl, ethyl, n-propyl, iso-propyl, n-butyl, iso-butyl, sec-butyl, tert-butyl, n-pentyl, neopentyl, methoxy, ethoxy, n-propoxy, iso-propoxy, n-butyloxy, iso-butyloxy, sec-butyloxy, tert-butyloxy, -S-methyl, -S-ethyl, -CF₃, -CHF₂, -CH₂F, -O-CF₃, -O-CHF₂, -O-CH₂ F, -S-CF₃, -S-CHF₂, -S-CH₂F, -S(=O)₂-phenyl, pyrazolyl, phenyl, -N(CH₃)₂, -N(C₂H₅)₂, -CH₂-O-C(=O)-phenyl, -O-C(=O)-phenyl, furyl (furanyl), thiadiazolyl, thiophenyl (thienyl), phenoxy and benzyl, and wherein the cyclic substituents may themselves be respectively mono- or polysubstituted, identically or differently, with a substituent selected from the group consisting of F, Cl, Br, I,
a -C(=O)-R⁷ radical, which may be attached via a -(CH₂)-, -(CH₂)₂ or -(CH₂)₃ group,
a -C(=O)-O-R⁸ radical, which may be attached via a -(CH₂), -(CH₂)₂, -(-CH₂)₃ or -C(H)(phenyl) group, or
an -N(H)-C(=O)-O-R⁹ radical, which may be attached via a -(CH₂), -(CH₂)₂, -(CH₂)₃, -(CH₂)₄, -(CH₂)₅, -C(H)(CH₂-phenyl), -C(H)(phenyl), -C(H)(C(H)(CH₃)₂) or -C(H)(CH₂-CH(CH₃)₂)-NH-C(=O)-CH₂ group,
R⁶ stands for a group -NR¹⁰R¹¹,
a methyl, ethyl, n-propyl, iso-propyl, n-butyl, iso-butyl, sec-butyl or tert-butyl radical,
a 7,7-dimethyl-2-oxo-bicyclo[2.2.1]heptyl radical possibly attached via a -(CH₂), -(CH₂)₂ or -(CH₂)₃ group, or
a phenyl, naphthyl, thiophenyl (thienyl), furanyl, (furyl), thiazolyl, pyrazolyl, 2,3-dihydro-benzo[1,4]-dioxinyl, 1,2,3,4-tetrahydroiso-quinolinyl or 3,4-dihydrobenzo[1,4]oxazinyl radical, which may be attached via a -(CH₂), -(CH₂)₂, or -(CH₂)₃ group and/or be mono- or polysubstituted, identically or differently, with a substituent selected from the group consisting of F, Cl, Br, I, methyl, ethyl, n-propyl, iso-propyl, n-butyl, iso-butyl, sec-butyl, tert-butyl, n-pentyl, iso-pentyl, neopentyl, methoxy, ethoxy, -CN, -CF₃, -CF₂H, -CFH₂, -NO₂, -C(=O)-CF₃, -O-CF₃, -C(=O)-O-CH₃, -C(=O)-O-C₂H₅, -NH-C(=O)-CH₃, -S(=O)₂-CH₃ and phenyl,
R⁷ stands for a methyl, ethyl, n-propyl, phenyl, indolyl, 2,3-dihydroindolyl, dimethylamino or diethylamino radical,
R⁸ stands for a methyl, ethyl, phenyl or benzyl radical,
R⁹ stands for a methyl, ethyl, n-propyl, iso-propyl, n-butyl, iso-butyl, sec-butyl, tert-butyl, or a phenyl or fluorenyl radical possibly attached via a -(CH₂) group,
R¹⁰,R¹¹, identical or different, respectively stand for a methyl, ethyl, n-propyl or iso-propyl radical,
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

11. Compounds according to one or more of claims 1 to 10, **characterised in that**
X stands for a C(H)(NHR²) group or an NR^{2a} group,
R¹ stands for a -C(=O)-R³ group or a -C(=O)-O-R⁴ group,
R², R^{2a}, independently of one another, respectively stand for a -C(=O)-R⁵ group or an -S(=O)₂-R⁶ group,
R³ stands for a linear or branched, possibly substituted C₁₋₁₀ alkyl radical, C₂₋₁₀ alkenyl radical or C₂₋₁₀ alkynyl radical possibly comprising 1, 2 or 3 heteroatom(s) selected from the group consisting of oxygen, sulphur and nitrogen (NH) as chain member(s);
an unsaturated or saturated, possibly substituted 3-, 4-, 5-, 6-, 7-, 8- or 9-membered cycloaliphatic radical;
a possibly substituted 5- to 14-membered aryl or heteroaryl radical;
-(CHR¹²)-Uₐ-(CH₂)_{b}-V_{c}-(CH₂)_{d}-R¹³ with a = 0 or 1, b = 0 or 1, c = 0 or 1 and d = 0 or 1, in which U and V, independently of one another, respectively stand for O, S and NH;
or -(CHR¹⁴)-(CH₂)ₑ-R¹⁵ with e = 0, 1, 2 or 3;
R⁴ stands for a possibly substituted 5- to 14-membered aryl or heteroaryl radical;
or -(CHR¹⁶)-(CH₂)_{f}-R¹⁷ with f= 0, 1, 2 or 3;
R⁵ stands for a linear or branched, possibly substituted C₁₋₁₀ alkyl radical, C₂₋₁₀ alkenyl radical or C₂₋₁₀ alkynyl radical possibly comprising 1, 2 or 3 heteroatom(s) selected from the group consisting of oxygen, sulphur and nitrogen (NH) as chain member(s);
an unsaturated or saturated, possibly substituted 3-, 4-, 5-, 6-, 7-, 8- or 9-membered cycloaliphatic radical, which may be condensed with a saturated, unsaturated or aromatic, possibly substituted mono- or polycyclic ring system;
a possibly substituted 5- to 14-membered aryl or heteroaryl radical, which may be condensed with a saturated or unsaturated, possibly substituted mono- or polycyclic ring system;
-(CHR¹⁸)_{g}-(CH₂)ₕ-C(=O)-R⁷ with g = 0 or 1 and h = 0, 1, 2 or 3;
-(CHR¹⁹)ⱼ-(CH₂)ₖ-C(=O)-O-R⁸ with j = 0 or 1 and k = 0, 1, 2 or 3;
-(CHR²⁰)ₘ-(CH₂)ₙNH-C(=O)]ₚ-(CH₂)_{q}-NH-C(=O)-O-R⁹ with m = 0 or 1; n = 0, 1, 2, 3, 4 or 5; p = 0 or 1 and q = 0, 1 or 2;
-(CR²¹R²²)-Wᵣ-(CH₂)ₛ-Xₜ-(CH₂)ᵤ-Yᵥ-R²³ with r = 0 or 1, s = 0 or 1, t = 0 or 1, u = 0, 1 or 2 and v = 0 or 1, in which W, X and Y, independently of one another, respectively stand for O, S and NH;
or -CH=CH-R²⁴;
R⁶ stands for a group -NR¹⁰R¹¹;
a linear or branched C₁₋₁₀ alkyl radical, C₂₋₁₀ alkenyl radical or C₂₋₁₀ alkynyl radical possibly comprising substituted 1, 2 or 3 heteroatom(s) selected from the group consisting of oxygen, sulphur and nitrogen (NH) as chain member(s);
an unsaturated or saturated, possibly substituted 3-, 4-, 5-, 6-, 7-, 8- or 9-membered cycloaliphatic radical, which may be condensed with a saturated, unsaturated or aromatic, possibly substituted mono- or polycyclic ring system and/or be bridged with a linear or branched C₁₋₅ alkylene group;
a possibly substituted 5- to 14-membered aryl or heteroaryl radical, which may be condensed with a saturated or unsaturated, possibly substituted mono- or polycyclic ring system;
-(CHR²⁵)-Z_{w}-(CH₂)ₓ-A)_{y}-(CH₂)_{z}-R²⁶ with w = 0 or 1, x = 0 or 1, y = 0 or 1 and z = 0 or 1, in which Z and A, independently of one another, respectively stand for O, S and NH;
or -(CHR²⁷)-(CH₂)ₐₐ-R²⁸ with aa = 0, 1, 2 or 3;
R⁷ stands for a linear or branched, possibly substituted C₁₋₁₀ alkyl radical;
an unsaturated or saturated, possibly substituted 3-, 4-, 5-, 6-, 7-, 8- or 9-membered cycloaliphatic radical, which may be condensed with a saturated, unsaturated or aromatic, possibly substituted mono- or polycyclic ring system;
a possibly substituted 5- to 14-membered aryl or heteroaryl radical;
or an -NR^{7a}R^{7b} radical, in which the radicals R^{7a} and R^{7b}, independently of one another, respectively stand for a linear or branched C₁₋₁₀ alkyl radical;
R⁸ stands for a linear or branched, possibly substituted C₁₋₁₀ alkyl radical;
or a possibly substituted 5- to 14-membered aryl or heteroaryl radical, which may be attached via a linear or branched C₁₋₅-alkylene group;
R⁹ stands for a linear or branched, possibly substituted C₁₋₁₀ alkyl radical;
an unsaturated or saturated, possibly substituted 3-, 4-, 5-, 6-, 7-, 8- or 9-membered cycloaliphatic radical, which radical may be condensed with a saturated, unsaturated or aromatic, possibly substituted mono- or polycyclic ring system and/or be attached via a linear or branched C₁₋₅ alkylene group;
or a possibly substituted 5- to 14-membered aryl or heteroaryl radical, which may be attached via a linear or branched C₁₋₅ alkylene group;
R¹⁰ and R¹¹, independently of one another, respectively stand for a linear or branched, possibly substituted C₁₋₁₀ alkyl radical;
R¹², R¹⁴, R¹⁶, R¹⁸, R²¹, R²², R²⁵ and R²⁷, independently of one another, respectively stand for
a hydrogen radical
or a linear or branched, possibly substituted C₁₋₁₀ alkyl radical;
R¹³ stands for an unsaturated or saturated, possibly substituted 3-, 4-, 5-, 6-, 7-, 8- or 9-membered cycloaliphatic radical;
R¹⁵ and R¹⁷, independently of one another, respectively stand for a possibly substituted 5- to 14-membered aryl or heteroaryl radical;
R¹⁹ stands for a hydrogen radical;
a respectively linear or branched possibly substituted C₁₋₁₀ alkyl radical or a phenyl radical;
R²⁰ stands for a hydrogen radical;
a respectively linear or branched, possibly substituted C₁₋₁₀ alkyl radical
or a phenyl radical, which may be attached via a linear or branched C₁₋₅ alkylene group;
R²³ stands for an unsaturated or saturated, possibly substituted 3-, 4-, 5-, 6-, 7-, 8- or 9-membered cycloaliphatic radical, which may be condensed with a saturated, unsaturated or aromatic, possibly substituted mono- or polycyclic ring system;
or a possibly substituted 5- to 14-membered aryl or heteroaryl radical, which may be condensed with a saturated or unsaturated, possibly substituted mono- or polycyclic ring system;
R²⁴ and R²⁸, independently of one another, respectively stand for
a possibly substituted 5- to 14-membered aryl or heteroaryl radical, which may be condensed with a saturated or unsaturated, possibly substituted mono- or polycyclic ring system;
and
R²⁶ stands for an unsaturated or saturated, possibly substituted 3-, 4-, 5-, 6-, 7-, 8- or 9-membered cycloaliphatic radical, which may be condensed with a saturated, unsaturated or aromatic, possibly substituted mono- or polycyclic ring system and/or may be bridged with a linear or branched C₁₋₅ alkylene group;
wherein
the aforementioned C₁₋₁₀ alkyl radicals may be unsubstituted or in each case possibly substituted with 1, 2, 3, 4 or 5 substituents selected independently of one another from the group consisting of F, Cl, Br, I, -CN, -NO₂, -OH, -SH and -NH₂;
the aforementioned C₂₋₁₀ alkenyl radicals may be unsubstituted or possibly substituted in each case with 1, 2, 3, 4 or 5 substituents selected independently of one another from the group consisting of F, Cl, Br, I, -CN, -NO₂, -OH, -SH and -NH₂;
the aforementioned C₂₋₁₀ alkynyl radicals may be unsubstituted or possibly substituted in each case with 1, 2, 3, 4 or 5 substituents selected independently of one another from the group consisting of F, Cl, Br, I, -CN, -NO₂, -OH, -SH and -NH₂;
the aforementioned cycloaliphatic radicals may be unsubstituted or possibly substituted in each case with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, C₁₋₅-alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-NH₂, -C(=O)-NH-C₁₋₅-alkyl, -C(=O)-N-(C₁₋₅-akyl)₂, -S(=O)₂-NH₂, -S(=O)₂-NH-C₁₋₅-akyl, -S(=O)₂-NH-phenyl, -S(=O)₂-C₁₋₅-alkyl, -S(=O)₂-phenyl, -(CH₂)-benzo[b]furanyl, -O-phenyl, -O-benzyl, phenyl, benzyl, naphthyl and -(CH₂)-naphthyl, wherein in each case the cyclic moiety of the radicals -O-phenyl, -S(=O)₂-NH-phenyl, -S(=O)₂-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, C₁₋₅-alkyl, -O-CF₃, -S-CF₃, phenyl and -O-benzyl,
and the aforementioned cycloaliphatic radicals may in each case possibly comprise 1, 2, 3, 4 or 5 heteroatom(s) selected independently of one another from the group consisting of oxygen, nitrogen and sulphur;
and the aforementioned aryl- or heteroaryl radicals may be unsubstituted or possibly substituted in each case with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, C₁₋₅-alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -(CH₂)-benzo[b]furanyl, -O-phenyl, -S-phenyl, -S-benzyl, -O-benzyl, phenyl, furanyl, thienyl (thiophenyl), pyrazolyl, thiadiazolyl and benzyl, wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl, furanyl, thienyl (thiophenyl), pyrazolyl, thiadiazolyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, C₁₋₅-alkyl, -O-CF₃, -S-CF₃, phenyl and -O-benzyl,
and the aforementioned heteroaryl radicals may possibly in each case comprise 1, 2, 3, 4 or 5 heteroatom(s) as ring member(s), which are selected independently of one another from the group consisting of oxygen, nitrogen and sulphur;
the rings of the aforementioned mono- or polycyclic ring systems may be unsubstituted or possibly substituted in each case with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, C₁₋₅-alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -(CH₂)-benzo[b]furanyl, -O-phenyl, -S-phenyl, -S-benzyl, -O-benzyl, phenyl, furanyl, thienyl (thiophenyl), pyrazolyl, thiadiazolyl and benzyl, wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl, furanyl, thienyl (thiophenyl), pyrazolyl, thiadiazolyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, C₁₋₅-alkyl, -O-CF₃, -S-CF₃, phenyl and -O-benzyl,
and the rings of the aforementioned mono- or polycyclic ring systems are in each case 5-, 6- or 7-membered and may in each case possibly comprise 1, 2, 3, 4 or 5 heteroatom(s) as ring member(s), which are selected independently of one another from the group consisting of oxygen, nitrogen and sulphur;
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

12. Compounds according to claim 11, **characterised in that**
X stands for a C(H)(NHR²) group or an NR^{2a} group,
R¹ stands for a -C(=O)-R³ group or a -C(=O)-O-R⁴ group,
R², R^{2a}, independently of one another, respectively stand for a -C(=O)-R⁵ group or an -S(=O)₂-R⁶ group,
R³ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl, -(CH₂)-(CH₂)-(CH)(CH₃)-(CH₂) -(CH₂)-(CH₂)-(CH₃), vinyl, 1-propenyl, 2-propenyl, 1-butenyl, 2-butenyl, 3-butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ and -CH₂-NH-CH₂-CH₃;
a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, C₁₋₅-alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, C₁₋₅-alkyl, -O-CF₃, -S-CF₃, phenyl and -O-benzyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, C₁₋₅-alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅ -alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, C₁₋₅-alkyl, -O-CF₃, -S-CF₃, phenyl and -O-benzyl;
-(CHR¹²)-R¹³, -(CHR¹²)-(CH₂)-R¹³, -(CHR¹²)-O-R¹³, -(CHR¹²)-S-R¹³, -(CHR¹²)-NH-R¹³, -(CHR¹²)-(CH₂)-(CH₂)-R¹³, -(CHR¹²)-(CH₂)-O-R¹³, -(CHR¹²)-(CH₂)-S-R¹³, -(CHR¹²)-(CH₂)-NH-R¹³, -(CHR¹²)-(CH₂)-O-(CH₂)-R¹³, -(CHR¹²)-(CH₂)-S-(CH₂)-R¹³ or -(CHR¹²)-(CH₂)-NH-R¹³;
or -(CHR¹⁴)-R¹⁵, -(CHR¹⁴)-(CH₂)-R¹⁵ or -(CHR¹⁴)-(CH₂)-(CH₂)-R¹⁵;
R⁴ stands for a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, C₁₋₅-alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH -phenyl, -S(=O)₂-C₁₋₅-alkyl, -S(=O)₂-phenyl, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
or -(CHR¹⁶)-R¹⁷, -(CHR¹⁶)-(CH₂)-R¹⁷ or -(CHR¹⁶)-(CH₂)-(CH₂)-R¹⁷;
R⁵ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl, -(CH₂)-(CH₂)-(CH)(CH₃)-(CH₂)-(CH₂)-(CH₂)-(CH₃), vinyl, 1-propenyl, 2-propenyl, 1-butenyl, 2-butenyl, 3-butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ and -CH₂-NH-CH₂-CH₃;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl and 3,4-dihydro-2H-benzo[1,4]oxazinyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, (3,4)-dihydro-2H-benzo[1,4]oxazinyl, (2,3)-dihydro-benzo[1,4]dioxinyl, benzo[1,3]dioxinyl, (2,3)-dihydro-benzofuranyl, 2H-chromenyl, chromanyl and (1,2,3,4)-tetrahydroisoquinolinyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -O-phenyl, -S-phenyl, -S-benzyl, -O-benzyl, phenyl, pyrazolyl, thienyl, furanyl, thiadiazolyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl, pyrazolyl, thienyl, furanyl, thiadiazolyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
-C(=O)-R⁷, -(CHR¹⁸)-C(=O)-R⁷, -(CHR¹⁸)-(CH₂)-C(=O)-R⁷ or -(CHR¹⁸)-(CH₂)-(CH₂)-C(=O)-R⁷; -C(=O)-O-R⁸, -(CHR¹⁹)-C(=O)-O-R⁸, -(CHR¹⁹)-(CH₂)-C(=O)-O-R⁸ or -(CHR¹⁹)-(CH₂)-(CH₂)-C(=O)-O-R⁸;
-(CHR²⁰)-NH-C(=O)-O-R⁹, -(CHR²⁰)-(CH₂)-NH-C(=O)-O-R⁹, -(CHR²⁰)-NH-C(=O)-(CH₂)-NH-C(=O)-O-R⁹, -(CHR²⁰)-(CH₂)-(CH₂)-NH-C(=O)-O-R⁹, -(CHR²⁰)-(CH₂)-(CH₂)-(CH₂)-NH-C(=O)-O-R⁹, -(CHR²⁰)-(CH₂)-(CH₂)-(CH₂)-NH-C(=O)-O-R⁹ or -(CHR²⁰)-(CH₂)-(CH₂)-(CH₂)-(CH₂)-NH-C(=O)-O-R⁹ ;
-(CR²¹R²²)-R²³, -(CR²¹R²²)-(CH₂)-R²³, -(CR²¹R²²)-O-R²³, -(CR²¹R²²)-S-R²³, -(CR²¹R²²)-NH-R²³, -(CR²¹R²²)-(CH₂)-(CH₂)-R²³, -(CR²¹R²²)-O-(CH₂)-R²³, -(CR²¹R²²)-S-(CH₂)-R²³, -(CR²¹R²²)-NH-(CH₂)-R²³, -(CR²¹R²²)-(CH₂)-(CH₂)-O-R²³, -(CR²¹R²²)-(CH₂)-(CH₂)-NH-R²³, -(CR²¹R²²)-(CH₂)-(CH₂)-S-R²³ or -(CR²¹R²²)-(CH₂)-(CH₂)-(CH₂)-R²³;
or -CH=CH-R²⁴;
R⁶ stands for a group -NR¹⁰R¹¹;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl, -(CH₂)-(CH₂)-(CH)(CH₃)-(CH₂)-(CH₂)-(CH₂)-(CH₃), vinyl, 1-propenyl, 2-propenyl, 1-butenyl, 2-butenyl, 3-butenyl, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ and -CH₂-NH-CH₂-CH₃;
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl, 3,4-dihydro -2H-benzo[1,4]oxazinyl and bicyclo[2.2.1]heptyl, wherein the (hetero)-cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, C₁₋₅-alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, (3,4)-dihydro-2H-benzo[1,4]oxazinyl, (2,3)-dihydro-benzo[1,4]dioxinyl, benzo[1,3]dioxinyl, (2,3)-dihydro-benzofuranyl, 2H-chromenyl, chromanyl and (1,2,3,4)-tetrahydroisoquinolinyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
-(CHR²⁵)-R²⁶, -(CHR²⁵)-(CH₂)-R²⁶, -(CHR²⁵)-O-R²⁶, -(CHR²⁵)-S-R²⁶, -(CHR²⁵)-NH-R²⁶, -(CHR²⁵)-(CH₂)-(CH₂)-R²⁶, -(CHR²⁵)-O-(CH₂)-R²⁶, -(CHR²⁵)-S-(CH₂)-R²⁶, -(CHR²⁵)-NH-(CH₂)-R²⁶, -(CHR²⁵)-(CH₂)-(CH₂) -O-R²⁶, -(CHR²⁵)-(CH₂)-(CH₂)-NH-R²⁶ or -(CHR²⁵)-(CH₂)-(CH₂)-S-R²⁶;
or -(CHR²⁷)-R²⁸, -(CHR²⁷)-(CH₂)-R²⁸ or -(CHR²⁷)-(CH₂)-(CH₂)-R²⁸;
R⁷ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl and 3,4-dihydro-2H-benzo[1,4]oxazinyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
or an -NR^{7a}R^{7b} radical, in which R^{7a} and R^{7b}, independently of one another, respectively stand for a radical selected from the group consisting of methyl, ethyl, n-propyl, isopropyl, n-butyl, sec-butyl, isobutyl, tert-butyl and n-pentyl;
R⁸ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
or a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅- perfluoroalkyl, -C(=O)-NH₂, -C(=O)-NH-C₁₋₅-alkyl, -C(=O)-N-(C₁₋₅-alkyl)₂, -S(=O)₂-NH₂ and -S(=O)₂-NH-C₁₋₅-alkyl and/or may be attached via a -(CH₂), -(CH₂)₂ or -(CH₂)₃ group;
R⁹ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl, 3,4-dihydro-2H-benzo[1,4]oxazinyl and 9H-fluorenyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl and/or attached via a -(CH₂), -(CH₂)₂ or -(CH₂)₃ group;
or a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂ and -S(=O)₂-NH-C₁₋₅-alkyl and/or attached via a -(CH₂), -(CH₂)₂ or -(CH₂)₃ group;
R¹⁰ and R¹¹, independently of one another, respectively stand for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
R¹², R¹⁴, R¹⁶, R¹⁸, R²¹, R²², R²⁵ and R²⁷, independently of one another, respectively stand for
a hydrogen radical
or for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
R¹³ stands for a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, C₁₋₅-alkyl, -C(=O)-H, -C(=O)-C₁₋₅alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
R¹⁵ and R¹⁷, independently of one another, respectively stand for
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -(CH₂)-O-C(=O)-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-C₁₋₅-alkyl, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃ and -S-CF₃;
R¹⁹ stands for a hydrogen radical;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
or a phenyl radical;
R²⁰ stands for a hydrogen radical;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl and -(CH₂)-(CH₂)-(CH)(CH₃)-(CH₂)-(CH₂)-(CH₂)-(CH₃);
or a phenyl radical, which may attached via a -(CH₂) or -(CH₂)₂ group;
R²³ stands for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl and 3,4-dihydro-2H-benzo[1,4]oxazinyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, and wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, (3,4)-dihydro-2H-benzo[1,4]oxazinyl, (2,3)-dihydro-benzo[1,4]dioxinyl, benzo[1,3]dioxinyl, (2,3)-dihydro-benzofuranyl, 2H-chromenyl, chromanyl and (1,2,3,4)-tetrahydroisoquinolinyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
R²⁴ and R²⁸, independently of one another, respectively stand for
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, (3,4)-dihydro-2H-benzo[1,4]oxazinyl, (2,3)-dihydro-benzo[1,4]dioxinyl, benzo[1,3]dioxinyl, (2,3)-dihydrobenzofuranyl, 2H-chromenyl, chromanyl and (1,2,3,4)-tetrahydroisoquinolinyl, wherein the radical may in each case possibly be substituted with 1, 2, 3, 4 or 5 substituents selected independently of one another from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, C₁₋₅-alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, -C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -S(=O)₂-phenyl, -O-phenyl, -S-phenyl, -S-benzyl, -O-benzyl, phenyl and benzyl, and wherein in each case the cyclic moiety of the radicals -O-C(=O)-phenyl, -(CH₂)-O-C(=O)-phenyl, -S(=O)₂-NH-phenyl, -S(=O)₂-phenyl, -O-phenyl, -S-phenyl, -S-benzyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl;
and
R²⁶ stands for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (1,2,3,4)-tetrahydronaphthyl, (1,3)-dihydroisoindolyl, (2,3)-dihydroindolyl, 3,4-dihydro-2H-benzo[1,4]oxazinyl and bicyclo[2.2.1]heptyl, wherein the (hetero)cycloaliphatic radical may in each case possibly be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅-alkyl, -N(C₁₋₅-alkyl)₂, -O-phenyl, -O-benzyl, phenyl and benzyl, wherein in each case the cyclic moiety of the radicals -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl.

13. Compounds according to claim 11 or 12, **characterised in that**
X stands for a C(H)(NHR²) group or an NR^{2a} group,
R¹ stands for a -C(=O)-R³ group or a -C(=O)-O-R⁴ group,
R², R^{2a}, independently of one another, respectively stand for a -C(=O)-R⁵ group or an -S(=O)₂-R⁶ group,
R³ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl and n-heptyl;
a (hetero)cycloaliphatic radical selected from the group consisting of cyclopentyl, cyclohexyl, cycloheptyl, tetrahydrofuranyl, pyrrolidinyl, piperidinyl, morpholinyl, piperazinyl, azepanyl and diazepanyl;
a radical selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl and oxadiazolyl, wherein the radical may in each case possibly be substituted with 1, 2, 3, 4 or 5 substituents selected independently of one another from the group consisting of F, Cl, Br, I, -CN, -CF₃, methoxy, ethoxy, n-propoxy, isopropoxy, n-butoxy, isobutoxy, tert-butoxy, methyl, ethyl, n-propyl, iso-propyl, n-butyl, iso-butyl, sec-butyl, tert-butyl and n-pentyl;
-(CHR¹²)-R¹³, -(CHR¹²)-(CH₂)-R¹³ or -(CHR¹²)-(CH₂)-(CH₂)-R¹³;
or -(CHR¹⁴)-R¹⁵, -(CHR¹⁴)-(CH₂)-R¹⁵ or -(CHR¹⁴)-(CH₂)-(CH₂)-R¹⁵;
R⁴ stands for -(CHR¹⁶)-R¹⁷ or -(CHR¹⁶)-(CH₂)-R¹⁷;
R⁵ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl, 3-hexyl, -(CH₂)-(CH₂)-(C(CH₃)₃), n-heptyl, 3-heptyl, 4-heptyl, n-octyl, -(CH₂)-(CH₂)-(CH)(CH₃)-(CH₂)-(CH₂)-(CH₂)-(CH₃), vinyl, 1-propenyl, 2-propenyl, 1-butenyl, 2-butenyl, 3-butenyl, -CH=CH-CH=CH-CH₃, -CH₂-O-CH₃ and -CH₂-O-CH₂-CH₂-O-CH;
a radical selected from the group consisting of cyclopropyl, cyclobutyl, cyclopentyl, cyclohexyl, cycloheptyl, tetrahydrofuranyl, piperidinyl, piperazinyl and (1,2,3,4)-tetrahydronaphthyl, wherein the (hetero)cycloaliphatic radical may in each case possibly be substituted with 1, 2, 3, 4 or 5 substituents selected independently of one another from the group consisting of -C(=O)-O-CH, -C(=O)-O-C₂H₅, -C(=O)-C(CH₃)₃ and phenyl, wherein in each case the cyclic moiety of the phenyl radical may be substituted with 1, 2, 3, 4 or 5 substituents selected independently of one another from the group consisting of F, Cl and Br;
a radical selected from the group consisting of phenyl, naphthyl, thiophenyl, furanyl, pyrrolyl, pyrazolyl, pyrazinyl, triazolyl, pyridinyl, indolyl, benzo[b]thiophenyl, thiazolyl, thiadiazolyl, oxazolyl, isoxazolyl, quinolinyl, isoquinolinyl, benzo[1,3]dioxinyl, (2,3)-dihydrobenzofuranyl, benzo[1,2,5]oxadiazolyl, 2H-chromenyl and chromanyl, wherein the radical may in each case possibly be substituted with 1, 2, 3, 4 or 5 substituents selected independently of one another from the group consisting of F, Cl, Br, -CN, -CF₃, methoxy, ethoxy, n-propoxy, isopropoxy, n-butoxy, isobutoxy, tert-butoxy, -NO₂, -O-CF₃, -O-CHF₂, -O-CH₂F, -S-CF₃, -S-CHF₂, -S-CH₂F, -S-CH₃, -S-C₂H₅, -S-n-C₃H₇, -S-C(CH₃)₃, methyl, ethyl, n-propyl, iso-propyl, n-butyl, iso-butyl, sec-butyl, tert-butyl, n-pentyl, -O-C(=O)-phenyl, -(CH₂)-O-C(=O)-phenyl, -N(CH₃)₂, -N(C₂H₅)₂, -S(=O)₂-phenyl, -O-phenyl, phenyl, furanyl, thienyl, pyrazolyl, thiadiazolyl and benzyl, wherein in each case the cyclic moiety of the radicals -O-C(=O)-phenyl, -(CH₂)-O-C(=O)-phenyl, furanyl, thienyl, pyrazolyl, thiadiazolyl, -S(=O)₂-phenyl, -O-phenyl, phenyl and benzyl may be substituted with 1, 2, 3, 4 or 5 substituents selected independently of one another from the group consisting of F, Cl and Br;
-C(=O)-R⁷, -(CHR¹⁸)-C(=O)-R⁷, -(CHR¹⁸)-(CH₂)-C(=O)-R⁷ or -(CHR¹⁸)-(CH₂)-(CH₂)-C(=O)-R⁷;
-C(=O)-O-R⁸, -(CHR¹⁹)-C(=O)-O-R⁸, -(CHR¹⁹)-(CH₂)-C(=O)-O-R⁸ or -(CHR¹⁹)-(CH₂)-(CH₂)-C(=O)-O-R⁸;
-(CHR²⁰)-NH-C(=O)-O-R⁹, -(CHR²⁰)-(CH₂)-NH-C(=O)-O-R⁹, -(CHR²⁰)-NH-C(=O)-(CH₂)-NH-C(=O)-O-R⁹, -(CHR²⁰)-(CH₂)-(CH₂)-NH-C(=O)-O-R⁹, -(CHR²⁰)-(CH₂)-(CH₂)-(CH₂)-NH-C(=O)-O-R⁹, -(CHR²⁰)-(CH₂)-(CH₂)-(CH₂)-NH-C(=O)-O-R⁹ or -(CHR²⁰)-(CH₂)-(CH₂)-(CH₂)-(CH₂)-NH-C(=O)-O-R⁹;
-(CR²¹R²²)-R²³, -(CR²¹R²²)-(CH₂)-R²³, -(CR²¹R²²)-O-R²³, -(CR²¹R²²)-S-R²³, -(CR²¹R²²)-(CH₂)-(CH₂)-R²³, -(CR²¹R²²)-O-(CH₂)-R²³, -(CR²¹R²²)-S-(CH₂)-R²³, -(CR²¹R²²)-(CH₂)-(CH₂)-O-R²³, -(CR²¹R²²)-(CH₂)-(CH₂)-S-R²³ or -(CR²¹R²²)-(CH₂)-(CH₂)-(CH₂)-R²³;
or -CH=CH-R²⁴;
R⁶ stands for a group -NR¹⁰R¹¹;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
a radical selected from the group consisting of phenyl, naphthyl, thiophenyl, furanyl, pyrazolyl, thiazolyl, (3,4)-dihydro-2H-benzo[1,4]oxazinyl, (2,3)-dihydro-benzo[1,4]dioxinyl and (1,2,3,4)-tetrahydroisoquinolinyl, wherein the radical may in each case possibly be substituted with 1, 2, 3, 4 or 5 substituents selected independently of one another from the group consisting of F, Cl, Br, -CN, -CF₃, methoxy, ethoxy, n-propoxy, isopropoxy, n-butoxy, isobutoxy, tert-butoxy, -NO₂, -O-CF₃, -O-CHF₂, -O-CH₂F, methyl, ethyl, n-propyl, iso-propyl, n-butyl, iso-butyl, sec-butyl, tert-butyl, n-pentyl, -C(CH₃)₂-CH₂-CH₃, -C(=O)-O-CH₃, -C(=O)-O-C₂H₅, -C(=O)-O-C(CH₃)₃, -NH-C(=O)-CH, -NH-C(=O)-C₂H₅, -NH-C(=O)-C(CH₃)₃, -C(=O)-CF₃, -C(=O)-C₂F₅, -S(=O)₂-CH₃, -S(=O)₂-C₂H₅, -S(=O)-C(CH₃)₃, phenyl and benzyl;
-(CHR²⁵)-R²⁶ or -(CHR²⁵)-(CH₂)-R²⁶;
or -(CHR²⁷)-R²⁸ or -(CHR²⁷)-(CH₂)-R²⁸;
R⁷ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
a (2,3)-dihydroindolyl radical;
a radical selected from the group consisting of phenyl, naphthyl and indolyl;
or an -NR^{7a}R^{7b} radical, in which R^{7a} and R^{7b}, independently of one another, respectively stand for a radical selected from the group consisting of methyl, ethyl, n-propyl, isopropyl, n-butyl, sec-butyl, isobutyl, tert-butyl and n-pentyl;
R⁸ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl and n-hexyl;
or a phenyl radical, which may be attached via a -(CH₂) group;
R⁹ stands for a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
a 9H-fluorenyl radical, which may be attached via a -(CH₂) group;
or a phenyl radical, which may be attached via a -(CH₂) group;
R¹⁰ and R¹¹, independently of one another, respectively stand for a radical selected from the group consisting of methyl, ethyl, n-propyl, isopropyl, n-butyl, sec-butyl, isobutyl and tert-butyl;
R¹², R¹⁴, R¹⁶, R¹⁸, R²¹, R²² and R²⁵ and R²⁷, independently of one another, respectively stand for
a hydrogen radical
or a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
R¹³ stands for a (hetero)cycloaliphatic radical selected from the group consisting of cyclopropyl, cyclobutyl, cyclopentyl, cyclohexyl, cycloheptyl, pyrrolidinyl, piperidinyl, morpholinyl and piperazinyl;
R¹⁵ and R¹⁷, independently of one another, respectively stand for a phenyl radical;
R¹⁹ stands for a hydrogen radical;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
or a phenyl radical;
R²⁰ stands for a hydrogen radical;
a radical selected from the group consisting of methyl, ethyl, n-propyl, isopropyl, n-butyl, sec-butyl, isobutyl, tert-butyl, n-pentyl, 2-pentyl, 3-pentyl, neopentyl, n-hexyl, 2-hexyl and 3-hexyl;
or a phenyl radical, which may be attached via a -(CH₂) group;
R²³ stands for a radical selected from the group consisting of cyclopropyl, cyclobutyl, cyclopentyl, cyclohexyl, cycloheptyl, pyrrolidinyl, piperidinyl, morpholinyl, piperazinyl, dithiolanyl, isoindole-1,3-dionyl, benzoxazolin-2-onyl and 2,5-dioxo-imidazolidinyl, wherein the radical may in each case be substituted with 1, 2 or 3 substituents selected independently of one another from the group consisting of methyl, ethyl, n-propyl, iso-propyl, n-butyl, iso-butyl, sec-butyl, tert-butyl and n-pentyl;
a radical selected from the group consisting of phenyl, naphthyl, thiophenyl, furanyl and thiazolyl, wherein the radical may in each case possibly be substituted with 1, 2, 3, 4 or 5 substituents selected independently of one another from the group consisting of F, Cl, Br, -CN, -CF₃, -OH, methoxy, ethoxy, n-propoxy, isopropoxy, n-butoxy, isobutoxy, tert-butoxy, methyl, ethyl, n-propyl, iso-propyl, n-butyl, iso-butyl, sec-butyl, tert-butyl, n-pentyl, -O-C(=O)-phenyl, phenyl and benzyl, wherein in each case the cyclic moiety of the radicals -O-C(=O)-phenyl, phenyl and benzyl may be substituted with 1, 2, 3, 4 or 5 substituents selected independently of one another from the group consisting of F, Cl and Br;
R²⁴ and R²⁸, independently of one another, respectively stand for
a phenyl radical, wherein the radical may in each case be substituted with 1, 2 or 3 substituents selected independently of one another from the group consisting of F, Cl, Br and -CF₃;
and
R²⁶ stands for a 7,7-dimethyl-2-oxo-bicyclo[2.2.1]hept-1-yl radical,
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

14. Compounds according to one or more of claims 1-13 selected from the group consisting of
[1] 3-fluoro-N-[6-(4-fluoro-benzoyl)-5,6,7,8-tetrahydro-pyrido[4,3-d]pyrimidin-2-yl] -benzamide,
[2] 3,5-dichloro-N-[6-(3-fluoro-4-methoxy-benzoyl)-5,6,7,8-tetrahydro-pyrido[4,3-d]pyrimidin-2-yl]-benzamide,
[3] 4-tert-butyl-N-(6-hexanoyl-5,6,7,8-tetrahydro-pyrido[4,3-d]pyrimidin-2-yl)-benzamide,
[4] N-(6-acetyl-5,6,7,8-tetrahydro-pyrido[4,3-d]pyrimidin-2-yl)-3,4-dichloro-benzamide,
[5] 3,5-dichloro-N-[6-(3-trifluoromethyl-benzoyl)-5,6,7,8-tetrahydro-pyrido[4,3-d]pyrimidin-2-yl]-benzamide,
[6] 3-chloro-N-[6-(4-methoxy-2,3,6-trimethyl-benzenesulphonyl)-5,6,7,8-tetrahydro-pyrido[4,3-d]pyrimidin-2-yl]-benzamide,
[7] N-[6-(2-ethoxy-benzoyl)-5,6,7,8-tetrahydro-pyrido[4,3-d]pyrimidin-2-yl]-3-fluoro-benzamide,
[8] 3-chloro-N-[6-(3-phenyl-propionyl)-5,6,7,8-tetrahydro-pyrido[4,3-d]pyrimidin-2-yl]-benzamide,
[9] 4-tert-butyl-N-[6-(isoxazole-5-carbonyl)-5,6,7,8-tetrahydro-pyrido[4,3-d]pyrimidin-2-yl]-benzamide,
[10] N-[6-(2-benzylsulphanyl-acetyl)-5,6,7,8-tetrahydro-pyrido[4,3-d]pyrimidin-2-yl]-2-methoxy-benzamide,
[11] thiophene-2-carboxylic acid {6-[2-(4-chloro-phenyl)-2-methyl-propionyl]-5,6,7,8-tetrahydro-pyrido[4,3-d]pyrimidin-2-yl}-amide,
[12] N-(6-benzoyl-5,6,7,8-tetrahydro-pyrido[4,3-d]pyrimidin-2-yl)-3-methyl-benzamide,
[13] N-[6-(2,3-dihydro-benzofuran-5-carbonyl)-5,6,7,8-tetrahydro-pyrido[4,3-d]pyrimidin-2-yl]-2-fluoro-benzamide,
[14] thiophene-2-carboxylic acid [6-(3,4,5-trimethoxy-benzoylamino)-5,6,7,8-tetrahydro-quinazolin-2-yl]-amide,
[15] naphthalene-1-carboxylic acid [6-(3-methyl-5-phenyl-isoxazole-4-carbonyl)-5,6,7,8-tetrahydro-pyrido[4,3-d]pyrimidin-2-yl]-amide,
[16] 3-chloro-N- {6-[2-(5-methyl-2-phenyl-thiazol-4-yl)-acetyl]-5,6,7,8-tetrahydro-pyrido[4,3-d]pyrimidin-2-yl}-benzamide,
[17] N-[6-(4-chloro-2,5-dimethyl-benzenesulphonyl)-5,6,7,8-tetrahydro-pyrido[4,3-d]pyrimidin-2-yl]-2-trifluoromethyl-benzamide,
[18] N-[6-(1-benzenesulphonyl-1H-indole-2-carbonyl)-5,6,7,8-tetrahydro-pyrido[4,3-d]pyrimidin-2-yl]-3-methoxy-benzamide,
[20] N-[6-(5-methyl-1-phenyl-1H-pyrazole-4-sulphonyl)-5,6,7,8-tetrahydro-pyrido[4,3-d]pyrimidin-2-yl]-2-trifluoromethyl-benzamide,
[21] N-[6-(3-chloro-2-methyl-benzenesulphonyl)-5,6,7,8-tetrahydro-pyrido[4,3-(d)]pyrimidin-2-yl]-3-fluoro-benzamide,
[22] 3,5-dichloro-N-[6-(3,5-dimethyl-isoxazole-4-carbonyl)-5,6,7,8-tetrahydro-pyrido[4,3-d]pyrimidin-2-yl]-benzamide,
[23] thiophene-2-carboxylic acid [6-(4-trifluoromethoxy-benzenesulphonylamino)-5,6,7,8-tetrahydro-quinazolin-2-yl]-amide,
[25] N-[6-(furan-2-carbonyl)-5,6,7,8-tetrahydro-pyrido[4,3-d]pyrimidin-2-yl]-3-methoxy-benzamide,
[26] N-{6-[4-(2,3-dihydro-indol-1-yl)-4-oxo-butyryl]-5,6,7,8-tetrahydro-pyrido[4,3-d]pyrimidin-2-yl} -3-methyl-benzamide,
[27] N-{6-[2-(4-methyl-cyclohexyl)-acetyl]-5,6,7,8-tetrahydro-pyrido[4,3-d]pyrimidin-2-yl} -2-trifluoromethyl-benzamide,
[28] 3,4-difluoro-N-[6-(6-phenoxy-pyridine-3-carbonyl)-5,6,7,8-tetrahydro-pyrido[4,3-d]pyrimidin-2-yl]-benzamide,
[29] 4-tert-butyl-N-[6-(2-phenyl-thiazole-4-carbonyl)-5,6,7,8-tetrahydro-pyrido[4,3-d]pyrimidin-2-yl]-benzamide,
[30] 3,5-dichloro-N-[6-(2-trifluoromethyl-benzenesulphonyl)-5,6,7,8-tetrahydro-pyrido[4,3-d]pyrimidin-2-yl]-benzamide,
[31] thiophene-2-carboxylic acid [6-(3-bromo-benzenesulphonylamino)-5,6,7,8-tetrahydro-quinazolin-2-yl]-amide,
[32] N-[6-(2-chloro-pyridine-4-carbonyl)-5,6,7,8-tetrahydro-pyrido[4,3-d]pyrimidin-2-yl]-3-methoxy-benzamide,
[33] 4-fluoro-N-[6-(2-methanesulphonyl-benzenesulphonylamino)-5,6,7,8-tetrahydro-quinazolin-2-yl]-benzamide,
[35] N-(6-dimethylsulphamoyl-5,6,7,8-tetrahydro-pyrido[4,3-(d)]pyrimidin-2-yl)-3-fluoro-benzamide,
[36] 3-fluoro-N-{6-[2-(4-trifluoromethyl-phenyl)-acetyl]-5,6,7,8-tetrahydro-pyrido[4,3-(d)]pyrimidin-2-yl} -benzamide,
[37] N-{6-[2-(1,3-dioxo-1,3-dihydro-isoindol-2-yl)-acetyl]-5,6,7,8-tetrahydro-pyrido[4,3-(d)]pyrimidin-2-yl}-2-trifluoromethyl-benzamide,
[38] thiophene-2-carboxylic acid {6-[4-(4-chloro-2-methyl-phenoxy)-butyrylamino]-5,6,7,8-tetrahydro-quinazolin-2-yl}-amide,
[39] N-[6-(butane-1-sulphonyl)-5,6,7,8-tetrahydro-pyrido[4,3-d]pyrimidin-2-yl]-3-fluoro-benzamide,
[40] 2-methoxy-N-[6-(2-propyl-pentanoyl)-5,6,7,8-tetrahydro-pyrido[4,3-d]pyrimidin-2-yl]-benzamide,
[41] N-[6-(4,5-dichloro-thiophene-2-sulphonylamino)-5,6,7,8-tetrahydro-quinazolin-2-yl]-4-fluoro-benzamide,
[42] 4-chloro-N-[6-(2-trifluoromethyl-benzenesulphonyl)-5,6,7,8-tetrahydro-pyrido[4,3-d]pyrimidin-2-yl]-benzamide,
[43] thiophene-2-carboxylic acid [6-(4-methoxy-benzenesulphonylamino)-5,6,7,8-tetrahydro-quinazolin-2-yl]-amide,
[44] 5-[2-(3,4-difluoro-benzoylamino)-7,8-dihydro-5H-pyrido[4,3-d]pyrimidin-6-yl]-5-oxo-valeric acid methyl ester,
[45] N-[6-(benzo[b]thiophene-3-carbonyl)-5,6,7,8-tetrahydro-pyrido[4,3-d]pyrimidin-2-yl]-4-chloro-benzamide,
[46] N-[6-(5-bromo-2-methoxy-benzenesulphonylamino)-5,6,7,8-tetrahydro-quinazolin-2-yl]-4-fluoro-benzamide,
[47] 4-fluoro-N-[6-(4-fluoro-benzenesulphonylamino)-5,6,7,8-tetrahydro-quinazolin-2-yl]-benzamide,
[48] N-{6-[2-(1H-indol-3-yl)-2-oxo-acetyl]-5,6,7,8-tetrahydro-pyrido[4,3-d]pyrimidin-2-yl} -2-trifluoromethyl-benzamide,
[49] 5-tert-butyl-2-methyl-furan-3-carboxylic acid {2-[(thiophene-2-carbonyl)-amino]-5,6,7,8-tetrahydro-quinazolin-6-yl}-amide,
[50] N-[6-(2,5-dimethoxy-benzenesulphonyl)-5,6,7,8-tetrahydro-pyrido[4,3-d]pyrimidin-2-yl]-3-methoxy-benzamide,
[51] benzoic acid 2-{2-[(thiophene-2-carbonyl)-amino]-5,6,7,8-tetrahydro-quinazolin-6-ylcarbamoyl}-benzyl ester,
[52] 3,4-difluoro-N-(6-hexanoyl-5,6,7,8-tetrahydro-pyrido[4,3-d]pyrimidin-2-yl)-benzamide,
[53] 4-ethyl-N-[6-(tetrahydro-furan-2-carbonyl)-5,6,7,8-tetrahydro-pyrido[4,3-d]pyrimidin-2-yl]-benzamide,
[54] N-[6-(2-chloro-benzenesulphonyl)-5,6,7,8-tetrahydro-pyrido[4,3-d]pyrimidin-2-yl]-3-fluoro-benzamide,
[55] 4-tert-butyl-N-[6-(5-tert-butyl-2-methyl-2H-pyrazole-3-carbonyl)-5,6,7,8-tetrahydro-pyrido[4,3-d]pyrimidin-2-yl]-benzamide,
[56] 3-methoxy-N-[6-(4-methoxy-benzenesulphonyl)-5,6,7,8-tetrahydro-pyrido[4,3-d]pyrimidin-2-yl]-benzamide,
[57] {2-[2-(3,4-difluoro-benzoylamino)-7,8-dihydro-5H-pyrido[4,3-d]pyrimidin-6-yl]-2-oxo-1-phenyl-ethyl}-carbamic acid benzyl ester,
[58] 5-phenyl-oxazole-4-carboxylic acid {2-[(thiophene-2-carbonyl)-amino]-5,6,7,8-tetrahydro-quinazolin-6-yl}-amide,
[59] 4-chloro-N-(6-dimethylsulphamoyl-5,6,7,8-tetrahydro-pyrido[4,3-d]pyrimidin-2-yl)-benzamide,
[60] 4-tert-butyl-N-[6-(2-fluoro-benzenesulphonyl)-5,6,7,8-tetrahydro-pyrido[4,3-d]pyrimidin-2-yl]-benzamide,
[61] 4-chloro-N-[6-(3-cyclopentyl-propionyl)-5,6,7,8-tetrahydro-pyrido[4,3-d]pyrimidin-2-yl]-benzamide,
[62] 4-chloro-N-[6-(4-phenyl-butyryl)-5,6,7,8-tetrahydro-pyrido[4,3-d]pyrimidin-2-yl]-benzamide,
[63] 4-tert-butyl-N-[6-(5-methyl-2-phenyl-2H-[1,2,3]triazole-4-carbonyl)-5,6,7,8-tetrahydro-pyrido[4,3-d]pyrimidin-2-yl]-benzamide,
[64] 2-chloro-N-{2-[(thiophene-2-carbonyl)-amino]-5,6,7,8-tetrahydro-quinazolin-6-yl}-isonicotinamide,
[66] N-[6-(5-chloro-thiophene-2-sulphonyl)-5,6,7,8-tetrahydro-pyrido[4,3-d]pyrimidin-2-yl]-3-fluoro-benzamide,
[67] N-[6-(4-diethylamino-benzoyl)-5,6,7,8-tetrahydro-pyrido[4,3-d]pyrimidin-2-yl]-3-methoxy-benzamide,
[68] N-[6-(2,4-dimethyl-thiazole-5-sulphonyl)-5,6,7,8-tetrahydro-pyrido[4,3-d]pyrimidin-2-yl]-3-methyl-benzamide,
[69] 4-chloro-N-[6-naphthalene-2-carbonyl)-5,6,7,8-tetrahydro-pyrido[4,3-d]pyrimidin-2-yl]-benzamide,
[70] N-[6-(2,4-difluoro-benzenesulphonyl)-5,6,7,8-tetrahydro-pyrido[4,3-d]pyrimidin-2-yl]-2-methoxy-benzamide,
[71] 3,4-dichloro-N-[6-(4-chloro-benzenesulphonyl)-5,6,7,8-tetrahydro-pyrido[4,3-d]pyrimidin-2-yl]-benzamide,
[72] 3,4-difluoro-N-[6-(thiophene-2-sulphonyl)-5,6,7,8-tetrahydro-pyrido[4,3-d]pyrimidin-2-yl]-benzamide,
[74] 4-ethyl-N-[6-(4-pyrazol-1-yl-benzoyl)-5,6,7,8-tetrahydro-pyrido[4,3-d]pyrimidin-2-yl]-benzamide,
[75] N-[6-(7,7-dimethyl-2-oxo-bicyclo[2.2.1]hept-1-ylmethanesulphonylamino)-5,6,7,8-tetrahydro-quinazolin-2-yl]-butyramide,
[76] 5-oxo-5-phenyl-valeric acid (2-butyrylamino-5,6,7,8-tetrahydro-quinazolin-6-yl)-amide,
[77] 3-[2-(4-chloro-benzoylamino)-7,8-dihydro-5H-pyrido[4,3-d]pyrimidin-6-yl]-3-oxo-propionic acid methyl ester,
[78] N-{6-[5-(4-chloro-phenyl)-2-methyl-furan-3-carbonyl]-5,6,7,8-tetrahydro-pyrido [4,3-d]pyrimidin-2-yl}-2-methoxy-benzamide,
[79] 4-chloro-N-[6-(2,4-dimethyl-thiazole-5-sulphonyl)-5,6,7,8-tetrahydro-pyrido[4,3-d]pyrimidin-2-yl]-benzamide,
[80] N-[6-(2,3-difluoro-benzoyl)-5,6,7,8-tetrahydro-pyrido[4,3-d]pyrimidin-2-yl]-3-methoxy-benzamide,
[81] 4-chloro-N-{6-[2-(2-methoxy-ethoxy)-acetyl]-5,6,7,8-tetrahydro-pyrido [4,3-d]pyrimidin-2-yl} -benzamide,
[82] 2-[2-(2-ethoxy-benzoylamino)-7,8-dihydro-5H-pyrido[4,3-d]pyrimidine-6-sulphonyl]-benzoic acid methyl ester,
[83] 4-tert-butyl-N-[6-(4-nitro-benzenesulphonyl)-5,6,7,8-tetrahydro-pyrido[4,3-d]pyrimidin-2-yl]-benzamide,
[86] N-[6-(5-chloro-thiophene-2-sulphonyl)-5,6,7,8-tetrahydro-pyrido[4,3-d]pyrimidin-2-yl]-2-methoxy-benzamide,
[87] 4-fluoro-N-[6-(furan-2-carbonyl)-5,6,7,8-tetrahydro-pyrido[4,3-d]pyrimidin-2-yl]-benzamide,
[88] 4- {2-[(thiophene-2-carbonyl)-amino]-5,6,7,8-tetrahydro-quinazolin-6-ylcarbamoyl}-piperidine-1-carboxylic acid tert-butyl ester,
[89] 1-(4-chloro-phenyl)-cyclopropanecarboxylic acid (2-butyrylamino-5,6,7,8-tetrahydro-quinazolin-6-yl)-amide,
[90] N-(6-ethanesulphonyl-5,6,7,8-tetrahydro-pyrido[4,3-d]pyrimidin-2-yl)-3-methoxy-benzamide,
[91] 5-methyl-thiophene-2-carboxylic acid (2-butyrylamino-5,6,7,8-tetrahydro-quinazolin-6-yl)-amide,
[92] 4-tert-butyl-N-[6-(4-methyl-3-nitro-benzoyl)-5,6,7,8-tetrahydro-pyrido[4,3-d]pyrimidin-2-yl]-benzamide,
[94] 4-fluoro-N-[6-(3-phenyl-propionylamino)-5,6,7,8-tetrahydro-quinazolin-2-yl]-benzamide,
[95] 3,4-dichloro-N-[6-(2,4,6-trimethyl-benzenesulphonyl)-5,6,7,8-tetrahydro-pyrido[4,3-d]pyrimidin-2-yl]-benzamide,
[96] N-[6-(4-methyl-3,4-dihydro-2H-benzo[1,4]oxazine-7-sulphonyl)-5,6,7,8-tetrahydro-pyrido[4,3-d]pyrimidin-2-yl]-nicotinamide,
[97] thiophene-2-carboxylic acid [6-(3,5-difluoro-benzoylamino)-5,6,7,8-tetrahydro-quinazolin-2-yl]-amide,
[98] N-[6-(2,4-difluoro-benzenesulphonylamino)-5,6,7,8-tetrahydro-quinazolin-2-yl]-butyramide,
[99] thiophene-2-carboxylic acid [6-(2,3,5,6-tetramethyl-benzenesulphonyl)-5,6,7,8-tetrahydro-pyrido[4,3-d]pyrimidin-2-yl]-amide,
[101] N-[6-(3,4-dichloro-benzoyl)-5,6,7,8-tetrahydro-pyrido[4,3-d]pyrimidin-2-yl]-4-ethyl-benzamide,
[102] N-[6-(7,7-dimethyl-2-oxo-bicyclo[2.2.1]hept-1-ylmethanesulphonyl)-5,6,7,8-tetrahydro-pyrido[4,3-d]pyrimidin-2-yl]-3-fluoro-benzamide,
[103] N-[6-(6-fluoro-4H-benzo[1,3]dioxin-8-carbonyl)-5,6,7,8-tetrahydro-pyrido[4,3-d]pyrimidin-2-yl]-2-trifluoromethyl-benzamide,
[104] [5-(2-butyrylamino-5,6,7,8-tetrahydro-quinazolin-6-ylcarbamoyl)-pentyl]-carbamic acid benzyl ester,
[105] 2-ethoxy-N-[6-(4-oxo-pentanoyl)-5,6,7,8-tetrahydro-pyrido[4,3-d]pyrimidin-2-yl]-benzamide,
[106] naphthalene-1-carboxylic acid [6-(propane-1-sulphonyl)-5,6,7,8-tetrahydro-pyrido[4,3-d]pyrimidin-2-yl]-amide,
[107] N-[6-(5-fluoro-2-methyl-benzenesulphonyl)-5,6,7,8-tetrahydro-pyrido[4,3-d]pyrimidin-2-yl]-4-methyl-benzamide,
[109] 3-chloro-N-[6-(2-methyl-benzoyl)-5,6,7,8-tetrahydro-pyrido[4,3-d]pyrimidin-2-yl]-benzamide,
[110] thiophene-2-carboxylic acid [6-(3-chloro-benzo[b]thiophene-2-carbonyl)-5,6,7,8-tetrahydro-pyrido[4,3-d]pyrimidin-2-yl]-amide,
[112] thiophene-2-carboxylic acid [6-(2-cyclopentyl-acetylamino)-5,6,7,8-tetrahydro-quinazolin-2-yl]-amide,
[113] N-[6-(3,4-dichloro-benzoyl)-5,6,7,8-tetrahydro-pyrido[4,3-d]pyrimidin-2-yl]-2-fluoro-benzamide,
[114] N-[6-(2-chloro-benzenesulphonyl)-5,6,7,8-tetrahydro-pyrido[4,3-d]pyrimidin-2-yl]-4-ethyl-benzamide,
[115] thiophene-2-carboxylic acid [6-(4-bromo-3-methyl-benzoylamino)-5,6,7,8-tetrahydro-quinazolin-2-yl]-amide,
[116] 3-methyl-N-[6-(toluene-3-sulphonyl)-5,6,7,8-tetrahydro-pyrido[4,3-d]pyrimidin-2-yl]-benzamide,
[117] naphthalene-1-carboxylic acid [6-(5-chloro-thiophene-2-sulphonyl)-5,6,7,8-tetrahydro-pyrido[4,3-d]pyrimidin-2-yl]-amide,
[118] N-(2-butyrylamino-5,6,7,8-tetrahydro-quinazolin-6-yl)-2,6-difluoro-benzamide,
[119] 3-chloro-N-[6-(3,4-dimethoxy-benzoyl)-5,6,7,8-tetrahydro-pyrido[4,3-d]pyrimidin-2-yl]-benzamide,
[120] N-[6-(3-chloro-benzo[b]thiophene-2-carbonyl)-5,6,7,8-tetrahydro-pyrido[4,3-d]pyrimidin-2-yl]-benzamide,
[121] 4-ethyl-N-[6-(thiophene-3-sulphonyl)-5,6,7,8-tetrahydro-pyrido[4,3-d]pyrimidin-2-yl]-benzamide,
[123] 2-ethoxy-N-[6-(2-ethylsulphanyl-pyridine-3-carbonyl)-5,6,7,8-tetrahydro-pyrido[4,3-d]pyrimidin-2-yl]-benzamide,
[124] 3-[2-(3-chloro-benzoylamino)-7,8-dihydro-5H-pyrido[4,3-d]pyrimidin-6-yl]-3-oxo-propionic acid ethyl ester,
[125] N-[6-(3-bromo-benzenesulphonyl)-5,6,7,8-tetrahydro-pyrido[4,3-d]pyrimidin-2-yl]-3-chloro-benzamide,
[126] 3,4-dichloro-N-{6-[2-(4-chloro-phenyl)-acetyl]-5,6,7,8-tetrahydro-pyrido[4,3-d]pyrimidin-2-yl}-benzamide,
[127] 3-chloro-N-[6-(2-chloro-6-fluoro-benzoyl)-5,6,7,8-tetrahydro-pyrido[4,3-d]pyrimidin-2-yl]-benzamide,
[128] 4-fluoro-N-(6-hexanoylamino-5,6,7,8-tetrahydro-quinazolin-2-yl)-benzamide,
[129] N-[6-(5-bromo-2-methoxy-benzenesulphonyl)-5,6,7,8-tetrahydro-pyrido[4,3-d]pyrimidin-2-yl]-3-methoxy-benzamide,
[132] N-[6-(3-cyclopentyl-propionyl)-5,6,7,8-tetrahydro-pyrido[4,3-d]pyrimidin-2-yl]-isonicotinamide,
[133] 5-benzyl-furan-2-carboxylic acid (2-butyrylamino-5,6,7,8-tetrahydro-quinazolin-6-yl)-amide
[134] 3,4-dichloro-N-[6-(4-trifluoromethoxy-benzenesulphonyl)-5,6,7,8-tetrahydro-pyrido[4,3-d]pyrimidin-2-yl]-benzamide,
[135] [((2-butyrylamino-5,6,7,8-tetrahydro-quinazolin-6-ylcarbamoyl)-phenyl-methyl]-carbamic acid benzyl ester,
[136] N-[6-(3,4-dimethoxy-benzenesulphonyl)-5,6,7,8-tetrahydro-pyrido[4,3-d]pyrimidin-2-yl]-4-fluoro-benzamide,
[137] 2-fluoro-N-[6-(2-methyl-5-phenyl-furan-3-carbonyl)-5,6,7,8-tetrahydro-pyrido[4,3-d]pyrimidin-2-yl]-benzamide,
[138] thiophene-2-carboxylic acid (6-pent-4-enoylamino-5,6,7,8-tetrahydro-quinazolin-2-yl)-amide,
[139] ((2-methyl-1-{2-[(thiophene-2-carbonyl)-amino]-5,6,7,8-tetrahydro-quinazolin-6-ylcarbamoyl}-propyl)-carbamic acid 9H-fluoren-9-yl methyl ester,
[140] ((5-{2-[(thiophene-2-carbonyl)-amino]-5,6,7,8-tetrahydro-quinazolin-6-ylcarbamoyl}-pentyl)-carbamic acid tert-butyl ester,
[141] 3-fluoro-N-[6-(1,2,3,4-tetrahydro-naphthalene-2-carbonyl)-5,6,7,8-tetrahydro-pyrido[4,3-d]pyrimidin-2-yl]-benzamide,
[142] 4-chloro-N-{6-[2-(2,5-dioxo-imidazolidin-4-yl)-acetyl]-5,6,7,8-tetrahydro-pyrido[4,3-d]pyrimidin-2-yl}-benzamide,
[143] 3-fluoro-N-[6-(toluene-4-sulphonyl)-5,6,7,8-tetrahydro-pyrido[4,3-d]pyrimidin-2-yl]-benzamide,
[144] 3,5-dichloro-N-[6-(4-thiophen-2-yl-benzoyl)-5,6,7,8-tetrahydro-pyrido[4,3-d]pyrimidin-2-yl]-benzamide,
[145] N-[6-(3-chloro-thiophene-2-carbonyl)-5,6,7,8-tetrahydro-pyrido[4,3-d]pyrimidin-2-yl]-2-ethoxy-benzamide,
[146] N-[6-(toluene-3-sulphonylamino)-5,6,7,8-tetrahydro-quinazolin-2-yl]-butyramide,
[147] thiophene-2-carboxylic acid {6-[2-(2,2,2-trifluoro-acetyl)-1,2,3,4-tetrahydro-isoquinoline-7-sulphonylamino]-5,6,7,8-tetrahydro-quinazolin-2-yl}-amide,
[148] 4-methyl-N-[6-(4-trifluoromethylsulphanyl-benzoyl)-5,6,7,8-tetrahydro-pyrido[4,3-d]pyrimidin-2-yl]-benzamide,
[149] ((2-{2-[(thiophene-2-carbonyl)-amino]-5,6,7,8-tetrahydro-quinazolin-6-ylcarbamoyl}-ethyl)-carbamic acid tert-butyl ester,
[150] 2-fluoro-N-[6-(2-methyl-5-nitro-benzenesulphonyl)-5,6,7,8-tetrahydro-pyrido[4,3-d]pyrimidin-2-yl]-benzamide,
[151] N-[6-(4-methoxy-benzenesulphonylamino)-5,6,7,8-tetrahydro-quinazolin-2-yl]-butyramide,
[152] 4-chloro-N-[6-(2,4-difluoro-benzoyl)-5,6,7,8-tetrahydro-pyrido[4,3-d]pyrimidin-2-yl]-benzamide
[153] 4-methyl-N-(6-pent-4-enoyl-5,6,7,8-tetrahydro-pyrido[4,3-d]pyrimidin-2-yl)-benzamide,
[154] naphthalene-1-carboxylic acid [6-(3-fluoro-4-methyl-benzoyl)-5,6,7,8-tetrahydro-pyrido[4,3-d]pyrimidin-2-yl]-amide,
[155] N-{6-[2-(4-trifluoromethyl-phenyl)-acetyl]-5,6,7,8-tetrahydro-pyrido[4,3-d]pyrimidin-2-yl}-benzamide,
[156] N-[6-(3,4-dichloro-benzoyl)-5,6,7,8-tetrahydro-pyrido[4,3-d]pyrimidin-2-yl]-nicotinamide,
[157] 4-ethyl-N-{6-[2-(2,2,2-trifluoro-acetyl)-1,2,3,4-tetrahydro-isoquinoline-7-sulphonyl]-5,6,7,8-tetrahydro-pyrido[4,3-d]pyrimidin-2-yl}-benzamide,
[158] 2-methoxy-N-[6-(4-methoxy-2,3,6-trimethyl-benzenesulphonyl)-5,6,7,8-tetrahydro-pyrido[4,3-d]pyrimidin-2-yl]-benzamide,
[159] 4-ethyl-N-[6-(2-methoxy-acetyl)-5,6,7,8-tetrahydro-pyrido[4,3-d]pyrimidin-2-yl]-benzamide,
[160] 4-fluoro-N-[6-(propane-1-sulphonylamino)-5,6,7,8-tetrahydro-quinazolin-2-yl]-benzamide,
[162] N-[6-(7,7-dimethyl-2-oxo-bicyclo[2.2.1]hept-1-ylmethanesulphonylammo)-5,6,7,8-tetrahydro-quinazolin-2-yl]-butyramide,
[163] N-{6-[2-(2,5-dimethyl-phenyl)-acetylamino]-5,6,7,8-tetrahydro-quinazolin-2-yl}-4-fluoro-benzamide,
[165] thiophene-2-carboxylic acid (6-phenylmethanesulphonylamino-5,6,7,8-tetrahydro-quinazolin-2-yl)-amide,
[166] N-[6-(3,4-dimethoxy-benzenesulphonylamino)-5,6,7,8-tetrahydro-quinazolin-2-yl]-butyramide,
[167] N-{6-[4-(1,1-dimethyl-propyl)-benzenesulphonylamino]-5,6,7,8-tetrahydro-quinazolin-2-yl}-butyramide,
[168] N-(2-butyrylamino-5,6,7,8-tetrahydro-quinazolin-6-yl)-3-(3-trifluoromethyl-phenyl)-acrylamide,
[169] N-[6-(butane-1-sulphonylamino)-5,6,7,8-tetrahydro-quinazolin-2-yl]-4-fluoro-benzamide,
[170] naphthalene-1-carboxylic acid (6-acetyl-5,6,7,8-tetrahydro-pyrido[4,3-d]pyrimidin-2-yl)-amide,
[171] 3,5-dichloro-N-[6-(3-diethylcarbamoyl-propionyl)-5,6,7,8-tetrahydro-pyrido[4,3-d]pyrimidin-2-yl]-benzamide,
[172] 4-ethyl-N-[6-(4-methoxy-benzenesulphonyl)-5,6,7,8-tetrahydro-pyrido[4,3-d]pyrimidin-2-yl]-benzamide,
[173] 4-ethyl-N-[6-(quinoline-6-carbonyl)-5,6,7,8-tetrahydro-pyrido[4,3-d]pyrimidin-2-yl]-benzamide,
[174] N-[6-(2-cyclopropyl-acetyl)-5,6,7,8-tetrahydro-pyrido[4,3-d]pyrimidin-2-yl]-2-fluoro-benzamide,
[175] N-[6-(2H-chromene-3-carbonyl)-5,6,7,8-tetrahydro-pyrido[4,3-d]pyrimidin-2-yl]-3,4-difluoro-benzamide,
[176] N-{6-[2-(4-chloro-phenyl)-propionyl]-5,6,7,8-tetrahydro-pyrido[4,3-d]pyrimidin-2-yl}-benzamide,
[177] thiophene-2-carboxylic acid [6-(2-naphthalen-1-yl-acetylamino)-5,6,7,8-tetrahydro-quinazolin-2-yl]-amide,
[178] 4-fluoro-N-(6-phenylmethanesulphonyl-5,6,7,8-tetrahydro-pyrido[4,3-d]pyrimidin-2-yl)-benzamide,
[180] thiophene-2-carboxylic acid [6-(2,5-dichloro-thiophene-3-sulphonylamino)-5,6,7,8-tetrahydro-quinazolin-2-yl]-amide,
[181] {5-[2-(4-fluoro-benzoylamino)-5,6,7,8-tetrahydro-quinazolin-6-ylcarbamoyl]-pentyl}-carbamic acid benzyl ester,
[182] N-[6-(2,5-dimethyl-2H-pyrazole-3-carbonyl)-5,6,7,8-tetrahydro-pyrido[4,3-d]pyrimidin-2-yl]-4-ethyl-benzamide,
[183] naphthalene-1-carboxylic acid [6-(3-fluoro-benzoyl)-5,6,7,8-tetrahydro-pyrido[4,3-d]pyrimidin-2-yl]-amide,
[184] N-[6-(benzo[b]thiophene-3-carbonyl)-5,6,7,8-tetrahydro-pyrido[4,3-d]pyrimidin-2-yl]-4-tert-butyl-benzamide,
[185] 4-fluoro-N-[6-(4-methoxy-benzenesulphonyl)-5,6,7,8-tetrahydro-pyrido[4,3-d]pyrimidin-2-yl]-benzamide,
[186] 4-tert-butyl-N-[6-(2,3-dihydro-benzo[1,4]dioxin-6-sulphonyl)-5,6,7,8-tetrahydro-pyrido[4,3-d]pyrimidin-2-yl]-benzamide,
[187] 3-chloro-N-{6-[2-(2,6-dichloro-phenyl)-acetyl]-5,6,7,8-tetrahydro-pyrido[4,3-d]pyrimidin-2-yl}-benzamide,
[188] thiophene-2-carboxylic acid [6-(2,3-dihydro-benzo[1,4]dioxin-6-sulphonylamino)-5,6,7,8-tetrahydro-quinazolin-2-yl]-amide,
[189] N-{6-[2-(3-chloro-phenoxy)-acetyl]-5,6,7,8-tetrahydro-pyrido[4,3-d]pyrimidin-2-yl}-4-methyl-benzamide,
[190] N-[6-(5-phenyl-pentanoyl)-5,6,7,8-tetrahydro-pyrido[4,3-d]pyrimidin-2-yl]-benzamide,
[191] 4-ethyl-N-[6-(2-ethyl-butyryl)-5,6,7,8-tetrahydro-pyrido[4,3-d]pyrimidin-2-yl]-benzamide,
[192] 3,5-dichloro-N-[6-(4-trifluoromethoxy-benzoyl)-5,6,7,8-tetrahydro-pyrido[4,3-d]pyrimidin-2-yl]-benzamide,
[193] naphthalene-1-carboxylic acid [6-(4-methyl-octanoyl)-5,6,7,8-tetrahydro-pyrido[4,3-d]pyrimidin-2-yl]-amide,
[194] 4-[2-(4-fluoro-benzoylamino)-5,6,7,8-tetrahydro-quinazolin-6-ylcarbamoyl]-piperidine-1-carboxylic acid tert-butyl ester,
[195] N-[6-(2-benzyloxy-acetyl)-5,6,7,8-tetrahydro-pyrido[4,3-d]pyrimidin-2-yl]-2-methoxy-benzamide,
[196] N-[6-(2-trifluoromethyl-benzenesulphonylamino)-5,6,7,8-tetrahydro-quinazolin-2-yl]-butyramide,
[197] N-[2-(4-fluoro-benzoylamino)-5,6,7,8-tetrahydro-quinazolin-6-yl]-2-methyl-6-trifluoromethyl-nicotinamide,
[198] N-[6-(3-cyano-benzoyl)-5,6,7,8-tetrahydro-pyrido[4,3-d]pyrimidin-2-yl]-2-trifluoromethyl-benzamide,
[199] 3-methoxy-N-[6-(3-trifluoromethoxy-benzoyl)-5,6,7,8-tetrahydro-pyrido[4,3-d]pyrimidin-2-yl]-benzamide,
[200] N-(6-butyryl-5,6,7,8-tetrahydro-pyrido[4,3-d]pyrimidin-2-yl)-2-fluoro-benzamide,
[201] N-(6-benzenesulphonylamino-5,6,7,8-tetrahydro-quinazolin-2-yl)-4-fluoro-benzamide,
[202] N-[6-(5-chloro-thiophene-2-sulphonylamino)-5,6,7,8-tetrahydro-quinazolin-2-yl]-4-fluoro-benzamide,
[203] naphthalene-1-carboxylic acid (6-hexanoyl-5,6,7,8-tetrahydro-pyrido[4,3-d]pyrimidin-2-yl)-amide,
[204] N-(6-propionyl-5,6,7,8-tetrahydro-pyrido[4,3-d]pyrimidin-2-yl)-2-trifluoromethyl-benzamide,
[205] N-[6-(2-ethyl-butyryl)-5,6,7,8-tetrahydro-pyrido[4,3-d]pyrimidin-2-yl]-4-methyl-benzamide,
[206] 2-fluoro-N-[6-(3-trifluoromethyl-benzenesulphonyl)-5,6,7,8-tetrahydro-pyrido[4,3-d]pyrimidin-2-yl]-benzamide,
[207] 5-benzyl-furan-2-carboxylic acid {2-[(thiophene-2-carbonyl)-amino]-5,6,7,8-tetrahydro-quinazolin-6-yl}-amide,
[208] thiophene-2-carboxylic acid {6-[2-(2,5-dimethyl-phenyl)-acetylamino]-5,6,7,8-tetrahydro-quinazolin-2-yl}-amide,
[210] 4-fluoro-N-[6-(propane-1-sulphonyl)-5,6,7,8-tetrahydro-pyrido[4,3-d]pyrimidin-2-yl]-benzamide,
[211] 2-[2-(3-fluoro-benzoylamino)-7,8-dihydro-5H-pyrido[4,3-d]pyrimidine-6-sulphonyl]-benzoic acid methyl ester,
[212] 3,5-dichloro-N-[6-(5-[1,2]dithiolan-3-yl-pentanoyl)-5,6,7,8-tetrahydro-pyrido[4,3-d]pyrimidin-2-yl]-benzamide,
[213] thiophene-2-carboxylic acid [6-(2-phenoxy-butyrylamino)-5,6,7,8-tetrahydro-quinazolin-2-yl]-amide,
[214] thiophene-2-carboxylic acid {6-[2-(4-methoxyphenyl)-acetylamino]-5,6,7,8-tetrahydro-quinazolin-2-yl}-amide,
[215] N-(6-cyclohexanecarboxylic-5,6,7,8-tetrahydro-pyrido[4,3-d]pyrimidin-2-yl)-2-trifluoromethyl-benzamide,
[216] N-[6-(2,4-dimethyl-thiazole-5-sulphonyl)-5,6,7,8-tetrahydro-pyrido[4,3-d]pyrimidin-2-yl]-2-trifluoromethyl-benzamide,
[217] 2-thiophen-2-yl-thiazole-4-carboxylic acid {2-[(thiophene-2-carbonyl)-amino]-5,6,7,8-tetrahydro-quinazolin-6-yl}-amide,
[219] thiophene-2-carboxylic acid {6-[3-(3-trifluoromethyl-phenyl)-acryloylamino]-5,6,7,8-tetrahydro-quinazolin-2-yl}-amide,
[220] thiophene-2-carboxylic acid [6-(5-phenyl-pentanoylamino)-5,6,7,8-tetrahydro-quinazolin-2-yl]-amide,
[221] thiophene-2-carboxylic acid [6-(2-chloro-5-trifluoromethyl-benzoylamino)-5,6,7,8-tetrahydro-quinazolin-2-yl]-amide,
[222] 4-fluoro-N-[6-(2,3,5,6-tetramethyl-benzenesulphonylamino)-5,6,7,8-tetrahydro-quinazolin-2-yl]-benzamide,
[223] 4-fluoro-N-[6-(4-methyl-octanoyl)-5,6,7,8-tetrahydro-pyrido[4,3-d]pyrimidin-2-yl]-benzamide,
[224] 2-{2-[(thiophene-2-carbonyl)-amino]-5,6,7,8-tetrahydro-quinazolin-6-ylsulphamoyl}-benzoic acid methyl ester,
[225] [((3-methyl-1-{2-[(thiophene-2-carbonyl)-amino]-5,6,7,8-tetrahydro-quinazolin-6-ylcarbamoyl}-butylcarbamoyl)-methyl]-carbamic acid benzyl ester,
[226] 3-chloro-N-(6-pentanoyl-5,6,7,8-tetrahydro-pyrido[4,3-d]pyrimidin-2-yl)-benzamide,
[227] 4-fluoro-N-[6-(5-oxo-5-phenyl-pentanoylamino)-5,6,7,8-tetrahydro-quinazolin-2-yl]-benzamide,
[228] thiophene-2-carboxylic acid [6-(3-phenyl-acryloylamino)-5,6,7,8-tetrahydro-quinazolin-2-yl]-amide,
[229] N-[6-(5-[1,2]dithiolan-3-yl-pentanoylamino)-5,6,7,8-tetrahydro-quinazolin-2-yl]-4-fluoro-benzamide,
[230] benzoic acid 2-(2-{2-[(thiophene-2-carbonyl)-amino]-5,6,7,8-tetrahydro-quinazolin-6-ylcarbamoyl}-ethyl)-phenyl ester,
[231] 4-fluoro-N-[6-(2-methyl-5-nitro-benzenesulphonylamino)-5,6,7,8-tetrahydro-quinazolin-2-yl]-benzamide,
[232] 4-chloro-N-[6-(2-phenoxy-acetyl)-5,6,7,8-tetrahydro-pyrido[4,3-d]pyrimidin-2-yl]-benzamide,
[234] N-[6-(5-bromo-thiophene-2-carbonyl)-5,6,7,8-tetrahydro-pyrido[4,3-d]pyrimidin-2-yl]-2-fluoro-benzamide,
[235] 4-fluoro-N-[6-(5-fluoro-2-methyl-benzenesulphonylamino)-5,6,7,8-tetrahydro-quinazolin-2-yl]-benzamide,
[236] N-[6-(4-acetylamino-benzenesulphonylamino)-5,6,7,8-tetrahydro-quinazolin-2-yl]-4-fluoro-benzamide,
[237] thiophene-2-carboxylic acid [6-(2-trifluoromethyl-benzoylamino)-5,6,7,8-tetrahydro-quinazolin-2-yl]-amide,
[238] 3,4-difluoro-N-[6-(quinoline-6-carbonyl)-5,6,7,8-tetrahydro-pyrido[4,3-d]pyrimidin-2-yl]-benzamide,
[239] {1-[2-(4-fluoro-benzoylamino)-5,6,7,8-tetrahydro-quinazolin-6-ylcarbamoyl]-2-phenyl-ethyl}-carbamic acid tert-butyl ester,
[240] thiophene-2-carboxylic acid [6-(2-methyl-benzoylamino)-5,6,7,8-tetrahydro-quinazolin-2-yl]-amide,
[241] 3-chloro-N-{6-[3-(2-oxo-benzooxazol-3-yl)-propionyl]-5,6,7,8-tetrahydro-pyrido [4,3-d]pyrimidin-2-yl} -benzamide,
[242] 3-chloro-N-[6-(5-chloro-thiophene-2-sulphonyl)-5,6,7,8-tetrahydro-pyrido[4,3-d]pyrimidin-2-yl]-benzamide,
[243] 3,5-dichloro-N-[6-(4-methoxy-benzenesulphonyl)-5,6,7,8-tetrahydro-pyrido[4,3-d]pyrimidin-2-yl]-benzamide,
[244] 4-tert-butyl-N-[6-(5-methyl-isoxazole-3-carbonyl)-5,6,7,8-tetrahydro-pyrido[4,3-d]pyrimidin-2-yl]-benzamide,
[245] N-[6-(5-bromo-2-methoxy-benzenesulphonyl)-5,6,7,8-tetrahydro-pyrido[4,3-d]pyrimidin-2-yl]-4-methyl-benzamide,
[246] 4-tert-butyl-N-{6-[3-(2-hydroxyphenyl)-propionyl]-5,6,7,8-tetrahydro-pyrido [4,3-d]pyrimidin-2-yl} -benzamide,
[247] 4-fluoro-N-[6-(2-phenyl-butyrylamino)-5,6,7,8-tetrahydro-quinazolin-2-yl]-benzamide,
[248] 4-tert-butyl-N-[6-(4-propyl-benzoyl)-5,6,7,8-tetrahydro-pyrido[4,3-d]pyrimidin-2-yl]-benzamide,
[249] thiophene-2-carboxylic acid [6-(2-bromo-benzoylamino)-5,6,7,8-tetrahydro-quinazolin-2-yl]-amide,
[250] 2-methoxy-N-[6-(6-phenoxy-pyridine-3-carbonyl)-5,6,7,8-tetrahydro-pyrido[4,3-d]pyrimidin-2-yl]-benzamide,
[251] N-[6-(4-acetylamino-benzenesulphonyl)-5,6,7,8-tetrahydro-pyrido [4,3-d]pyrimidin-2-yl]-4-ethyl-benzamide,
[252] 3,5-dichloro-N-[6-(4-fluoro-benzenesulphonyl)-5,6,7,8-tetrahydro-pyrido[4,3-d]pyrimidin-2-yl]-benzamide,
[253] N-[6-(1-benzenesulphonyl-1H-indole-2-carbonyl)-5,6,7,8-tetrahydro-pyrido[4,3-d]pyrimidin-2-yl]-4-fluoro-benzamide and
[255] N-[6-(3-chloro-benzenesulphonylamino)-5,6,7,8-tetrahydro-quinazolin-2-yl]-4-fluoro-benzamide,
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

15. A process for the production of substituted 5,6,7,8-tetrahydro-pyrido[4,3-d]pyrimidin-2-yl compounds according to one or more of claims 1-14, in which X stands for an NR^{2a} group, **characterised in that** a protected piperidin-4-one of the general formula II 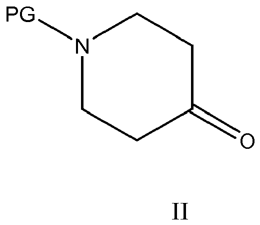 in which PG stands for a protective group, is converted by reaction with dimethoxy-methyl-dimethyl-amine of formula III 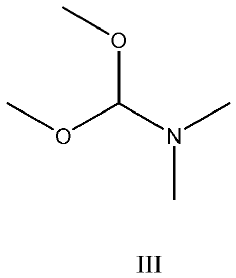 in an organic reaction medium to yield a compound of the general formula IV, 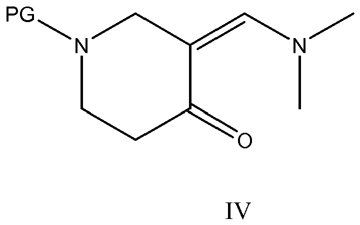 in which PG has the aforementioned meaning, this latter compound is possibly purified and/or isolated, and is converted by reaction with guanidine of formula V 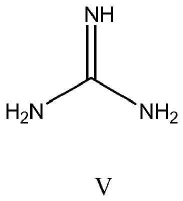 possibly in the form of a corresponding salt, in an organic reaction medium, possibly in the presence of at least one base to yield a compound of the general formula VI 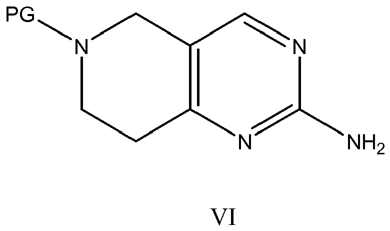 in which PG has the aforementioned meaning, this latter compound is possibly purified and/or isolated, and is reacted in an organic reaction medium, possibly in the presence of at least one base and/or at least one catalyst, with a compound of the general formula R³-C(=O)-X¹ or a compound of the general formula (R³-C(=O))₂O, or by reaction with a compound of the general formula R³-COOH in an organic reaction medium in the presence of at least one coupling agent, possibly in the presence of at least one base, or by reaction with a compound of the general formula R⁴-O-C(=O)-X^{1a} in an organic reaction medium, possibly in the presence at least one base, wherein R³ and R⁴ in each case have the aforementioned meaning according to one or more of claims 1-15 and X¹ and X^{1a} respectively stand for a leaving group, to yield a compound of the general formula VII 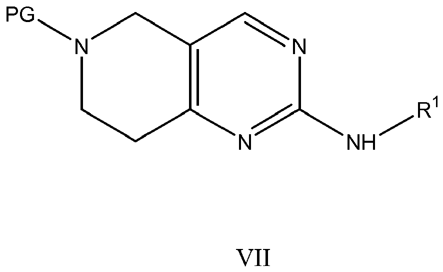 in which PG has the aforementioned meaning and R¹ has the meaning according to one or more of claims 1-15, this latter compound is possibly purified and/or isolated, and by elimination of the protective group PG is converted to yield a compound of the general formula VIII 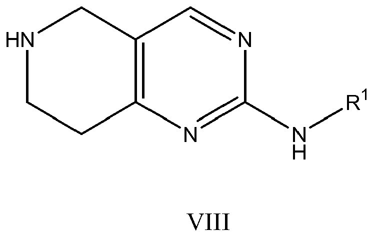 possibly in the form of a corresponding salt, this latter compound is possibly purified and/or isolated, and is converted by reaction with a compound of the general formula R⁵-C(=O)-X² or (R⁵-C(=O))₂O, in which R⁵ in each case has the meaning according to one or more of claims 1-15 and X² stands for a leaving group, in an organic reaction medium, possibly in the presence of at least one base and/or in the presence at least one catalyst,
or by reaction with a compound of the general formula R⁶-SO₂-X³, in which R⁶ has the meaning according to one or more of claims 1-15 and X³ stands for a leaving group, in an organic reaction medium, possibly in the presence of at least one base, or by reaction with a compound of the general formula R⁵-COOH, in which R⁵ has the meaning according to one or more of claims 1-15, in an organic reaction medium, in the presence of at least one suitable coupling agent, possibly in the presence of at least one base, to yield a compound of the general formula IX 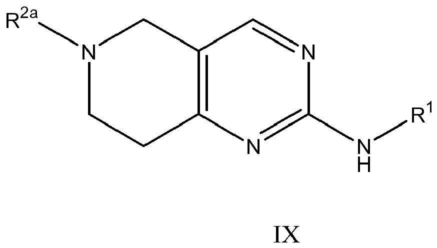 in which R¹ and R^{2a} have the meaning according to one or more of claims 1-15, this latter compound is possibly purified and/or isolated, and possibly converted to yield a corresponding salt and this is possibly purified and/or isolated,
or piperidin-4-one of the general formula X 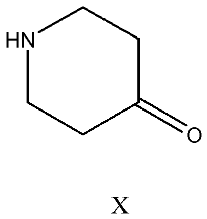 possibly in the form of a corresponding salt, is converted by reaction with a compound of the general formula R⁵-C(=O)-X² or (R⁵-C(=O))₂O, in which R⁵ in each case has the meaning according to one or more of claims 1-15 and X² stands for a leaving group, in an organic reaction medium, possibly in the presence of at least one base and/or in the presence of at least one catalyst, or by reaction with a compound of the general formula R⁶-SO₂-X³, in which R⁶ has the meaning according to one or more of claims 1-15 and X³ stands for a leaving group, in an organic reaction medium, possibly in the presence of at least one base, or by reaction with a compound of the general formula R⁵-COOH, in which R⁵ has the meaning according to one or more of claims 1-15, in an organic reaction medium, in the presence of a suitable coupling agent, possibly in the presence of at least one base, to yield a compound of the general formula XI 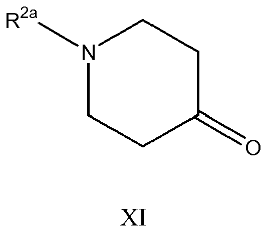 in which R^{2a} has the meaning according to one or more of claims 1-15, this latter compound is possibly purified and/or isolated, and is converted by reaction with dimethoxy-methyl-dimethyl-amine of formula III 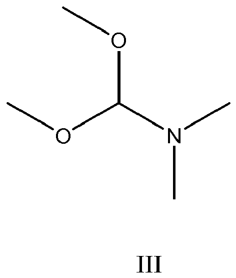 in an organic reaction medium to yield a compound of the general formula XII 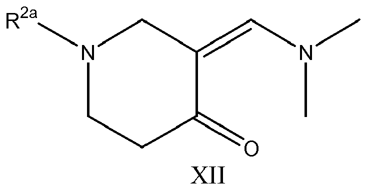 in which R^{2a} has the aforementioned meaning, this compound is possibly purified and/or isolated, and is converted with guanidine of formula V 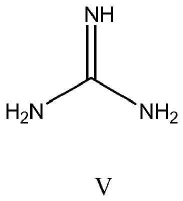 possibly in the form of a corresponding salt, in an organic reaction medium, possibly in the presence of at least one base, to yield a compound of the general formula XIII 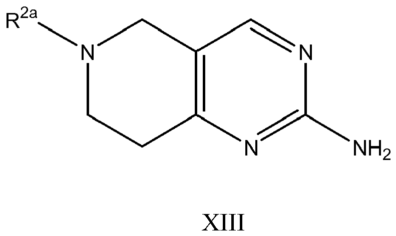 this compound is possibly purified and/or isolated, and is reacted in an organic reaction medium, possibly in the presence of at least one base and/or at least one catalyst, with a compound of the general formula R³-C(=O))₂O or a compound of the general formula R³-C(=O)-X¹, or by reaction with a compound of the general formula R³-COOH in an organic reaction medium in the presence of at least one coupling agent, possibly in the presence of at least one base, or by reaction with a compound of the general formula R⁴-O-C(=O)-X^{1a} in an organic reaction medium, possibly in the presence of at least one base, wherein R³ and R⁴ in each case have the meaning according to one or more of claims 1-15 and X¹ and X^{1a} respectively stand for a leaving group, to yield a compound of the general formula XIV 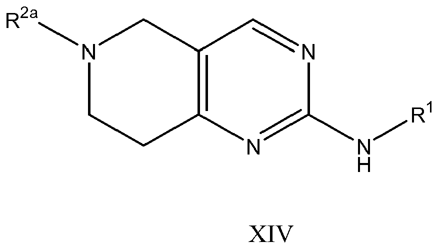 in which R¹ and R^{2a} have the meaning according to one or more of claims 1-15, and this latter compound is possibly purified and/or isolated, and possibly converted to yield a corresponding salt and this salt is possibly purified and/or isolated.

16. A process for the production of substituted 5,6,7,8-tetrahydro-quinazolin-2-yl compounds according to one or more of claims 1-14, in which X stands for a C(H)(NHR²) group, **characterised in that** 1,4-dioxa-spiro[4.5]dec-8-yl-amine of formula C 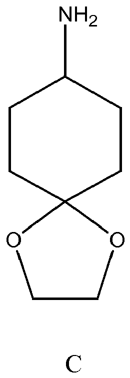 is converted by reaction with a compound of the general formula R⁵-C(=O)-X⁴ or (R⁵-C(=O))₂O, in which R⁵ in each case has the meaning according to one or more of claims 1-15 and X⁴ stands for a leaving group, in an organic reaction medium, possibly in the presence of at least one base and/or in the presence at least one catalyst,
or by reaction with a compound of the general formula R⁶-SO₂-X⁵, in which R⁶ has the meaning according to one or more of claims 1-15 and X⁵ stands for a leaving group, in an organic reaction medium, possibly in the presence of at least one base, or by reaction with a compound of the general formula R⁵-COOH, in which R⁵ has the meaning according to one or more of claims 1-15, in an organic reaction medium, in the presence of a suitable coupling agent, possibly in the presence of at least one base, to yield a compound of the general formula D 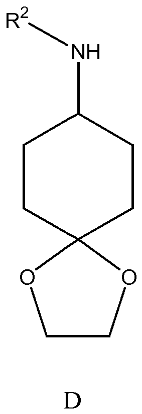 in which R² has the meaning according to one or more of claims 1 to 15, this latter compound is possibly purified and/or isolated, and converted by elimination of the acetal group in an organic reaction medium in the presence of water and at least one acid to yield a compound of the general formula E 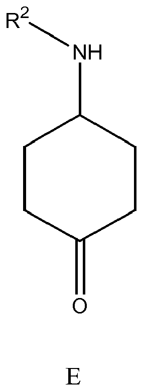 in which R² has the aforementioned meaning, and this latter compound is converted by introduction of a protective group (PG¹) in an organic reaction medium to yield a compound of the general formula F 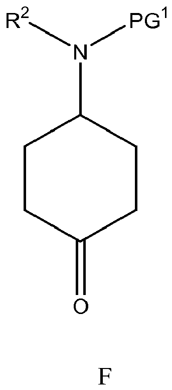 in which R² has the aforementioned meaning and PG¹ stands for a protective group, this latter compound is possibly purified and/or isolated, and is converted by reaction with dimethoxymethyl-dimethyl-amine of the general formula III 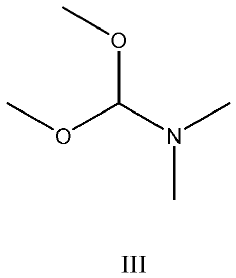 in an organic reaction medium to yield a compound of the general formula G 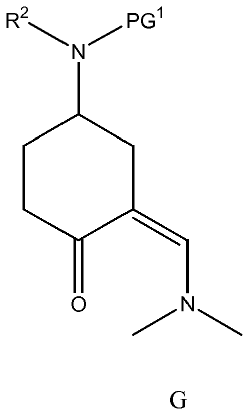 in which R² and PG¹ have the aforementioned meaning, and this latter compound is possibly purified and/or isolated, and possibly converted in the presence of at least one base in an organic reaction medium with a guanidine compound of formula V, possibly in the form of a corresponding salt, 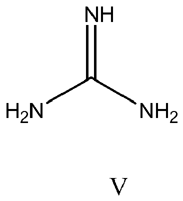 to yield a compound of the general formula H 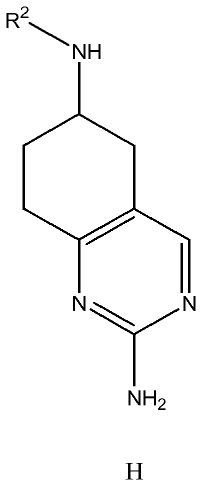 this latter compound is possibly purified and/or isolated, and is converted by introduction of a protective group (PG2) in an organic reaction medium to yield a compound of the general formula I 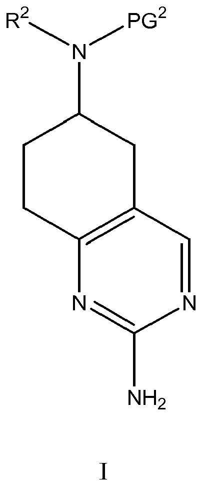 in which R² and PG² have the aforementioned meaning, this latter compound is possibly purified and/or isolated, and converted by reaction with a compound of the general formula R³-C(=O)-X⁶ or a compound of the general formula (R³-C(=O))₂O, in which R³ in each case has the meaning according to one or more of claims 1-15 and X⁶ stands for a leaving group, or by reaction with a compound of the general formula R³COOH, in which R³ has the aforementioned meaning, in an organic reaction medium in the presence of a coupling agent, possibly in the presence of at least one base, or by reaction with a compound of the general formula R⁴-O-C(=O)-X^{1a} in an organic reaction medium, possibly in the presence of a base, to yield a compound of the general formula J 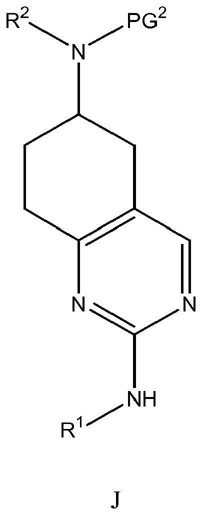 in which R² and PG² have the aforementioned meaning and R¹ has the meaning according to one or more of claims 1-15, this latter compound is possibly purified and/or isolated, and converted by elimination of the protective group to yield a 5,6,7,8-tetrahydro-quinazolin-2-yl compound according to one or more of claims 1-15, in which X stands for a C(H)(NHR²) group, this latter compound is possibly purified and/or isolated and then possibly converted to yield a corresponding salt, and this is possibly purified and/or isolated.

17. A pharmaceutical preparation containing at least one compound according to one or more of claims 1-14 and possibly one or more physiologically acceptable auxiliary substances.

18. A pharmaceutical preparation according to claim 17 for noradrenalin receptor regulation, in particular for inhibiting noradrenalin reuptake (noradrenalin uptake), for 5-HT receptor regulation, in particular for inhibiting 5-hydroxy-tryptophan reuptake (5-HT uptake), for mGluR5 receptor regulation and/or for batrachotoxin (BTX) receptor regulation.

19. A pharmaceutical preparation according to claim 17 for the prevention and/or treatment of disorders and/or diseases, which are mediated at least in part by one or more of the receptors selected from the group consisting of noradrenalin-receptors, 5-HT receptors, mGluR5 receptors and batrachotoxin (BTX) receptors.

20. A pharmaceutical preparation according to one or more of claims 17-19 for the prevention and/or treatment of pain, preferably of pain selected from the group consisting of acute pain, chronic pain, neuropathic pain and visceral pain.

21. A pharmaceutical preparation according to one or more of claims 17-19 for the prevention and/or treatment of migraine, depression, urinary incontinence, coughing, neurodegenerative diseases, preferably selected from the group consisting of Parkinson's disease, Huntington's disease, Alzheimer's disease and multiple sclerosis, eating disorders, preferably selected from the group consisting of bulimia, anorexia, obesity and cachexia, cognitive dysfunction, preferably memory disorders, cognitive deficiency states (attention deficit syndrome, ADS), disorders of the nervous system, epilepsy, schizophrenia, cerebral ischaemia, muscle spasms, cramps, diarrhoea, pruritus, gastro-oesophageal reflux syndrome, panic attacks, alcohol and/or drug abuse and/or abuse of medicines, alcohol and/or drug dependency and/or dependency on medicines, preferably for the prevention and/or reduction of withdrawal symptoms associated with alcohol and/or drug dependency and/or dependency on medicines, for the prevention and/or reduction of a development of tolerance to medicines, in particular medicines based on opioids, for regulating food intake, for modulating locomotor activity, for regulating the cardiovascular system, for local anaesthesia, for anxiolysis, for increasing vigilance, for increasing libido, for diuresis, and/or for antinatriuresis.

22. Use of at least one compound according to one or more the claims 1-14 and possibly one or more pharmaceutically compatible auxiliary substances for the production of a pharmaceutical preparation for noradrenalin receptor regulation, in particular for inhibiting noradrenalin reuptake (noradrenalin uptake), for 5-HT receptor regulation, in particular for inhibiting 5-hydroxy-tryptophan reuptake (5-HT uptake), for mGluR5 receptor regulation and/or for batrachotoxin (BTX) receptor regulation.

23. Use of at least one compound according to one or more the claims 1-14 and possibly one or more pharmaceutically compatible auxiliary substances for the production of a pharmaceutical preparation for the prevention and/or treatment of disorders and/or diseases which are mediated at least in part by one or more receptors selected from the group consisting of noradrenalin receptors, 5-HT receptors, mGluR5 receptors and batrachotoxin (BTX) receptors.

24. Use of at least one compound according to one or more the claims 1-14 and possibly one or more pharmaceutically compatible auxiliary substances for the production of a pharmaceutical preparation for the prevention and/or treatment of pain, preferably of pain selected from the group consisting of acute pain, chronic pain, neuropathic pain and visceral pain.

25. Use of at least one compound according to one or more the claims 1-14 and possibly one or more pharmaceutically compatible auxiliary substances for the production of a pharmaceutical preparation for the prevention and/or treatment of migraine, depression, urinary incontinence, coughing, neurodegenerative diseases, preferably selected from the group consisting of Parkinson's disease, Huntington's disease, Alzheimer's disease and multiple sclerosis, eating disorders, preferably selected from the group consisting of bulimia, anorexia, obesity and cachexia, cognitive dysfunction, preferably memory disorders, cognitive deficiency states (attention deficit syndrome, ADS), disorders of the nervous system, epilepsy, schizophrenia, cerebral ischaemia, muscle spasms, cramps, diarrhoea, pruritus, gastro-oesophageal reflux syndrome, panic attacks, alcohol and/or drug abuse and/or abuse of medicines, preferably for the prevention and/or reduction of withdrawal symptoms associated with alcohol and/or drug dependency and/or dependency on medicines, for the prevention and/or reduction of a development of tolerance to medicines, in particular medicines based on opioids, for regulating food intake, for modulating locomotor activity, for regulating the cardiovascular system, for local anaesthesia, for anxiolysis, for increasing vigilance, for increasing libido, for diuresis, and/or for antinatriuresis.

## Revendications

1. Composés de 5,6,7,8-tétrahydro-pyrido[4,3-d]pyrimidin-2-yle et 5,6,7,8-tétrahydro-quinazolin-2-yle substitués de formule générale I 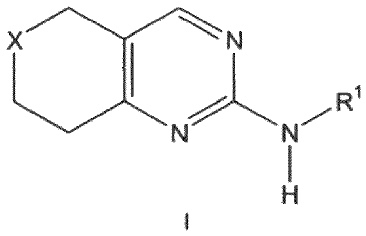 dans laquelle
X représente un groupe C(H)(NHR²) ou un groupe NR^{2a}, R¹ représente un groupe -C(=O)-R³ ou un groupe -C(=O)-O-R⁴,
R², R^{2a} représentent chacun indépendamment l'un de l'autre un groupe -C(=O)-R⁵ ou un groupe -S(=O)₂-R⁶,
R³ représente un radical en C₁₋₈ aliphatique, linéaire ou ramifié, non substitué ou substitué au moins une fois, saturé ou insaturé, comprenant éventuellement au moins un hétéroatome en tant qu'élément de chaîne, un radical cycloaliphatique de 3 à 8 éléments, non substitué ou substitué au moins une fois, saturé ou insaturé, comprenant éventuellement au moins un hétéroatome en tant qu'élément de cycle, qui peut être relié par un groupe alkylène en C₁₋₃ linéaire ou ramifié, non substitué ou substitué au moins une fois, comprenant éventuellement au moins un hétéroatome en tant qu'élément de chaîne, ou un radical aryle ou hétéroaryle de 5 à 14 éléments non substitué ou substitué au moins une fois, qui peut être relié par un groupe alkylène en C₁₋₃ linéaire ou ramifié, non substitué ou substitué au moins une fois,
R⁴ représente un radical aryle ou hétéroaryle de 5 à 14 éléments non substitué ou substitué au moins une fois, qui peut être relié par un groupe alkylène en C₁₋₃ linéaire ou ramifié, non substitué ou substitué au moins une fois,
R⁵ représente un radical en C₁₋₁₀ aliphatique, linéaire ou ramifié, non substitué ou substitué au moins une fois, saturé ou insaturé, comprenant éventuellement au moins un hétéroatome en tant qu'élément de chaîne,
un radical cycloaliphatique de 3 à 8 éléments, non substitué ou substitué ou moins une fois, saturé ou insaturé, comprenant éventuellement au moins un hétéroatome en tant qu'élément de cycle, qui peut être relié par un groupe alkylène en C₁₋₆ linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un radical aryle ou hétéroaryle de 5 à 14 éléments non substitué ou substitué au moins une fois, qui peut être relié par un groupe alkylène en C₁₋₆, alcénylène en C₂₋₆ ou alcynylène en C₂₋₆ linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un radical -C(=O)-R⁷, qui peut être relié par un groupe alkylène en C₁₋₃ linéaire ou ramifié, non substitué ou substitué au moins une fois,
un radical -C(=O)-O-R⁸, qui peut être relié par un groupe alkylène en C₁₋₃ linéaire ou ramifié, non substitué ou substitué au moins une fois, ou
un radical -N(H)-C(=O)-O-R⁹, qui peut être relié par un groupe alkylène en C₁₋₈ linéaire ou ramifié, non substitué ou substitué au moins une fois, comprenant éventuellement au moins un groupe -N(H)-C(=O) ou au moins un groupe -C(=O)-N(H) en tant qu'élément de chaîne,
R⁶ représente un groupe -NR¹⁰R¹¹,
un radical en C₁₋₁₀ aliphatique, linéaire ou ramifié, non substitué ou substitué au moins une fois, saturé ou insaturé, comprenant éventuellement au moins un hétéroatome en tant qu'élément de chaîne,
un radical cycloaliphatique de trois à huit éléments, non substitué ou substitué au moins une fois, saturé ou insaturé, comprenant éventuellement au moins un hétéroatome en tant qu'élément de cycle, éventuellement condensé avec un système cyclique mono- ou polycyclique non substitué ou substitué au moins une fois, qui peut être relié par un groupe alkylène en C₁₋₆ linéaire ou ramifié, non substitué ou substitué au moins une fois, comprenant éventuellement au moins un hétéroatome en tant qu'élément de chaîne, et/ou ponté avec un groupe alkylène en C₁₋₆ linéaire ou ramifié, ou
un radical aryle ou hétéroaryle de 5 à 14 éléments non substitué ou substitué au moins une fois, qui peut être relié par un groupe alkylène en C₁₋₆ linéaire ou ramifié, non substitué ou substitué au moins une fois, et/ou condensé avec un système cyclique mono- ou polycyclique non substitué ou substitué au moins une fois,
R⁷ représente un radical alkyle en C₁₋₃ linéaire ou ramifié, non substitué ou substitué au moins une fois, un radical aryle ou hétéroaryle de 5 à 14 éléments non substitué ou substitué au moins une fois, un radical cycloaliphatique de 5, 6 ou 7 éléments, non substitué ou substitué au moins uns fois, saturé ou insaturé, comprenant éventuellement au moins un hétéroatome en tant qu'élément de cycle, qui peut être condensé avec un système cyclique monocyclique non substitué ou substitué au moins une fois, ou un radical -NR^{7a}R^{7b}, les radicaux R^{7a} et R^{7b}, identiques ou différents, représentant chacun un radical alkyle en C₁₋₅ linéaire ou ramifié,
R⁸ représente un radical alkyle en C₁₋₃ linéaire ou ramifié, non substitué ou substitué au moins une fois, ou un radical aryle ou hétéroaryle de 5 ou 6 éléments, non substitué ou substitué au moins une fois, éventuellement relié par un groupe alkylène en C₁₋₃ linéaire ou ramifié,
R⁹ représente un radical alkyle en C₁₋₃ linéaire ou ramifié, non substitué ou substitué au moins une fois, un radical aryle ou hétéroaryle de 5 ou 6 éléments, non substitué ou substitué au moins une fois, éventuellement relié par un groupe alkylène en C₁₋₃ linéaire ou ramifié, ou un radical cycloaliphatique de 5 ou 6 éléments, non substitué ou substitué au moins une fois, saturé ou insaturé, qui peut être condensé avec au moins un système cyclique monocyclique non substitué ou substitué au moins une fois,
R¹⁰ et R¹¹ identiques ou différents, représentent chacun un radical alkyle en C₁₋₅ linéaire ou ramifié,
si un ou plusieurs des substituants R³ et R⁵ à R⁹ représentent un radical aliphatique saturé ou insaturé, c.-à-d. un radical alkyle, alcényle ou alcynyle, qui est substitué 1, 2, 3, 4, 5, 6, 7, 8 ou 9 fois, ses substituants sont choisis, à chaque fois indépendamment les uns des autres, dans le groupe constitué par F, Cl, Br, -OH, -SH et -NH₂ ;
si un ou plusieurs des substituants R³, R⁵ et R⁶ représentent un radical aliphatique saturé ou insaturé, c.-à-d. un radical alkyle, alcényle ou alcynyle, qui comprend 1, 2, 3, 4 ou 5 hétéroatomes en tant qu'éléments de chaîne, ces hétéroatomes sont choisis, à chaque fois indépendamment les uns des autres, dans le groupe constitué par oxygène, soufre et azote (NH) ;
si un ou plusieurs des substituants R³, R⁵ à R⁷ et R⁹ représentent un radical cycloaliphatique ou comprennent un radical cycloaliphatique, qui est substitué 1, 2, 3, 4 ou 5 fois, ses substituants sont choisis, à chaque fois indépendamment les uns des autres, dans le groupe constitué par -alkyle en C₁₋₅, -C(=O)-O-alkyle en C₁₋₅, - C(=O)-CF₃, -S(=O)₂-alkyle en C₁₋₅, -S(=O)₂-phényle, oxo (=O) et phényle, les radicaux alkyle en C₁₋₅ pouvant à chaque fois être linéaires ou ramifiés et les radicaux phényle pouvant à chaque fois être substitués 1, 2, 3, 4 ou 5 fois, de manière identique ou différente, avec un substituant choisi dans le groupe constitué par F, Cl, Br et I ;
si les radicaux cycloaliphatiques comprennent 1, 2 ou 3 hétéroatomes en tant qu'éléments de cycle, ceux-ci sont choisis, à chaque fois indépendamment les uns des autres, dans le groupe constitué par azote, oxygène et soufre ;
si un ou plusieurs des substituants R³ à R⁹ représentent un radical aryle ou hétéroaryle ou comprennent un radical aryle ou hétéroaryle, qui est substitué 1, 2, 3, 4 ou 5 fois, ses substituants sont choisis, à chaque fois indépendamment les uns des autres, dans le groupe constitué par halogène, -CN, -NO₂. -OH, -SH, alkyle en C₁₋₅, alcoxy en C₁₋₅, -S-alkyle en C₁₋₅, -CF₃, -CHF₂,-CH₂F, -O-CF₃, -O-CHF₂, -O-CH₂F, -C(=O)-CF₃, -S-CF₃, -S-CHF₂, -S-CH₂F, -S (=O)₂-phényle, -S(=O)₂-alkyle en C₁₋₅, N (alkyle en C₁₋₅) (alkyle en C₁₋₅), -C(=O)-O-alkyle en C₁₅, -CH₂-O-C(=O)-phényle, -O-C(=O)-phényle, -NH-C(=O)-alkyle en C₁₋₅, -C=C-phényle, -C=C-naphtyle, -C=C-pyrrolyle, -C=C-indolyle, -C≡C-furyle (-C≡C-furanyle), -C≡C-benzo[b]furanyle, -C≡C-thiényle (-C=C-thiophényle), -C≡C-benzo[b]thiényle, -C≡C-pyrazolyle,-C=C-imidazolyle, -C≡C-thiazolyle, -C=C-thiadiazolyle,-C=C-triazolyle, -C≡C-oxazolyle, -C≡C-isoxazolyle, -C=C-pyridinyle, -C≡C-pyridazinyle, -C≡C-pyrimidinyle, -C≡C-pyrazinyle, -C≡C-pyranyle, -C≡C-indazolyle, -C=C-purinyle, -C≡C-indolizinyle, -C≡C-quinolinyle, -C≡C-isoquinolinyle, -C≡C-quinazolinyle, pyrazolyle, phényle, furyle (furanyle), thiadiazolyle, thiophényle (thiényle), phénoxy et benzyle, les substituants cycliques pouvant eux-mêmes à chaque fois être substitués 1, 2, 3, 4 ou 5 fois, de manière identique ou différente, avec un substituant choisi dans le groupe constitué par F, Cl, Br, I ;
si un ou plusieurs des substituants R³ à R⁹ représentent un radical hétéroaryle ou comprennent un radical hétéroaryle, ses 1, 2, 3, 4 ou 5 hétéroatomes sont choisis, à chaque fois indépendamment les uns des autres dans le groupe constitué par oxygène, soufre et azote ;
si un des substituants R³ à R⁶ mentionnés précédemment comprend un groupe alkylène, alcénylène ou alcynylène linéaire ou ramifié, qui est substitué 1, 2, 3, 4 ou 5 fois, ses substituants sont choisis, à chaque fois indépendamment les uns des autres, dans le groupe constitué par F, Cl, Br, hydroxy et phényle non substitué ;
si le groupe alkylène, alcénylène ou alcynylène comprend 1, 2, 3, 4 ou 5 hétéroatomes en tant qu'éléments de chaîne, ceux-ci sont choisis dans le groupe constitué par oxygène, soufre et azote (NH) ;
et
un système cyclique mono- ou polycyclique se rapporte à des radicaux hydrocarbonés mono- ou polycycliques qui peuvent être saturés, insaturés ou aromatiques et qui peuvent éventuellement comprendre 1, 2, 3, 4 ou 5 hétéroatomes en tant qu'éléments de cycle, les différents cycles pouvant, à chaque fois indépendamment les uns des autres, présenter un degré de saturation différent, c.-à-d. pouvant être saturés, insaturés ou aromatiques ; et
si un système cyclique polycyclique est présent, les différents cycles peuvent, à chaque fois indépendamment les uns des autres, présenter un degré de saturation différent, c.-à-d. peuvent être saturés, insaturés ou aromatiques, et les hétéroatomes de chaque cycle, à chaque fois identiques ou différents, peuvent être choisis dans le groupe constitué par oxygène, azote et soufre ; et
si un ou plusieurs des substituants R⁵, R⁶, R⁷ et R⁹ comprennent un système cyclique monocyclique ou polycyclique, qui est substitué 1, 2, 3, 4 ou 5 fois, ses substituants sont choisis, à chaque fois indépendamment les uns des autres, dans le groupe constitué par halogène, -CN, -NO₂, -OH, -SH, alkyle en C₁₋₅, alcoxy en C₁₋₅, -S-alkyle en C₁₋₅, -CF₃, -CHF₂,-CH₂F, -O-CF₃, -O-CHF₂, -O-CH₂F, -C(=O)-CF₃, -S-CF₃, -S-CHF₂, -S-CH₂F, -S(=O)₂-phényle, -S(=O)₂-alkyle en C₁₋₅, N (alkyle en C₁₋₅) (alkyle en C₁₋₅), -C(=O)-O-alkyle en C₁₅, CH₂-O-C(=O)-phényle, -O-C(=O) -phényle, -NH-C(=O)-alkyle en C₁₋₅, -C≡C-phényle, -C≡C-naphtyle, -C=C-pyrrolyle, -C≡C-indolyle, -C≡C-furyle (-C≡C-furanyle), -C≡C-benzo[b]furanyle, -C≡C-thiényle (-C=C-thiophényle), -C≡C-benzo[b]thiényle, -C≡C-pyrazolyle, - C=C-imidazolyle, -C≡C-thiazolyle, -C=C-thiadiazolyle, - C=C-triazolyle, -C≡C-oxazolyle, -C≡C-isoxazolyle, -C=C-pyridinyle, -C≡C-pyridazinyle, -C≡C-pyrimidinyle, -C≡C-pyrazinyle, -C≡C-pyranyle, -C≡C-indazolyle, -C=C-purinyle, -C≡C-indolizinyle, -C≡C-quinolinyle, -C≡C-isoquinolinyle, -C≡C-quinazolinyle, pyrazolyle, phényle, furyle (furanyle), thiadiazolyle, thiophényle (thiényle), phénoxy et benzyle, les substituants cycliques pouvant eux-mêmes à chaque fois être substitués 1, 2, 3, 4 ou 5 fois, de manière identique ou différente, avec un substituant choisi dans le groupe constitué par F, Cl, Br, I ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme des sels correspondants, ou à chaque fois sous la forme des solvates correspondants.

2. Composés selon la revendication 1, **caractérisés en ce que** R³ représente un radical alkyle en C₁₋₈ linéaire ou ramifié, non substitué ou substitué au moins une fois, comprenant éventuellement un ou plusieurs atomes d'oxygène et/ou un ou plusieurs groupes NH en tant qu'éléments de chaîne, un radical cycloaliphatique de 5, 6 ou 7 éléments, non substitué ou substitué au moins une fois, saturé ou insaturé, comprenant éventuellement au moins un hétéroatome en tant qu'élément de cycle, qui peut être relié par un groupe alkylène en C₁₋₃ linéaire ou ramifié, non substitué ou substitué au moins une fois, comprenant éventuellement au moins un hétéroatome en tant qu'élément de chaîne, ou un radical phényle, un radical thiophényle (thiényle), un radical furanyle (furyle), un radical pyridinyle ou un radical naphtyle non substitué ou substitué au moins une fois, qui peut être relié par un groupe alkylène en C₁₋₃ linéaire ou ramifié, non substitué ou substitué au moins une fois.

3. Composés selon la revendication 1 ou 2, **caractérisés en ce que** le radical R⁴ représente un radical phényle éventuellement relié par un pont -(CH₂)-, -(CH₂)₂- ou -(CH₂)₃-, le cycle phényle pouvant être non substitué ou substitué au moins une fois, de manière identique ou différente, avec un substituant choisi dans le groupe constitué par méthyle, éthyle, n-propyle, isopropyle, n-butyle, isobutyle, sec-butyle, tert.-butyle, méthoxy, éthoxy, n-propoxy, iso-propoxy, n-butoxy, iso-butoxy, sec-butoxy, tert.-butoxy, F, Cl, Br, I, -CN et -CF₃.

4. Composés selon une ou plusieurs des revendications 1 à 3, **caractérisés en ce que** R⁵ représente un radical alkyle en C₁₋₁₀, linéaire ou ramifié, comprenant éventuellement un ou plusieurs atomes d'oxygène et/ou éventuellement un ou plusieurs atomes de soufre et/ou éventuellement un ou plusieurs groupes -N(H) en tant qu'éléments de chaîne, un radical alcényle en C₂₋₁₀ linéaire ou ramifié, comprenant éventuellement un ou plusieurs atomes d'oxygène et/ou éventuellement un ou plusieurs atomes de soufre et/ou éventuellement un ou plusieurs groupes -N(H) en tant qu'éléments de chaîne, un radical alcynyle en C₂₋₁₀ linéaire ou ramifié, comprenant éventuellement un ou plusieurs atomes d'oxygène et/ou éventuellement un ou plusieurs atomes de soufre et/ou éventuellement un ou plusieurs groupes -N(H) en tant qu'éléments de chaîne,
un radical cycloaliphatique de 3 à 8 éléments, non substitué ou substitué ou moins une fois, saturé ou insaturé, comprenant éventuellement un ou plusieurs hétéroatomes, choisis indépendamment les uns des autres dans le groupe constitué par oxygène, soufre et azote, en tant qu'éléments de cycle, qui peut être relié par un groupe -(CH₂)- -(CH₂)₂-, -(CH₂)₃-, -(CH₂)₄-,-C(C₂H₅)(H)-, -(CH₂)-O-, -(CH₂)₂-O-, -(CH₂)₃-O-, -(CH₂)₄-O-, -O-(CH₂)-, -O-(CH₂)₂, -O-(CH₂)₃-, -O-(CH₂)₄-,-C(C₂H₅)(H)-O-, -O-C(C₂H₅)(H)-, -CH₂-O-CH₂-, -CH₂-S-CH₂-,-C(CH₃)₂- ou -C (H) (CH₃)-, et/ou condensé avec un système cyclique monocyclique de 5 ou 6 éléments non substitué ou substitué au moins une fois,
un radical aryle ou hétéroaryle de 5 ou 6 éléments non substitué ou substitué au moins une fois, qui peut être relié par un groupe -(CH₂)-, -(CH₂)₂-, -(CH₂)₃-,-(CH₂)₄-, -C(C₂H₅)(H)-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -C(CH₃)₂-, -C(H)(CH₃)- ou -CH=CH-, et/ou condensé avec un système cyclique monocyclique de 5 ou 6 éléments non substitué ou substitué au moins une fois,
un radical -C(=O)-R⁷, qui peut être relié par un groupe -(CH₂)-, -(CH₂)₂- ou -(CH₂)₃-,
un radical -C(=O)-O-R⁸, qui peut être relié par un groupe alkylène en C₁₋₃ linéaire ou ramifié, non substitué ou substitué une ou plusieurs fois avec un radical phényle, ou
un radical -N(H)-C(=O)-O-R⁹, qui peut être relié par un groupe alkylène en C₁₋₈ linéaire ou ramifié, non substitué ou substitué une ou plusieurs fois avec un radical phényle, comprenant éventuellement au moins un groupe -N(H)-C(=O) ou au moins un groupe -C(=O)-N(H) en tant qu'élément de chaîne.

5. Composés selon une ou plusieurs des revendications 1 à 4, **caractérisés en ce que** R⁶ représente un groupe -NR¹⁰R¹¹,
un radical alkyle en C₁₋₁₀ linéaire ou ramifié, non substitué, un radical alcényle en C₂₋₁₀ linéaire ou ramifié, non substitué, un radical alcynyle en C₂₋₁₀ linéaire ou ramifié, non substitué,
un radical cycloaliphatique de 3, 4, 5, 6, 7 ou 8 éléments, non substitué ou substitué au moins une fois, saturé ou insaturé, qui peut être relié par un groupe alkylène en C₁₋₃ linéaire ou ramifié et/ou ponté avec un groupe alkylène en C₁₋₃ linéaire ou ramifié,
un radical aryle ou hétéroaryle de 5 ou 6 éléments non substitué ou substitué au moins une fois, qui peut être relié par un groupe alkylène en C₁₋₆ linéaire ou ramifié et/ou condensé avec un système cyclique monocyclique de 5 ou 6 éléments, non substitué ou substitué au moins une fois.

6. Composés selon une ou plusieurs des revendications 1 à 5, **caractérisés en ce que** le radical R⁷ représente un radical alkyle en C₁₋₃ linéaire ou ramifié, non substitué, un radical aryle ou hétéroaryle de 5 ou 6 éléments non substitué, un radical cycloaliphatique de 5, 6 ou 7 éléments, non substitué, saturé ou insaturé, comprenant éventuellement un atome d'azote en tant qu'élément de cycle, qui peut être condensé avec un système cyclique monocyclique à 6 éléments non substitué, ou un radical -NR^{7a}R^{7b}, les radicaux R^{7a} et R^{7b}, identiques ou différents, représentant chacun un radical alkyle en C₁₋₃ linéaire ou ramifié.

7. Composés selon une ou plusieurs des revendications 1 à 6, **caractérisés en ce que** R⁸ représente un radical alkyle en C₁₋₃ linéaire ou ramifié, non substitué, ou un radical phényle éventuellement relié par un groupe alkylène en C₁₋₃ linéaire ou ramifié.

8. Composés selon une ou plusieurs des revendications 1 à 7, **caractérisés en ce que** R⁹ représente un radical alkyle en C₁₋₃ linéaire ou ramifié, non substitué, un radical aryle ou hétéroaryle de 5 ou 6 éléments, non substitué, éventuellement relié par un groupe alkylène en C₁₋₃ linéaire ou ramifié, ou un radical cycloaliphatique de 5 ou 6 éléments, non substitué ou substitué au moins une fois, saturé ou insaturé, qui peut être condensé avec au moins un système cyclique monocyclique à 6 éléments non substitué.

9. Composés selon une ou plusieurs des revendications 1 à 8, **caractérisés en ce que** R¹⁰ et R¹¹, identiques ou différents, représentent chacun un radical méthyle, éthyle, n-propyle ou isopropyle.

10. Composés selon une ou plusieurs des revendications 1 à 9, **caractérisés en ce que**
X représente un groupe C(H)(NHR²) ou un groupe NR^{2a},
R¹ représente un groupe -C(=O)-R³ ou un groupe -C(=O)-O-R⁴,
R², R^{2a} représentent chacun indépendamment l'un de l'autre un groupe -C(=O)-R⁵ ou un groupe -S(=O)₂-R⁶,
R³ représente un radical méthyle, éthyle, n-propyle, isopropyle, n-butyle, isobutyle, sec-butyle ou tert.-butyle, un radical cycloaliphatique de 5, 6 ou 7 éléments, non substitué, saturé ou insaturé, comprenant éventuellement un ou plusieurs atomes d'oxygène et/ou un ou plusieurs atomes d'azote en tant qu'éléments de cycle, qui peut être relié par un groupe -(CH₂)-, -(CH₂)₂- ou -(CH₂)₃-, ou un radical phényle, un radical thiophényle (radical thiényle), un radical furanyle (radical furyle), un radical pyridinyle ou un radical naphtyle, qui peut être relié par un groupe - (CH₂)-, -(CH₂)₂- ou -(CH₂)₃- et/ou substitué une ou plusieurs fois, de manière identique ou différente, avec un substituant choisi dans le groupe constitué par méthyle, éthyle, n-propyle, isopropyle, n-butyle, isobutyle, sec-butyle, tert.-butyle, méthoxy, éthoxy, n-propoxy, isopropoxy, n-butoxy, isobutoxy, sec-butoxy, tert.-butoxy, F, Cl, Br, I, -CN et -CF₃,
R⁴ représente un radical benzyle non substitué,
R⁵ représente un radical choisi dans le groupe constitué par méthyle, éthyle, n-propyle, isopropyle, n-butyle, sec-butyle, tert-butyle, n-pentyle, isopentyle, néo-pentyle, -C (H) (C₂H₅)₂, -C (H) (n-C₃H₇)₂,-CH=CH-CH=CH-CH₃, -CH₂-CH₂-CH=CH₂, -CH₂-CH₂-C(H) (CH₃)-(CH₂)₃-CH₃, -CH₂-O-CH₃ et -CH₂-O-CH₂-CH₂-O-CH₃,
un radical choisi dans le groupe constitué par cyclopropyle, cyclobutyle, cyclopentyle, cyclohexyle, cycloheptyle, cyclooctyle, pipéridinyle, pipérazinyle, morpholinyle, tétrahydrofuranyle (tétrahydrofuryle), dithiolanyle, 1,2,3,4-tétrahydroindolyle, 1,2,3,4-tétrahydronaphtyle, 1,3-dihydroisoindolyle, benzooxazolyle et imidazolidinyle, le radical cyclique pouvant à chaque fois être relié par un groupe -(CH₂)-, -(CH₂)₂-, -(CH₂)₃-, -(CH₂)₄-, -C(C₂H₅)(H)-, -(CH₂)-O-,-(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, - CH₂-O-CH₂-, -CH₂-S-CH₂-, -C(CH₃)₂- ou -C(H)(CH₃)- et/ou substitué une ou plusieurs fois, de manière identique ou différente, avec un substituant choisi dans le groupe constitué par phényle, -C(=O)-O-tert.-butyle, oxo (=O), -S(=O)₂-méthyle et -S(=O)₂-phényle, et les radicaux phényle mentionnés précédemment pouvant à chaque fois être substitués une ou plusieurs fois, de manière identique ou différente, avec un substituant choisi dans le groupe constitué par F, Cl, Br et I,
un radical choisi dans le groupe constitué par phényle, naphtyle, furanyle (furyle), thiophényle (thiényle), pyridinyle, isoxazolyle, triazolyle, pyrazolyle, thiazolyle, benzo[1,2,5]-oxadiazolyle, 2,3-dihydrobenzofuranyle, quinolinyle, chromanyle, chroményle, benzo[b]furanyle, benzo[b]thiophényle, benzo[1,3]-dioxinyle, indolyle, 2,3-dihydro-indolyle, 3,4-dihydro-benzo[1,4]oxazinyle et 1,2,3,4-tétrahydroisoquinolinyle, le radical cyclique pouvant à chaque fois être relié par un groupe -(CH₂)-, -(CH₂)₂-, -(CH₂)₃-, -(CH₂)₄-, -C(C₂H₅)(H)-, -(CH₂)-O-, -(CH₂)₂-O-, - (CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂O-CH₂-,-CH₂-S-CH₂-, -C(CH₃)₂-, -C(H)(CH₃)- ou -CH=CH-, et/ou substitué une ou plusieurs fois, de manière identique ou différente, avec un substituant choisi dans le groupe constitué par F, Cl, Br, I, -CN, -NO₂, -OH, -SH, méthyle, éthyle, n-propyle, isopropyle, n-butyle, isobutyle, sec-butyle, tert.-butyle, n-pentyle, néo-pentyle, méthoxy, éthoxy, n-propoxy, isopropoxy, n-butyloxy, iso-butyloxy, sec-butyloxy, tert.-butyloxy, - S-méthyle, -S-éthyle, -CF₃, -CHF₂, -CH₂F, -O-CF₃, -O-CHF₂, -O-CH₂F, -S-CF₃, -S-CHF₂, -S-CH₂F, -S(=O)₂-phényle, pyrazolyle, phényle, -N(CH₃)₂, -N (C₂H₅)₂, -CH₂-O-C(=O)-phényle, -O-C(=O)-phényle, furyle (furanyle), thiadiazolyle, thiophényle (thiényle), phénoxy et benzyle, et les substituants cycliques pouvant eux-mêmes à chaque fois être substitués une ou plusieurs fois, de manière identique ou différente, avec un substituant choisi dans le groupe constitué par F, Cl, Br, I,
un radical -C(=O)-R⁷, qui peut être relié par un groupe -(CH₂)-, -(CH₂)₂-, -(CH₂)₃-,
un radical -C(=O)-O-R⁸, qui peut être relié par un groupe -(CH₂)-, -(CH₂)₂-, -(CH₂)₃- ou -C (H) (phényle), ou
un radical -N(H)-C(=O)-O-R⁹, qui est relié par un groupe -(CH₂)-, -(CH₂)₂-, -(CH₂)₃-, -(CH₂)₄-, -(CH₂)₅-, - C (H)(CH₂-phényle) -, -C (H)(phényle), -C (H) (C (H) (CH₃)₂)-ou -C(H)(CH₂-CH(CHF₃)₂)-NH-C (=O)-CH₂-,
R⁶ représente un groupe -NR¹⁰R¹¹,
un radical méthyle, éthyle, n-propyle, isopropyle, n-butyle, isobutyle, sec-butyle ou tert.-butyle,
un radical 7,7-diméthyl-2-oxo-bicyclo[2.2.1]heptyle, éventuellement relié par un groupe -(CH₂)-, -(CH₂)₂- ou -(CH₂)₃-, ou
un radical phényle, naphtyle, thiophényle (thiényle), furanyle (furyle), thiazolyle, pyrazolyle, 2,3-dihydro-benzo[1,4]-dioxinyle, 1,2,3,4-tétrahydroisoquinolinyle ou 3,4-dihydrobenzo[1,4]-oxazinyle, qui peut être relié par un groupe -(CH₂)-,-(CH₂)₂- ou -(CH₂)₃-, et/ou substitué une ou plusieurs fois, de manière identique ou différente, avec un substituant choisi dans le groupe constitué par F, Cl, Br, I, méthyle, éthyle, n-propyle, iso-propyle, n-butyle, iso-butyle, sec-butyle, tert.-butyle, n-pentyle, isopentyle, néo-pentyle, méthoxy, éthoxy, -CN, -CF₃, -CF₂H, -CFH₂, -NO₂, -C(=O)-CF₃, -O-CF₃, -C(=O)-O-CH₃, -C(=O)-O-C₂H₅, -NH-C(=O)-CH₃, -S(=O)₂-CH₃ et phényle,
R⁷ représente un radical méthyle, éthyle, n-propyle, phényle, indolyle, 2,3-dihydro-indolyle, diméthylamino ou diéthylamino,
R⁸ représente un radical méthyle, éthyle, phényle ou benzyle,
R⁹ représente un radical méthyle, éthyle, n-propyle, isopropyle, n-butyle, isobutyle, sec-butyle, tert.-butyle, ou un radical phényle ou fluorényle éventuellement relié par un groupe -(CH₂)-, R¹⁰, R¹¹ identiques ou différents, représentent chacun un radical méthyle, éthyle, n-propyle ou isopropyle,
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme des sels correspondants, ou à chaque fois sous la forme des solvates correspondants.

11. Composés selon une ou plusieurs des revendications 1 à 10, **caractérisés en ce que**
X représente un groupe C(H)(NHR²) ou un groupe NR^{2a},
R¹ représente un groupe -C(=O)-R³ ou un groupe -C(=O)-O-R⁴,
R², R^{2a} représentent chacun indépendamment l'un de l'autre un groupe -C(=O)-R⁵ ou un groupe -S(=O)₂-R⁶,
R³ représente un radical alkyle en C₁₋₁₀, alcényle en C₂₋₁₀ ou alcynyle en C₂₋₁₀ linéaire ou ramifié, éventuellement substitué, comprenant éventuellement 1, 2 ou 3 hétéroatomes choisis dans le groupe constitué par oxygène, soufre et azote (NH) en tant qu'éléments de chaîne ;
un radical cycloaliphatique de 3, 4, 5, 6, 7, 8 ou 9 éléments insaturé ou saturé, éventuellement substitué ;
un radical aryle ou hétéroaryle de 5 à 14 substituants éventuellement substitué ;
-(CHR¹²)-Uₐ-(CH₂)_{b}-V_{c}-(CH₂)_{d}-R¹³ avec a = 0 ou 1, b = 0 ou 1, c = 0 ou 1 et d = 0 ou 1, U et V représentant chacun indépendamment l'un de l'autre 0, S et NH ;
ou -(CHR¹⁴)-(CH₂)ₑ-R¹⁵ avec e = 0, 1, 2 ou 3 ;
R⁴ représente un radical aryle ou hétéroaryle de 5 à 14 éléments éventuellement substitué ;
ou (CHR¹⁶)-(CH₂)_{f}-R¹⁷ avec f = 0, 1, 2 ou 3 ;
R⁵ représente un radical alkyle en C₁₋₁₀, un radical alcényle en C₂₋₁₀ ou un radical alcynyle en C₂₋₁₀ linéaire ou ramifié, éventuellement substitué, comprenant éventuellement 1, 2 ou 3 hétéroatomes choisis dans le groupe constitué par oxygène, soufre et azote (NH) en tant qu'éléments de chaîne ;
un radical cycloaliphatique de 3, 4, 5, 6, 7, 8 ou 9 éléments insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments éventuellement substitué, qui peut être condensé avec un système cyclique mono- ou polycyclique saturé ou insaturé, éventuellement substitué ;
-(CHR¹⁸)_{g}-(CH₂)ₕ-C(=O)-R⁷ avec g = 0 ou 1 et h = 0, 1, 2 ou 3 ;
-(CHR¹⁹)ⱼ(CH₂)ₖ-C(=O)-O-R⁸ avec j = 0 ou 1 et k = 0, 1, 2 ou 3 ;
-(CHR²⁰)ₘ-(CH₂)ₙ-[NH-C(=O)]ₚ-(CH₂)_{q}-NH-C(=O)-O-R⁹ avec m = 0 ou 1 ; n = 0, 1, 2, 3, 4 ou 5 ; p = 0 ou 1 et q = 0, 1 ou 2 ;
-(CR²¹R²²)-Wᵣ-(CH₂)ₛ-XₜCH₂)ᵤ-Yᵥ-R²³ avec r = 0 ou 1, s = 0 ou 1, t = 0 ou 1, u = 0, 1 ou 2 et v = 0 ou 1, W, X et Y représentant chacun indépendamment les uns des autres 0, S et NH ;
ou -CH=CH-R²⁴ ;
R⁶ représente un groupe -NR¹⁰R¹¹ ;
un radical alkyle en C₁₋₁₀, un radical alcényle en C₂₋₁₀ ou un radical alcynyle en C₂₋₁₀ linéaire ou ramifié, éventuellement substitué, comprenant éventuellement 1, 2 ou 3 hétéroatomes choisis dans le groupe constitué par oxygène, soufre et azote (NH) en tant qu'éléments de chaîne ;
un radical cycloaliphatique de 3, 4, 5, 6, 7, 8 ou 9 éléments insaturé ou saturé, éventuellement substitué, qui peut être condensé avec un système cyclique mono- ou polycyclique saturé, insaturé ou aromatique, éventuellement substitué, et/ou ponté avec un groupe alkylène en C₁₋₅ linéaire ou ramifié ;
un radical aryle ou hétéroaryle de 5 à 14 éléments éventuellement substitué, qui peut être condensé avec un système cyclique mono- ou polycyclique saturé ou insaturé, éventuellement substitué ;
-(CHR²⁵)-Z_{w}-(CH₂)ₓ-A_{y}-(CH₂)_{z}-R²⁶ avec w = 0 ou 1, x = 0 ou 1, y = 0 ou 1 et z = 0 ou 1, Z et A représentant chacun indépendamment l'un de l'autre 0, S et NH ;
ou -(CHR²⁷)-(CH₂)ₐₐ-R²⁸ avec aa = 0, 1, 2 ou 3 ;
R⁷ représente un radical alkyle en C₁₋₁₀ linéaire ou ramifié, éventuellement substitué ;
un radical cycloaliphatique de 3, 4, 5, 6, 7, 8 ou 9 éléments insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué ;
ou un radical -NR^{7a}R^{7b}, les radicaux R^{7a} et R^{7b} représentant chacun indépendamment l'un de l'autre un radical alkyle en C₁₋₁₀ linéaire ou ramifié ;
R⁸ représente un radical alkyle en C₁₋₁₀ linéaire ou ramifié, éventuellement substitué ;
ou un radical aryle ou hétéroaryle de 5 à 14 éléments éventuellement substitué, qui peut être relié par un groupe alkylène en C₁₋₅ linéaire ou ramifié ;
R⁹ représente un radical alkyle en C₁₋₁₀ linéaire ou ramifié, éventuellement substitué ;
un radical cycloaliphatique de 3, 4, 5, 6, 7, 8 ou 9 éléments insaturé ou saturé, éventuellement substitué, qui peut être condensé avec un système cyclique mono- ou polycyclique saturé, insaturé ou aromatique, éventuellement substitué, et/ou relié par un groupe alkylène en C₁₋₅ linéaire ou ramifié ;
ou un radical aryle ou hétéroaryle de 5 à 14 éléments, éventuellement substitué, qui peut être relié par un groupe alkylène en C₁₋₅ linéaire ou ramifié ;
R¹⁰ et R¹¹ représentent chacun, indépendamment l'un de l'autre, un radical alkyle en C₁₋₁₀ linéaire ou ramifié, éventuellement substitué ;
R¹², R¹⁴, R¹⁶, R¹⁰, R²¹, R²², R²⁵ et R²⁷ représentent chacun indépendamment les uns des autres,
un radical hydrogène,
ou un radical alkyle en C₁₋₁₀ linéaire ou ramifié, éventuellement substitué ;
R¹³ représente un radical cycloaliphatique de 3, 4, 5, 6, 7, 8 ou 9 éléments, insaturé ou saturé, éventuellement substitué ;
R¹⁵ et R¹⁷ représentent chacun indépendamment l'un de l'autre un radical aryle ou hétéroaryle de 5 à 14 éléments, éventuellement substitué ;
R¹⁹ représente un radical hydrogène ;
un radical alkyle en C₁₋₁₀ linéaire ou ramifié, éventuellement substitué ;
ou un radical phényle ;
R²⁰ représente un radical hydrogène ;
un radical alkyle en C₁₋₁₀ linéaire ou ramifié, éventuellement substitué ;
ou un radical phényle, qui peut être relié par un groupe alkylène en C₁₋₅ linéaire ou ramifié ;
R²³ représente un radical cycloaliphatique de 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
ou un radical aryle ou hétéroaryle de 5 à 14 éléments éventuellement substitué, qui peut être condensé avec un système cyclique mono- ou polycyclique saturé ou insaturé, éventuellement substitué ;
R²⁴ et R²⁸ représentent chacun indépendamment l'un de l'autre
un radical aryle ou hétéroaryle de 5 à 14 éléments éventuellement substitué, qui peut être condensé avec un système cyclique mono- ou polycyclique saturé ou insaturé, éventuellement substitué ;
et
R²⁶ représente un radical cycloaliphatique de 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué et/ou ponté avec un groupe alkylène en C₁₋₅ linéaire ou ramifié ;
les radicaux alkyle en C₁₋₁₀ mentionnés précédemment pouvant être non substitués ou chacun éventuellement substitués avec 1, 2, 3, 4 ou 5 substituants choisis indépendamment les uns des autres dans le groupe constitué par F, Cl, Br, I, -CN, -NO₂, -OH, -SH et - NH₂ ;
les radicaux alcényle en C₂₋₁₀ mentionnés précédemment pouvant être non substitués ou chacun éventuellement substitués avec 1, 2, 3, 4 ou 5 substituants choisis indépendamment les uns des autres dans le groupe constitué par F, Cl, Br, I, -CN, -NO₂, -OH, -SH et - NH₂ ;
les radicaux alcynyle en C₂₋₁₀ mentionnés précédemment pouvant être non substitués ou chacun éventuellement substitués avec 1, 2, 3, 4 ou 5 substituants choisis indépendamment les uns des autres dans le groupe constitué par F, Cl, Br, I, -CN, -NO₂, -OH, -SH et - NH₂ ;
les radicaux cycloaliphatiques mentionnés précédemment pouvant être non substitués ou chacun éventuellement substitués avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃,-S-CF₃, -SH, -S-alkyle en C₁₋₅, -alkyle en C₁₋₅, -C(=O)-H, -C(=O)-alkyle en C₁₋₅, -C(=O)-perfluoroalkyle en C₁₋₅, - C(=O)-OH, -C(=O)-O-alkyle en C₁₋₅, -(CH₂)-C(=O)-OH,-(CH₂) -C (=O)-O-alkyle en C₁₋₅, -NH-alkyle en C₁₋₅, - N (alkyle en C₁₋₅)₂, -NH-C(=O)-O-alkyle en C₁₋₅, -NH-C(=O)-alkyle en C₁₋₅, -C(=O)-NH₂, -C(=O)-NH-alkyle en C₁₋₅, - C(=O)-N-(alkyle en C₁₋₅)₂, -S(=O)₂-NH₂, -S(=O)₂-NH-alkyle en C₁₋₅, -S(=O)₂-NH-phényle, -S(=O)₂-alkyle en C₁₋₅, - S(=O)₂-phényle, -(CH₂)-benzo[b]furanyle, -0-phényle, -O-benzyle, phényle, benzyle, naphtyle et -(CH₂)-naphtyle, la partie cyclique des radicaux -0-phényle, -S(=O)₂-NH-phényle, -S(=O)₂-phényle, -0-benzyle, phényle, -(CH₂)-benzo[b]furanyle, benzyle, naphtyle et -(CH₂)-naphtyle pouvant à chaque fois être substituée avec 1, 2, 3, 4 ou 5 substituants choisis indépendamment les uns des autres dans le groupe constitué par F, Cl, Br, -OH,-CF₃, -SF₅, -CN, -NO₂, -0-alkyle en C₁₋₅, -alkyle en C₁₋₅, -O-CF₃, -S-CF₃, phényle et -0-benzyle,
et les radicaux cycloaliphatiques mentionnés précédemment pouvant à chaque fois éventuellement comprendre 1, 2, 3, 4 ou 5 hétéroatomes choisis indépendamment les uns des autres dans le groupe constitué par oxygène, azote et soufre ;
et les radicaux aryle ou hétéroaryle mentionnés précédemment pouvant être non substitués ou chacun éventuellement substitués avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅,-NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -(CH₂)-O-C(=O)-phényle, -NH-alkyle en C₁₋₅, -N (alkyle en C₁₋₅)₂,-NH-C(=O)-O-alkyle en C₁₋₅, -NH-C(=O)-alkyle en C₁₋₅,-C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S (=O)₂-NH₂, -S(=O)₂-NH-alkyle en C₁₋₅, -S (=O)₂-NH-phényle, -S(=O)₂-alkyle en C₁₋₅, -S(=O)₂-phényle, -(CH₂)-benzo[b]furanyle, -O-phényle, -S-phényle, -S-benzyle, -0-benzyle, phényle, furanyle, thiényle (thiophényle), pyrazolyle, thiadiazolyle et benzyle, la partie cyclique des radicaux -O-C(=O)-phényle, -(CH₂)-O-C(=O)-phényle, -S(=O)₂-NH-phényle, - S(=O)₂-phényle, -0-phényle, -S-phényle, -S-benzyle, -O-benzyle, phényle, furanyle, thiényle (thiophényle), pyrazolyle, thiadiazolyle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, -alkyle en C₁₋₅, -O-CF₃, -S-CF₃, phényle et -0-benzyle,
et les radicaux hétéroaryle mentionnés précédemment pouvant à chaque fois éventuellement comprendre 1, 2, 3, 4 ou 5 hétéroatomes en tant qu'éléments de cycle, qui sont choisis indépendamment les uns des autres dans le groupe constitué par oxygène, azote et soufre ;
les cycles des systèmes cycliques mono- ou polycycliques mentionnés précédemment pouvant être non substitués ou chacun éventuellement substitués avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -0-alcényle en C₂₋₅, -NH₂, -NO₂, -O-CF₃,-O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -O-C(=O)-phényle, - (CH₂) -0-C(=O)-alkyle en C₁₋₅, -(CH₂) -O-C(=O)-phényle, -NH-alkyle en C₁₋₅, -N (alkyle en C₁₋₅)₂, -NH-C(=O)-O-alkyle en C₁₋₅, - NH-C(=O)-alkyle en C₁₋₅, -C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S(=O)₂-NH₂,-S (=O)₂-NH-alkyle en C₁₋₅, -S (=O)₂-NH-phényle, -S(=O)₂-alkyle en C₁₋₅, -S(=O)₂-phényle, -(CH₂)-benzo[b]furanyle, -0-phényle, -S-phényle, -S-benzyle, -0-benzyle, phényle, furanyle, thiényle (thiophényle), pyrazolyle, thiadiazolyle et benzyle, la partie cyclique des radicaux -O-C(=O)-phényle, - (CH₂)-O-C(=O)-phényle, - S(=O)₂-NH-phényle, -S(=O)₂-phényle, -0-phényle, -S-phényle, -S-benzyle, -0-benzyle, phényle, furanyle, thiényle (thiophényle), pyrazolyle, thiadiazolyle,-(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, - NO₂, -0-alkyle en C₁₋₅, -alkyle en C₁₋₅, -O-CF₃, -S-CF₃, phényle et -0-benzyle,
et les cycles des systèmes cycliques mono- ou polycycliques mentionnés précédemment étant à chaque fois à 5, 6 ou 7 éléments et pouvant à chaque fois éventuellement comprendre 1, 2, 3, 4 ou 5 hétéroatomes en tant qu'éléments de cycle, choisis indépendamment les uns des autres dans le groupe constitué par oxygène, azote et soufre ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme des sels correspondants, ou à chaque fois sous la forme des solvates correspondants.

12. Composés selon la revendication 11, **caractérisés en ce que**
X représente un groupe C(H)(NHR²) ou un groupe NR^{2a},
R¹ représente un groupe -C(=O)-R³ ou un groupe -C(=O)-O-R⁴,
R², R^{2a} représentent chacun, indépendamment l'un de l'autre, un groupe -C(=O)-R⁵ ou un groupe -S(=O)₂-R⁶,
R³ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle, - (CH₂)-(CH₂)-(CH)(CH₃-(CH₂)-(CH₂)-(CH₂)-(CH₃), vinyle, 1-propényle, 2-propényle, 1-butényle, 2-butényle, 3-butényle, -CH=CH-CH=CH-CH₃, -CH₂-O-CH₃, -CH₂-O-CH₂-CH₂-O-CH₃, -CH₂-S-CH₃, - CH₂-S-CH₂-CH₂-S-CH₃, -CH₂-NH-CH₃ et -CH₂-NH-CH₂-CH₃ ;
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -(CH₂)-OC(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-perfluoroalkyle en C₁₋₅, -C(=O)-NH₂, -C (=0) -NH-alkyle en C₁₋₅, -C (=0) -N-(alkyle en C₁₋₅)₂, -S(=O)₂-NH₂, -S(=O)₂-NH-alkyle en C₁₋₅, -S(=O)₂-NH-phényle, -S(=O)₂-alkyle en C₁₋₅, -S(=O)₂-phényle, -0-phényle, -S-phényle, -S-benzyle, -0-benzyle, phényle et benzyle, et la partie cyclique des radicaux -O-C(=O)-phényle, -(CH₂)-O-C(=O)-phényle, - S(=O)₂-NH-phényle, -S(=O)₂-phényle, -0-phényle, -S-phényle, -S-benzyle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
-(CHR¹²)-R¹³, -(CHR¹²)-(CH₂)-R¹³, -(CHR¹²)-O-R¹³, - (CHR¹²)-S-R¹³, -(CHR¹²)-NH-R¹³, -(CHR¹²)-(CH₂)-(CH₂)-R¹³, - (CHR¹²)-(CH₂)-O-R¹³, -(CHR¹²)-(CH₂)-S-R¹³, -(CHR¹²)-(CH₂)-NH-R¹³, -(CHR¹²)-(CH₂)-O-(CH₂)-R¹³, -(CHR¹²)-(CH₂)-S-(CH₂)-R¹³ ou -(CHR¹²)-(CH₂)-NH-R¹³
ou -(CHR¹⁴)-R¹⁵, -(CHR¹⁴)-(CH₂)-R¹⁵ ou -(CHR¹⁴)-(CH₂)-(CH₂)-R¹⁵ ;
R⁴ représente un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment dans le groupe constitué par F, Cl, Br, I, -CN, -CF₃, -SF₅, -OH, -0-alkyle en C₁₋₅, -O-alcényle en C₂₋₅, -NH₂, -NO₂, -0-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -O-C(=O)-phényle, -(CH₂)-O-C(=O)-alkyle en C₁₋₅, -(CH₂)-O-C(=O)-phényle, -NH-alkyle en C₁₋₅, -N (alkyle en C₁₋₅)₂, -NH-C(=O)-O-alkyle en C₁₋₅, -NH-C(=O)-alkyle en C₁₋₅, -C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S (=O)₂-NH₂, -S (=O)₂-NH-alkyle en C₁₋₅, -S(=O)₂-NH-phényle, -S(=O)₂-alkyle en C₁₋₅, -S(=O)₂-phényle, -0-phényle, -S-phényle, -S-benzyle, -0-benzyle, phényle et benzyle, et la partie cyclique des radicaux -O-C(=O)-phényle, -(CH₂)-O-C(=O)-phényle, -S(=O)₂-NH-phényle, - S(=O)₂-phényle, -0-phényle, -S-phényle, -S-benzyle, -O-benzyle, phényle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, -alkyle en C₁₋₅, -O-CF₃, -S-CF₃, phényle et -O-benzyle ;
ou -(CHR¹⁶)-R¹⁷, -(CHR¹⁶)-(CH₂)-R¹⁷ ou -(CHR¹⁶)-(CH₂)-(CH₂)-R¹⁷ ;
R⁵ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle, -(CH₂)-(CH₂)-(CH)(CH₃)-(CH₂)-(CH₂)-(CH₂)-(CH₃), vinyle, 1-propényle, 2-propényle, 1-butényle, 2-butényle, 3-butényle, -CH=CH-CH=CH-CH₃, -CH₂-O-CH₃, -CH₂-O-CH₂-CH₂-O-CH₃, -CH₂-S-CH₃, - CH₂-S-CH₂-CH₂-S-CH₃, -CH₂-NH-CH₃ et -CH₂-NH-CH₂-CH₃ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle et 3,4-dihydro-2H-benzo[1.4]oxazinyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle, oxadiazolyle, benzo[1,2,5]oxadiazolyle, (3,4)-dihydro-2H-benzo[1.4]oxazinyle, (2,3)-dihydrobenzo[1,4]dioxinyle, benzo[1,3]dioxinyle, (2,3)-dihydrobenzofuranyle, 2H-chroményle, chromanyle et (1,2,3,4)-tétrahydroisoquinolinyle, le radical pouvant à chaque fois éventuellement être substitué avec 1, 2, 3, 4 ou 5 substituants choisis indépendamment les uns des autres dans le groupe constitué par F, Cl, Br, I, - CN, -CF₃, -SF₅, -OH, -0-alkyle en C₁₋₅, -O-alcényle en C₂₋₅, -NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -(CH₂)-OC(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-perfluoroalkyle en C₁₋₅, -C(=O)-NH₂, -C (=0) -NH-alkyle en C₁₋₅, -C (=0) -N-(alkyle en C₁₋₅)₂, -S(=O)₂-NH₂, -S(=O)₂-NH-alkyle en C₁₋₅, -S (=O)₂-NH-phényle, -S(=O)₂-alkyle en C₁₋₅, -S(=O)₂-phényle, -0-phényle, -S-phényle, -S-benzyle, -0-benzyle, phényle, pyrazolyle, thiényle, furanyle, thiadiazolyle et benzyle, et la partie cyclique des radicaux -O-C(=O)-phényle, -(CH₂)-O-C(=O)-phényle, - S(=O)₂-NH-phényle, -S(=O)₂-phényle, -0-phényle, -S-phényle, -S-benzyle, -0-benzyle, phényle, pyrazolyle, thiényle, furanyle, thiadiazolyle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
-C(=O)-R⁷, -(CHR¹⁸)-C(=O)-R⁷, -(CHR¹⁸)-(CH₂)-C(=O)-R⁷ ou -(CHR¹⁸)-(CH₂)-(CH₂)-C(=O)-R⁷ ;
-C(=O)-O-R⁸, -(CHR¹⁹)-C(=O)-O-R⁸, -(CHR¹⁹)-(CH₂)-C (=O)-O-R⁸ ou -(CHR¹⁹)-(CH₂)-(CH₂)-C (=O)-O-R⁸ ;
-(CHR²⁰)-NH-C (=O)-O-R⁹, -(CHR²⁰)-(CH₂)-NH-C (=O)-O-R⁹, - (CHR²⁰)-NH-C(=O)-(CH₂)-NH-C(=O)-O-R⁹, -(CHR²⁰)-(CH₂)-(CH₂)-NH-C(=O)-O-R⁹, -(CHR²⁰)-(CH₂)-(CH₂)-(CH₂)-NH-C(=O)-0-R⁹, -(CHR²⁰)-(CH₂)-(CH₂)-(CH₂)-NH-C (=O)-O-R⁹ ou -(CHR²⁰)-(CH₂)-(CH₂)-(CH₂)-(CH₂)-NH-C (=O)-O-R⁹ ;
-(CR²¹R²²)-R²³, -(CR²¹R²²)-(CH₂)-R²³, -(CR²¹R²²)-O-R²³, - (CR²¹R²²)-S-R²³, -(CR²¹R²²)-NH-R²³, -(CR²¹R²²)-(CH₂)-(CH₂)-R²³, -(CR²¹R²²)-O-(CH₂)-R²³, -(CR²¹R²²)-S-(CH₂)-R²³, - (CR²¹R²²)-NH-(CH₂)-R²³, -(CR²¹R²²)-(CH²)-(CH₂)-O-R²³, - (CR²¹R²²)-(CH₂)-(CH₂)-NH-R²³, -(CR²¹R²²)-(CH₂)-(CH₂)-S-R²³ ou - (CR²¹R²²)-(CH²)-(CH²)-(CH₂)-R²³ ;
ou -(CH=CH)-R²⁴ ;
R⁶ représente un groupe -NR¹⁰R¹¹ ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, - (CH₂)-(CH₂)-(C(CH₃)₃), n-heptyle, 3-heptyle, 4-heptyle, n-octyle, -(CH₂)-(CH₂)-(CH) (CH₃)-(CH₂)-(CH₂)-(CH₂)-(CH₃), vinyle, 1-propényle, 2-propényle, 1-butényle, 2-butényle, 3-butényle, -CH=CH-CH=CH-CH₃, -CH₂-O-CH₃, -CH₂-O-CH₂-CH₂-O-CH₃, -CH₂-S-CH₃, -CH₂-S-CH₂-CH₂-S-CH₃, -CH₂-NH-CH₃ et -CH₂-NH-CH₂-CH₃ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle, 3,4-dihydro-2H-benzo[1.4]oxazinyle et bicyclo[2.2.1]heptyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N(alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle, oxadiazolyle, benzo[1,2,5]oxadiazolyle, (3,4)-dihydro-2H-benzo[1.4]oxazinyle, (2,3)- dihydrobenzo[1,4]dioxinyle, benzo[1,3]dioxinyle, (2,3)-dihydrobenzofuranyle, 2H-chroményle, chromanyle et (1,2,3,4)-tétrahydroisoquinolinyle, le radical pouvant à chaque fois éventuellement être substitué avec 1, 2, 3, 4 ou 5 substituants choisis indépendamment les uns des autres dans le groupe constitué par F, Cl, Br, I, - CN, -CF₃, -SF₅, -OH, -0-alkyle en C₁₋₅, -O-alcényle en C₂₋₅, -NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -(CH₂)-O-C(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S (=O)₂-NH₂, -S (=O)₂-NH-alkyle en C₁₋₅, -S(=O)₂-NH-phényle, -S(=O)₂-alkyle en C₁₋₅, -S(=O)₂-phényle, -0-phényle, -S-phényle, -S-benzyle, -0-benzyle, phényle et benzyle, et la partie cyclique des radicaux -O-C(=O)-phényle, - (CH₂)-O-C(=O)-phényle, - S(=O)₂-NH-phényle, -S(=O)₂-phényle, -0-phényle, -S-phényle, -S-benzyle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
- (CHR²⁵)-R²⁶, -(CHR²⁵)-(CH₂)-R²¹, -(CHR²⁵)-O-R²⁶, - (CHR²⁵)-S-R²⁶, -(CHR²⁵)-NH-R²⁶, -(CHR²⁵)-(CH₂)-(CH₂)-R²⁶, - (CHR²⁵)-O-(CH₂)-R²⁶, -(CHR²⁵)-S-(CH₂)-R²⁶, -(CHR²⁵)-NH-(CH₂)-R²⁶, -(CHR²⁵)-(CH₂)-(CH₂)-O-R²⁶, -(CHR²⁵)-(CH₂)-(CH₂)-NH-R²⁶ ou -(CHR²⁵)-(CH₂)-(CH₂)-S-R²⁶ ;
ou -(CHR²⁷)-R²⁸, -(CHR²⁷)-(CH₂)-R²⁸ ou -(CHR²⁷)-(CH₂)-(CH₂)-R²⁸ ;
R⁷ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle et -(CH₂)-(CH₂)-(CH)(CH₃)-(CH₂)-(CH₂)-(CH₂)-(CH₃) ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle et 3,4-dihydro-2H-benzo[1.4]oxazinyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N(alkyle en C₁₋₅)₂, -0-phényle, -O-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -(CH₂)-OC(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -0-phényle, -S-phényle, -S-benzyle, - O-benzyle, phényle et benzyle, et la partie cyclique des radicaux -O-C(=O)-phényle, -(CH₂)-O-C(=O)-phényle, - 0-phényle, -S-phényle, -S-benzyle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, -alkyle en C₁₋₅, -O-CF₃, -S-CF₃, phényle et -O-benzyle ;
ou un radical -NR^{7a}R^{7b}, R^{7a} et R^{7b} représentant chacun indépendamment l'un de l'autre un radical choisi dans le groupe constitué par méthyle, éthyle, n-propyle, isopropyle, n-butyle, sec-butyle, isobutyle, tert-butyle et n-pentyle ;
R⁸ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle et -(CH₂)-(CH₂)-(CH)(CH₃)-(CH₂)-(CH₂)-(CH₂)-(CH₃) ;
ou un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C(=O)-phényle, -(CH₂)-O-C(=O)-alkyle en C₁₋₅, -NH-alkyle en C₁₋₅, -N (alkyle en C₁₋₅)₂, -NH-C(=O)-O-alkyle en C₁₋₅, - NH-C(=O)-alkyle en C₁₋₅, -C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S(=O)₂-NH₂ et -S(=O)₂-NH-alkyle en C₁₋₅ et/ou relié par un groupe - (CH₂)-, -(CH₂)₂- ou -(CH₂)₃- ;
R⁹ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle et - (CH₂)-(CH₂)-(CH)(CH₃)-(CH₂)-(CH₂)-(CH₂)-(CH₃) ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle, 3,4-dihydro-2H-benzo[1.4]oxazinyle et 9H-fluorényle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O) - perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle et/ou reliée par un groupe -(CH₂)-, -(CH₂)₂- ou -(CH₂)₃- ;
ou un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C (=O)-phényle, - (CH₂)-O-C(=O)-alkyle en C₁₋₅, -NH-alkyle en C₁₋₅, -N (alkyle en C₁₋₅)₂, -NH-C(=O)-O-alkyle en C₁₋₅, - NH-C(=O)-alkyle en C₁₋₅, -C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S(=O)₂-NH₂ et -S(=O)₂-NH-alkyle en C₁₋₅ et/ou relié par un groupe - (CH₂)-, -(CH₂)₂- ou -(CH₂)₃- ;
R¹⁰ et R¹¹ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, - (CH₂)-(CH₂)-(C(CH₃)₃), n-heptyle, 3-heptyle, 4-heptyle, n-octyle et - (CH₂)-(CH₂)-(CH)(CH₃)-(CH₂)-(CH₂)-(CH₂)-(CH₃) ;
R¹², R¹⁴, R¹⁶, R¹⁸, R²¹, R²², R²⁵ et R²⁷ représentent chacun indépendamment les uns des autres
un radical hydrogène,
ou un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, - (CH₂)-(CH₂)-(C(CH₃)₃), n-heptyle, 3-heptyle, 4-heptyle, n-octyle et - (CH₂)-(CH₂)-(CH)(CH₃)-(CH₂)-(CH₂)-(CH₂)-(CH₃) ;
R¹³ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, - S-CF₃, -SH, -S-alkyle en C₁₋₅, -alkyle en C₁₋₅, -C(=O)-H, -C(=O)-alkyle en C₁₋₅, -C(=O)-perfluoroalkyle en C₁₋₅, - C(=O)-OH, -C(=O)-O-alkyle en C₁₋₅, -(CH₂)-C(=O)-OH, - (CH₂)-C(=O)-O-alkyle en C₁₋₅, -NH-alkyle en C₁₋₅, - N (alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, - NO₂, -0-alkyle en C₁₋₅, -alkyle en C₁₋₅, -O-CF₃, -S-CF₃, phényle et -O-benzyle ;
R¹⁵ et R¹⁷ représentent chacun indépendamment les uns des autres
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -(CH₂)-O-C(=O)-alkyle en C₁₋₅, -NH-alkyle en C₁₋₅, -N(alkyle en C₁₋₅)₂, -NH-C(=O)-O-alkyle en C₁₋₅, -NH-C(=O)-alkyle en C₁₋₅, -C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, *-*S(=O)₂-NH₂, -S(=O)₂-NH-alkyle en C₁₋₅, -S(=O)₂-alkyle en C₁₋₅, -0-phényle, -0-benzyle, phényle et benzyle, et la partie cyclique des radicaux 0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃ et -S-CF₃ ;
R¹⁹ représente un radical hydrogène ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, - (CH₂)-(CH₂)-(C(CH₃)₃), n-heptyle, 3-heptyle, 4-heptyle, n-octyle et - (CH₂)-(CH₂)-(CH)(CH₃)-(CH₂)-(CH₂)-(CH₂)-(CH₃) ;
ou un radical phényle ;
R²⁰ représente un radical hydrogène ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, - (CH₂)-(CH₂)-(C(CH₃)₃), n-heptyle, 3-heptyle, 4-heptyle, n-octyle et -(CH₂)-(CH₂)-(CH)(CH₃)-(CH₂)-(CH₂)-(CH₂)-(CH₃) ;
ou un radical phényle, qui peut être relié par un groupe -(CH₂)- ou -(CH₂)₂- ;
R²³ représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle et 3,4-dihydro-2H-benzo[1.4]oxazinyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -0-phényle, -0-benzyle, phényle et benzyle, et la partie cyclique des radicaux -0-phényle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle, oxadiazolyle, benzo[1,2,5]oxadiazolyle, (3,4)-dihydro-2H-benzo[1.4]oxazinyle, (2,3)-dihydrobenzo[1,4]dioxinyle, benzo[1,3]dioxinyle, (2,3)-dihydrobenzofuranyle, 2H-chroményle, chromanyle et (1,2,3,4)-tétrahydroisoquinolinyle, le radical pouvant à chaque fois éventuellement être substitué avec 1, 2, 3, 4 ou 5 substituants choisis indépendamment les uns des autres dans le groupe constitué par F, Cl, Br, I, - CN, -CF₃, -SF₅, -OH, -0-alkyle en C₁₋₅, -O-alcényle en C₂₋₅, -NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -0-C (=O)-phényle, - (CH₂)-O-C(=O)-alkyle en C₁₋₅, - (CH₂)-0-C(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S(=O)₂-NH₂, -S(=O)₂-NH-alkyle en C₁₋₅, -S(=O)₂-NH-phényle, -S(=O)₂-alkyle en C₁₋₅, -S(=O)₂-phényle, -0-phényle, -S-phényle, -S-benzyle, -0-benzyle, phényle et benzyle, et la partie cyclique des radicaux -O-C(=O)-phényle, - (CH₂)-O-C(=O)-phényle, - S(=O)₂-NH-phényle, -S(=O)₂-phényle, -0-phényle, -S-phényle, -S-benzyle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
R²⁴ et R²⁸ représentent chacun indépendamment l'un de l'autre
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle, oxadiazolyle, benzo[1,2,5]oxadiazolyle, (3,4)-dihydro-2H-benzo[1.4]oxazinyle, (2,3)-dihydrobenzo[1,4]dioxinyle, benzo[1,3]dioxinyle, (2,3)-dihydrobenzofuranyle, 2H-chroményle, chromanyle et (1,2,3,4)-tétrahydroisoquinolinyle, le radical pouvant à chaque fois éventuellement être substitué avec 1, 2, 3, 4 ou 5 substituants choisis indépendamment les uns des autres dans le groupe constitué par F, Cl, Br, I, - CN, -CF₃, -SF₅, -OH, -0-alkyle en C₁₋₅, -O-alcényle en C₂₋₅, -NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -O-C (=O)-phényle, - (CH₂)-O-C(=O)-alkyle en C₁₋₅, - (CH₂)-0-C(=O)-phényle, -NH-alkyle en C₁₋₅, -N (alkyle en C₁₋₅)₂, - NH-C(=O)-O-alkyle en C₁₋₅, -NH-C(=O)-alkyle en C₁₋₅,-C(=O)-H, -C(=O)-alkyle en C₁₋₅, -C(=O)-perfluoroalkyle en C₁₋₅, -C(=O)-NH₂, -C(=O)-NH-alkyle en C₁₋₅, -C(=O)-N-(alkyle en C₁₋₅)₂, -S(=O)₂-NH₂, -S(=O)₂-NH-alkyle en C₁₋₅, -S(=O)₂-NH-phényle, -S(=O)₂-alkyle en C₁₋₅, -S(=O)₂-phényle, -0-phényle, -S-phényle, -S-benzyle, -0-benzyle, phényle et benzyle, et la partie cyclique des radicaux -O-C(=O)-phényle, - (CH₂)-O-C(=O)-phényle, - S(=O)₂-NH-phényle, -S(=O)₂-phényle, -0-phényle, -S-phényle, -S-benzyle, -0-benzyle, phényle et benzyle pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -O-CF₃, -S-CF₃, phényle et -O-benzyle ;
et
R²⁶ représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (1,2,3,4)-tétrahydronaphtyle, (1,3)-dihydroisoindolyle, (2,3)-dihydroindolyle, 3,4-dihydro-2H-benzo[1.4]oxazinyle et bicyclo[2.2.1]heptyle, le radical (hétéro)cycloaliphatique pouvant à chaque fois éventuellement être substitué avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, - alkyle en C₁₋₅, -C(=O)-H, -C(=O)-alkyle en C₁₋₅, -C(=O)-perfluoroalkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N(alkyle en C₁₋₅)₂, -0-phényle, -O-benzyle, phényle et benzyle, et la partie cyclique des radicaux -0-phényle, -0-benzyle, phényle et benzyle, pouvant à chaque fois être substituée avec 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, -alkyle en C₁₋₅, -0-CF3, -S-CF₃, phényle et -0-benzyle.

13. Composés selon la revendication 11 ou 12, **caractérisés en ce que**
X représente un groupe C(H)(NHR²) ou un groupe NR^{2a},
R¹ représente un groupe -C(=O)-R³ ou un groupe -C(=O) - O-R⁴,
R², R^{2a} représentent chacun indépendamment l'un de l'autre un groupe -C(=O)-R⁵ ou un groupe -S(=O)₂-R⁶,
R³ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle et n-heptyle ;
un radical (hétéro)cycloaliphatique choisi dans le groupe constitué par cyclopentyle, cyclohexyle, cycloheptyle, tétrahydrofuranyle, pyrrolidinyle, pipéridinyle, morpholinyle, pipérazinyle, azépanyle et diazépanyle ;
un radical choisi dans le groupe constitué par phényle, naphtyle, thiophényle, furanyle, pyrrolyle, pyrazolyle, pyrazinyle, triazolyle, pyridinyle, imidazolyle, indolyle, isoindolyle, benzo[b]furanyle, benzo[b]thiophényle, thiazolyle, thiadiazolyle, oxazolyle, isoxazolyle, pyridazinyle, pyrazinyle, pyrimidinyle, indazolyle, quinazolinyle, quinolinyle, isoquinolinyle, phénazinyle, phénothiazinyle et oxadiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, méthoxy, éthoxy, n-propoxy, isopropoxy, n-butoxy, isobutoxy, tert-butoxy, méthyle, éthyle, n-propyle, isopropyle, n-butyle, isobutyle, sec-butyle, tert-butyle et n-pentyle ;
-(CHR¹²)-R¹³, -(CHR¹²)-(CH²)-R¹³ ou -(CHR¹²)-(CH₂)-(CH₂)-R¹³ ;
ou -(CHR¹⁴)-R¹⁵, -(CHR¹⁴)-(CH²)-R¹⁵ ou -(CHR¹⁴)-(CH₂)-(CH₂)-R¹⁵ ;
R⁴ représente -(CHR¹⁶)-R¹⁷ ou -(CHR¹⁶)-(CH₂)-R¹⁷ ;
R⁵ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle, 3-hexyle, -(CH₂)-(CH₂)-(C(CH₃)₃), n-heptyle, 3-heptyle, 4-heptyle, n-octyle, -(CH₂)-(CH₂)-(CH)(CH₃)-(CH₂)-(CH₂)-(CH₂)-(CH₃), vinyle, 1-propényle, 2-propényle, 1-butényle, 2-butényle, 3-butényle, -CH=CH-CH=CH-CH₃, -CH₂-O-CH₃ et -CH₂-O-CH₂-CH₂-O-CH₃ ;
un radical choisi dans le groupe constitué par cyclopropyle, cyclobutyle, cyclopentyle, cyclohexyle, cycloheptyle, tétrahydrofuranyle, pipéridinyle, pipérazinyle et (1,2,3,4)-tétrahydronaphtyle, le radical (hétéro)cycloaliphatique pouvant à chaque fois éventuellement être substitué avec 1, 2, 3, 4 ou 5 substituants choisis indépendamment les uns des autres dans le groupe constitué par -C(=O)-O-CH₃, - C(=O)-O-C₂H₅, -C(=O)-O-C(CH₃)₃ et phényle, la partie cyclique du radical phényle pouvant à chaque fois être substituée avec 1, 2, 3, 4 ou 5 substituants choisis indépendamment les uns des autres dans le groupe constitué par F, Cl et Br ;
un radical choisi dans le groupe constitué par phényle, naphtyle, thiophényle, furanyle, pyrrolyle, pyrazolyle, pyrazinyle, triazolyle, pyridinyle, indolyle, benzo[b]thiophényle, thiazolyle, thiadiazolyle, oxazolyle, isoxazolyle, quinolinyle, isoquinolinyle, benzo[1,3]dioxinyle, (2,3)-dihydrobenzofuranyle, benzo[1,2,5]oxadiazolyle, 2H-chroményle et chromanyle, le radical pouvant à chaque fois éventuellement être substitué avec 1, 2, 3, 4 ou 5 substituants choisis indépendamment les uns des autres dans le groupe constitué par F, Cl, Br, -CN, - CF₃, méthoxy, éthoxy, n-propoxy, isopropoxy, n-butoxy, isobutoxy, tert-butoxy, -NO₂, -O-CF₃, -O-CHF₂, -O-CH₂F, - S-CF₃, -S-CHF₂, -S-CH₂F, -S-CH₃, -S-C₂H₅, -S-n-C₃H₇, -S-C(CH₃)₃, méthyle, éthyle, n-propyle, isopropyle, n-butyle, isobutyle, sec-butyle, tert-butyle, n-pentyle, -O-C(=O)-phényle, - (CH₂)-O-C(=O)-phényle, -N(CH₃)₂, - N(C₂H₅)₂, -S(=O)₂-phényle, -0-phényle, phényle, furanyle, thiényle, pyrazolyle, thiadiazolyle et benzyle, la partie cyclique des radicaux -O-C(=O)-phényle, -(CH₂)-OC(=O)-phényle, furanyle, thiényle, pyrazolyle, thiadiazolyle, -S(=O)₂-phényle, -0-phényle, phényle et benzyle pouvant à chaque fois être substituée avec 1, 2, 3, 4 ou 5 substituants choisis indépendamment les uns des autres dans le groupe constitué par F, Cl et Br ;
C(=O)-R⁷, -(CHR¹⁸)-C(=O)R⁷, -(CHR¹⁸)-(CH₂)-C(=O)-R⁷ ou -(CHR¹⁸)-(CH₂)-(CH₂)-C(=O)-R⁷ ;
-C (=O)-O-R⁸, -(CHR¹⁹)-C (=O)-O-R⁸, -(CHR¹⁹)-(CH₂)-C (=O)-O-R⁸ ou - (CHR¹⁹)-(CH₂)-(CH₂)-C (=O)-O-R⁸ ;
-(CHR²⁰)-NH-C (=O)-O-R⁹, -(CHR²⁰)-(CH₂)-NH-C (=O)-O-R⁹, -(CHR²⁰)-NH-C(=O)-(CH₂)-NH-C (=O)-O-R⁹, -(CHR²⁰)-(CH₂)-(CH₂)-NH-C(=O)-O-R⁹, -(CHR²⁰)-(CH₂)-(CH₂)-(CH₂)-NH-C(=O)-OR⁹, -(CHR²⁰)-(CH₂)-(CH₂)-(CH₂)-NH-C (=O)-O-R⁹ ou -(CHR²⁰)-(CH₂)-(CH₂)-(CH₂)-(CH₂)-NH-C (=O)-O-R⁹ ;
-(CR²¹R²²)-R²³, -(CR²¹R²²)-(CH₂)-R²³, -(CR²¹R²²)-O-R²³, - (CR²¹R²²)-S-R²³, -(CR²¹R²²)-(CH₂)-(CH₂)-R²³, -(CR²¹R²²)-O-(CH₂)-R²³, -(CR²¹R²²)-S-(CH₂)-R²³, -(CR²¹R²²)-(CH₂)-(CH₂)-OR²³, -(CR²¹R²²)-(CH₂)-(CH₂)-S-R²³ ou -(CR²¹R²²)-(CH₂)-(CH₂)-(CH₂)-R²³ ;
ou -(CH=CH)-R²⁴ ;
R⁶ représente un groupe -NR¹⁰R¹¹ ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
un radical choisi dans le groupe constitué par phényle, naphtyle, thiophényle, furanyle, pyrazolyle, thiazolyle, (3,4)-dihydro-2H-benzo[1.4]oxazinyle, (2,3)-dihydrobenzo[1,4]dioxinyle et (1,2,3,4)-tétrahydroisoquinolinyle, le radical pouvant à chaque fois éventuellement être substitué avec 1, 2, 3, 4 ou 5 substituants choisis indépendamment les uns des autres dans le groupe constitué par F, Cl, Br, -CN, - CF₃, méthoxy, éthoxy, n-propoxy, isopropoxy, n-butoxy, isobutoxy, tert-butoxy, -NO₂, -O-CF₃, -O-CHF₂, -O-CH₂F, méthyle, éthyle, n-propyle, isopropyle, n-butyle, isobutyle, sec-butyle, tert-butyle, n-pentyle, -C(CH₃)₂-CH₂-CH₃, -C(=O)-O-CH₃, -C(=O)-O-C₂H₅, -C(=O)-O-C(CH₃)₃, - NH-C(=O)-CH₃, -NH-C(=O)-C₂H₅, -NH-C(=O)-C(CH₃)₃, -C(=O)-CF₃, -C (=O)-C₂F₅, -S(=O)₂-CH₃, -S(=O)₂-C₂H₅, -S(=O)-C(CH₃)₃, phényle et benzyle ;
-(CHR²⁵)-R²⁶ ou -(CHR²⁵)-(CH₂)-R²⁶ ;
ou -(CHR²⁷)-R²⁸ ou -(CHR²⁷)-(CH₂)-R²⁸ ;
R⁷ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
un radical (2,3)-dihydroindolyle ;
un radical choisi dans le groupe constitué par phényle, naphtyle et indolyle ;
ou un radical -NR^{7a}R^{7b}, R^{7a} et R^{7b} représentant chacun indépendamment l'un de l'autre un radical choisi dans le groupe constitué par méthyle, éthyle, n-propyle, isopropyle, n-butyle, sec-butyle, isobutyle, tert-butyle et n-pentyle ;
R⁸ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle et n-hexyle ;
ou un radical phényle, qui peut être relié par un groupe -(CH₂)- ;
R⁹ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
un radical 9H-fluorényle, qui peut être relié par un groupe -(CH₂)- ;
ou un radical phényle, qui peut être relié par un groupe -(CH₂)- ;
R¹⁰ et R¹¹ représentent chacun indépendamment l'un de l'autre un radical choisi dans le groupe constitué par méthyle, éthyle, n-propyle, isopropyle, n-butyle, sec-butyle, isobutyle et tert-butyle ;
R¹², R¹⁴, R¹⁶, R¹⁸, R²¹, R²², R²⁵ et R²⁷ représentent chacun indépendamment les uns des autres
un radical hydrogène,
ou un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
R¹³ représente un radical (hétéro)cycloaliphatique choisi dans le groupe constitué par cyclopropyle, cyclobutyle, cyclopentyle, cyclohexyle, cycloheptyle, pyrrolidinyle, pipéridinyle, morpholinyle et pipérazinyle ;
R¹⁵ et R¹⁷ représentent chacun indépendamment l'un de l'autre
un radical phényle ;
R¹⁹ représente un radical hydrogène ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
ou un radical phényle ;
R²⁰ représente un radical hydrogène ;
un radical choisi dans le groupe constitué par méthyle, éthyle, n-propyle, isopropyle, n-butyle, sec-butyle, isobutyle, tert-butyle, n-pentyle, 2-pentyle, 3-pentyle, néo-pentyle, n-hexyle, 2-hexyle et 3-hexyle ;
ou un radical phényle, qui peut être relié par un groupe -(CH₂)- ;
R²³ représente un radical choisi dans le groupe constitué par cyclopropyle, cyclobutyle, cyclopentyle, cyclohexyle, cycloheptyle, pyrrolidinyle, pipéridinyle, morpholinyle, pipérazinyle, dithiolanyle, isoindol-1,3-dionyle, benzoxazolin-2-onyle et 2,5-dioxo-imidazolidinyle, le radical pouvant à chaque fois être substitué avec 1, 2 ou 3 substituants choisis indépendamment les uns des autres dans le groupe constitué par méthyle, éthyle, n-propyle, isopropyle, n-butyle, isobutyle, sec-butyle, tert-butyle et n-pentyle ;
un radical choisi dans le groupe constitué par phényle, naphtyle, thiophényle, furanyle et thiazolyle, le radical pouvant à chaque fois éventuellement être substitué avec 1, 2, 3, 4 ou 5 substituants choisis indépendamment les uns des autres dans le groupe constitué par F, Cl, Br, -CN, -CF₃, -OH, méthoxy, éthoxy, n-propoxy, isopropoxy, n-butoxy, isobutoxy, tert-butoxy, méthyle, éthyle, n-propyle, isopropyle, n-butyle, isobutyle, sec-butyle, tert-butyle, n-pentyle, -O-C(=O)-phényle, phényle et benzyle, la partie cyclique des radicaux -O-C(=O)-phényle, phényle et benzyle pouvant à chaque fois être substituée avec 1, 2, 3, 4 ou 5 substituants choisis indépendamment les uns des autres dans le groupe constitué par F, Cl et Br ;
R²⁴ et R²⁸ représentent chacun indépendamment l'un de l'autre
un radical phényle, le radical pouvant à chaque fois être substitué avec 1, 2 ou 3 substituants choisis indépendamment les uns des autres dans le groupe constitué par F, Cl, Br et -CF₃ ;
et
R²⁶ représente un radical 7,7-diméthyl-2-oxo-bicyclo[2.2.1]hept-1-yle,
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme des sels correspondants, ou à chaque fois sous la forme des solvates correspondants.

14. Composés selon une ou plusieurs des revendications 1 à 13, choisis dans le groupe constitué par
[1] 3-fluoro-N-[6-(4-fluoro-benzoyl)-5,6,7,8-tétrahydro-pyrido[4,3-d]pyrimidin-2-yl]-benzamide,
[2] 3,5-dichloro-N-[6-(3-fluoro-4-méthoxy-benzoyl)-5,6,7,8-tétrahydro-pyrido[4,3-d]pyrimidin-2-yl]-benzamide,
[3] 4-tert-butyl-N-(6-hexanoyl-5,6,7,8-tétrahydro-pyrido[4,3-d]pyrimidin-2-yl)-benzamide,
[4] N-(6-acétyl-5,6,7,8-tétrahydro-pyrido[4,3-d]pyrimidin-2-yl)-3,4-dichloro-benzamide,
[5] 3,5-dichloro-N-[6-(3-trifluorométhyl-benzoyl)-5,6,7,8-tétrahydro-pyrido[4,3-d]pyrimidin-2-yl]-benzamide,
[6] 3-chloro-N-[6-(4-méthoxy-2,3,6-triméthylbenzènesulfonyl)-5,6,7,8-tétrahydro-pyrido[4,3-d]pyrimidin-2-yl]-benzamide,
[7] N-[6-(2-éthoxy-benzoyl)-5,6,7,8-tétrahydro-pyrido[4,3-d]pyrimidin-2-yl]-3-fluoro-benzamide,
[8] 3-chloro-N-[6-(3-phényl-propionyl)-5,6,7,8-tétrahydro-pyrido[4,3-d]pyrimidin-2-yl]-benzamide,
[9] 4-tert-butyl-N-[6-(isoxazol-5-carbonyl)-5,6,7,8-tétrahydro-pyrido[4,3-d]pyrimidin-2-yl]-benzamide,
[10] N-[6-(2-benzylsulfanyl-acétyl)-5,6,7,8-tétrahydro-pyrido[4,3-d]pyrimidin-2-yl]-2-méthoxy-benzamide,
[11] {6-[2-(4-chloro-phényl)-2-méthyl-propionyl]-5,6,7,8-tétrahydro-pyrido[4,3-d]pyrimidin-2-yl}-amide de l'acide thiophène-2-carboxylique,
[12] N-(6-benzoyl-5,6,7,8-tétrahydro-pyrido[4,3-d]pyrimidin-2-yl)-3-méthyl-benzamide,
[13] N-[6-(2,3-dihydro-benzofurane-5-carbonyl)-5,6,7,8-tétrahydro-pyrido[4,3-d]pyrimidin-2-yl]-2-fluoro-benzamide,
[14] [6-(3,4,5-triméthoxy-benzoylamino)-5,6,7,8-tétrahydro-quinazolin-2-yl]-amide de l'acide thiophène-2-carboxylique,
[15] [6-(3-méthyl-5-phényl-isoxazol-4-carbonyl)-5,6,7,8-tétrahydro-pyrido[4,3-d]pyrimidin-2-yl]-amide de l'acide naphtaline-1-carboxylique,
[16] 3-chloro-N-{6-[2-(5-méthyl-2-phényl-thiazol-4-yl)-acétyl]-5,6,7,8-tétrahydro-pyrido[4,3-d]pyrimidin-2-yl}-benzamide,
[17] N-[6-(4-chloro-2,5-diméthyl-benzènesulfonyl)-5,6,7,8-tétrahydro-pyrido[4,3-d]pyrimidin-2-yl]-2-trifluorométhyl-benzamide,
[18] N-[6-(1-benzènesulfonyl-1H-indol-2-carbonyl)-5,6,7,8-tétrahydro-pyrido[4,3-d]pyrimidin-2-yl]-3-méthoxy-benzamide,
[20] N-[6-(5-méthyl-1-phényl-1H-pyrazol-4-sulfonyl)-5,6,7,8-tétrahydro-pyrido[4,3-d]pyrimidin-2-yl]-2-trifluorométhyl-benzamide,
[21] N-[6-(3-chloro-2-méthyl-benzènesulfonyl)-5,6,7,8-tétrahydro-pyrido[4,3-d]pyrimidin-2-yl]-3-fluoro-benzamide,
[22] 3,5-dichloro-N-[6-(3,5-diméthyl-isoxazol-4-carbonyl)-5,6,7,8-tétrahydro-pyrido[4,3-d]pyrimidin-2-yl]-benzamide,
[23] [6-(4-trifluorométhoxy-benzènesulfonylamino)-5,6,7,8-tétrahydro-quinazolin-2-yl]-amide de l'acide thiophène-2-carboxylique,
[25] N-[6-(furane-2-carbonyl)-5,6,7,8-tétrahydro-pyrido[4,3-d]pyrimidin-2-yl]-3-méthoxy-benzamide,
[26] N-{6-[4-(2,3-dihydro-indol-1-yl)-4-oxo-butyryl]-5,6,7,8-tétrahydro-pyrido[4,3-d]pyrimidin-2-yl}-3-méthyl-benzamide,
[27] N-{6-[2-(4-méthyl-cyclohexyl)-acétyl]-5,6,7,8-tétrahydro-pyrido[4,3-d]pyrimidin-2-yl}-2-trifluorométhyl-benzamide,
[28] 3,4-difluoro-N-[6-(6-phénoxy-pyridine-3-carbonyl)-5,6,7,8-tétrahydro-pyrido[4,3-d]pyrimidin-2-yl]-benzamide,
[29] 4-tert-butyl-N-[6-(2-phényl-thiazol-4-carbonyl)-5,6,7,8-tétrahydro-pyrido[4,3-d]pyrimidin-2-yl]-benzamide,
[30] 3,5-dichloro-N-[6-(2-trifluorométhyl-benzènesulfonyl)-5,6,7,8-tétrahydro-pyrido[4,3-d]pyrimidin-2-yl]-benzamide,
[31] [6-(3-bromo-benzènesulfonylamino)-5,6,7,8-tétrahydro-quinazolin-2-yl]-amide de l'acide thiophène-2-carboxylique,
[32] N-[6-(2-chloro-pyridine-4-carbonyl)-5,6,7,8-tétrahydro-pyrido[4,3-d]pyrimidin-2-yl]-3-méthoxy-benzamide,
[33] 4-fluoro-N-[6-(2-méthanesulfonyl-benzènesulfonylamino)-5,6,7,8-tétrahydro-quinazolin-2-yl]-benzamide,
[35] N-(6-diméthylsulfamoyl-5,6,7,8-tétrahydro-pyrido[4,3-d]pyrimidin-2-yl)-3-fluoro-benzamide,
[36] 3-fluoro-N-{6-[2-(4-trifluorométhyl-phényl)-acétyl]-5,6,7,8-tétrahydro-pyrido[4,3-d]pyrimidin-2-yl}-benzamide,
[37] N-{6-[2-(1,3-dioxo-1,3-dihydro-isoindol-2-yl)-acétyl]-5,6,7,8-tétrahydro-pyrido[4,3-d]pyrimidin-2-yl}-2-trifluorométhyl-benzamide,
[38] {6-[4-(4-chloro-2-méthyl-phénoxy)-butyrylamino]-5,6,7,8-tétrahydro-quinazolin-2-yl}-amide de l'acide thiophène-2-carboxylique,
[39] N-[6-(butane-1-sulfonyl)-5,6,7,8-tétrahydro-pyrido[4,3-d]pyrimidin-2-yl]-3-fluoro-benzamide,
[40] 2-méthoxy-N-[6-(2-propyl-pentanoyl)-5,6,7,8-tétrahydro-pyrido[4,3-d]pyrimidin-2-yl]-benzamide,
[41] N-[6-(4,5-dichloro-thiophène-2-sulfonylamino)-5,6,7,8-tétrahydro-quinazolin-2-yl]-4-fluoro-benzamide,
[42] 4-chloro-N-[6-(2-trifluorométhyl-benzènesulfonyl)-5,6,7,8-tétrahydro-pyrido[4,3-d]pyrimidin-2-yl]-benzamide,
[43] [6-(4-méthoxy-benzènesulfonylamino)-5,6,7,8-tétrahydro-quinazolin-2-yl]-amide de l'acide thiophène-2-carboxylique,
[44] ester méthylique de l'acide 5-[2-(3,4-difluoro-benzoylamino)-7,8-dihydro-5H-pyrido[4,3-d]pyrimidin-6-yl]-5-oxo-valérique,
[45] N-[6-(benzo[b]thiophène-3-carbonyl)-5,6,7,8-tétrahydro-pyrido[4,3-d]pyrimidin-2-yl]-4-chloro-benzamide,
[46] N-[6-(5-bromo-2-méthoxy-benzènesulfonylamino)-5,6,7,8-tétrahydro-quinazolin-2-yl]-4-fluoro-benzamide,
[47] 4-fluoro-N-[6-(4-fluoro-benzènesulfonylamino)-5,6,7,8-tétrahydro-quinazolin-2-yl]-benzamide,
[48] N-{6-[2-(1H-indol-3-yl)-2-oxo-acétyl]-5,6,7,8-tétrahydro-pyrido[4,3-d]pyrimidin-2-yl}-2-trifluorométhyl-benzamide,
[49] {2-[(thiophène-2-carbonyl)-amino]-5,6,7,8-tétrahydro-quinazolin-6-yl}-amide de l'acide 5-tert-butyl-2-méthyl-furane-3-carboxylique,
[50] N-[6-(2,5-diméthoxy-benzènesulfonyl)-5,6,7,8-tétrahydro-pyrido[4,3-d]pyrimidin-2-yl]-3-méthoxy-benzamide,
[51] ester 2-{2-[(thiophène-2-carbonyl)-amino]-5,6,7,8-tétrahydro-quinazolin-6-ylcarbamoyl}-benzylique de l'acide benzoïque,
[52] 3,4-difluoro-N-(6-hexanoyl-5,6,7,8-tétrahydro-pyrido[4,3-d]pyrimidin-2-yl)-benzamide,
[53] 4-éthyl-N-[6-(tétrahydro-furane-2-carbonyl)-5,6,7,8-tétrahydro-pyrido[4,3-d]pyrimidin-2-yl]-benzamide,
[54] N-[6-(2-chloro-benzènesulfonyl)-5,6,7,8-tétrahydro-pyrido[4,3-d]pyrimidin-2-yl]-3-fluoro-benzamide,
[55] 4-tert-butyl-N-[6-(5-tert-butyl-2-méthyl-2H-pyrazol-3-carbonyl)-5,6,7,8-tétrahydro-pyrido[4,3-d]pyrimidin-2-yl]-benzamide,
[56] 3-méthoxy-N-[6-(4-méthoxy-benzènesulfonyl)-5,6,7,8-tétrahydro-pyrido[4,3-d]pyrimidin-2-yl]-benzamide,
[57] ester benzylique de l'acide {2-[2-(3,4-difluoro-benzoylamino)-7,8-dihydro-5H-pyrido[4,3-d]pyrimidin-6-yl]-2-oxo-1-phényl-éthyl}-carbamique,
[58] {2-[(thiophène-2-carbonyl)-amino]-5,6,7,8-tétrahydro-quinazolin-6-yl}-amide de l'acide 5-phényl-oxazol-4-carboxylique,
[59] 4-chloro-N-(6-diméthylsulfamoyl-5,6,7,8-tétrahydro-pyrido[4,3-d]pyrimidin-2-yl)-benzamide,
[60] 4-tert-butyl-N-[6-(2-fluoro-benzènesulfonyl)-5,6,7,8-tétrahydro-pyrido[4,3-d]pyrimidin-2-yl]-benzamide,
[61] 4-chloro-N-[6-(3-cyclopentyl-propionyl)-5,6,7,8-tétrahydro-pyrido[4,3-d]pyrimidin-2-yl]-benzamide,
[62] 4-chloro-N-[6-(4-phényl-butyryl)-5,6,7,8-tétrahydro-pyrido[4,3-d]pyrimidin-2-yl]-benzamide,
[63] 4-tert-butyl-N-[6-(5-méthyl-2-phényl-2H-[1,2,3]triazol-4-carbonyl)-5,6,7,8-tétrahydro-pyrido[4,3-d]pyrimidin-2-yl]-benzamide,
[64] 2-chloro-N-{2-[(thiophène-2-carbonyl)-amino]-5,6,7,8-tétrahydro-quinazolin-6-yl}-isonicotinamide, [66] N-[6-(5-chloro-thiophène-2-sulfonyl)-5,6,7,8-tétrahydro-pyrido[4,3-d]pyrimidin-2-yl]-3-fluoro-benzamide,
[67] N-[6-(4-diéthylamino-benzoyl)-5,6,7,8-tétrahydro-pyrido[4,3-d]pyrimidin-2-yl]-3-méthoxy-benzamide,
[68] N-[6-(2,4-diméthyl-thiazol-5-sulfonyl)-5,6,7,8-tétrahydro-pyrido[4,3-d]pyrimidin-2-yl]-3-méthyl-benzamide,
[69] 4-chloro-N-[6-(naphtaline-2-carbonyl)-5,6,7,8-tétrahydro-pyrido[4,3-d]pyrimidin-2-yl]-benzamide,
[70] N-[6-(2,4-difluoro-benzènesulfonyl)-5,6,7,8-tétrahydro-pyrido[4,3-d]pyrimidin-2-yl]-2-méthoxy-benzamide,
[71] 3,4-dichloro-N-[6-(4-chloro-benzènesulfonyl)-5,6,7,8-tétrahydro-pyrido[4,3-d]pyrimidin-2-yl]-benzamide,
[72] 3,4-difluoro-N-[6-(thiophène-2-sulfonyl)-5,6,7,8-tétrahydro-pyrido[4,3-d]pyrimidin-2-yl]-benzamide,
[74] 4-éthyl-N-[6-(4-pyrazol-1-yl-benzoyl)-5,6,7,8-tétrahydro-pyrido[4,3-d]pyrimidin-2-yl]-benzamide,
[75] N-[6-(7,7-diméthyl-2-oxo-bicyclo[2.2.1]hept-1-ylméthanesulfonylamino)-5,6,7,8-tétrahydro-quinazolin-2-yl]-butyramide,
[76] ((2-butyrylamino-5,6,7,8-tétrahydro-quinazolin-6-yl)-amide de l'acide 5-oxo-5-phényl-valérique,
[77] ester méthylique de l'acide 3-[2-(4-chloro-benzoylamino)-7,8-dihydro-5H-pyrido[4,3-d]pyrimidin-6-yl]-3-oxo-propionique,
[78] N-{6-[5-(4-chloro-phényl)-2-méthyl-furane-3-carbonyl]-5,6,7,8-tétrahydro-pyrido[4,3-d]pyrimidin-2-yl}-2-méthoxy-benzamide,
[79] 4-chloro-N-[6-(2,4-diméthyl-thiazol-5-sulfonyl)-5,6,7,8-tétrahydro-pyrido[4,3-d]pyrimidin-2-yl]-benzamide,
[80] N-[6-(2,3-difluoro-benzoyl)-5,6,7,8-tétrahydro-pyrido[4,3-d]pyrimidin-2-yl]-3-méthoxy-benzamide,
[81] 4-chloro-N-{6-[2-(2-méthoxy-éthoxy)-acétyl]-5,6,7,8-tétrahydro-pyrido[4,3-d]pyrimidin-2-yl}-benzamide,
[82] ester méthylique de l'acide 2-[2-(2-éthoxy-benzoylamino)-7,8-dihydro-5H-pyrido[4,3-d]pyrimidine-6-sulfonyl]-benzoïque,
[83] 4-tert-butyl-N-[6-(4-nitro-benzènesulfonyl)-5,6,7,8-tétrahydro-pyrido[4,3-d]pyrimidin-2-yl]-benzamide,
[86] N-[6-(5-chloro-thiophène-2-sulfonyl)-5,6,7,8-tétrahydro-pyrido[4,3-d]pyrimidin-2-yl]-2-méthoxy-benzamide,
[87] 4-fluoro-N-[6-(furane-2-carbonyl)-5,6,7,8-tétrahydro-pyrido[4,3-d]pyrimidin-2-yl]-benzamide,
[88] ester tert-butylique de l'acide 4-{2-[(thiophène-2-carbonyl)-amino]-5,6,7,8-tétrahydro-quinazolin-6-ylcarbamoyl}-pipéridine-1-carboxylique,
[89] ((2-butyrylamino-5,6,7,8-tétrahydro-quinazolin-6-yl)-amide de l'acide 1-(4-chloro-phényl)-cyclopropanecarboxylique,
[90] N-(6-éthanesulfonyl-5,6,7,8-tétrahydro-pyrido[4,3-d]pyrimidin-2-yl)-3-méthoxy-benzamide,
[91] ((2-butyrylamino-5,6,7,8-tétrahydro-quinazolin-6-yl)-amide de l'acide 5-méthyl-thiophène-2-carboxylique,
[92] 4-tert-butyl-N-[6-(4-méthyl-3-nitro-benzoyl)-5,6,7,8-tétrahydro-pyrido[4,3-d]pyrimidin-2-yl]-benzamide,
[94] 4-fluoro-N-[6-(3-phényl-propionylamino)-5,6,7,8-tétrahydro-quinazolin-2-yl]-benzamide,
[95] 3,4-dichloro-N-[6-(2,4,6-triméthyl-benzènesulfonyl)-5,6,7,8-tétrahydro-pyrido[4,3-d]pyrimidin-2-yl]-benzamide,
[96] N-[6-(4-méthyl-3,4-dihydro-2H-benzo[1,4]oxazine-7-sulfonyl)-5,6,7,8-tétrahydro-pyrido[4,3-d]pyrimidin-2-yl]-nicotinamide,
[97] [6-(3,5-difluoro-benzoylamino)-5,6,7,8-tétrahydro-quinazolin-2-yl]-amide de l'acide thiophène-2-carboxylique,
[98] N-[6-(2,4-difluoro-benzènesulfonylamino)-5,6,7,8-tétrahydro-quinazolin-2-yl]-butyramide,
[99] [6-(2,3,5,6-tétraméthyl-benzènesulfonyl)-5,6,7,8-tétrahydro-pyrido[4,3-d]pyrimidin-2-yl]-amide de l'acide thiophène-2-carboxylique,
[101] N-[6-(3,4-dichloro-benzoyl)-5,6,7,8-tétrahydro-pyrido[4,3-d]pyrimidin-2-yl]-4-éthyl-benzamide,
[102] N-[6-(7,7-diméthyl-2-oxo-bicyclo[2.2.1]hept-1-ylméthanesulfonyl)-5,6,7,8-tétrahydro-pyrido[4,3-d]pyrimidin-2-yl]-3-fluoro-benzamide,
[103] N-[6-(6-fluoro-4H-benzo[1,3]dioxine-8-carbonyl)-5,6,7,8-tétrahydro-pyrido[4,3-d]pyrimidin-2-yl]-2-trifluorométhyl-benzamide,
[104] ester benzylique de l'acide [5-(2-butyrylamino-5,6,7,8-tétrahydro-quinazolin-6-ylcarbamoyl)-pentyl]-carbamique,
[105] 2-éthoxy-N-[6-(4-oxo-pentanoyl)-5,6,7,8-tétrahydro-pyrido[4,3-d]pyrimidin-2-yl]-benzamide,
[106] [6-(propane-1-sulfonyl)-5,6,7,8-tétrahydro-pyrido[4,3-d]pyrimidin-2-yl]-amide de l'acide naphtaline-1-carboxylique,
[107] N-[6-(5-fluoro-2-méthyl-benzènesulfonyl)-5,6,7,8-tétrahydro-pyrido[4,3-d]pyrimidin-2-yl]-4-méthyl-benzamide,
[109] 3-chloro-N-[6-(2-méthyl-benzoyl)-5,6,7,8-tétrahydro-pyrido[4,3-d]pyrimidin-2-yl]-benzamide,
[110] [6-(3-chloro-benzo[b]thiophène-2-carbonyl)-5,6,7,8-tétrahydro-pyrido[4,3-d]pyrimidin-2-yl]-amide de l'acide thiophène-2-carboxylique,
[112] [6-(2-cyclopentyl-acétylamino)-5,6,7,8-tétrahydro-quinazolin-2-yl]-amide de l'acide thiophène-2-carboxylique,
[113] N-[6-(3,4-dichloro-benzoyl)-5,6,7,8-tétrahydro-pyrido[4,3-d]pyrimidin-2-yl]-2-fluoro-benzamide,
[114] N-[6-(2-chloro-benzènesulfonyl)-5,6,7,8-tétrahydro-pyrido[4,3-d]pyrimidin-2-yl]-4-éthyl-benzamide,
[115] [6-(4-bromo-3-méthyl-benzoylamino)-5,6,7,8-tétrahydro-quinazolin-2-yl]-amide de l'acide thiophène-2-carboxylique,
[116] 3-méthyl-N-[6-(toluène-3-sulfonyl)-5,6,7,8-tétrahydro-pyrido[4,3-d]pyrimidin-2-yl]-benzamide,
[117] [6-(5-chloro-thiophène-2-sulfonyl)-5,6,7,8-tétrahydro-pyrido[4,3-d]pyrimidin-2-yl]-amide de l'acide naphtaline-1-carboxylique,
[118] N-(2-butyrylamino-5,6,7,8-tétrahydro-quinazolin-6-yl)-2,6-difluoro-benzamide,
[119] 3-chloro-N-[6-(3,4-diméthoxy-benzoyl)-5,6,7,8-tétrahydro-pyrido[4,3-d]pyrimidin-2-yl]-benzamide,
[120] N-[6-(3-chloro-benzo[b]thiophène-2-carbonyl)-5,6,7,8-tétrahydro-pyrido[4,3-d]pyrimidin-2-yl]-benzamide,
[121] 4-éthyl-N-[6-(thiophène-3-sulfonyl)-5,6,7,8-tétrahydro-pyrido[4,3-d]pyrimidin-2-yl]-benzamide,
[123] 2-éthoxy-N-[6-(2-éthylsulfanyl-pyridine-3-carbonyl)-5,6,7,8-tétrahydro-pyrido[4,3-d]pyrimidin-2-yl]-benzamide,
[124] ester éthylique de l'acide 3-[2-(3-chloro-benzoylamino)-7,8-dihydro-5H-pyrido[4,3-d]pyrimidin-6-yl]-3-oxo-propionique,
[125] N-[6-(3-bromo-benzènesulfonyl)-5,6,7,8-tétrahydro-pyrido[4,3-d]pyrimidin-2-yl]-3-chloro-benzamide,
[126] 3,4-dichloro-N-{6-[2-(4-chloro-phényl)-acétyl]-5,6,7,8-tétrahydro-pyrido[4,3-d]pyrimidin-2-yl}-benzamide,
[127] 3-chloro-N-[6-(2-chloro-6-fluoro-benzoyl)-5,6,7,8-tétrahydro-pyrido[4,3-d]pyrimidin-2-yl]-benzamide,
[128] 4-fluoro-N-(6-hexanoylamino-5,6,7,8-tétrahydro-quinazolin-2-yl)-benzamide,
[129] N-[6-(5-bromo-2-méthoxy-benzènesulfonyl)-5,6,7,8-tétrahydro-pyrido[4,3-d]pyrimidin-2-yl]-3-méthoxy-benzamide,
[132] N-[6-(3-cyclopentyl-propionyl)-5,6,7,8-tétrahydro-pyrido[4,3-d]pyrimidin-2-yl]-isonicotinamide,
[133] ((2-butyrylamino-5,6,7,8-tétrahydro-quinazolin-6-yl)-amide de l'acide 5-benzyl-furane-2-carboxylique,
[134] 3,4-dichloro-N-[6-(4-trifluorométhoxy-benzènesulfonyl)-5,6,7,8-tétrahydro-pyrido[4,3-d]pyrimidin-2-yl]-benzamide,
[135] ester benzylique de l'acide [(2-butyrylamino-5,6,7,8-tétrahydro-quinazolin-6-ylcarbamoyl)-phényl-méthyl]-carbamique,
[136] N-[6-(3,4-diméthoxy-benzènesulfonyl)-5,6,7,8-tétrahydro-pyrido[4,3-d]pyrimidin-2-yl]-4-fluoro-benzamide,
[137] 2-fluoro-N-[6-(2-méthyl-5-phényl-furane-3-carbonyl)-5,6,7,8-tétrahydro-pyrido[4,3-d]pyrimidin-2-yl]-benzamide,
[138] ((6-pent-4-énoylamino-5,6,7,8-tétrahydro-quinazolin-2-yl)-amide de l'acide thiophène-2-carboxylique,
[139] ester 9H-fluorén-9-ylméthylique de l'acide (2-méthyl-1-{2-[(thiophène-2-carbonyl)-amino]-5,6,7,8-tétrahydro-quinazolin-6-ylcarbamoyl}-propyl)-carbamique,
[140] ester tert-butylique de l'acide (5-{2-[(thiophène-2-carbonyl)-amino]-5,6,7,8-tétrahydro-quinazolin-6-ylcarbamoyl}-pentyl)-carbamique,
[141] 3-fluoro-N-[6-(1,2,3,4-tétrahydro-naphtaline-2-carbonyl)-5,6,7,8-tétrahydro-pyrido[4,3-d]pyrimidin-2-yl]-benzamide,
[142] 4-chloro-N-{6-[2-(2,5-dioxo-imidazolidin-4-yl)-acétyl]-5,6,7,8-tétrahydro-pyrido[4,3-d]pyrimidin-2-yl}-benzamide,
[143] 3-fluoro-N-[6-(toluène-4-sulfonyl)-5,6,7,8-tétrahydro-pyrido[4,3-d]pyrimidin-2-yl]-benzamide,
[144] 3,5-dichloro-N-[6-(4-thiophène-2-yl-benzoyl)-5,6,7,8-tétrahydro-pyrido[4,3-d]pyrimidin-2-yl]-benzamide,
[145] N-[6-(3-chloro-thiophène-2-carbonyl)-5,6,7,8-tétrahydro-pyrido[4,3-d]pyrimidin-2-yl]-2-éthoxy-benzamide,
[146] N-[6-(toluène-3-sulfonylamino)-5,6,7,8-tétrahydro-quinazolin-2-yl]-butyramide,
[147] {6-[2-(2,2,2-trifluoro-acétyl)-1,2,3,4-tétrahydro-isoquinoline-7-sulfonylamino]-5,6,7,8-tétrahydro-quinazolin-2-yl}-amide de l'acide thiophène-2-carboxylique,
[148] 4-méthyl-N-[6-(4-trifluorométhylsulfanyl-benzoyl)-5,6,7,8-tétrahydro-pyrido[4,3-d]pyrimidin-2-yl]-benzamide,
[149] ester tert-butylique de l'acide (2-{2-[(thiophène-2-carbonyl)-amino]-5,6,7,8-tétrahydro-quinazolin-6-ylcarbamoyl}-éthyl)-carbamique,
[150] 2-fluoro-N-[6-(2-méthyl-5-nitro-benzènesulfonyl)-5,6,7,8-tétrahydro-pyrido[4,3-d]pyrimidin-2-yl]-benzamide,
[151] N-[6-(4-méthoxy-benzènesulfonylamino)-5,6,7,8-tétrahydro-quinazolin-2-yl]-butyramide,
[152] 4-chloro-N-[6-(2,4-difluoro-benzoyl)-5,6,7,8-tétrahydro-pyrido[4,3-d]pyrimidin-2-yl]-benzamide
[153] 4-méthyl-N-(6-pent-4-énoyl-5,6,7,8-tétrahydro-pyrido[4,3-d]pyrimidin-2-yl)-benzamide,
[154] [6-(3-fluoro-4-méthyl-benzoyl)-5,6,7,8-tétrahydro-pyrido[4,3-d]pyrimidin-2-yl]-amide de l'acide naphtaline-1-carboxylique,
[155] N-{-6[2-(4-trifluorométhyl-phényl)-acétyl]-5,6,7,8-tétrahydro-pyrido[4,3-d]pyrimidin-2-yl}-benzamide,
[156] N-[6-(3,4-dichloro-benzoyl)-5,6,7,8-tétrahydro-pyrido[4,3-d]pyrimidin-2-yl]-nicotinamide,
[157] 4-éthyl-N-{6-[2-(2,2,2-trifluoro-acétyl)-1,2,3,4-tétrahydro-isoquinoline-7-sulfonyl]-5,6,7,8-tétrahydro-pyrido[4,3-d]pyrimidin-2-yl}-benzamide,
[158] 2-méthoxy-N-[6-(4-méthoxy-2,3,6-triméthyl-benzènesulfonyl)-5,6,7,8-tétrahydro-pyrido[4,3-d]pyrimidin-2-yl]-benzamide,
[159] 4-éthyl-N-[6-(2-méthoxy-acétyl)-5,6,7,8-tétrahydro-pyrido[4,3-d]pyrimidin-2-yl]-benzamide,
[160] 4-fluoro-N-[6-(propane-1-sulfonylamino)-5,6,7,8-tétrahydro-quinazolin-2-yl]-benzamide,
[162] N-[6-(7,7-diméthyl-2-oxo-bicyclo[2.2.1]hept-1-ylméthanesulfonylamino)-5,6,7,8-tétrahydro-quinazolin-2-yl]-butyramide,
[163] N-{6-[2-(2,5-diméthyl-phényl)-acétylamino]-5,6,7,8-tétrahydro-quinazolin-2-yl}-4-fluoro-benzamide, [165] (6-phénylméthanesulfonylamino-5,6,7,8-tétrahydro-quinazolin-2-yl)-amide de l'acide thiophène-2-carboxylique,
[166] N-[6-(3,4-diméthoxy-benzènesulfonylamino)-5,6,7,8-tétrahydro-quinazolin-2-yl]-butyramide,
[167] N-{6-[4-(1,1-diméthyl-propyl)-benzènesulfonylamino]-5,6,7,8-tétrahydro-quinazolin-2-yl}-butyramide,
[168] N-(2-butyrylamino-5,6,7,8-tétrahydro-quinazolin-6-yl)-3-(3-trifluorométhyl-phényl)-acrylamide,
[169] N-[6-(butane-1-sulfonylamino)-5,6,7,8-tétrahydro-quinazolin-2-yl]-4-fluoro-benzamide,
[170] ((6-acétyl-5,6,7,8-tétrahydro-pyrido[4,3-d]pyrimidin-2-yl)-amide de l'acide naphtaline-1-carboxylique,
[171] 3,5-dichloro-N-[6-(3-diéthylcarbamoyl-propionyl)-5,6,7,8-tétrahydro-pyrido[4,3-d]pyrimidin-2-yl]-benzamide,
[172] 4-éthyl-N-[6-(4-méthoxy-benzènesulfonyl)-5,6,7,8-tétrahydro-pyrido[4,3-d]pyrimidin-2-yl]-benzamide,
[173] 4-éthyl-N-[6-(quinoline-6-carbonyl)-5,6,7,8-tétrahydro-pyrido[4,3-d]pyrimidin-2-yl]-benzamide,
[174] N-[6-(2-cyclopropyl-acétyl)-5,6,7,8-tétrahydro-pyrido[4,3-d]pyrimidin-2-yl]-2-fluoro-benzamide,
[175] N-[6-(2H-chromène-3-carbonyl)-5,6,7,8-tétrahyclro-pyrido[4,3-d]pyrimidin-2-yl]-3,4-difluoro-benzamide,
[176] N-{6-[2-(4-chloro-phényl)-propionyl]-5,6,7,8-tétrahydro-pyrido[4,3-d]pyrimidin-2-yl}-benzamide,
[177] [6-(2-naphtaline-1-yl-acétylamino)-5,6,7,8-tétrahydro-quinazolin-2-yl]-amide de l'acide thiophène-2-carboxylique,
[178] 4-fluoro-N-(6-phénylméthanesulfonyl-5,6,7,8-tétrahydro-pyrido[4,3-d]pyrimidin-2-yl)-benzamide,
[180] [6-(2,5-dichloro-thiophène-3-sulfonylamino)-5,6,7,8-tétrahydro-quinazolin-2-yl]-amide de l'acide thiophène-2-carboxylique,
[181] ester benzylique de l'acide {5-[2-(4-fluoro-benzoylamino)-5,6,7,8-tétrahydro-quinazolin-6-ylcarbamoyl]-pentyl}-carbamique,
[182] N-[6-(2,5-diméthyl-2H-pyrazol-3-carbonyl)-5,6,7,8-tétrahydro-pyrido[4,3-d]pyrimidin-2-yl]-4-éthyl-benzamide,
[183] [6-(3-fluoro-benzoyl)-5,6,7,8-tétrahydro-pyrido[4,3-d]pyrimidin-2-yl]-amide de l'acide naphtaline-1-carboxylique,
[184] N-[6-(benzo[b]thiophène-3-carbonyl)-5,6,7,8-tétrahydro-pyrido[4,3-d]pyrimidin-2-yl]-4-tert-butyl-benzamide,
[185] 4-fluoro-N-[6-(4-méthoxy-benzènesulfonyl)-5,6,7,8-tétrahydro-pyrido[4,3-d]pyrimidin-2-yl]-benzamide,
[186] 4-tert-butyl-N-[6-(2,3-dihydro-benzo[1,4]dioxine-6-sulfonyl)-5,6,7,8-tétrahydro-pyrido[4,3-d]pyrimidin-2-yl]-benzamide,
[187] 3-chloro-N-{6-[2-(2,6-dichloro-phényl)-acétyl]-5,6,7,8-tétrahydro-pyrido[4,3-d]pyrimidin-2-yl}-benzamide,
[188] [6-(2,3-dihydro-benzo[1,4]dioxine-6-sulfonylamino)-5,6,7,8-tétrahydro-quinazolin-2-yl]-amide de l'acide thiophène-2-carboxylique,
[189] N-{6-[2-(3-chloro-phénoxy)-acétyl]-5,6,7,8-tétrahydro-pyrido[4,3-d]pyrimidin-2-yl}-4-méthyl-benzamide,
[190] N-[6-(5-phényl-pentanoyl)-5,6,7,8-tétrahydro-pyrido[4,3-d]pyrimidin-2-yl]-benzamide,
[191] 4-éthyl-N-[6-(2-éthyl-butyryl)-5,6,7,8-tétrahydro-pyrido[4,3-d]pyrimidin-2-yl]-benzamide,
[192] 3,5-dichloro-N-[6-(4-trifluorométhoxy-benzoyl)-5,6,7,8-tétrahydro-pyrido[4,3-d]pyrimidin-2-yl]-benzamide,
[193] [6-(4-méthyl-octanoyl)-5,6,7,8-tétrahydro-pyrido[4,3-d]pyrimidin-2-yl]-amide de l'acide naphtaline-1-carboxylique,
[194] ester tert-butylique de l'acide 4-[2-(4-fluoro-benzoylamino)-5,6,7,8-tétrahydro-quinazolin-6-ylcarbamoyl]-pipéridine-1-carboxylique,
[195] N-[6-(2-benzyloxy-acétyl)-5,6,7,8-tétrahydro-pyrido[4,3-d]pyrimidin-2-yl]-2-méthoxy-benzamide,
[196] N-[6-(2-trifluorométhyl-benzènesulfonylamino)-5,6,7,8-tétrahydro-quinazolin-2-yl]-butyramide,
[197] N-[2-(4-fluoro-benzoylamino)-5,6,7,8-tétrahydro-quinazolin-6-yl]-2-méthyl-6-trifluorométhyl-nicotinamide,
[198] N-[6-(3-cyano-benzoyl)-5,6,7,8-tétrahydro-pyrido[4,3-d]pyrimidin-2-yl]-2-trifluorométhyl-benzamide,
[199] 3-méthoxy-N-[6-(3-trifluorométhoxy-benzoyl)-5,6,7,8-tétrahydro-pyrido[4,3-d]pyrimidin-2-yl]-benzamide,
[200] N-(6-butyryl-5,6,7,8-tétrahydro-pyrido[4,3-d]pyrimidin-2-yl)-2-fluoro-benzamide,
[201] N-(6-benzènesulfonylamino-5,6,7,8-tétrahydro-quinazolin-2-yl)-4-fluoro-benzamide,
[202] N-[6-(5-chloro-thiophène-2-sulfonylamino)-5,6,7,8-tétrahydro-quinazolin-2-yl]-4-fluoro-benzamide,
[203] ((6-hexanoyl-5,6,7,8-tétrahydro-pyrido[4,3-d]pyrimidin-2-yl)-amide de l'acide naphtaline-1-carboxylique,
[204] N-(6-propionyl-5,6,7,8-tétrahydro-pyrido[4,3-d]pyrimidin-2-yl)-2-trifluorométhyl-benzamide,
[205] N-[6-(2-éthyl-butyryl)-5,6,7,8-tétrahydro-pyrido[4,3-d]pyrimidin-2-yl]-4-méthyl-benzamide,
[206] 2-fluoro-N-[6-(3-trifluorométhyl-benzènesulfonyl)-5,6,7,8-tétrahydro-pyrido[4,3-d]pyrimidin-2-yl]-benzamide,
[207] {2-[(thiophène-2-carbonyl)-amino]-5,6,7,8-tétrahydro-quinazolin-6-yl}-amide de l'acide 5-benzyl-furane-2-carboxylique,
[208] {6-[2-(2,5-diméthyl-phényl)-acétylamino]-5,6,7,8-tétrahydro-quinazolin-2-yl}-amide de l'acide thiophène-2-carboxylique,
[210] 4-fluoro-N-[6-(propane-1-sulfonyl)-5,6,7,8-tétrahydro-pyrido[4,3-d]pyrimidin-2-yl]-benzamide,
[211] ester méthylique de l'acide 2-[2-(3-fluoro-benzoylamino)-7,8-dihydro-5H-pyrido[4,3-d]pyrimidine-6-sulfonyl]-benzoïque,
[212] 3,5-dichloro-N-[6-(5-[1,2]dithiolan-3-yl-pentanoyl)-5,6,7,8-tétrahydro-pyrido[4,3-d]pyrimidin-2-yl]-benzamide,
[213] [6-(2-phénoxy-butyrylamino)-5,6,7,8-tétrahydro-quinazolin-2-yl]-amide de l'acide thiophène-2-carboxylique,
[214] {6-[2-(4-méthoxy-phényl)-acétylamino]-5,6,7,8-tétrahydro-quinazolin-2-yl}-amide de l'acide thiophène-2-carboxylique,
[215] N-(6-cyclohexanecarbonyl-5,6,7,8-tétrahydro-pyrido[4,3-d]pyrimidin-2-yl)-2-trifluorométhyl-benzamide,
[216] N-[6-(2,4-diméthyl-thiazol-5-sulfonyl)-5,6,7,8-tétrahydro-pyrido[4,3-d]pyrimidin-2-yl]-2-trifluorométhyl-benzamide,
[217] {2-[(thiophène-2-carbonyl)-amino]-5,6,7,8-tétrahydro-quinazolin-6-yl}-amide de l'acide 2-thiophén-2-yl-thiazol-4-carboxylique,
[219] {6-[3-(3-trifluorométhyl-phényl)-acryloylamino]-5,6,7,8-tétrahydro-quinazolin-2-yl}-amide de l'acide thiophène-2-carboxylique,
[220] [6-(5-phényl-pentanoylamino)-5,6,7,8-tétrahydro-quinazolin-2-yl]-amide de l'acide thiophène-2-carboxylique,
[221] [6-(2-chloro-5-trifluorométhyl-benzoylamino)-5,6,7,8-tétrahydro-quinazolin-2-yl]-amide de l'acide thiophène-2-carboxylique,
[222] 4-fluoro-N-[6-(2,3,5,6-tétraméthyl-benzènesulfonylamino)-5,6,7,8-tétrahydro-quinazolin-2-yl]-benzamide,
[223] 4-fluoro-N-[6-(4-méthyl-octanoyl)-5,6,7,8-tétrahydro-pyrido[4,3-d]pyrimidin-2-yl]-benzamide,
[224] ester méthylique de l'acide 2-{2-[(thiophène-2-carbonyl)-amino]-5,6,7,8-tétrahydro-quinazolin-6-ylsulfamoyl}-benzoïque,
[225] ester benzylique de l'acide [(3-méthyl-1-{2-[(thiophène-2-carbonyl)-amino]-5,6,7,8-tétrahydro-quinazolin-6-ylcarbamoyl}-butylcarbamoyl)-méthyl]-carbamique,
[226] 3-chloro-N-(6-pentanoyl-5,6,7,8-tétrahydro-pyrido[4,3-d]pyrimidin-2-yl)-benzamide,
[227] 4-fluoro-N-[6-(5-oxo-5-phényl-pentanoylamino)-5,6,7,8-tétrahydro-quinazolin-2-yl]-benzamide,
[228] [6-(3-phényl-acryloylamino)-5,6,7,8-tétrahydro-quinazolin-2-yl]-amide de l'acide thiophène-2-carboxylique,
[229] N-[6-(5-[1,2]dithiolan-3-yl-pentanoylamino)-5,6,7,8-tétrahydro-quinazolin-2-yl]-4-fluoro-benzamide,
[230] ester 2-(2-{2-[(thiophène-2-carbonyl)-amino]-5,6,7,8-tétrahydro-quinazolin-6-ylcarbamoyl}-éthyl)-phénylique de l'acide benzoïque,
[231] 4-fluoro-N-[6-(2-méthyl-5-nitro-benzènesulfonylamino)-5,6,7,8-tétrahydro-quinazolin-2-yl]-benzamide,
[232] 4-chloro-N-[6-(2-phénoxy-acétyl)-5,6,7,8-tétrahydro-pyrido[4,3-d]pyrimidin-2-yl]-benzamide,
[234] N-[6-(5-bromo-thiophène-2-carbonyl)-5,6,7,8-tétrahydro-pyrido[4,3-d]pyrimidin-2-yl]-2-fluoro-benzamide,
[235] 4-fluoro-N-[6-(5-fluoro-2-méthyl-benzènesulfonylamino)-5,6,7,8-tétrahydro-quinazolin-2-yl]-benzamide,
[236] N-[6-(4-acétylamino-benzènesulfonylamino)-5,6,7,8-tétrahydro-quinazolin-2-yl]-4-fluoro-benzamide,
[237] [6-(2-trifluorométhyl-benzoylamino)-5,6,7,8-tétrahydro-quinazolin-2-yl]-amide de l'acide thiophène-2-carboxylique,
[238] 3,4-difluoro-N-[6-(quinoline-6-carbonyl)-5,6,7,8-tétrahydro-pyrido[4,3-d]pyrimidin-2-yl]-benzamide,
[239] ester tert-butylique de l'acide {1-[2-(4-fluoro-benzoylamino)-5,6,7,8-tétrahydro-quinazolin-6-ylcarbamoyl]-2-phényl-éthyl}-carbamique
[240] [6-(2-méthyl-benzoylamino)-5,6,7,8-tétrahydro-quinazolin-2-yl]-amide de l'acide thiophène-2-carboxylique,
[241] 3-chloro-N-{6-[3-(2-oxo-benzooxazol-3-yl)-propionyl]-5,6,7,8-tétrahydro-pyrido[4,3-d]pyrimidin-2-yl}-benzamide,
[242] 3-chloro-N-[6-(5-chloro-thiophène-2-sulfonyl)-5,6,7,8-tétrahydro-pyrido[4,3-d]pyrimidin-2-yl]-benzamide,
[243] 3,5-dichloro-N-[6-(4-méthoxy-benzènesulfonyl)-5,6,7,8-tétrahydro-pyrido[4,3-d]pyrimidin-2-yl]-benzamide,
[244] 4-tert-butyl-N-[6-(5-méthyl-isoxazol-3-carbonyl)-5,6,7,8-tétrahydro-pyrido[4,3-d]pyrimidin-2-yl]-benzamide,
[245] N-[6-(5-bromo-2-méthoxy-benzènesulfonyl)-5,6,7,8-tétrahydro-pyrido[4,3-d]pyrimidin-2-yl]-4-méthyl-benzamide,
[246] 4-tert-butyl-N-{6-[3-(2-hydroxy-phényl)-propionyl]-5,6,7,8-tétrahydro-pyrido[4,3-d]pyrimidin-2-yl}-benzamide,
[247] 4-fluoro-N-[6-(2-phényl-butyrylamino)-5,6,7,8-tétrahydro-quinazolin-2-yl]-benzamide,
[248] 4-tert-butyl-N-[6-(4-propyl-benzoyl)-5,6,7,8-tétrahydro-pyrido[4,3-d]pyrimidin-2-yl]-benzamide,
[249] [6-(2-bromo-benzoylamino)-5,6,7,8-tétrahydro-quinazolin-2-yl]-amide de l'acide thiophène-2-carboxylique,
[250] 2-méthoxy-N-[6-(6-phénoxy-pyridin-3-carbonyl)-5,6,7,8-tétrahydro-pyrido[4,3-d]pyrimidin-2-yl]-benzamide,
[251] N-[6-(4-acétylamino-benzènesulfonyl)-5,6,7,8-tétrahydro-pyrido[4,3-d]pyrimidin-2-yl]-4-éthyl-benzamide,
[252] 3,5-dichloro-N-[6-(4-fluoro-benzènesulfonyl)-5,6,7,8-tétrahydro-pyrido[4,3-d]pyrimidin-2-yl]-benzamide,
[253] N-[6-(1-benzènesulfonyl-1H-indol-2-carbonyl)-5,6,7,8-tétrahydro-pyrido[4,3-d]pyrimidin-2-yl]-4-fluoro-benzamide et
[255] N-[6-(3-chloro-benzènesulfonylamino)-5,6,7,8-tétrahydro-quinazolin-2-yl]-4-fluoro-benzamide, à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme des sels correspondants, ou à chaque fois sous la forme des solvates correspondants.

15. Procédé de fabrication de composés de 5,6,7,8-tétrahydro-pyrido[4,3-d]pyrimidin-2-yle substitués selon une ou plusieurs des revendications 1 à 14, X représentant un groupe NR^{2a}, **caractérisé en ce qu'**une pipéridin-4-one protégée de formule générale II 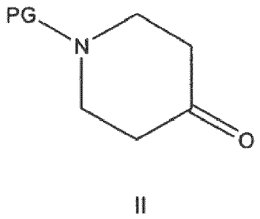 dans laquelle PG représente un groupe protecteur, est transformée par mise en réaction avec la diméthoxyméthyl-diméthyl-amine de formule III 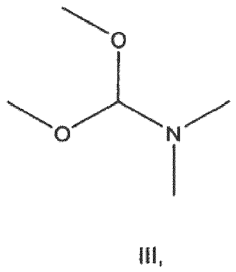 dans un milieu réactionnel organique en un composé de formule générale IV 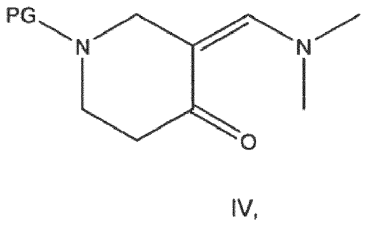 dans laquelle PG a la signification indiquée précédemment, celui-ci est éventuellement purifié et/ou isolé, et transformé par mise en réaction avec la guanidine de formule V 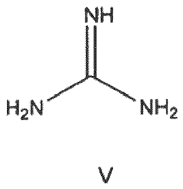 éventuellement sous la forme d'un sel correspondant, dans un milieu réactionnel organique, éventuellement en présence d'au moins une base, en un composé de formule générale VI 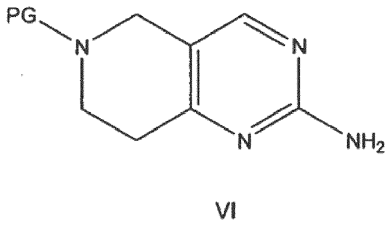 dans laquelle PG a la signification indiquée précédemment, celui-ci est éventuellement purifié et/ou isolé, et transformé dans un milieu réactionnel organique, éventuellement en présence d'au moins une base et/ou d'au moins un catalyseur, avec un composé de formule générale R³-C(=O)-X¹ ou un composé de formule générale (R³-C(=O))₂O, ou par mise en réaction avec un composé de formule générale R³-COOH dans un milieu réactionnel organique en présence d'un agent de couplage approprié, éventuellement en présence d'au moins une base, ou par mise en réaction avec un composé de formule générale R⁴-O-C(=O)-X^{1a} dans un milieu réactionnel organique, éventuellement en présence d'une base, R³ et R⁴ ayant chacun la signification selon une ou plusieurs des revendications 1 à 15 et X¹ et X^{1a} représentant chacun un groupe partant, en un composé de formule générale VII 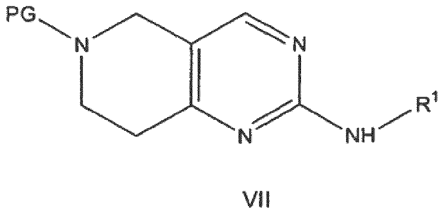 dans laquelle PG a la signification indiquée précédemment et R¹ a la signification selon une ou plusieurs des revendications 1 à 15, celui-ci est éventuellement purifié et/ou isolé, et transformé par clivage du groupe protecteur PG en un composé de formule générale VIII 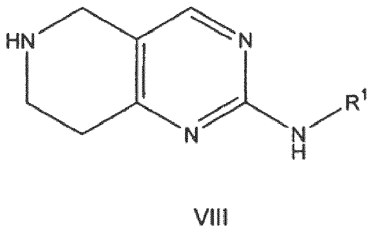 éventuellement sous la forme d'un sel correspondant, celui-ci est éventuellement purifié et/ou isolé, et transformé par mise en réaction avec un composé de formule générale R⁵-C(=O)-X² ou (R⁵-C(=O))₂O, R⁵ ayant à chaque fois la signification selon une ou plusieurs des revendications 1 à 15 et X² représentant un groupe partant, dans un milieu réactionnel organique, éventuellement en présence d'au moins une base et/ou en présence d'au moins un catalyseur, ou par mise en réaction avec un composé de formule générale R⁶-SO₂-X³, R⁶ ayant la signification selon une ou plusieurs des revendications 1 à 15 et X³ représentant un groupe partant, dans un milieu réactionnel organique, éventuellement en présence d'au moins une base, ou par mise en réaction avec un composé de formule générale R⁵-COOH, R⁵ ayant la signification selon une ou plusieurs des revendications 1 à 15, dans un milieu réactionnel organique, en présence d'un agent de couplage approprié, éventuellement en présence d'au moins une base, en un composé de formule générale IX 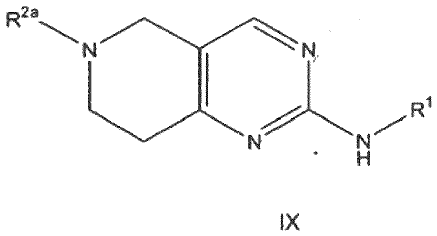 dans laquelle R¹ et R^{2a} ont la signification selon une ou plusieurs des revendications 1 à 15, celui-ci est éventuellement purifié et/ou isolé, et éventuellement transformé en un sel correspondant, et celui-ci est éventuellement purifié et/ou isolé,
ou une pipéridin-4-one de formule générale X 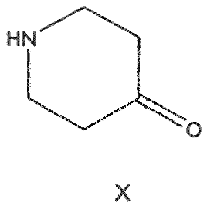 éventuellement sous la forme d'un sel correspondant, est transformée par mise en réaction avec un composé de formule générale R⁵-C(=O)-X² ou (R⁵-C(=O))₂O, R⁵ ayant à chaque fois la signification selon une ou plusieurs des revendications 1 à 15 et X² représentant un groupe partant, dans un milieu réactionnel organique, éventuellement en présence d'au moins une base et/ou en présence d'au moins un catalyseur, ou par mise en réaction avec un composé de formule générale R⁶-SO₂-X³, R⁶ ayant la signification selon une ou plusieurs des revendications 1 à 15 et X³ représentant un groupe partant, dans un milieu réactionnel organique, éventuellement en présence d'au moins une base, ou par mise en réaction avec un composé de formule générale R⁵-COOH, R⁵ ayant la signification selon une ou plusieurs des revendications 1 à 15, dans un milieu réactionnel organique, en présence d'un agent de couplage approprié, éventuellement en présence d'au moins une base, en un composé de formule générale XI 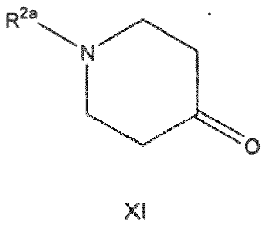 dans laquelle R^{2a} a la signification selon une ou plusieurs des revendications 1 à 15, celui-ci est éventuellement purifié et/ou isolé, et transformé par mise en réaction avec la diméthoxy-méthyl-diméthyl-amine de formule III 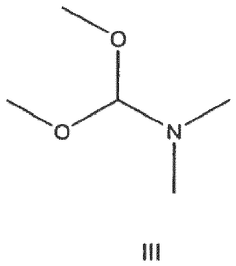 dans un milieu réactionnel organique en un composé de formule générale XII 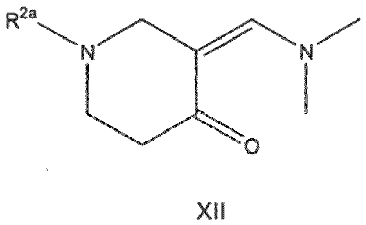 dans laquelle R^{2a} a la signification indiquée précédemment, celui-ci est éventuellement purifié et/ou isolé, et transformé avec la guanidine de formule V 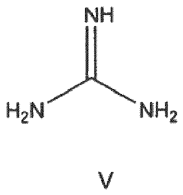 éventuellement sous la forme d'un sel correspondant, dans un milieu réactionnel organique, éventuellement en présence d'au moins une base, en un composé de formule générale XIII 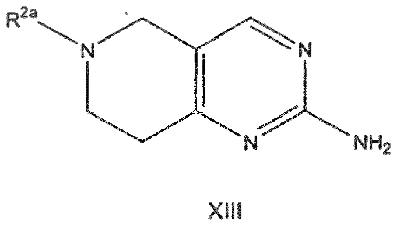 celui-ci est éventuellement purifié et/ou isolé, et transformé dans un milieu réactionnel organique, éventuellement en présence d'au moins une base et/ou d'au moins un catalyseur, avec un composé de formule générale (R³-C(=O))₂O ou un composé de formule générale R³-C(=O)-X¹ ou par mise en réaction avec un composé de formule générale R³-COOH dans un milieu réactionnel organique, en présence d'un agent de couplage approprié, éventuellement en présence d'au moins une base, ou par mise en réaction avec un composé de formule générale R⁴-O-C(=O)-X^{1a} dans un milieu réactionnel organique, éventuellement en présence d'une base, R³ et R⁴ ayant chacun la signification selon une ou plusieurs des revendications 1 à 15 et X¹ et X^{1a} représentant chacun un groupe partant, en un composé de formule générale XIV 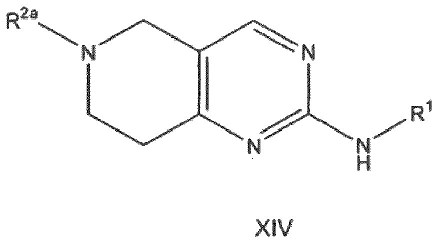 dans laquelle R¹ et R^{2a} ont la signification selon une ou plusieurs des revendications 1 à 15, et celui-ci est éventuellement purifié et/ou isolé, et celui-ci est éventuellement transformé en un sel correspondant et ce sel est éventuellement purifié et/ou isolé.

16. Procédé de fabrication de composés de 5,6,7,8-tétrahydro-quinazolin-2-yle substitués selon une ou plusieurs des revendications 1 à 14, X représentant un groupe C(H)(NHR²), **caractérisé en ce qu'**une 1,4-dioxaspiro[4.5]déc-8-yl-amine de formule C 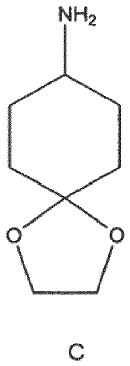 est transformée par mise en réaction avec un composé de formule générale R⁵-C(=O)-X⁴ ou (R⁵-C(=O))₂O, R⁵ ayant à chaque fois la signification selon une ou plusieurs des revendications 1 à 15 et X⁴ représentant un groupe partant, dans un milieu réactionnel organique, éventuellement en présence d'au moins une base et/ou en présence d'au moins un catalyseur, ou par mise en réaction avec un composé de formule générale R⁶-SO₂-X⁵, R⁶ ayant la signification selon une ou plusieurs des revendications 1 à 15 et X⁵ représentant un groupe partant, dans un milieu réactionnel organique, éventuellement en présence d'au moins une base, ou par mise en réaction avec un composé de formule générale R⁵-COOH, R⁵ ayant la signification selon une ou plusieurs des revendications 1 à 15, dans un milieu réactionnel organique, en présence d'au moins un agent de couplage approprié, éventuellement en présence d'au moins une base, en un composé de formule générale D 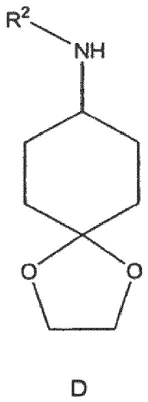 R² ayant la signification selon une ou plusieurs des revendications 1 à 15, celui-ci est éventuellement purifié et/ou isolé, et celui-ci est transformé par clivage du groupe acétal dans un milieu réactionnel organique en présence d'eau et d'au moins un acide en un composé de formule générale E 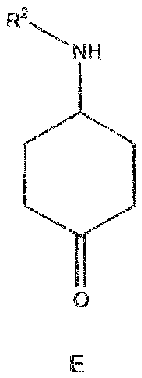 R² ayant la signification indiquée précédemment, et celui-ci est transformé par introduction d'un groupe protecteur (PG¹) dans un milieu réactionnel organique en un composé de formule générale F 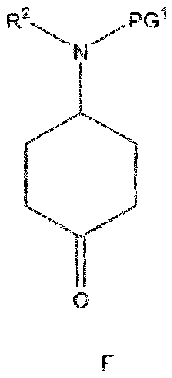 R² ayant la signification indiquée précédemment et PG¹ représentant un groupe protecteur, celui-ci est éventuellement purifié et/ou isolé, et celui-ci est transformé par mise en réaction avec la diméthoxyméthyl-diméthyl-amine de formule générale III 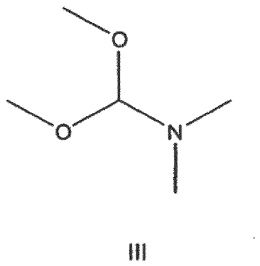 dans un milieu réactionnel organique en un composé de formule générale G 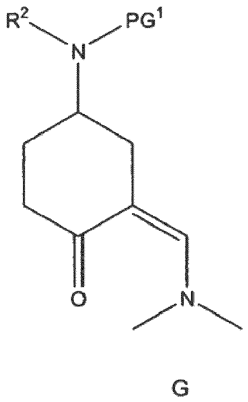 R² et PG¹ ayant la signification indiquée précédemment, et celui-ci est éventuellement purifié et/ou isolé, et transformé éventuellement en présence d'au moins une base dans un milieu réactionnel organique avec un composé de guanidine de formule V, éventuellement sous la forme d'un sel correspondant 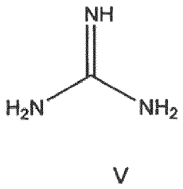 en un composé de formule générale H 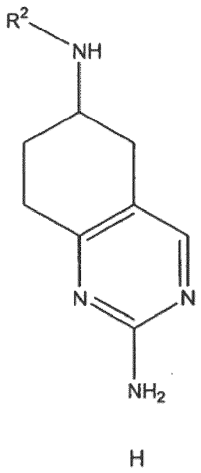 celui-ci est éventuellement purifié et/ou isolé, et celui-ci est transformé par introduction d'un groupe protecteur (PG²) dans un milieu réactionnel organique en un composé de formule générale I 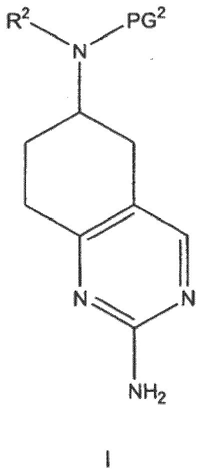 R² et PG² ayant la signification indiquée précédemment, celui-ci est éventuellement purifié et/ou isolé, et celui-ci est transformé par mise en réaction avec un composé de formule générale R³-C(=O)-X⁶ ou un composé de formule générale (R³-C(=O))₂O, R³ ayant à chaque fois la signification selon une ou plusieurs des revendications 1 à 15 et X⁶ représentant un groupe partant, ou par mise en réaction avec un composé de formule générale R³COOH, R³ ayant la signification indiquée précédemment, dans un milieu réactionnel organique en présence d'un agent de couplage, éventuellement en présence d'au moins une base, ou par mise en réaction avec un composé de formule générale R⁴-O-C(=O)-X^{1a} dans un milieu réactionnel organique, éventuellement en présence d'une base, en un composé de formule générale J 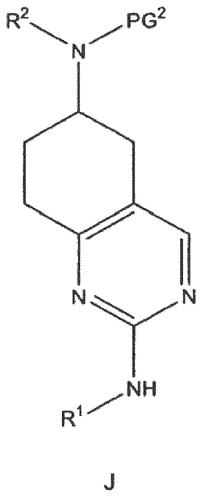 dans laquelle R² et PG² ont la signification indiquée précédemment et R¹ ont la signification selon une ou plusieurs des revendications 1 à 15, celui-ci est éventuellement purifié et/ou isolé, et celui-ci est transformé par clivage du groupe protecteur en un composé de 5,6,7,8-tétrahydro-quinazolin-2-yle substitué correspondant selon une ou plusieurs des revendications 1 à 15, X représentant un groupe C(H)(NHR²), celui-ci est éventuellement purifié et/ou isolé, puis éventuellement transformé en un sel correspondant, et celui-ci est éventuellement purifié et/ou isolé.

17. Médicament contenant au moins un composé selon une ou plusieurs des revendications 1 à 14 et éventuellement un ou plusieurs adjuvants pharmaceutiquement compatibles.

18. Médicament selon la revendication 17 pour la régulation des récepteurs de noradrénaline, notamment pour l'inhibition du recaptage de noradrénaline (noradrenaline uptake), pour la régulation des récepteurs de 5-HT, notamment pour l'inhibition du recaptage de 5-hydroxy-tryptophane (5-HT uptake), pour la régulation des récepteurs de mGluR5 et/ou pour la régulation des récepteurs de batrachotoxine (BTX).

19. Médicament selon la revendication 17 pour la prophylaxie et/ou le traitement de troubles et/ou de maladies, qui sont médiés au moins en partie par un ou plusieurs des récepteurs choisis dans le groupe constitué par les récepteurs de noradrénaline, les récepteurs de 5-HT, les récepteurs de mGluR5 et les récepteurs de batrachotoxine (BTX).

20. Médicament selon une ou plusieurs des revendications 17 à 19 pour la prophylaxie et/ou le traitement de la douleur, de préférence de la douleur choisie dans le groupe constitué par la douleur aiguë, la douleur chronique, la douleur neuropathique et la douleur viscérale.

21. Médicament selon une ou plusieurs des revendications 17 à 19 pour la prophylaxie et/ou le traitement de la migraine, des dépressions, de l'incontinence urinaire, de la toux, des maladies neurodégénératives, de préférence choisies dans le groupe constitué par la maladie de Parkinson, la maladie d'Huntington, la maladie d'Alzheimer et la sclérose en plaques, des troubles de l'alimentation, de préférence choisis dans le groupe constitué par la boulimie, l'anorexie, l'obésité et la cachexie, des dysfonctionnements cognitifs, de préférence les troubles de la mémoire, du trouble déficitaire de l'attention (attention deficit syndrom, ADS), des troubles du système nerveux, de l'épilepsie, de la schizophrénie, des ischémies cérébrales, des spasmes musculaires, des crampes, de la diarrhée, du prurit, du syndrome du reflux gastro-oesophagien, des états de panique, de l'abus d'alcool et/ou de drogues et/ou de médicaments, de la dépendance à l'alcool et/ou à des drogues et/ou à des médicaments, de préférence pour la prophylaxie et/ou la réduction des phénomènes de sevrage en cas de dépendance à l'alcool et/ou à des drogues et/ou à des médicaments, pour la prophylaxie et/ou la réduction du développement d'une tolérance à des médicaments, notamment des médicaments à base d'opioïdes, pour la régulation de l'alimentation, pour la modulation de l'activité physique, pour la régulation du système cardiovasculaire, pour l'anesthésie locale, pour l'anxiolyse, pour l'augmentation de la vigilance, pour l'augmentation de la libido, pour la diurèse et/ou pour l'antinatriurèse.

22. Utilisation d'au moins un composé selon une ou plusieurs des revendications 1 à 14, ainsi qu'éventuellement d'un ou de plusieurs adjuvants pharmaceutiquement compatibles pour la fabrication d'un médicament pour la régulation des récepteurs de noradrénaline, notamment pour l'inhibition du recaptage de noradrénaline (noradrenaline uptake), pour la régulation des récepteurs de 5-HT, notamment pour l'inhibition du recaptage de 5-hydroxy-tryptophane (5-HT uptake), pour la régulation des récepteurs de mGluR5 et/ou pour la régulation des récepteurs de batrachotoxine (BTX).

23. Utilisation d'au moins un composé selon une ou plusieurs des revendications 1 à 14, ainsi qu'éventuellement d'un ou de plusieurs adjuvants pharmaceutiquement compatibles pour la fabrication d'un médicament pour la prophylaxie et/ou le traitement de troubles et/ou de maladies, qui sont médiés au moins en partie par un ou plusieurs récepteurs choisis dans le groupe constitué par les récepteurs de noradrénaline, les récepteurs de 5-HT, les récepteurs de mGluR5 et les récepteurs de batrachotoxine (BTX).

24. Utilisation d'au moins un composé selon une ou plusieurs des revendications 1 à 14, ainsi qu'éventuellement d'un ou de plusieurs adjuvants pharmaceutiquement compatibles pour la fabrication d'un médicament pour la prophylaxie et/ou le traitement de la douleur, de préférence de la douleur choisie dans le groupe constitué par la douleur aiguë, la douleur chronique, la douleur neuropathique et la douleur viscérale.

25. Utilisation d'au moins un composé selon une ou plusieurs des revendications 1 à 14, ainsi qu'éventuellement d'un ou de plusieurs adjuvants pharmaceutiquement compatibles pour la fabrication d'un médicament pour la prophylaxie et/ou le traitement de la migraine, des dépressions, de l'incontinence urinaire, de la toux, des maladies neurodégénératives, de préférence choisies dans le groupe constitué par la maladie de Parkinson, la maladie d'Huntington, la maladie d'Alzheimer et la sclérose en plaques, des troubles de l'alimentation, de préférence choisis dans le groupe constitué par la boulimie, l'anorexie, l'obésité et la cachexie, des dysfonctionnements cognitifs, de préférence les troubles de la mémoire, du trouble déficitaire de l'attention (attention deficit syndrom, ADS), des troubles du système nerveux, de l'épilepsie, de la schizophrénie, des ischémies cérébrales, des spasmes musculaires, des crampes, de la diarrhée, du prurit, du syndrome du reflux gastro-oesophagien, des états de panique, de l'abus d'alcool et/ou de drogues et/ou de médicaments, de la dépendance à l'alcool et/ou à des drogues et/ou à des médicaments, de préférence pour la prophylaxie et/ou la réduction des phénomènes de sevrage en cas de dépendance à l'alcool et/ou à des drogues et/ou à des médicaments, pour la prophylaxie et/ou la réduction du développement d'une tolérance à des médicaments, notamment des médicaments à base d'opioïdes, pour la régulation de l'alimentation, pour la modulation de l'activité physique, pour la régulation du système cardiovasculaire, pour l'anesthésie locale, pour l'anxiolyse, pour l'augmentation de la vigilance, pour l'augmentation de la libido, pour la diurèse et/ou pour l'antinatriurèse.
